# EUROPEAN PATENT SPECIFICATION

(11) **EP 0 527 889 B1**
(45) Date of publication and mention of the grant of the patent: **02.08.2000**
(21) Application number: 91909628.9
(22) Date of filing: 01.05.1991
(51) Int. Cl.: C07D 471/04, C07D 455/02, C07D 491/16, C07D 495/16, C07D 471/16, C07D 519/00, A61K 31/435, A61K 31/505, A61K 31/495, A61K 31/54, A61K 31/535

(54) **QUINOLIZINONE TYPE COMPOUNDS**
VERBINDUNGEN VOM CHINOLIZINON-TYP
COMPOSES DE TYPE QUINOLIZINONE

(30) Priority: 02.05.1990 US 517780
(43) Date of publication of application: 24.02.1993
(73) Proprietor: ABBOTT LABORATORIES, Abbott Park, Illinois 60064-3500 (US)
(72) Inventor: CHU, Daniel, T., Vernon Hills, IL 60061 (US); LEE, Cheuk, M., Libertyville, IL 60048 (US); LI, Qun, Gurnee, IL 60031 (US); COOPER, Curt, S., Gurnee, IL 60031 (US); PLATTNER, Jacob, J., Libertyville, IL 60048 (US)
(74) Representative: Modiano, Guido, Dr.-Ing.
(86) International application number: US9102998
(87) International publication number: WO9116894

(56) References cited:
- EP-A- 0 159 174
- JP-A- 57 203 085
- US-A- 4 288 438
- US-A- 4 291 036
- US-A- 4 650 804
- US-A- 4 758 567
- US-A- 4 877 795
- US-A- 4 921 857
- US-A- 4 980 353
- US-A- 5 004 745

## Description

### TECHNICAL FIELD

This invention relates to compounds having biological activity and to pharmaceutical compositions containing the compounds. More particularly, this invention concerns novel quinolizinone compounds that are useful in the treatment of microbial infections, to pharmaceutical compositions containing the new quinolizinone compounds and the use of such compounds for manufacturing a medicament for treating microbial infections.

### BACKGROUND OF THE INVENTION

There is a continuing need for new antibiotics. Although many antibiotics are known which are useful in the treatment of Gram-positive and Gram-negative bacterial infections as well as other microbial infections, widespread use of antibiotics continues to give rise to resistant strains of microorganisms, i.e., strains of microorganisms against which a particular antibiotic or group of antibiotics, which was previously effective, is no longer useful. Also, known antibiotics may be effective against only certain strains of microorganisms or have limited activity against either Gram-positive or Gram-negative, aerobic or anaerobic organisms.

Certain quinolizinone derivatives are known. For example, Y. Kitaura *et al*., in U.S. Patent No. 4,650,804, issued March 17, 1987, have disclosed quinolizinone compounds having a tetrazolylcarbamoyl substituent which are useful for the treatment of allergic and ulcer diseases. J.V. Heck and E.D. Thorsett, in European Patent Application No. 0308019, published March 22, 1989, have disclosed the use of certain 4-oxo-4H-quinoline-3-carboxylic adds and derivatives thereof for treating bacterial infections.

### SUMMARY OF THE INVENTION

The compounds of the present invention are represented by the following structural formula (I): 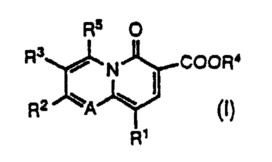 or a pharmaceutically acceptable salt, ester or amide thereof,
wherein R¹ is selected from (a) loweralkyl, (b) loweralkenyl, (c) halo(lower-alkyl), (d) loweralkoxy, (e) cycloalkyl of from 3 to 8 carbons, (f) phenyl, (g) halo, (h) cyano, (i) nitro, (j) bicycloalkyl, (k) loweralkynyl, (l) alkoxycarbonyl, (m) nitrogen-containing aromatic heterocycle and (n) a group of the formula -NR⁷R⁸, wherein R⁷ and R⁸ are independently hydrogen, loweralkyl, alkanoyl of from 1 to 8 carbons or,
alternatively, R⁷ and R⁸ taken together with the nitrogen atom to which they are attached form a 5-, 6-, or 7-membered saturated heterocycle.
R² in formula (I) is selected from the group consisting of halogen, loweralkoxy, carbocyclic aryloxy or carbocyclic aryl(loweralkyl)oxy, loweralkyl, loweralkenyl, cycloalkyl of from 3 to 8 carbons, cycloalkenyl of from 4 to 8 carbons, carbocyclic aryl(loweralkyl), cycloalkyl(loweralkyl), phenyl, amino, (loweralkyl)amino, carbocyclic aryl(loweralkyl)amino, hydroxy-substituted (loweralkyl)amino, nitrogen-containing aromatic heterocycle, bicyclic nitrogen-containing heterocycle and nitrogen-containing heterocycle having the formula 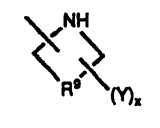 wherein x is 0 to 3; R⁹ is selected from (a) -(CH₂)ₘ-, wherein m is 1, 2 or 3, and (b) -(CH₂)ₙR¹⁰(CH₂)ₚ-; wherein R¹⁰ is selected from S, O and N n is 1 or 2, and p is 1 or 2; and Y is a non-hydrogen substituent independently selected from loweralkyl, halo(loweralkyl), loweralkoxy, hydroxy-substituted loweralkyl, hydroxy, amino(loweralkyl), halogen and a group having the formula -NR¹¹R¹², wherein R¹¹ and R¹² are independently selected from hydrogen and loweralkyl, or where one of R¹¹ and R¹² is hydrogen and the other is an alkanoyl of from 1 to 8 carbons, an α-amino acid or a polypeptide residue of from 2 to 5 amino acids.
R³ in formula (I) is hydrogen, halogen or loweralkoxy.
R⁴ is selected from the group consisting of hydrogen, loweralkyl, a pharmaceutically acceptable cation and a prodrug ester group.
R⁵ in formula (I) is selected from the group consisting of hydrogen, halogen, hydroxy, loweralkyl, halo(loweralkyl), loweralkoxy and a group having the formula -NR¹³R¹⁴ wherein R¹³ and R¹⁴ are independently selected from the group consisting of hydrogen, loweralkyl, hydroxy-substituted loweralkyl, alkoxy-substituted loweralkyl and alkanoyl of from 1 to 8 carbons.
A in the above formula is N or CR⁶, wherein R⁶ is selected from halogen, loweralkyl, halo(loweralkyl), hydroxy-substituted loweralkyl, loweralkoxy(loweralkyl), loweralkoxy and amino(loweralkyl).
Alternatively, R¹ and R⁶ in formula (I), taken together with the atoms to which they are attached, form a 6-membered saturated ring which may contain an oxygen or a sulfur atom and which may be substituted with loweralkyl.

The compounds of the present invention have antimicrobial activity, and may be prepared as pharmaceutical compositions useful in the treatment and prophylaxis of bacterial infection in humans and other animals.

### DETAILED DESCRIPTION OF THE INVENTION

In one aspect of the present invention, novel compounds are disclosed which have the formula 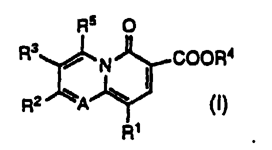 or which are a pharmaceutically acceptable salt, ester or amide thereof,
wherein R¹ is selected from the group consisting of (a) loweralkyl, (b) loweralkenyl, (c) halo(lower-alkyl), (d) loweralkoxy, (e) cycloalkyl of from 3 to 8 carbons, (f) phenyl, (g) halo, (h) cyano, (i) nitro, (j) bicycloalkyl, (k) loweralkynyl, (l) alkoxycarbonyl, (m) nitrogen-containing aromatic heterocycle and (n) a group of the formula -NR⁷R⁸ wherein R⁷ and R⁸ are independently selected from the group consisting of hydrogen, loweralkyl and alkanoyl of from 1 to 8 carbons or, taken together with the nitrogen atom to which they are attached, R⁷ and R⁸ form a 5-, 6-, or 7-membered saturated heterocycle;
R² is selected from the group consisting of halogen, loweralkoxy, carbocyclic aryloxy or carbocyclic aryl(loweralkyl)oxy, loweralkyl, loweralkenyl, cycloalkyl of from 3 to 8 carbons, cycloalkenyl of from 4 to 8 carbons, carbocyclic aryl(loweralkyl), cycloalkyl(loweralkyl), phenyl, amino, (loweralkyl)amino, carbocyclic aryl(loweralkyl)amino, hydroxy-substituted (loweralkyl)amino, bicyclic nitrogen-containing heterocycle and nitrogen-containing heterocycle having the formula 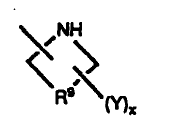 wherein x is 0 to 3; R⁹ is selected from the group consisting of (a) -(CH₂)ₘ-wherein m is 1, 2 or 3, and (b) -(CH₂)ₙR¹⁰(CH₂)ₚ- wherein R¹⁰ is S, O and N, n is 1 or 2, and p is 1 or 2; and Y is a non-hydrogen substituent independently selected from the group consisting of loweralkyl, halo(loweralkyl), loweralkoxy, hydroxy-substituted loweralkyl, hydroxy, amino(loweralkyl), halogen and a group having the formula -NR¹¹R¹² wherein x is 0 to 3, and R¹¹ and R¹² are independently selected from the group consisting of hydrogen and loweralkyl or, when one is hydrogen, the other is selected from the group consisting of an alkanoyl of from 1 to 8 carbons, an α-amino acid and a polypeptide residue of from 2 to 5 amino acids;
R³ is selected from the group consisting of hydrogen, halogen and loweralkoxy;
R⁴ is selected from the group consisting of hydrogen, loweralkyl, a pharmaceutically acceptable cation and a prodrug ester group;
R⁵ is selected from the group consisting of hydrogen, halogen, hydroxy, loweralkyl, halo(loweralkyl), loweralkoxy and a group having the formula -NR¹³R¹⁴ wherein R¹³ and R¹⁴ are independently selected from the group consisting of hydrogen, loweralkyl, hydroxy-substituted loweralkyl, alkoxy-substituted loweralkyl and alkanoyl of from 1 to 8 carbons; and
A is N or CR⁶, wherein R⁶ is selected from the group consisting of halogen, loweralkyl, halo(loweralkyl), hydroxy-substituted loweralkyl, loweralkoxy(loweralkyl), loweralkoxy and amino(loweralkyl) or wherein R¹ and R⁶, taken together with the atoms to which they are attached, form a 6-membered saturated ring which may contain an oxygen or a sulfur atom and which may be substituted with loweralkyl.

Representative of the compounds of the present invention are those having the formula 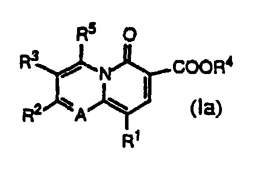 wherein A is N or CR⁶; R⁶ is selected from the group consisting of halogen, loweralkyl, halo(loweralkyl), hydroxy-substituted loweralkyl, loweralkoxy(loweralkyl), loweralkoxy and amino(loweralkyl); and R¹-R⁵ are as defined above.

Preferred compounds of the present invention include those of formula (Ia) in which R² is a nitrogen-containing heterocycle having the formula 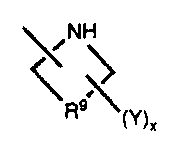 wherein x is 0 to 3; R⁹ is selected from the group consisting of (a) -(CH₂)ₘ-wherein m is 1, 2 or 3, and (b) -(CH₂)ₙR¹⁰(CH₂)ₚ- wherein R¹⁰ is S, O or N, n is 1 or 2, and p is 1 or 2; and Y is a non-hydrogen substituent independently selected from the group consisting of loweralkyl, halo(loweralkyl), loweralkoxy, hydroxy-substituted loweralkyl, hydroxy, amino(loweralkyl), halogen and a group having the formula -NR¹¹R¹² wherein R¹¹ and R¹² are independently selected from hydrogen and loweralkyl or when one is hydrogen, the other is selected from the group consisting of an alkanoyl of from 1 to 8 carbons, an α-amino acid and a polypeptide residue of from 2 to 5 amino acids.

Other embodiments of the compounds of the present invention are those in which R¹ and R⁶, taken together with the atoms to which they are attached, form a 6-membered saturated ring which may contain an oxygen or a sulfur atom and which may be substituted with loweralkyl, as represented in the formula 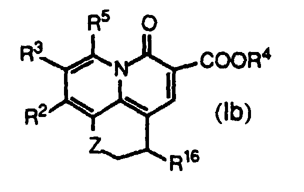 wherein Z is selected from the group consisting of CH₂, O and S; R¹⁶ is loweralkyl; and R²-R⁵ are as defined above.

Preferred compounds of the present invention also include those of formula (Ib) in which Z is O; R² is a nitrogen-containing heterocycle having the formula 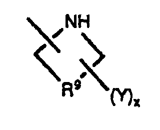 wherein x is 0 to 3; R⁹ is selected from the group consisting of (a) -(CH₂)ₘ-wherein m is 1, 2 or 3, and (b) -(CH₂)ₙR¹⁰(CH₂)ₚ- wherein R¹⁰ is S, O or N, n is 1 or 2, and p is 1 or 2; and Y is a non-hydrogen substituent independently selected from the group consisting of loweralkyl, halo(loweralkyl), loweralkoxy, hydroxy-substituted loweralkyl, hydroxy, amino(loweralkyl), halogen and a group having the formula -NR¹¹R¹² wherein R¹¹ and R¹² are independently selected from hydrogen and loweralkyl or, when one is hydrogen, the other is selected from the group consisting of an alkanoyl of from 1 to 8 carbons, an a-amino acid and a polypeptide residue of from 2 to 5 amino acids; and R¹⁶ is methyl.

Preferred compounds of the present invention include the following:
3-fluoro-9-(4-fluorophenyl)-2-(4-methylpiperazin-1-yl)-6(H)-6-oxo-pyrido[1,2-a]pyrimidine-7-carboxylic acid;
9-(2,4-difluorophenyl)-3-fluoro-2-(4-methylpiperazin-1-yl)-6(H)-6-oxo-pyrido[1,2-a]pyrimidine-7-carboxylic acid;
3-fluoro-9-cyclopropyl-2-(4-methylpiperazin-1-yl)-6(H)-6-oxo-pyrido[1,2-a]pyrimidine-7-carboxylic acid;
2-(3-aminopyrrolidin-1-yl)-9-cyclopropyl-3-fluoro-6H-6-oxo-pyrido[1,2-a]pyrimidine-7-carboxylic acid hydrochloride salt;
2-(3-aminopyrrolidin-1-yl)-9-cyclopropyl-3-fluoro-6H-6-oxo-pyrido[1,2-a]pyrimidine-7-carboxylic acid;
9-(2,4-difluorophenyl)-3-fluoro-2-(4-methylpiperazin-1-yl)-6H-6-oxopyrido[1,2-a]pyrimidine-7-carboxylic acid;
2-(3-aminopyrrolidin-1-yl)-9-(2,4-difluorophenyl)-3-fluoro-6H-6-oxopyrido[1,2-a]pyrimidine-7-carboxylic acid;
2-(3-(N-*t*-butoxycarbonyl)aminopyrrolidin-1-yl)-9-(2,4-difluorophenyl)-3-fluoro-6H-6-oxopyrido[1,2-a]pyrimidine-7-carboxylic acid;
2-(3-aminopyrrolidin-1-yl)-9-(2,4-difluorophenyl)-3-fluoro-6H-6-oxopyrido[1,2-a]pyrimidine-7-carboxylic acid;
9-cyclopropyl-3-fluoro-2-(4-methylpiperazin-1-yl)-6H-6-oxo-pyrido[1,2-a]pyrimidine-7-carboxylic acid;
9-cyclopropyl-3-fluoro-2-(piperazin-1-yl)-6H-6-oxo-pyrido[1,2-a]pyrimidine-7-carboxylic acid;
9-cyclopropyl-3-fluoro-2-(morpholin-1-yl)-6H-6-oxo-pyrido[1,2-a]pyrimidine-7-carboxylic acid;
9-(2,4-difluorophenyl)-3-fluoro-2-(3-(N-(S)-norvalyl)aminopyrrolidin-1-yl)-6H-6-oxopyrido[1,2-a]pyrimidine-7-carboxylic acid hydrochloride salt;
2-(3-(N-(S)-alanyl)aminopyrrolidin-1-yl)-9-(2,4-difluorophenyl)-3-fluoro-6H-6-oxopyrido[1,2-a]pyrimidine-7-carboxylic acid hydrochloride;
2-(3-(N-(S)-alanyl-(S)-alanyl)aminopyrrolidin-1-yl)-9-(2,4-difluorophenyl)-3-fluoro-6H-6-oxopyrido[1,2-a]pyrimidine-7-carboxylic acid hydrochloride;
2-((2S,4S)-4-acetamido-2-methylpyrrolidin-1-yl)-9-(2,4-difluorophenyl)-3-fluoro-6H-6-oxopyrido[1,2-a]pyrimidine-7-carboxylic acid;
9-(2,4-difluorophenyl)-3-fluoro-2-(3-hydroxypyrrolin-1-yl)-6H-6-oxopyrido[1,2-a]pyrimidine-7-carboxylic acid; and
2-((2S,4S)-4-amino-2-methylpyrrolidin-1-yl)-9-(2,4-difluorophenyl)-3-fluoro-6H-6-oxopyrido[1,2-a]pyrimidine-7-carboxylic acid hydrochloride.

In another aspect of the present invention are disclosed compositions comprising a therapeutically effective amount of a compound of the invention and a pharmaceutically acceptable carrier or diluent.

In yet another aspect of the present invention is disclosed the use of the compounds according to the invention for manufacturing a medicament for treating and preventing bacterial infections in an animal host (including but not limited to humans) in need thereof, said treatment and prevention comprising the administration of a therapeutically effective amount of a compound of the invention to such a host. It has been found that the compounds of the present invention possess antibacterial activity against a wide spectrum of Gram-positive and Grain-negative bacteria as well as enterobacteria. In addition, the compounds, by reason of their *in vitro* activity, may be used in scrub solutions for surface inhibition of bacterial growth. Susceptible organisms generally include those aerobic and anaerobic organisms whose growth can be inhibited by the compounds of the invention such as *Staphylococcus, Lactobacillus, Micrococcus, Enterococcus, Streptococcus,* *Sarcina, Escherichia, Enterobacter, Klebsiella, Pseudomonas, Acinobacter, Proteus, Providencia, Citrobacter, Nisseria, Baccillus, Bacteroides, Camphylobacter, Peptococcus, Clostridium, Salmonella, Shigella, Legionella, Serratia, Haemophilus, Brucella* and the like. The compounds of this invention are, therefore, useful in the antibiotic treatment of susceptible bacterial infections in both humans and animals.

The term "alkanoyl of from 1 to 8 carbons" as used herein refers to a substituent of the formula R¹⁵C(O)- wherein R¹⁵ is hydrogen or an alkyl group of from 1 to 7 carbon atoms, and includes acetylamino and pivaloylamino.

The terms "α-amino acid" and "polypeptide residue" refer to a single amino acid or two or more amino acids joined by amide (peptide) bonds. The amino acids can be any of the naturally occurring amino acids such as valine, phenylalanine or glycine or they may be synthetic α-amino acids such as cyclohexylalanine. The amino acids can either be in the L or D configuration or a mixture of the two isomers. If not specified, amino acid substituents are optically active and have the L configuration.

The term "amino(loweralkyl)" refers to loweralkyl groups, as defined below, having at least one amino substituent, which may have one or two loweralkyl substituents or one alpha-amino acid or polypeptide residue substituent. Examples of amino(loweralkyl) groups include aminoethyl, aminomethyl, N,N-dimethylaminoethyl and the like.

The term "aromatic group" refers to a C6 to C10 cyclic group which is aromatic according to Huckel's rule, for example phenyl and naphthyl.

The term "bicycloalkyl" refers to cyclic hydrocarbon rings wherein non-adjacent carbon atoms are bridged by 1-3 additional carbon atoms and having from four to nine carbon atoms in the ring system including, but not limited to, norbornyl, bicylo[2.2.1]hept-2-enyl, bicyclo[1.1.1]pentanyl and the like. The cyclic group may be optionally substituted with, for example, carbocyclic aryl(loweralkyl), alkoxycarbonyl, loweralkyl, halo(loweralkyl), amino(loweralkyl), hydroxy-substituted loweralkyl, hydroxy, loweralkoxy, halogen, or an amino, loweralkylamino, or alkanoylamino group of from 1 to 8 carbon atoms, wherein the amino group may be substituted with alkanoyl of from 1 to 8 carbons, an α-amino acid or a polypeptide of from two to five amino acids.

The term "bicyclic nitrogen-containing heterocyclic group" refers to a group selected from one of the formulae 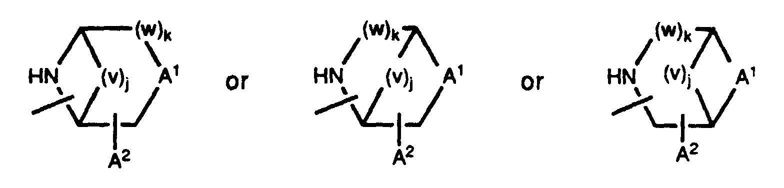 wherein v and w are CH₂; j and k are independently 1 , 2 or 3; A¹ is a carbon atom or a heteroatom selected from S, O and N; and A² is one or more non-hydrogen substituents selected independently from the group consisting of loweralkyl, halo(loweralkyl), hydroxy-substituted loweralkyl, hydroxy, halogen, amino(loweralkyl), alkanoylamino of from 1 to 8 carbons, phenyl and a group having the formula -NR⁷R⁸ wherein R⁷ and R⁸ are independently selected from hydrogen and loweralkyl or, when one is hydrogen, the other is an α-amino acid or a polypeptide residue of from two to five amino acids.

The term carbocyclic aryl(loweralkyl)" refers to an aromatic group, such as a phenyl group, which is bonded to a loweralkyl group. Examples of carbocyclic aryl(loweralkyl) groups include benzyl, phenylethyl and the like.

The term "carbocyclic aryl(loweralkyl)amino" refers to an carbocyclic aryl(loweralkyl) group as defined above bonded to an amino group. Examples of carbocyclic aryl(loweralkyl)amino groups include benzylamino, phenylethylamino and the like.

The term carbocyclic aryl(loweralkyl)oxy" refers to an carbocyclic aryl(loweralkyl) group, as defined above, which is bonded to an oxygen atom in an ether linkage. Examples of carbocyclic aryl(loweralkyl)oxy groups include benzyloxy, phenylethyloxy and the like.

The term "carbocyclic aryloxy" refers to an aromatic group, as defined above, bonded to an oxygen atom in an ether linkage, for example phenoxy.

The tem "cycloalkenyl of from 3 to 8 carbons" refers to a mono-unsaturated monocyclic hydrocarbon groups having from three to seven carbon atoms in the ring, including, but not limited to, cyclobutenyl, cyclopentenyl, cyclohexenyl, cycloheptenyl and the like. The cyclic group may be optionally substituted with, for example, carbocyclic aryl(loweralkyl), alkoxycarbonyl, loweralkyl, halo(loweralkyl), amino(loweralkyl), hydroxy-substituted loweralkyl, hydroxy, loweralkoxy, halogen, or an amino, loweralkylamino, or alkanoylamino group of from 1 to 8 carbon atoms, wherein the amino group may be substituted with alkanoyl of from 1 to 8 carbons, an α-amino acid or a polypeptide of from two to five amino acids.

The term "cycloalkyl of from 3 to 8 carbons" refers to saturated monocyclic hydrocarbon groups having from three to eight carbon atoms in the ring, including, but not limited to, cyclopropyl, cyclobutyl, cyclopentyl, cyclohexyl, cycloheptyl and the like. The cyclic group may be optionally substituted with, for example, carbocyclic aryl(loweralkyl), alkoxycarbonyl, loweralkyl, halo(loweralkyl), amino(loweralkyl), hydroxy-substituted loweralkyl, hydroxy, loweralkoxy, halogen, or an amino, loweralkylamino, or alkanoylamino group of from 1 to 8 carbon atoms, wherein the amino group may be substituted with alkanoyl offrom 1 to 8 carbons, an α-amino acid or a polypeptide of from two to five amino acids.

The term "cycloalkyl(loweralkyl)" refers to a cycloalkyl of from 3 to 8 carbon atoms, which is bonded to a loweralkyl group. The cyclic group may be optionally substituted with, for example, carbocyclic aryl(loweralkyl), alkoxycarbonyl, loweralkyl, halo(loweralkyl), amino(loweralkyl), hydroxy-substituted loweralkyl, hydroxy, loweralkoxy, halogen, or an amino, loweralkylamino, or alkanoylamino group of from 1 to 8 carbon atoms, wherein the amino group may be substituted with alkanoyl of rom 1 to 8 carbons, an α-amino acid or a polypeptide of from two to five amino acids.

The term "fused" as used herein refers to two cyclic groups having at least two atoms in common to both rings.

As used herein, the term "halogen" refers to chloro (Cl), bromo (Br), fluoro (F) and iodo (I).

The term "halo(loweralkyl)" refers to a loweralkyl group, as defined above, in which at least one hydrogen atom is replaced with a halogen atom. Examples of halo(loweralkyl) groups include fluoromethyl, trifluoromethyl, fluoroethyl and the like.

The term "hydroxy-substituted loweralkyl" refers to loweralkyl groups, as defined above, having at least one hydroxyl substituent, as for example hydroxyethyl and the like.

The term "loweralkenyl" refers to straight or branched chain hydrocarbon groups containing from two to six carbon atoms and possessing at least one carbon-carbon double bond. Examples of loweralkenyl groups include vinyl, allyl, 2- or 3-butenyl, 2-,3- or 4-pentenyl, 2-,3-,4- or 5-hexenyl, isomeric forms thereof and the like.

The term "loweralkoxy" refers to a loweralkyl group, as defined below, which is bonded to an oxygen atom in an ether linkage. Examples of loweralkoxy groups include methoxy, ethoxy, propoxy, t-butoxy, pentyloxy, hexyloxy, isomeric forms thereof and the like.

The term "loweralkoxycarbonyl" refers to a loweralkoxy group as defined above bonded through an ester linkage to a carbonyl group, as for example ethoxycarbonyl, methoxycarbonyl and the like.

The term" loweralkoxy(loweralkyl)" refers to a loweralkoxy group as defined above bonded through an ether linkage to a loweralkyl substituent, as for example methoxyethyl, ethoxymethyl and the like.

The term "loweralkyl" refers to branched or straight chain loweralkyl groups containing one to six carbon atoms including, but not limited to, methyl, ethyl, propyl, isopropyl, n-butyl, t-butyl, neopentyl and the like.

The term "(loweralkyl)amino" refers to amino groups substituted with one to three loweralkyl groups, as defined above, including methylamino, ethylamino, dimethylamino, propylamino, ethylmethylamino and the like.

The term "loweralkynyl" refers to straight or branched chain hydrocarbon groups containing from two to six carbon atoms and possessing at least one carbon-carbon triple bond. Examples of loweralkynyl groups include ethynyl, 2-hexyn-1-yl, 3,3-dimethyl-1-butyn-1-yl, 3-methylbutyn-3-yl and the like.

The term "nitrogen-containing aromatic heterocycle" refers to monocyclic aromatic ring systems having from five to seven ring atoms of which one, two or three ring atoms are independently heteroatoms selected from S, O and N, at least one heteroatom being nitrogen, with the remaining atoms being carbon. Examples of nitrogen-containing aromatic heterocycles include pyridine, pyrazine, pyrimidine, pyrrole, pyrazole, imidazole, thiazole, oxazole, isooxazole, substituted derivatives thereof and the like.

The term "nitrogen-containing heterocycle" as used herein refers to a 3- to 7-atom cyclic group containing one, two or three heteroatoms selected from S, O and N, at least one heteroatom being nitrogen. The cyclic group may be optionally substituted, either on a heteroatom or on a carbon atom, as for example with carbocyclic aryl(loweralkyl), alkoxycarbonyl, loweralkyl, halo(loweralkyl), amino(loweralkyl), hydroxy-substituted loweralkyl, hydroxy, loweralkoxy or halogen, or an amino, loweralkylamino, or alkanoylamino group of from 1 to 8 carbon atoms, wherein the amino group may be substituted with alkanoyl of from 1 to 8 carbons, an α-amino acid or a polypeptide of from two to five amino acids. Examples of nitrogen-containing heterocycles include pyrrolidine, isooxazolidine, oxazolidine, piperidine, piperazine, morpholine, thiomorpholine, aziridine, azetidine and the like.

The term "pharmaceutically acceptable cation" refers to a positively charged inorganic or organic ion that is generally considered suitable for human consumption. Examples of pharmaceutically acceptable cations are hydrogen, alkali metal (lithium, sodium and potassium), magnesium, calcium, ferrous, ferric, ammonium, alkylammonium, dialkylammonium, trialkylammonium, tetraalkylammonium, diethanolammmonium, triethanolammonium, and guanidinium ions, and protonated forms of lysine, procaine and choline. Cations may be interchanged by methods known in the art, such as ion exchange. Where compounds of the present invention are prepared in the carboxylic acid form (that is, where R₄ is hydrogen) addition of a base form of the cation, (such as a hydroxide or a free amine) will yield the appropriate cationic form.

By "pharmaceutically acceptable salts, esters and amides" is meant those carboxylate salts, amino acid addition salts, esters and amides of the compounds of of formula I which are, within the scope of sound medical judgement, suitable for use in contact with the tissues of humans and lower animals without undue toxicity, irritation, allergic response, and the like, commensurate with a reasonable benefit/risk ratio, and effective for their intended use, as well as the zwitterionic forms of the compounds of formula I.

Pharmaceutically acceptable salts are well known in the art. For example, S. M Berge *et al.* describe pharmaceutically salts in detail in J. Pharmaceutical Sciences, 66:1-19 (1977). Examples of pharmaceutically acceptable, nontoxic acid addition salts are salts of an amino group formed with inorganic acids such as hydrochloric acid, hydrobromic add, phosphoric acid, sulfuric acid and perchloric acid or with organic adds such as acetic acid, oxalic add, maleic acid, tartaric acid, citric acid, succinic add or malonic acid or by using other methods used in the art such as ion exchange. Other pharmaceutically acceptable salts include nitrate, bisulfate, borate, formate, butyrate, valerate, 3-phenylpropionate, camphorate, adipate, benzoate, oleate, palmitate, stearate, laurate, lactate, fumarate, ascorbate, aspartate, nicotinate, p-toluenesulfonate, camphorsulfonate, methanesulfonate, 2-hydroxyethanesulfonate, gluconate, glucoheptonate, lactobionate, glycerophosphate, pectinate, lauryl sulfate, and the like or metal salts such as sodium, potassium, magnesium or calcium salts or amino salts such as ammonium, triethylamine salts and the like, which may be prepared according to conventional methods.

Examples of pharmaceutically acceptable, non-toxic esters of the compounds of formula I include C1 to C6 alkyl esters wherein the alkyl group is straight or branched chain. Acceptable esters also include C5 to C7 cycloalkyl esters, although C1 to C4 alkyl esters are preferred. Esters of the compounds of formula I may be prepared according to conventional methods.

Examples of pharmaceutically acceptable, nontoxic amides of the compounds of formula I include amides derived from ammonia, primary C1 to C6 alkyl amines and secondary C1 to C6 dialkyl amines wherein the alkyl groups are straight or branched chain. In the case of secondary amines, the amine may also be in the form of a 5- or 6-membered heterocycle containing one nitrogen atom. Amides derived from ammonia, C1 to C3 alkyl primary amides and C1 to C2 dialkyl secondary amides are preferred. Amides of the compounds of formula I may be prepared according to conventional methods. It is understood that amides of the compounds of the present invention include amino acid and peptide derivatives.

As used herein, the term "pharmaceutically acceptable carrier means a non-toxic, inert solid, semi-solid or liquid filler, diluent, encapsulating material or formulation auxillary of any type. Some examples of the materials that can serve as pharmaceutically acceptable carriers are sugars, such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository waxes; oils such as peanut oil, cottonseed oil, safflower oil, sesame oil, olive oil, corn oil and soybean oil: glycols, such as propylene glycol; polyols such as glycerin, sorbitol, mannitol and polyethylene glycol; esters such as ethyl oleate and ethyl laurate; agar; buffering agents such as magnesium hydroxide and aluminum hydroxide; alginic acid; pyrogen-free water; isotonic saline; Ringer's solution; ethyl alcohol and phosphate buffer solutions, as well as other non-toxic compatible substances used in pharmaceutical formulations. Wetting agents, emulsifiers and lubricants such as sodium lauryl sulfate and magnesium stearate, as well as coloring agents, releasing agents, coating agents, sweetening, flavoring and perfuming agents, and preservatives can also be present in the composition, according to the judgement of the formulator.

The term "phenyl" refers to optionally substituted benzene rings having zero to five non-hydrogen substituents independently selected from the group consisting of halogen, hydroxy, loweralkoxy, loweralkyl, hydroxy-substituted loweralkyl, amino, (loweralkyl)amino, amino(lower)alkyl and a nitrogen-containing heterocycle, as for example aziridinyl, pyrrolidinyl, piperidinyl and the like.

The term "prodrug" refers to compounds that are rapidly transformed *in vivo* to yield the parent compound of the formula I, as for example by hydrolysis in blood. T. Higuchi and V. Stella provide a thorough discussion of the prodrug concept in "Pro-drugs as Novel Delivery Systems", Vol 14 of the A.C.S. Symposium Series, American Chemical Society (1975). Examples of esters useful as prodrugs for compounds containing carboxyl groups can be found on pages 14-21 of "Bioreversible Carriers in Drug Design: Theory and Application", edited by E.B. Roche, Pergamon Press:New York (1987).

The term "prodrug ester group" refers to any of several ester forming groups that are hydrolyzed under physiological conditions. Examples of prodrug ester groups include pivoyloxymethyl, acetoxymethyl, phthalidyl, indanyl and methoxymethyl, as well as other such groups known in the art, including a (5-R-2-oxo-1,3-dioxolen-4-yl)methyl group. Other examples of prodrug ester groups can be found in the book "Pro-drugs as Novel Delivery Systems", by Higuchi and Stella, cited above.

The term "protecting group" is well-known in the art and refers to substituents on functional groups of compounds undergoing chemical transformation which prevent undesired reactions and degradations during a synthesis; see, for example, T.H. Greene, "Protective Groups in Organic Synthesis", John Wiley & Sons, New York (1981).

The compounds of the present invention may be administered alone or in combination or in concurrent therapy with other agents.

The specific therapeutically effective dose level for any particular patient will depend upon a variety of factors including the disorder being treated and the severity of the disorder; activity of the specific compound employed; the specific composition employed; the age, body weight, general health, sex and diet of the patient; the time of administration, route of administration, and rate of excretion of the specific compound employed; the duration of the treatment; drugs used in combination or coincidently with the specific compound employed; and like factors well known in the medical arts.

The total daily dose of the compounds of this invention administered to a host in single or in divided doses can be in amounts, as for example from 0.1 to 750 mg/kg body weight or more usually from 0.25 to 500 mg/kg body weight. Single dose compositions may contain such amounts or submultiples thereof to make up the daily dose.

The compounds of the present invention may be administered orally, parenterally, by inhalation spray, rectally, or topically in dosage form unit formulations containing conventional nontoxic pharmaceutically acceptable carriers, adjuvants and vehicles as desired. The term "parenteral" as used herein includes subcutaneous injections, intravenous, intramuscular, intrasternal injection or infusion techniques.

Injectable preparations, as for example sterile injectable aqueous or oleaginous suspensions, may be formulated according to the known art using suitable dispersing or wetting agents and suspending agents. The sterile injectable preparation may also be a sterile injectable solution or suspension in a nontoxic parenterally acceptable diluent or solvent, as for example as a solution in 1,3-butanediol. Among the acceptable vehicles and solvents that may be employed are water, Ringer's solution, U.S.P. and isotonic sodium chloride solution. In addition, sterile, fixed oils are conventionally employed as a solvent or suspending medium. For this purpose any bland fixed oil can be employed including synthetic mono- or diglycerides. In addition, fatty acids such as oleic acid are used in the preparation of injectables.

In order to prolong the effect of a drug, it is often desirable to slow the absorption of a drug from subcutaneous or intramuscular injection. The most common way to accomplish this is to inject a suspension of crystalline or amorphous material with poor water solubility The rate of absorption of the drug becomes dependent on the rate of dissolution of the drug which is, in turn, dependent on the physical state of the drug, for example, the crystal size and the crystalline form. Another approach to delaying absorption of a drug is to administer the drug as a solution or suspension in oil. Injectable depot forms can also be made by forming microcapsule matrices of drugs and biodegradable polymers such as polylactide-polyglycolide. Depending on the ratio of drug to polymer and the composition of the polymer, the rate of drug release can be controlled. Examples of other biodegradable polymers include poly-orthoesters and polyanhydrides. Depot injectables can also be made by entrapping the drug in liposomes or microemulsions which are compatible with body tissues.

Suppositories for rectal or vaginal administration of the drug can be prepared by mixing the drug with a suitable nonirritating excipient such as cocoa butter and polyethylene glycol which are solid at ordinary temperature but will melt in the rectum or in the vagina and release the drug.

Solid dosage forms for oral administration may include capsules, tablets, pills, powders, prills and granules. In such solid dosage forms the active compound may be admixed with at least one inert diluent such as sucrose, lactose or starch. Such dosage forms may also comprise, as is normal practice, additional substances other than inert diluents, e.g., tableting lubricants and other tableting aids such as magnesium stearate and microcrystalline cellulose. In the case of capsules, tablets and pills, the dosage forms may also comprise buffering agents. Tablets and pills can additionally be prepared with enteric coatings and other release-controlling coatings.

Liquid dosage forms for oral administration may include pharmaceutically acceptable emulsions, microemulsions, solutions, suspensions, syrups and elixirs containing inert diluents commonly used in the art such as water. Such compositions may also comprise adjuvants, such as wetting agents; emulsifying and suspending agents; and sweetening, flavoring and perfuming agents.

If desired, the compounds of the present invention can be incorporated into slow release or targeted delivery systems such as polymer matrices, liposomes and microspheres. They may be sterilized, for example, by filtration through a bacteria-retaining filter, or by incorporating sterilizing agents in the form of sterile solid compositions which can dissolve in sterile water, or some other sterile injectable medium immediately before use.

The active compounds can also be in micro-encapsulated form with one or more excipients as noted above.

Dosage forms for topical or transdermal administration of a compound of this invention further include ointments, pastes, creams, lotions, gels, powders, solutions, sprays, inhalants or patches. The active component is admixed under sterile conditions with a pharmaceutically acceptable carrier and any needed preservatives or buffers as may be required. Ophthalmic formulations, ear drops, eye ointments, powders and solutions are also contemplated as being within the scope of this invention.

The ointments, pastes, creams and gels may contain, in addition to an active compound of this invention, excipients such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to the compounds of this invention, excipients such as lactose, talc, silicic acid, aluminum hydroxide, calcium silicates and polyamide powder, or mixtures of these substances. Sprays can additionally contain customary propellants such as chlorofluorohydrocarbons or substitutes therefor.

Transdermal patches have the added advantage of providing controlled delivery of a compound to the body. Such dosage forms can be made by dissolving or dispersing the compound in the proper medium. Absorption enhancers can also be used to increase the flux of the compound across the skin. The rate can be controlled by either providing a rate controlling membrane or by dispersing the compound in a polymer matrix or gel.

In general, the compounds of the present invention are synthesized according to reaction Schemes I through XI presented below, in which R -R¹⁶, A, X, Y and Z correspond to the groups defined in connection with formula (I), R is a loweralkyl group, X is a halogen atom, P is a protecting group and L is a good leaving group, as for example a halogen atom.

For the preparation of the compounds of formula I which are α-amino acid or peptide derivatives of amine groups at R², the condensation of the amino group with amino acids and peptides may be effected in accordance with conventional condensation methods such as the azide method, the mixed acid anhydride method, the DCC (dicyclohexylcarbodiimide) method, the active ester method (p-nitrophenyl ester method, N-hydroxysuccinic acid imide ester method, cyanomethyl ester method and the like), the Woodward reagent K method, the DCC-HOBT (1-hydroxy-benzotriazole) method and the like. Classical methods for amino acid condensation reactions are described in "Peptide Synthesis", Second Edition, M. Bodansky, Y.S. Klausner and M.A. Ondetti (1976). It is contemplated that the amino acid coupling reaction could be carried out before or after the amino-containing group is incorporated into the compound by displacement of the 7-fluorine atom of the appropriate intermediate.

As in conventional peptide synthesis, branched chain amino and carboxyl groups at alpha and omega positions in amino acids may be protected and deprotected if necessary. The protecting groups for amino groups which can be used involve, for example, benzyloxycarbonyl (Z), o-chloro-benzyloxycarbonyl((2-Cl)Z), p-nitrobenzyloxycarbonyl (Z(NO2)), p-methoxybenzyloxycarbonyl (Z(OMe)), t-butoxycarbonyl (Boc), t-amyloxycarbonyl (Aoc), isobornealoxycarbonyl, adamantyloxycarbonyl (Adoc), 2-(4-biphenyl)-2-propyloxy carbonyl (Bpoc), 9-fluorenyl-methoxycarbonyl (Fmoc), methylsulfonylethoxy carbonyl (Msc), trifluoroacetyl, phthalyl, formyl, 2-nitrophenylsulfenyl (Nps), diphenylphosphinothioyl (Ppt) and dimethylphosphino-thioyl (Mpt).

The examples of protecting groups for carboxyl groups involve, for example, benzyl ester (OBzl), cyclohexyl ester, 4-nitrobenzyl ester (OBzINO2), t-butyl ester (OtBu), 4-pyridylmethyl ester (OPic) and the like.

In the course of the synthesis of certain of the compounds of the present invention, specific amino acids having functional groups other than amino and carboxyl groups in the branched chain such as arginine, cysteine, serine and the like may be protected, if necessary, with suitable protecting groups. It is preferable that, for example, the guanidino group (NG) in arginine be protected with nitro, p-toluenesulfonyl (Tos), benzyloxycarbonyl (Z), adamantyloxycarbonyl (Adoc), p-methoxybenzenesulfonyl, 4-methoxy-2,6-dimethyl-benzenesulfonyl (Mts) or the like; that the thiol group in cysteine be protected with benzyl, p-methoxybenzyl, triphenylmethyl, acetamidomethyl, ethylcarbamyl, 4-methylbenzyl (4-MeBzl), 2,4,6,-trimethylbenzyl (Tmb) or the like; and that the hydroxy group in serine may be protected with benzyl (Bzl), t-butyl, acetyl, tetrahydropyranyl (THP) or the like.

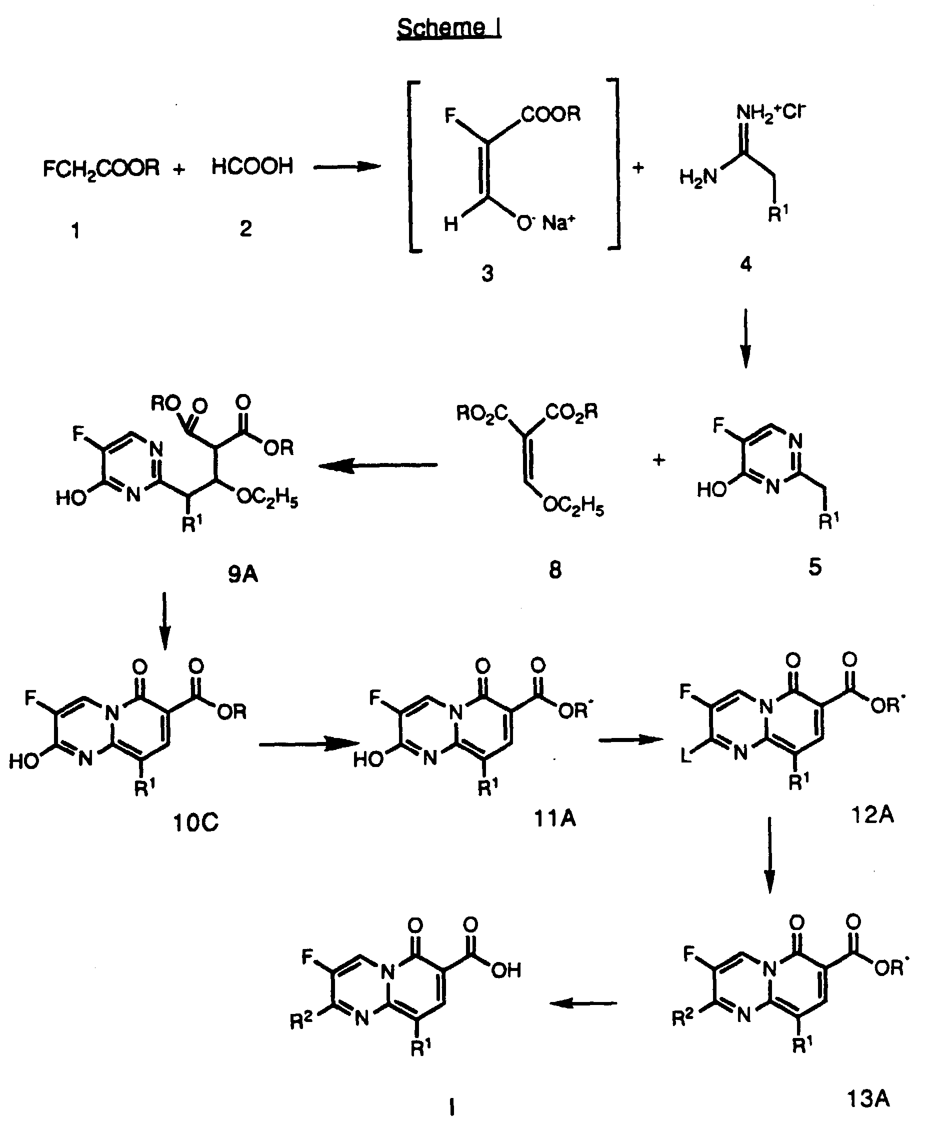

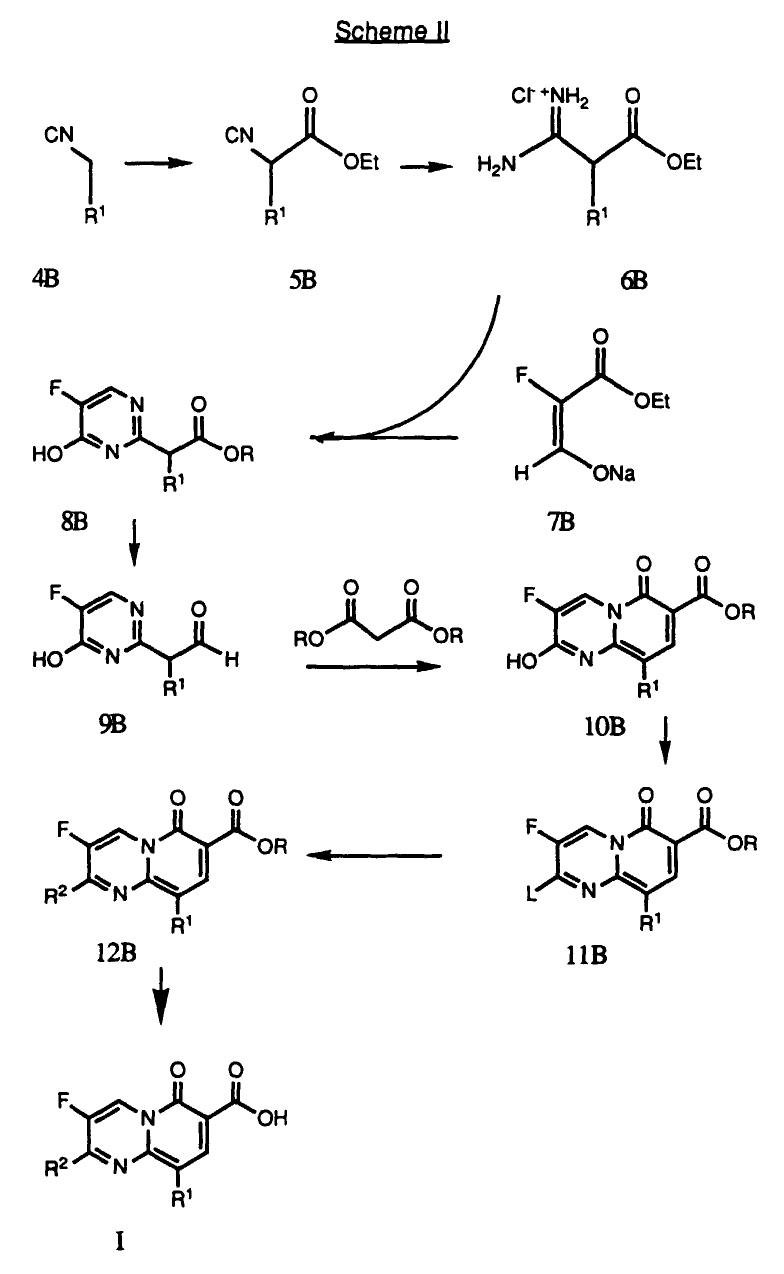

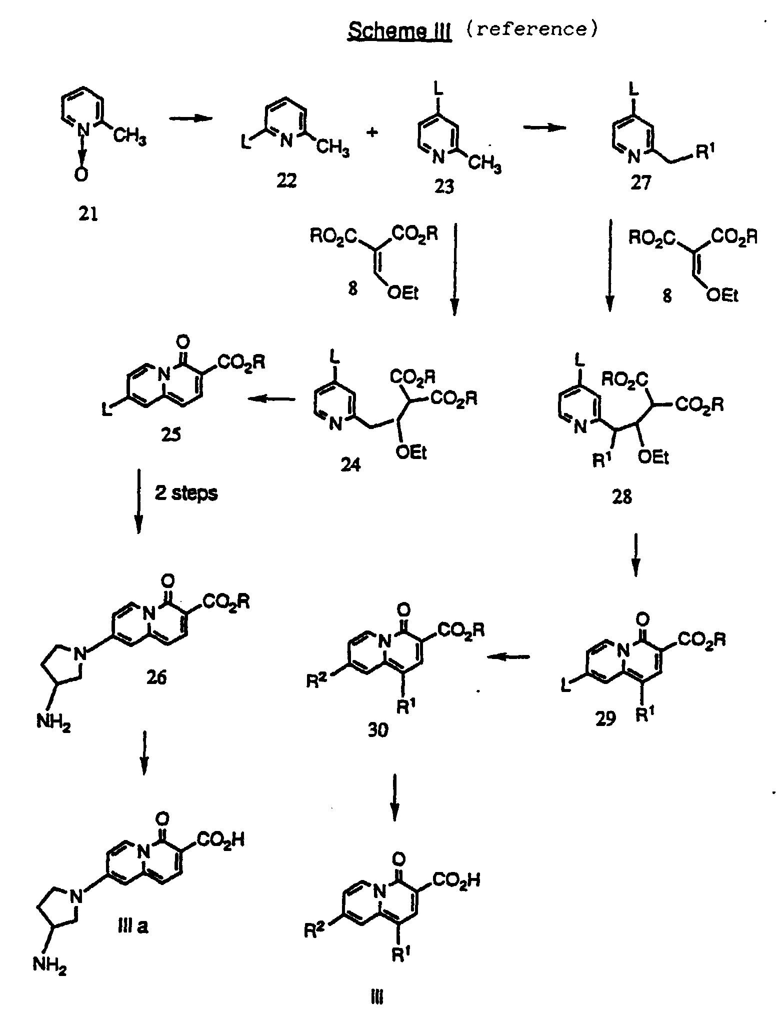

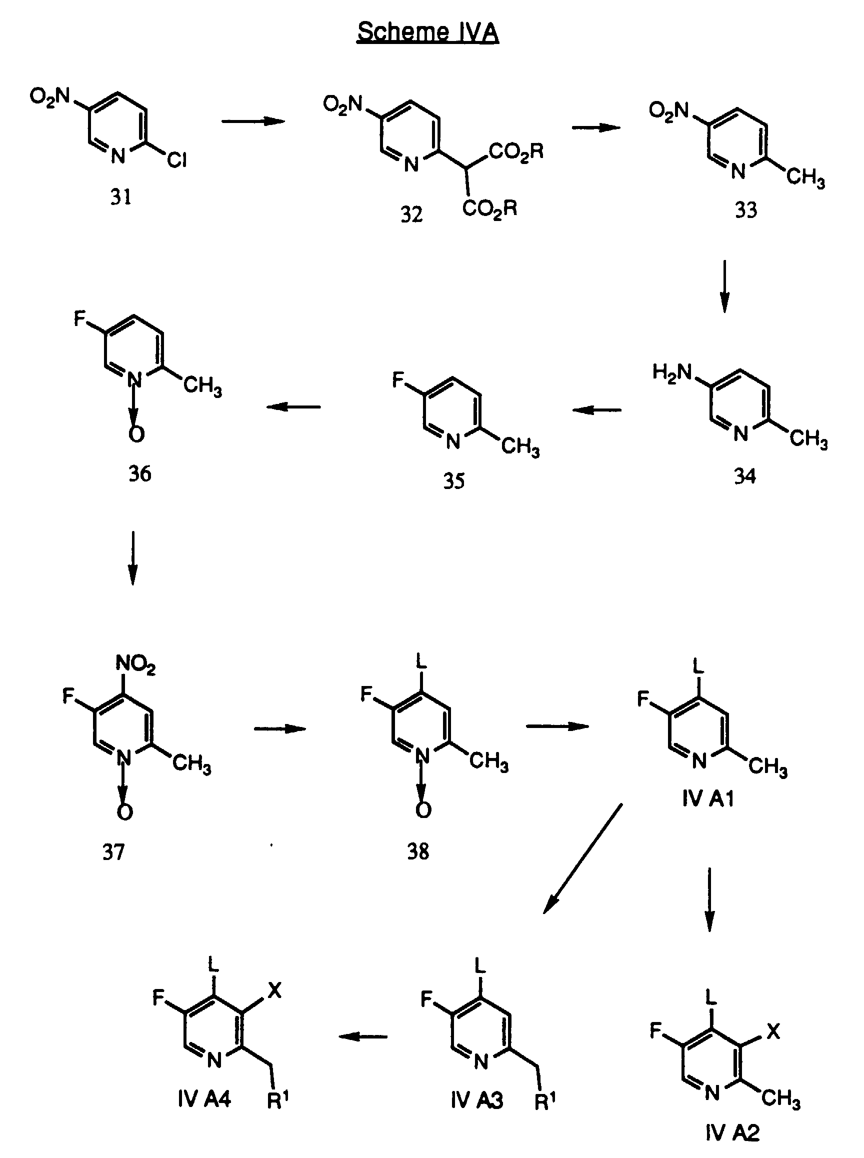

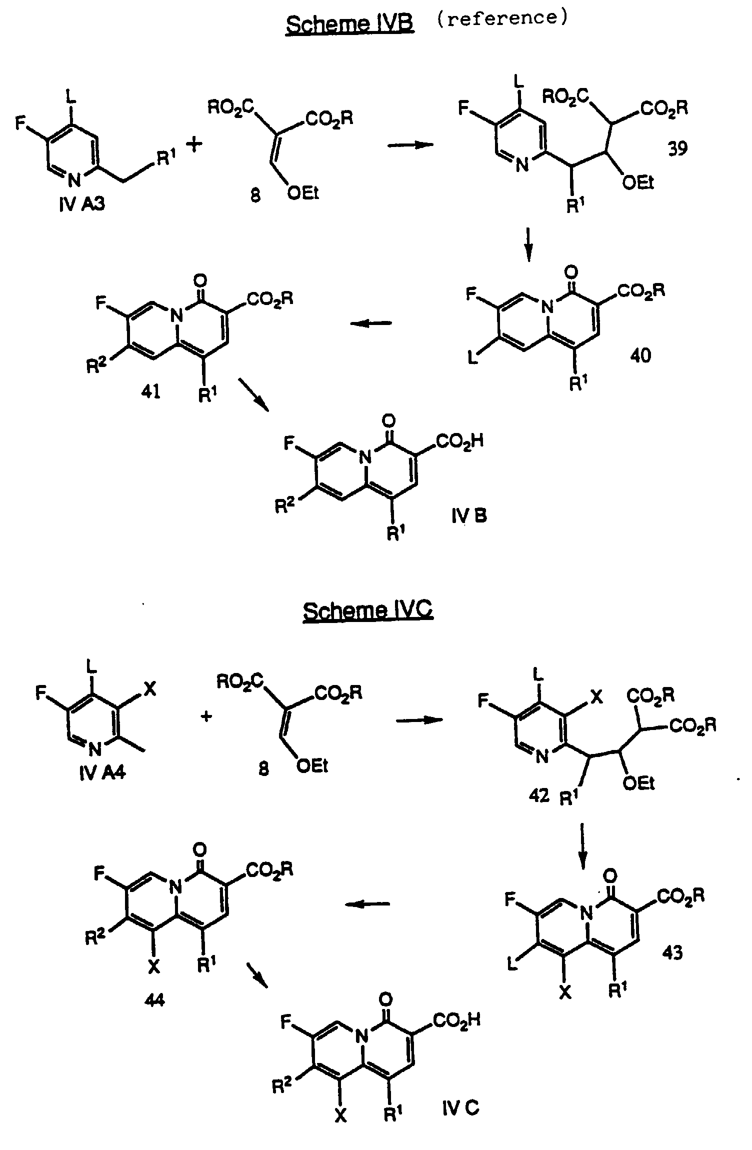

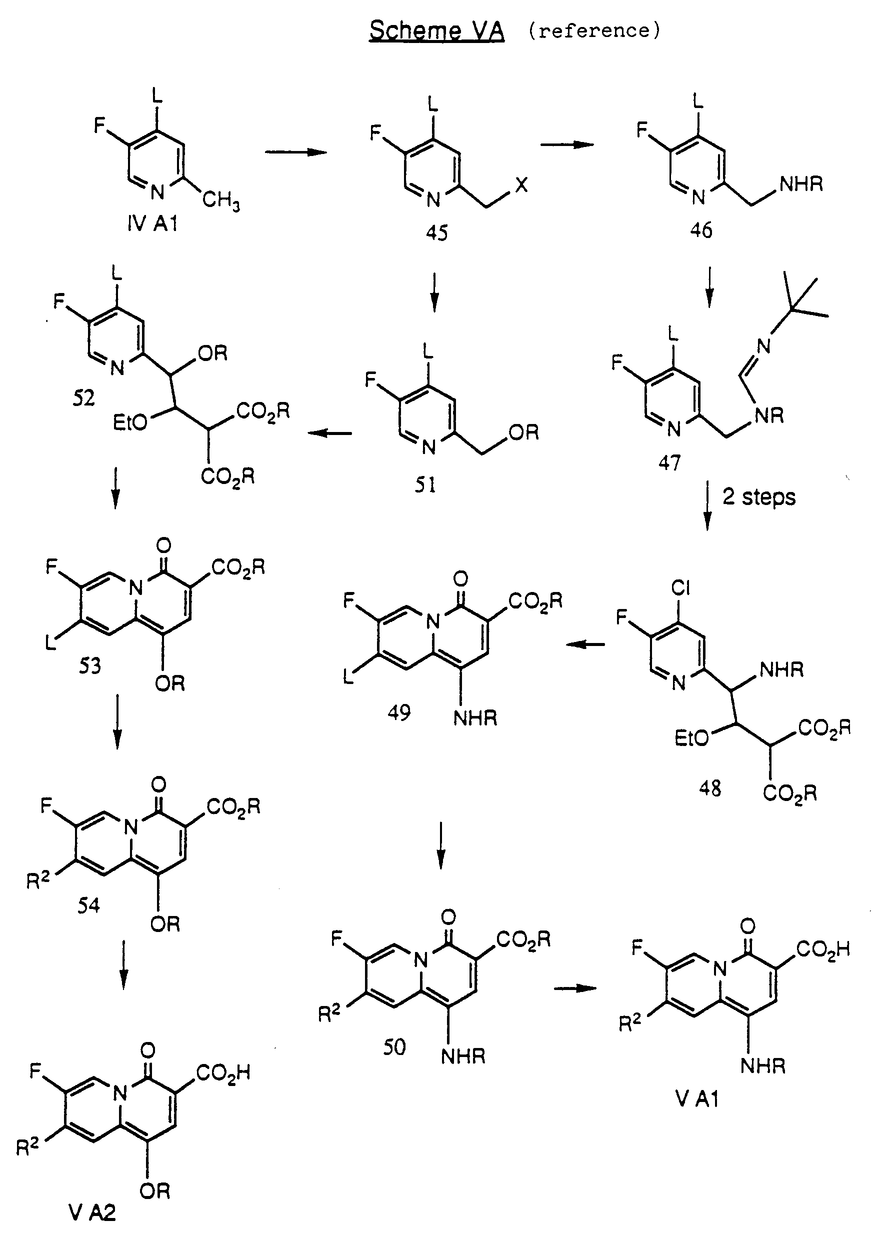

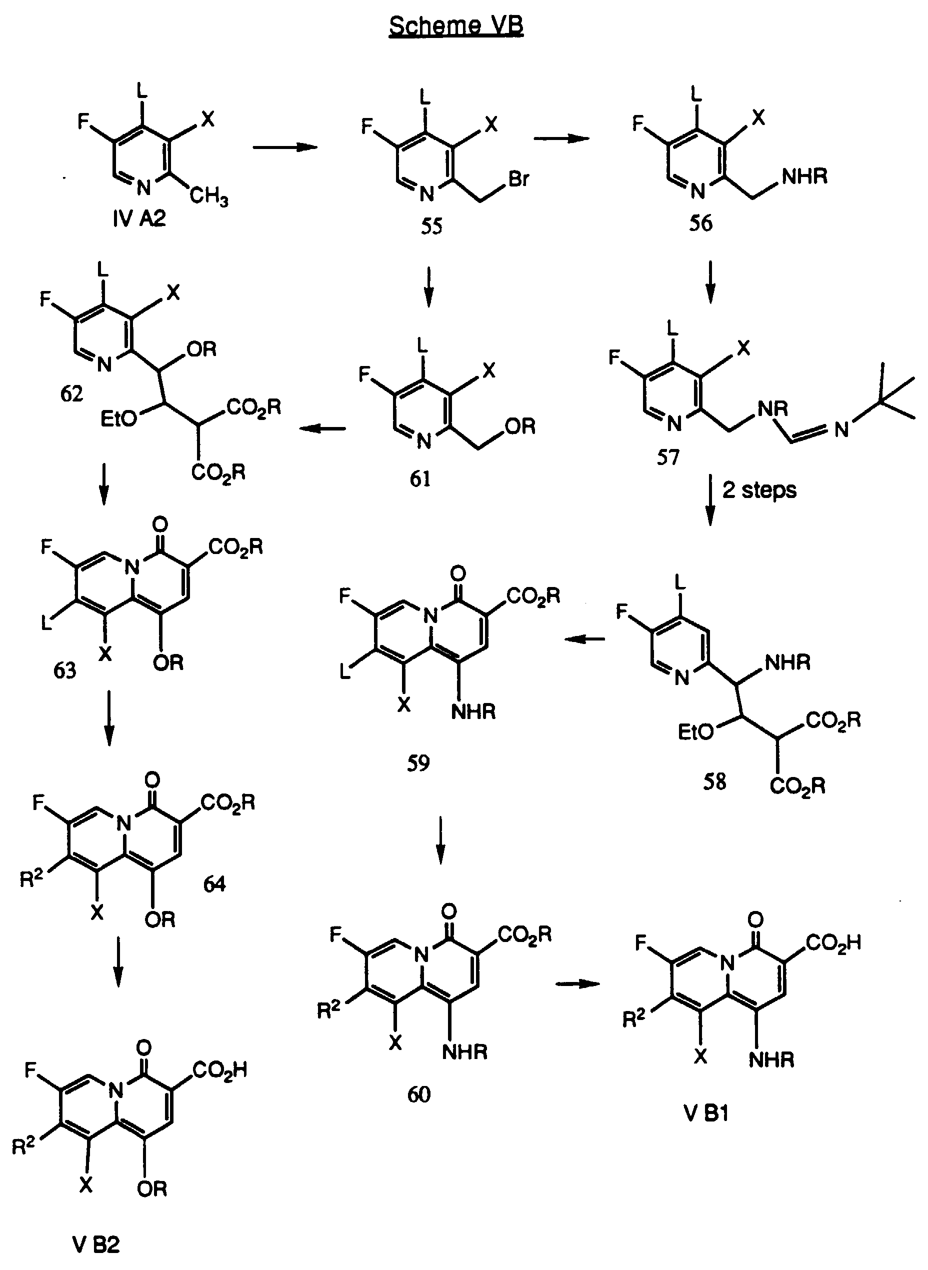

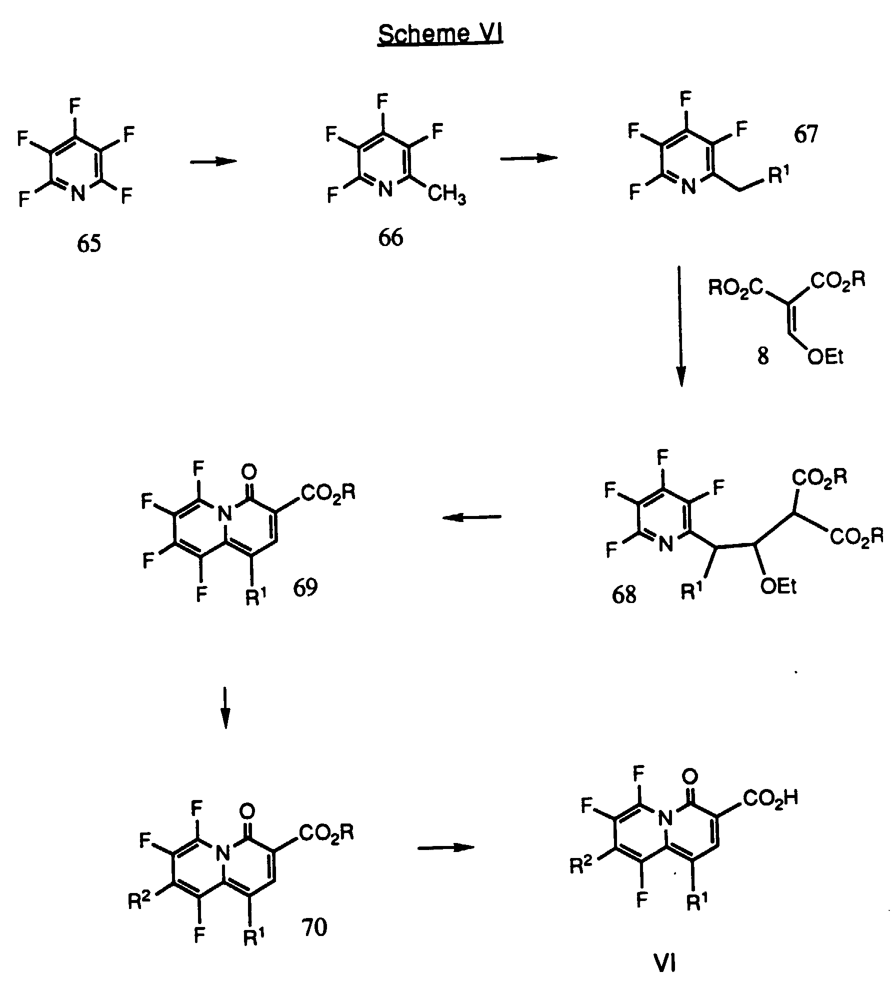

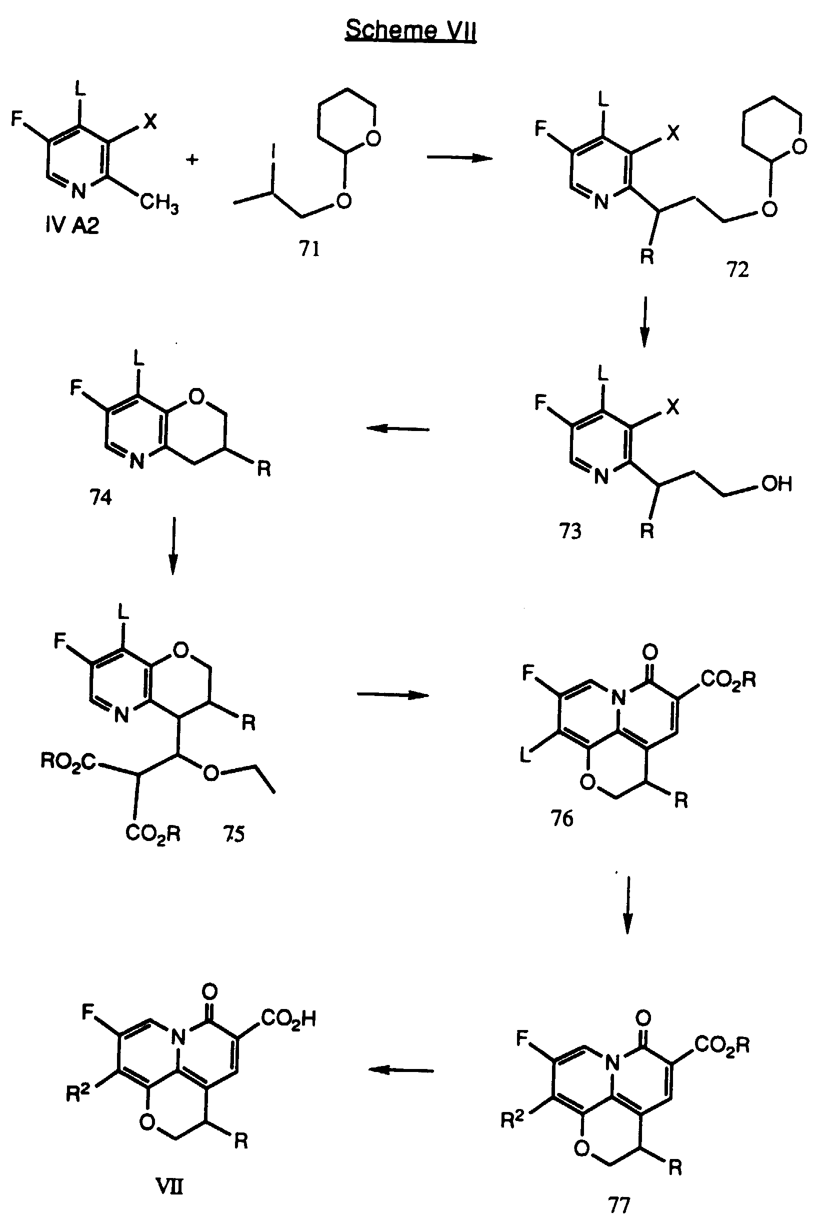

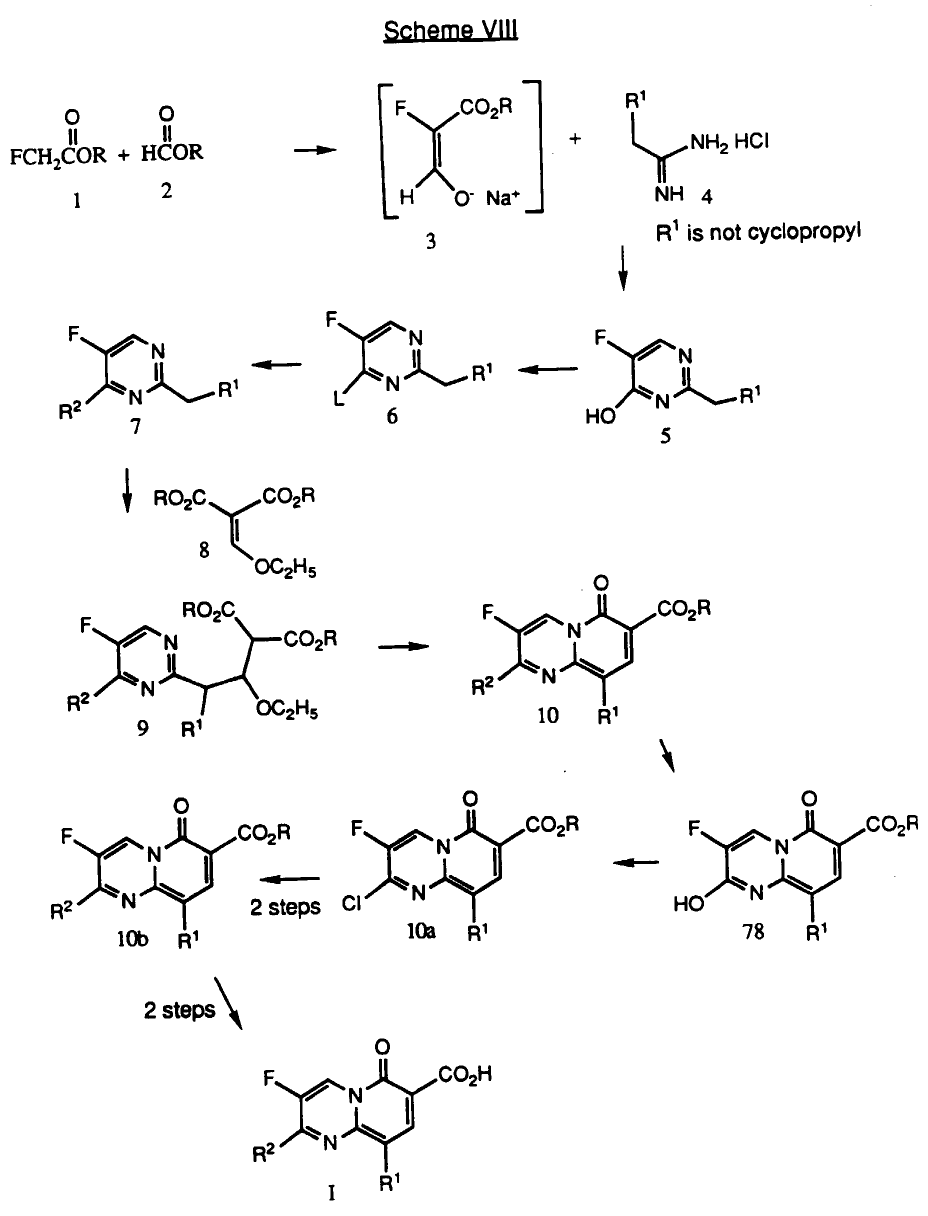

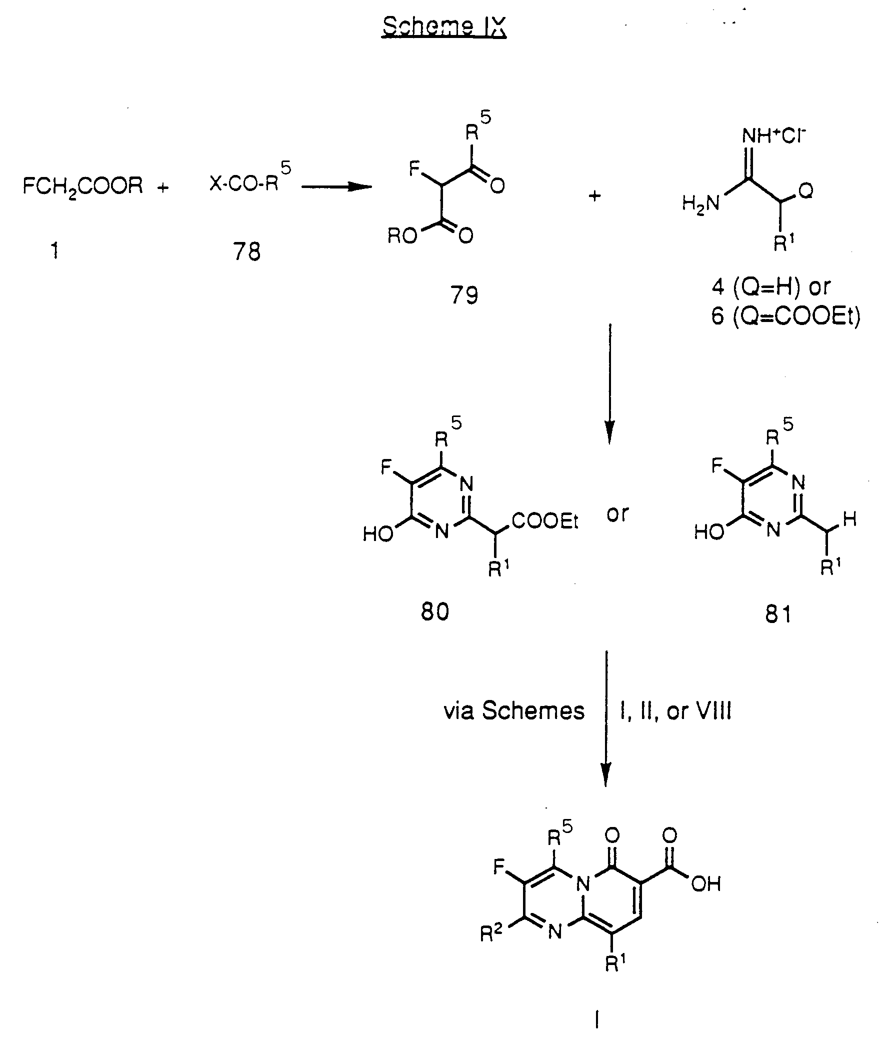

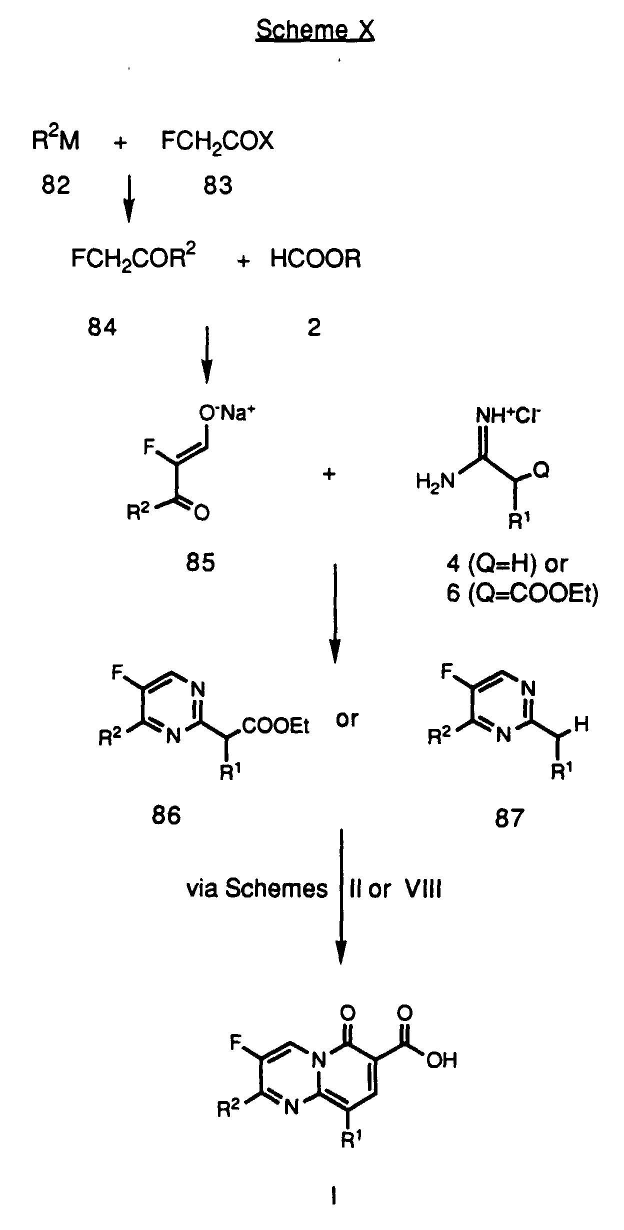

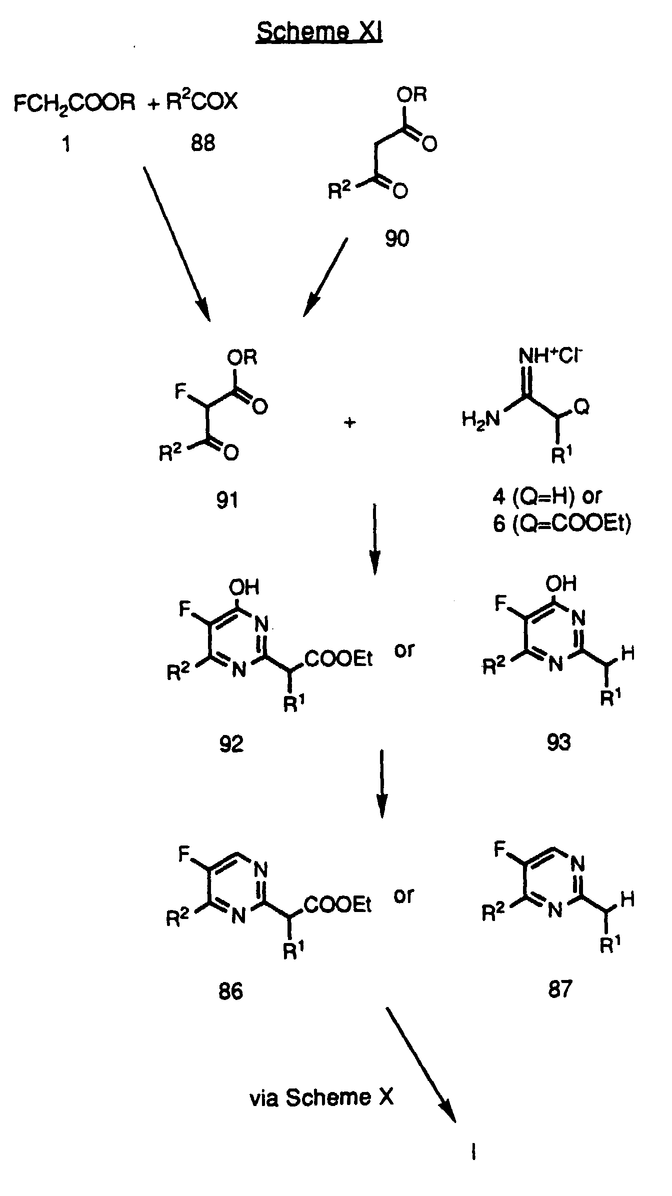

In accordance with reaction Scheme 1 illustrated above, an alpha-halo acetate derivative of formula 1, such as ethyl 2-fluoroacetate, is condensed with a formate ester of formula 2, in the presence of a suitable base, as for example sodium ethoxide, in an inert solvent such as diethyl ether to to give an enolate compound of formula 3. Compounds of formula 3 are, in turn, converted to compounds of formula 5 by condensation with an amidine derivative of formula 4, in which R¹ is an electron withdrawing group such as phenyl, trifluoromethyl, cyano, perfluoroalkyl, vinyl, substituted vinyl, fluorine, nitro, acetylene, substituted acetylene, alkoxycarbonyl, or a nitrogen-containing aromatic heterocycle. Compounds of formula 5 are reacted with an alkoxymethylene malonate derivative of formula 8 in the presence of a suitable strong base, for example lithium diisopropylamide (LDA) or n-butyl lithium, preferably at a temperature below 0°C, and conveniently at -78°C to afford the compounds of formula 9A.

The compounds of formula 9A are cyclized in the presence of a base, as for example DBU or piperidine, or in the presence of an acid, such as sulfuric acid, in a solvent such as toluene, THF, ethanol or chlorobenzene, or by heating the compound in a solvent, as for example xylene, diglyme, triglyme, sulfolane or Dowtherm A® (a eutectic mixture of biphenyl and diphenyl ether) at a temperature greater than 120°C, to give the compounds of formula 10C. The esters 10C are convened into the esters 11A via transesterification with an alcohol suitable for selective hydrolysis, such as benzyl alcohol or 2-(trimethylsilyl)ethanol (TMSE), in the presence of a catalyst, as for example titanium tetraethoxide.

The 2-hydroxy compounds of formula 11A are converted to the corresponding halo-derivatives of formula 12A by treatment with a halogenating agent, for example phosphorous oxychloride to afford the chloro derivative, optionally in an inert solvent at a temperature between about 20°C and 145°C, depending on the halogenating agent and the boiling point of the solvent if one is used, and conveniently at room temperature. The leaving group L in the compounds of formula 12A is then displaced by a nucleophile such as a nucleophilic amine, for example N-methylpiperazine or 2-methylpiperazine, to give the compounds of formula 13A. The reaction may be conducted at a temperature from about 20°C to about 130°C in a suitable organic solvent such as pyridine, methylene chloride, chloroform or 1-methyl-2-pyrrolidinone. It is desirable to carry out the reaction in the presence of an acid-acceptor such as triethylamine, potassium carbonate and the like, at a molar ratio of 1.0 to 2.0 moles of the acid acceptor per mole of compound of the formula 6. The amine can also be used as an acid acceptor in which case two or more equivalents of this reagent are used.

The benzyl ester group of compounds of formula 13A is then removed by hydrogenolysis when R* is benzyl, or with tetrabutylammonium fluoride when R* is TMSE, to afford a compound of formula I.

In accordance with Scheme II above, the substituted acetonitrile compounds of formula 4B, where R¹ is an alkyl, cycloalkyl, halo(loweralkyl) group or a (loweralkyl)amino group protected with a protecting group such as benzyloxycarbonyl, or may be an electron withdrawing group as described above for Scheme I, are reacted with diethyl carbonate and sodium hydride in an inert organic solvent, such as toluene, THF or the like, to give the substituted cyanoacetic acid ester of formula 5B. The cyano group of the compounds of formula 5B is then reacted with an inorganic acid, such as hydrochloric acid, in the presence of one equivalent of anhydrous alcohol, such as ethanol, followed by reaction with ammonia to give the substituted amidine ester of formula 6B, which is then condensed with an enolate compound of formula 7B, prepared in a manner similar to compounds of formula 3 in Scheme I, in the presence of a suitable base, for example triethylamine, in a polar solvent such as methanol to give the substituted hydroxypyrimidine ester compounds of formula 8B. The ester function of the compounds of formula 8B is converted into an aldehyde function by reduction, for example with a hindered aluminum hydride, such as diisobutylaluminum hydride or LiAlH(O-*t*-butyl)₃, or with N,N-dimethylchloromethyleneiminium chloride in pyridine or diaminoaluminum hydride to produce a compound of formula 9B. This reaction may be conducted at a temperature below -20°C, and conveniently at -78°C in the presence of a aprotic solvent such as hexane, toluene, methylene chloride or THF.

The aldehyde compounds of formula 9B are reacted with a malonic acid diester, such as diethyl malonate, dibenzyl malonate, *t*-butyl malonate or di-*t*-butyl malonate, in the presence of a suitable base such as piperidine and a catalytic amount of an acid, such as acetic acid or sulfuric acid, in a polar solvent, such as ethanol, to afford the pyridopyrimidine compounds of formula 10B. The compounds of formula 10B are reacted with a suitable halogenating agent such as phosphoryl chloride at room temperature to afford the compounds of formula 11B. The halo group is displaced as discussed in reaction Scheme I to afford the compounds of formula 12B, which are in turn convened into the compounds of formula I as described in Scheme I for the conversion of compounds of formula 10 into compounds of formula I.

According to reference reaction Scheme III illustrated above, 2-picoline-N-oxide is converted to a mixture of compounds of formulae 22 and 23 by treatment with a halogenating agent, for example phosphorus oxychloride, optionally in an inert solvent. The reaction may be run at a temperature between about 25°C and 125°C, depending on the halogenating agent selected. When the halogenating agent is phosphorus oxychloride the reaction temperature is preferably between 60°C and 120°C. A compound of formula 23 is, in turn, reacted with an alkoxymethylene malonate derivative of formula 8 in the presence of a suitably strong and hindered base, for example lithium diisopropylamide (LDA), preferably at a temperature below 0°C, and conveniently at -78°C to afford the compounds of formula 24. Compounds of formula 24 are cyclized by heating the compound in a solvent with a boiling point greater than 120°C, for example xylene, diglyme, triglyme, sulfolane or Dowtherm A® (a eutectic mixture of biphenyl and diphenyl ether), to afford compounds of formula 25. The leaving group in the 8-position of the quinolizinone compound of formula 25 is then displaced using 3-aminopyrrolidine with the primary amino group protected, for example with t-butoxycarbonyl,. The protecting group is then removed to give the compounds of formula 26.

The esters of formula 26 are than converted to the carboxylic acids of formula III as described in Scheme 1 for the conversion of compounds of formula 10 to compounds of formula I.

Alternately, compounds of formula 23 are converted to compounds of formula 27, wherein R¹ is alkyl, cycloalkyl or carbocyclic aryl(loweralkyl), by treatment with an alkyl, cycloalkyl or carbocyclic aryl(loweralkyl) halide in the presence of a suitable base such as LDA. Compounds of formula 23 are converted to compounds of formula 27, wherein R¹ is a phenyl group as defined herein or an alkylamino group by conversion to the corresponding halomethyl compound and treatment of the halomethyl compound with an aryl metal compound such as phenyllithium as described above, or with an alkylamine such as methylamine as shown in reaction Scheme VA. The compounds of formula 27 are converted to the compounds of formula 29 by the sequence of reactions described above for the conversion of compounds of formula 25. The leaving group in the 8-position of the quinolizinone compound of formula 29 is then displaced, for example by a nucleophilic amine such as N-methylpiperazine or 2-methylpiperazine, to give the the compounds of formula 30. The reaction may be conducted at a temperature from about 20°C to about 130°C in a suitable organic solvent such as pyridine, methylene chloride, chloroform or 1-methyl-2-pyrrolidinone. It is desirable to carry out the reaction in the presence of an acid-acceptor such as triethylamine, potassium carbonate and the like, at a molar ratio of 1.0 to 2.0 moles of the acid acceptor per mole of compound of the formula29. The amine can also be used as an acid acceptor in which case two or more equivalents of this reagent are used.

In the case where R² is a phenyl group as defined herein, compounds of formula 30 are formed by coupling the compound of formula 29 with an aryl metal compound, for example phenyllithium, to replace the 8-leaving group with an unsubstituted phenyl group. The coupling reaction is carried in a reaction-inert solvent, i.e., a solvent which does not interfere with the coupling reaction of the aryl metal compound with a compound of formula29. Suitable reaction-inert solvents include ethers, for example diethyl ether, dimethoxyethane and tetrahydrofuran (THF). Co-solvents may be used with ethers if desired. These co-solvents may be benzene, toluene, tetramethylethyleneamine (TMEDA) and hexamethylphosphoramide (HMPA). The aryl metal compounds may be prepared by known methods. For example, they may be prepared by direct lithium-halogen exchange of the corresponding aryl halide using n-butyl-, sec-butyl- or t-butyl-lithium followed by transmetallation by a wide variety of salts by known methods such as described by E. Negishi in "Organometallics in Organic Sysnthesis", Vol. 1, page 104.

According to Scheme IV A illustrated above, a compound of formula 31 is treated with a malononic acid ester, for example diethyl malonate, in the presence of a suitable base such as sodium hydride in a polar nonprotic solvent such as an ether, for example diethyl ether or THF, to afford a compound of formula 32. Compounds of formula 32 are, in turn, decarboxylated, for example by heating them in strong mineral acid such as aqueous sulfuric acid, to afford the compounds of formula 33. The nitro-compound of formula 33 is reduced to the corresponding amino-compound of formula 34. The nitro group may be reduced by catalytic hydrogenation using standard techniques or by any of a variety of known reducing agents such as using a metal, for example zinc, tin or iron, in the presence of a mineral acid, usually hydrochloric acid. The amino-compound of formula 34 is converted to the corresponding fluoro-compound of formula 35 by treatment with ethyl nitrite and tetrafluoroboric acid, followed by treatment with potassium fluoride. The compound of formula 35 is then converted into the corresponding N-oxide of formula 36 by oxidation, for example using peracetic acid. The reaction is carried out in the range from about 20°C up to the reflux temperature of the solvent employed, preferably at about 50°C. The compound of formula 36 is nitrated to afford compounds of formula 37. The nitration reaction can be carried out using a variety of known nitrating agents, for example a mixture of nitric acid and sulfuric add or a mixture of sulfuric acid and potassium nitrate, or by using nitronium salts such as nitronium trifluoromethanesulfonate. The nitro compound of formula 37 is, in turn, converted to the corresponding halo compound of formula 38 by treatment with mineral acid at ambient or elevated temperature as desired. For example, the compound of formula 37 is treated with aqueous hydrochloric acid at a temperature of about 100-120°C to afford the compound of formula 38 wherein L is Cl. The compound of formula 38 is, in turn converted to the compound of formula IV A1 by reduction, for example using a metal such as iron or zinc in the presence of an acid such as acetic acid. The compound of formula IV A1 is, in turn, converted to the compound of formula IV A2 by treatment with a suitable base, such as LDA, followed by treatment with a halogenating agent, for example N-chloro or N-bromo succinimide. Alternately, the compounds of formula IV A1 are converted to compounds of formula IV A3, wherein R¹ is alkyl, cycloalkyl or carbocyclic aryl(loweralkyl), by treatment with an alkyl, cycloalkyl or carbocyclic aryl(loweralkyl) halide in the presence of a suitable base such as LDA. The compounds of formula IV A3 are further treated with a a suitable base, such as LDA, followed by treatment with a halogenating agent for example N-chloro or N-bromo succinimide to afford the compounds of formula IV A4. Compounds of formulae IV A1 - IV A4 are key intermediates used in the synthesis of quinolizinone compounds.

According to Schemes IV B (reference) and IV C illustrated above, the compounds of formulae IV A3 and IV A4 are converted to the quinolizinone compounds of formula IV B and IV C, respectively, by the following series of reactions: (1) reaction with an alkoxymethylene malonate derivative of formula 8 in the presence of a suitably strong and hindered base, for example lithium diisopropylamide (LDA), preferably at a temperature below 0°C, and conveniently at -78°C, to afford the compounds of formulae 39 and 42, respectively (2) cyclization as discussed in reaction Scheme III, to afford the compounds of formulae 40 and 43, respectively (3) displacement of the leaving group in the 8-position as discussed in reaction Scheme III to afford the compounds of formulae 41 and 44, respectively and (4) hydrolysis or hydrogenolysis as discussed in reaction Scheme III of the carboxylic acid ester to the corresponding carboxylic acids of formulae IV B and IV C, respectively.

According to Scheme V A illustrated above, compounds of formula IV A1 are treated with a halogenating agent under suitable conditions for generating halogen radicals, for example using N-bromo- or N-chlorosuccinimide in the presence of a free radical initiator such as AIBN to afford the compounds of formula 45. The halogen on the alpha carbon atom is then displaced by a nucleophile, for example an alkoxide to give the compounds of formula 51 or an amine to give the compounds of formula 46. The amine function is protected during synthesis by converting it to the corresponding formamidine function affording compounds of formula 47. Compounds of formula 47 are reacted with an alkoxymethylene malonate derivative of formula 8 in the presence of a suitably strong and hindered base, for example lithium diisopropylamide (LDA), preferably at a temperature below 0°C, and conveniently at -78°C. The formamidine group is then removed by reaction with hydrazine and acetic acid to afford the compounds of formula 48. The compounds of formula 48 are cyclized as discussed in reaction Scheme III, to afford the compounds of formula 49. The leaving group, L, is then displaced as discussed in reaction Scheme III to afford the compounds of formula 50. The compounds of formula 50 are, in turn, convened to the compounds of formula V A1 as discussed in reaction Scheme I.

The compounds of formula 51 are converted to the compounds of formula V A2 by the following series of reactions: (1) reaction with an alkoxymethylene malonate derivative of formula 8 in the presence of a suitably strong and hindered base, for example lithium diisopropylamide (LDA), preferably at a temperature below 0°C, and conveniently at -78°C, to afford the compounds of formula 52 (2) cyclization as discussed in reaction Scheme III, to afford the compounds of formula 53 (3) displacement of the leaving group in the 8-position as discussed in reaction Scheme III to afford the compounds of formula 54 and (4) conversion of the carboxylic acid ester to the corresponding carboxylic acids of formula V A2.

According to reaction Scheme V B illustrated above, compounds of formula IV A2 are converted to compounds of formulae V B1 and V B2 by the same procedures discussed in reaction Scheme V A for the conversion of compounds of formula IV A1 to compounds of formulae V A1 and V A2.

According to reaction Scheme VI illustrated above, perfluoroinated pyridine is converted to the compound of formula 66 by the procedures described in reaction Scheme IV A for the preparation of compounds of formula 33. Compounds of formula 66 are, in turn, convened to the compounds of formula VI A and VI B by the series of reactions discussed in reaction Scheme III for the conversion of compounds of formula 23 to compounds of formula III.

According to reaction Scheme VII illustrated above, compounds of formula IV A2 are reacted with a protected alcohol of formula 71, in the presence of a suitable base such as LDA, to afford compounds of formula 72. The hydroxy protecting group is preferably a THP (tetrahydopyranyl) ether group. The compounds of formula 72 are, in turn, deprotected by standard methods to afford the compounds of formula 73. The compounds of formula 73 are cyclized, in the presence of a suitable non-nucleophilic base such as sodium hydride, to afford the compounds of formula 74. The compounds of formula 74 are then comverted to the compounds of formula 77 by the series of reactions described in reaction Scheme IV B for the conversion of the compounds of formula IV A3 to the compounds of formula IV B.

Compounds of formula I, wherein R² contains a free primary amino group are synthesized according to reaction Scheme VIII illustrated above. In accordance with reaction Scheme VIII, an alpha-halo acetate derivative of formula 1, such as ethyl 2-fluoroacetate, is condensed with a formate ester of formula 2, in the presence of a suitable base, for example sodium ethoxide, in an inert solvent such as diethyl ether to give an enolate compound of formula 3. Compounds of formula 3 are, in turn, convened to compounds of formula 5 by condensation with an amidine derivative of formula 4, in the presence of a suitable base, for example triethylamine, in a polar solvent such as methanol. The hydroxy-substituted compounds of formula 5 are convened to the corresponding halo-derivatives of formula 6 by treatment with a halogenating agent, for example phosphorus oxychloride to afford the chloro derivative, optionally in an inert solvent at a temperature between about 20°C and 145°C, depending on the halogenating agent and the boiling point of the solvent if one is used. When phosphorus oxychloride is the halogenating agent, the reaction temperature is preferably between about 80°C and 100°C. The leaving group in the 5-position of the pyrimidine ring of compounds of formula 6 is then displaced by a nucleophile such as a nucleophilic amine, for example N-methylpiperazine or 2-methylpiperazine, to give the the compounds of formula 7. The reaction may be conducted at a temperature from about 20°C to about 130°C in a suitable organic solvent such as pyridine, methylene chloride, chloroform or 1-methyl-2-pyrrolidinone. It is desirable to carry out the reaction in the presence of an acid-acceptor such as triethylamine, potassium carbonate and the like, at a molar ratio of 1.0 to 2.0 moles of the acid acceptor per mole of compound of the formula 6. The amine can also be used as an acid acceptor in which case two or more equivalents of this reagent are used.

The compounds of formula 7 are reacted with an alkoxymethylene malonate derivative of formula 8 in the presence of a suitably strong hindered base, for example lithium diisopropylamide (LDA), preferably at a temperature below 0°C, and conveniently at -78°C to afford the compounds of formula 9. The compounds of formula 9 are cyclized in the presence of a suitable hindered base, for example DBU, in an aprotic solvent, such as toluene, THF or chlorobenzene to give the compounds of formula 10. The cyclization is carried out at a temperature in the range of about 30°C to about 130°C, preferably at the reflux temperture of the reaction mixture. The compounds of formula 10 are hydrolyzed in the presence of a suitable base such as sodium or potasium hydroxide to afford the compounds of formula 78. The compounds of formula 78 are, in turn, chlorinated to afford the compounds of formula 10a using an appropriate chlorinating agent such as phosphorus oxychloride. The leaving group in the 8-position of the quinolizinone compound of formula 10a is then displaced using a nucleophilic amine such as 3-aminopyrrolidine (with the primary amino group protected, for example with t-butoxycarbonyl). The protecting group is then removed to give the compounds of formula 10b. The esters of formula 10b are then converted to the carboxylic acids of formula I. The conversion may be achieved by conventional hydrolysis or by converting a compound of formula 10b to the corresponding ester, via transesterification with an alcohol suitable for selective hydrolysis, such as benzyl alcohol or 2-(trimethylsilyl)ethanol (TMSE), in the presence of a catalyst, for example titanium tetraethoxide, and then, in turn, removing the alcohol group by hydrogenolysis when R* is benzyl or tetrabutylammonium fluoride when R* is TMSE to afford a compound of formula I.

Compounds of formula I, where R⁵ is loweralkyl or halo(loweralkyl), are synthesized according to reaction Scheme IX. In accordance with reaction Scheme IX illustrated above, an alpha-halo acetate derivative of formula 1, such as ethyl 2-fluoroacetate, is condensed with a compound of formula 78 where X may be a halogen or alkanoyl and R⁵ may be loweralkyl or halo(loweralkyl), for example acetyl chloride or ethyl trifluoroacetate, in the presence of a suitable base, for example sodium methoxide or sodium ethoxide, and in a suitable solvent, such as methanol, ethanol or ether, to give an alpha-fluoro beta-keto ester compound of formula 79. Compounds of formula 79 are then reacted with amidine compounds of formula 4 or formula 6, in which R¹ is an alkyl, halo(loweralkyl) or cycloalkyl group, or may be an electron withdrawing group such as phenyl, trifluoromethyl, cyano, perfluoroalkyl, vinyl, substituted vinyl, fluorine, nitro, acetylene, substituted acetylene, alkoxycarbonyl, or a nitrogen-containing aromatic heterocycle, in the presence of a suitable base, such as sodium methoxide or sodium ethoxide, in the presence of a suitable solvent, such as methanol or ethanol, to give compounds of formulae 81 or 80, respectively. Compounds of formula 80 may be substituted for compounds of formula 8B in Scheme II and converted via the reactions in that Scheme, described above, into compounds of formula I. Compounds of formula 81 may be substituted for compounds of formula 5 in Scheme I and converted into compounds of formula I via the reactions of Scheme I described above. Alternatively, the compounds of formula 81 may be substituted for compounds of formula 5 in Scheme VIII and converted via the reactions in that scheme, described above, into compounds of formula I.

Compounds of formula I, where R² is loweralkyl, cycloalkyl, carbocyclic aryl(loweralkyl), cycloalkyl(loweralkyl), phenyl, nitrogen-containing aromatic heterocycle, or nitrogen-containing heterocycle are synthesized according to reaction Scheme X. In accordance with reaction Scheme X illustrated above, an organo-metallic derivative of formula 82, such as phenyl magnesium bromide, cyclopentyl magnesium bromide, or N-methylpiperidin-4-yl magnesium bromide is condensed with an alpha-haloacetate derivative of formula 83, where X may be a halogen or alkoxy group, such as ethyl 2-fluoroacetate or 2-fluoroacetyl chloride, in an anhydrous solvent, for example ether or THF, to produce the alpha-fluoro compounds of formula 84. Compounds of formula 84, may in turn be reacted with a formate ester of formula 2, in the presence of a suitable base, for example sodium ethoxide, in an inert solvent such as diethyl ether to give an enolate derivative of formula 85. The compounds of formula 85 are in turn converted to compounds of formula 86 or 87 by condensation with an amidine derivative of formula 4 or 6, in which R¹ is loweralkyl, halo(loweralkyl) or cycloalkyl, or is an electron withdrawing group such as phenyl, trifluoromethyl, cyano, perfluoroalkyl, vinyl, substituted vinyl, fluorine, nitro, acetylene, substituted acetylene, alkoxycarbonyl, or a nitrogen-containing aromatic heterocycle, in the presence of a suitable base, for example triethylamine, in a polar solvent such as methanol. Compounds of formula 87 may be substituted for compounds of formula 7 in Scheme VIII, and converted via the reactions in that scheme, described above, into compounds of formula I. Compounds of formula 86 may be substituted for compounds of formula 9B in Scheme II and, by reaction with a malonic acid diester as described for Scheme II above, converted directly into compounds of formula 12B and, thence, into compounds of formula I.

Alternatively, compounds of formula I, where R² is loweralkyl, cycloalkyl, carbocyclic aryl(loweralkyl), cycloalkyl(loweralkyl), phenyl, nitrogen-containing aromatic heterocycle, or nitrogen-containing heterocycle are synthesized according to reaction Scheme XI. An alpha-haloacetate derivative of formula 1 is condensed with an acid halide or ester derivative of formula 88, for example acetyl chloride, benzoyl chloride, isonicotinoyl chloride, or 2,6-dimethylisonicotinoyl chloride, in an anhydrous solvent, for example ether, THF, anhydrous methanol or an hydrous ethanol, in the presence of a suitable base, such as sodium methoxide or NaN(TMS)2, to produce the beta-ketoester derivative of formula 91, which is converted into compounds of formula 92 in the presence of a suitable base, such as sodium methoxide or sodium ethoxide, in the presence of a suitable solvent, such as methanol, ethanol or ether, to give the hydroxy-substituted compounds of formulae 92 or 93. These compounds, in turn, are converted into the corresponding halo- derivatives of formulae 94 and 95 under conditions as described for conversion of compounds of formula 5 to compounds of formula 6 in Scheme VIII. The compounds of formulae 94 and 95 are then reacted with reducing agents such as zinc in acetic acid or hydrogen in the presence of catalytic agents such as Ni, Pd, or Pt in suitable solvents such as ethanol or methanol to produce the compounds of formula 86 and 87, which are converted as described in Scheme X into compounds of formula I.

In addition, the non-fluorinated derivatives of formula 90, where R2 is as described above, may be converted to the beta-ketoester derivatives of formula 91 using a reagent such as N-fluoropyridinium triflate, N-fluorosulfonyl amide, cesium fluorooxysulfate, or acetyl hypofluoride.

The foregoing may be better understood from the following examples, which are presented for the purpose of illustration and are not intended as a limitation upon the scope of the invention.

### Example 1

### 3-Fluoro-9-(4-fluorophenyl)-2-(4-methylpiperazin-1-yl)-6H-6-oxo-pyrido[1,2-a]pyrimidine-7-carboxylic acid

### Step 1; 5-Fluoro-2-(4-fluorobenzyl)-4-hydroxypyrimidine

Sodium hydride (4.36 g of 60% NaH in mineral oil, 107.6 mmol) was suspended, under a nitrogen atmosphere, in 125 mL of anhydrous diethyl ether in a 500 mL round-bottom flask fitted with a mechanical stirrer, a thermometer and a condenser. To this mixture, with vigorous stirring, was slowly added 6.28 mL (107.6 mmol) of anhydrous ethyl alcohol. After the evolution of gas ceased, a mixture of ethyl 2-fluoroacetate (10 mL, 102.5 mmol) and ethyl formate (12.5 mL, 153.7 mmol) was added, dropwise, to the ethoxide solution. The reaction mixture was cooled when necessary in order to maintain the reaction temperature between 18°C and 20°C. The reaction mixture was stirred, under a nitrogen atmosphere, at 18-20°C for 4.75 h. The solvent was removed under aspirator pressure, fresh anhydrous diethyl ether was added to the residue and the ether solution was concentrated under reduced pressure to afford, as a solid residue, the sodium enolate of ethyl 2-fluoro-3-oxo-2-propanecarboxylate, as described by E.Elkik and M. Imbeaux-Oudotte in Bull Soc Chim, 1165-1169, 1975. To this residue was added 20.3 g (107.6 mmol) of 4-fluorobenzylamidine hydrochloride, followed by 250 mL of methanol and 28.8 mL (205 mmol) of triethylamine (TEA). The reaction mixture was heated, with stirring, at reflux temperature for 16 h and then concentrated *in vacuo*. The residue was triturated with hexane and the hexane was decanted. Water was added to the residue and the aqueous mixture was acidified with glacial acetic acid and extracted with 4 X 150 mL of methylene chloride. The combined organic extract was washed with 200 mL of water and concentrated *in vacuo*. The residue was recrystallized twice from ethyl acetate containing Norite® charcoal to afford the title compound, m.p. 169-170°C; MS DCl-NH₃ M/Z: 223 (M+H)+; ¹H NMR (DMSO-d6) δ 3.87 (s, 2H), 7.14 (m, 2H), 7.33 (m, 2H), 7.98 (d, 1H). Analysis calculated for C₁₁H₈F₂N₂O: C, 59.46; H, 3.63; N, 12.61. Found: C, 59.08; H, 3.70; N, 12.57.

### Step 2: 4-Chloro-5-fluoro-2-(4-fluorobenzyl)-pyrimidine

A mixture of 1.93 g (8.7 mmol) of 5-fluoro-2-(4-fluorobenzyl)-4-hydroxypyrimidine, from Step 1, and 15 mL of phosphorus oxychloride was heated in an oil bath at 90°C for 1.5 h and then concentrated *in vacuo*. The residue was triturated with 75 mL of ice water and the aqueous mixture was adjusted to pH 8 - 9 by the addition of solid sodium bicarbonate. The mixture was extracted with 3 X 70 mL of methylene chloride. The combined organic extracts were dried over anhydrous magnesium sulfate, filtered and concentrated *in vacuo* to a light brown residue. The residue was purified by flash chromatography on a 230-400 mesh silica gel column (4.8 X 14.6 cm) eluted with hexane:methylene chloride (1:1 v/v) to afford 1.94 g (90% yield) of the title compound; MS DCl-NH₃ M/Z: 241 (M+H)+; ¹H NMR (CDCl₃) δ 4.22 (s, 2H), 7.00 (m, 2H), 7.30 (m, 2H), 8.48 (s, 1H).

### Step 3: 5-Fluoro-2-(4-fluorobenzyl)-4-(4-methylpiperazin-1-yl)-pyrimidine

A mixture of 0.48 g (2 mmol) of 4-chloro-5-fluoro-2-(4-fluorobenzyl)-pyrimidine from Step 2 and 1.53 mL (14 mmol) of 4-methylpiperazine in 10 mL of methylene chloride was stirred at ambient temperature for 1.5 h. The reaction mixture was concentrated *in vacuo* and the residue was dissolved in methylene chloride. The resultant solution was washed with 4 X 30 mL of water, dried over anhydrous magnesium sulfate, filtered and concentrated *in vacuo* to give 0.59 g (95% yield) of the title compound as an oil; ¹H NMR (CDCl₃) δ 2.32 (s, 3H), 2.47 (t, 4H), 3.78 (t, 4H), 3.99 (s, 2H), 6.97 (m, 2H), 7.29 (m, 2H), 7.97 (d, 1H). The product was carried on to the next step without purification.

### Step 4: Diethyl 2-ethoxy-3-(4-fluorophenyl)-3-[5-fluoro-4-(4-methypiperazin-1-yl)pyrimidin-2-yl]-propane-1,1-dicarboxylate

A solution of 0.35 mL (2.5 mmol) of diisopropylamine in 5 mL of anhydrous tetrahydrofuran (THF) was prepared under a nitrogen atmosphere and cooled in an ice/water bath. To this solution was added via syringe, 1.0 ml of a 2.5 M solution of n-butyllithium (2.5 mmol) in hexane. The solution was stirred for 15 minutes at 0°C and then cooled to -78°C. To the mixture at -78°C, was added a solution of 0.7 g (2.3 mmol) of 5-fluoro-2-(4-fluorobenzyl)-4-(4-methylpiperazin-1-yl)-pyrimidine, from Step 3, in 5 mL of anhydrous THF and a dark red-colored solution was formed. The solution was stirred at -78°C for 1 h and then 0.46 mL (2.3 mmol) of ethyl 2-carboethoxy-3-ethoxy-2-propenecarboxylate was added. Stirring was continued at -78°C for 3 h and the reaction mixture turned a tight yellow color. The reaction mixture was poured into 30 mL of water, with 6 g of solid ammonium chloride. The aqueous mixture was extracted with 4 X 50 mL of methylene chloride. The combined organic extract was dried over magnesium sulfate, filtered and concentrated *in vacuo*. The residue was dissolved in 300 mL of methylene chloride. The resultant solution was washed with a 50 mL portion of water, followed by a 75 mL portion of water, dried over anhydrous magnesium sulfate, filtered and concentrated *in vacuo* to afford the title compound; MS DCl-NH₃ M/Z: 521 (M+H)⁺; ¹H NMR (CDCl₃) δ 0.84 (2 X t, 3H), 1.18 (t, 3H), 1.28 (t, 3H), 2.33 (s, 3H), 2.50 (m, 4H), 3.36-3.53 (m, 2H), 3.83 (s, 4H), 3.96-4.22 (m, 4H), 4.42 (t, 1H), 4.98 (dd, 1H), 6.95 (m, 2H), 7.48 (m, 2H), 7.99 (d, 1H).

### Step 5: Ethyl 3-fluoro-9-(4-fluorophenyl)-2-(4-methylpiperazin-1-yl)-6H-6-oxo-pyrido[1,2-a]pyrimidine-7-carboxylate

A solution of 0.57 g (1.1 mmol) of diethyl 2-ethoxy-3-(4-fluorophenyl)-3-[5-fluoro-4-(4-methypiperazin-1-ylpyrimidin-2-yl]-propane-1,1-dicarboxylate, from Step 4, and 0.2 mL of 1,8-diazabicyclo[5.4.0]undec-7-ene (DBU) in 200 mL of toluene was heated at reflux temperature, with stirring, for 20.5 h. During the first 0.5 h, 125 mL of toluene was removed via Dean Stark trap and 100 mL of fresh toluene was added through a dropping funnel. Water (75 mL) was added to the reaction mixture and stirring was continued at ambient temperature for 3 h. The organic layer was separated and washed with 75 mL of water. The combined aqueous layers were extracted with 3 X 75 mL of toluene. The organic layers were all combined, dried over anhydrous magnesium sulfate, filtered and concentrated *in vacuo*. The residue (0.32 g) was purified on a 70-230 mesh silica gel column (2.4 X 43 cm) eluted with ethyl alcohol:chloroform (1:10 v/v) to afford 0.26 g (56% yield) of the title compound, m.p. 202-204°C; MS DCl-NH₃ M/Z: 429 (M+H)⁺; ¹H NMR (CDCl₃) δ 1.40 (t, 3H), 2.33 (s, 3H), 2.51 (m, 4H), 3.93 (m, 4H), 4.40 (q, 2H), 7.08 (t, 2H), 7.50 (m, 2H), 8.43 (s, 1H), 9.20 (d, 1H).

### Step 6: Benzyl 3-fluoro-9-(4-fluorophenyl)-2-(4-methylpiperazin-1-yl)-6H-6-oxo-pyrido[1,2-a]pyrimidine-7-carboxylate

A mixture of 0.11 g (0.26 mmol) of ethyl 3-fluoro-9-(4-fluorophenyl)-2-(4-methylpiperazin-1-yl)-6H-6-oxo-pyrido[1,2-a]pyrimidine-7-carboxylate, from Step 5, 50 mL of dry benzyl alcohol and 0.05 mL of titanium tetraethoxide was heated, with stirring, at 100°C for 22 h. The benzyl alcohol was removed by distillation under reduced pressure and the residue was dissolved in 75 mL of methylene chloride. To this solution was added 5 mL of saturated aqueous lithium fluoride solution and the resultant mixture was stirred at ambient temperature for 20 minutes. The layers were separated and the organic layer was diluted with 75 mL of methylene chloride and washed with 20 mL of water. The aqueous layer was extracted with 25 mL of methylene chloride and the methylene chloride layer from this extraction was combined with the organic layer. The combined organic layers were dried over anhydrous magnesium sulfate, filtered and concentrated. The residue (0.18 g) was chromatographed on a 70-230 mesh silica gel column (1.8 X 34 cm) eluted with ethanol:chloroform (1:13 v/v) to afford 87 mg (67% yield) of the title compound; ¹H NMR (CDCl₃) δ 2.33 (s, 3H), 2.52 (m, 4H), 3.94 (m, 4H), 5.40 (s, 2H), 7.08 (s, 2H), 7.27 (m, 5H), 8.44 (s, 1H), 9.21 (d, 1H). The product was carried on to the next step without further purification.

### Step 7: 3-Fluoro-9-(4-fluorophenyl)-2-(4-methylpiperazin-1-yl)-6H-6-oxo-pyrido[1,2-a]pyrimidine-7-carboxylic acid

Benzyl 3-fluoro-9-(4-fluorophenyl)-2-(4-methylpiperazin-1-yl)-6H-6-oxo-pyrido[1,2-a]pyrimidine-7-carboxylate (87 mg, 0.177 mmol), from Step 6, was dissolved in 20 mL of ethyl acetate. To this solution was added 20 mg of 10% palladium on carbon and the resultant mixture was hydrogenated at ambient temperature, under 4 atmospheres of hydrogen, for approximately 19 h. The catalyst was removed by filtration and washed with 400 mL of ethyl acetate The filtrate was concentrated *in vacuo* to give 65.2 mg of solid. The solid was purified by chromatography on a 70-230 mesh silica gel column (1.8 X 18.5 cm) eluted with chloroform:methanol:acetic acid:water (100:25:5:2.5 v/v/v/v). The fractions containing the desired product were combined and concentrated. Toluene was added to the residue and evaporated *in vacuo*. Chloroform was then added to the residue and evaporated *in vacuo* to afford the title compound as a yellow solid, m.p. 225-230°C; MS DCl-NH₃ M/Z: 401 (M+H)⁺; ¹H NMR (CDCl₃) δ 1.68 (brs, 1H), 2.33 (s, 3H), 2.53 (brs, 4H), 3.98 (brs, 4H), 7.10 (t, 2H), 7.48 (m, 2H), 8.57 (s, 1H), 9.08 (d, 2H). Analysis calculated for C₂₀H₁₈F₂N₄O₃+0.75H₂O: C,58.03; H, 4.75; N, 13.54. Found: C, 57.98; H, 4.32; N, 13.22.

### Example 2

### 3-Fluoro-9-(4-fluorophenyl)-2-(4-methylpiperazin-1-yl)-6H-6-oxo-pyrido[1,2-a]pyrimidine-7-carboxylic acid

### Step 1: Ethyl 3-fluoro-9-(4-fluorophenyl)-2-hydroxy-6H-6-oxo-pyrido[1,2-a]pyrimidine-7-carboxylate

To a stirred solution of 0.87 g (2.05 mmol) of ethyl 3-fluoro-9-(4-fluorophenyl)-2-(4-methylpiperazin-1-yl)-6H-6-oxo-pyrido[1,2-a]pyrimidine-7-carboxylate, the product of Step 5 of Example 1, in 54 mL of THF/water (1:1) was added 6 mL of 1 N aqueous sodium hydroxide solution. The reaction mixture was stirred at ambient temperature for 6 h and then was allowed to stand overnight at ambient temperature. The solid was filtered and dried to give the title compound; ¹H NMR (d₆-DMSO) δ 1.23 (t, 3H). 4.15 (q, 2H), 7.17 (m, 2H), 7.52 (m, 2H), 7.91 (s, 1H), 8.77 (d, 1H).

### Step 2: Ethyl 2-chloro-3-fluoro-9-(4-fluorophenyl)-6H-6-oxo-pyrido[1,2-a]pyrimidine-7-carboxylate

A mixture of 55.7 mg of ethyl 3-fluoro-9-(4-fluorophenyl)-2-hydroxy-6H-6-oxo-pyrido[1,2-a]pyrimidine-7-carboxylate from Step 1 and 0.5 mL of phosphorus oxychloride was stirred and heated at 90°C for 1.25 h. The mixture was evaporated under reduced pressure to yield the title compound which can be reacted with amines without purification. A pure sample of the title compound is obtained by treatment of the crude product with aqueous sodium bicarbonate solution and extracting the aqueous mixture with methylene chloride. The organic solution is concentrated and chromatographed on silica gel eluting with ethyl acetate.

### Step 3: Ethyl 3-fluoro-9-(4-fluorophenyl)-2-(4-methylpiperazin-1-yl)-6H-6-oxo-pyrido[1,2-a]pyrimidine-7-carboxylate

Following the procedures described in Step 3 of Example 1, ethyl 2-chloro-3-fluoro-9-(4-fluorophenyl)-6H-6-oxo-pyrido[1,2-a]pyrimidine-7-carboxylate from Step 2 is reacted with 4-methylpiperazine to afford the title compound.

### Step 4: Benzyl 3-fluoro-9-(4-fluorophenyl)-2-(4-methylpiperazin-1-yl)-6H-6-oxo-pyrido[1,2-a]pyrimidine-7-carboxylate

A mixture of 0.11 g (0.26 mmol) of ethyl 3-fluoro-9-(4-fluorophenyl)-2-(4-methylpiperazin-1-yl)-6H-6-oxo-pyrido[1,2-a]pyrimidine-7-carboxylate, from Step 3, 50 mL of dry benzyl alcohol and 0.05 mL of titanium tetraethoxide was heated, with stirring, at 100°C for 22 h. The benzyl alcohol was removed by distillation under reduced pressure and the residue was dissolved in 75 mL of methylene chloride. To this solution was added 5 mL of saturated aqueous lithium fluoride solution and the resultant mixture was stirred at ambient temperature for 20 minutes. The layers were separated and the organic layer was diluted with 75 mL of methylene chloride and washed with 20 mL of water. The aqueous layer was extracted with 25 mL of methylene chloride and the methylene chloride layer from this extraction was combined with the organic layer. The combined organic layers were dried over anhydrous magnesium sulfate, filtered and concentrated. The residue (0.18 g) was chromatographed on a 70-230 mesh silica gel column (1.8 X 34 cm) eluted with ethanol:chloroform (1:13 v/v) to afford 87 mg (67% yield) of the title compound; ¹H NMR (CDCl₃) δ 2.33 (s, 3H), 2.52 (m, 4H), 3.94 (m, 4H), 5.40 (s, 2H), 7.08 (s, 2H), 7.27 (m, 5H), 8.44 (s, 1H), 9.21 (d, 1H). The product was carried on to the next step without further purification.

### Step 5: 3-Fluoro-9-(4-fluorophenyl)-2-(4-methylpiperazin-1-yl)-6H-6-oxo-pyrido[1,2-a]pyrimidine-7-carboxylic acid

Benzyl 3-fluoro-9-(4-fluorophenyl)-2-(4-methylpiperazin-1-yl)-6H-6-oxo-pyrido[1,2-a]pyrimidine-7-carboxylate (87 mg, 0.177 mmol), from Step 4, was dissolved in 20 mL of ethyl acetate. To this solution was added 20 mg of 10% palladium on carbon and the resultant mixture was hydrogenated at ambient temperature, under 4 atmospheres of hydrogen, for approximately 19 hours. The catalyst was removed by filtration and washed with 400 mL of ethyl acetate The filtrate was concentrated *in vacuo* to give 65.2 mg of solid. The solid was purified by chromatography on a 70-230 mesh silica gel column (1.8 X 18.5 cm) eluted with chloroform:methanol:acetic acid:water (100:25:5:2.5 v/v/v/v). The fractions containing the desired product were combined and concentrated. Toluene was added to the residue and evaporated *in vacuo*. Chloroform was then added to the residue and evaporate*d in vacuo* to afford the title compound as a yellow solid, m.p. 225-230°C; MS DCl-NH₃ M/Z: 401 (M+H)⁺; ¹H NMR (CDCl₃) δ 1.68 (brs, 1H), 2.33 (s, 3H), 2.53 (brs, 4H), 3.98 (brs, 4H), 7.10 (t, 2H), 7.48 (m, 2H), 8.57 (s, 1H), 9.08 (d, 2H). Analysis calculated for C₂₀H₁₈F₂N₄O₃+0.75H₂O: C, 58.03; H, 4.75; N, 13.54. Found: C, 57.98; H, 4.32; N, 13.22.

### Examples 3-38

By following the procedures described in Example 2 and using the appropriate amine, Examples 3-20, as disclosed in Table 1, may be prepared which have the general formula

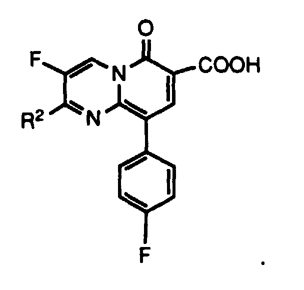

Likewise, Examples 21-38, as also disclosed in Table 1, may be prepared by using the appropriate amine and 2,4-difluorobenzylamidine instead of 4-fluorobenzylamidine to produce the general formula

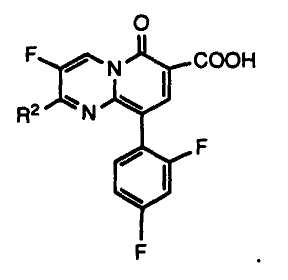

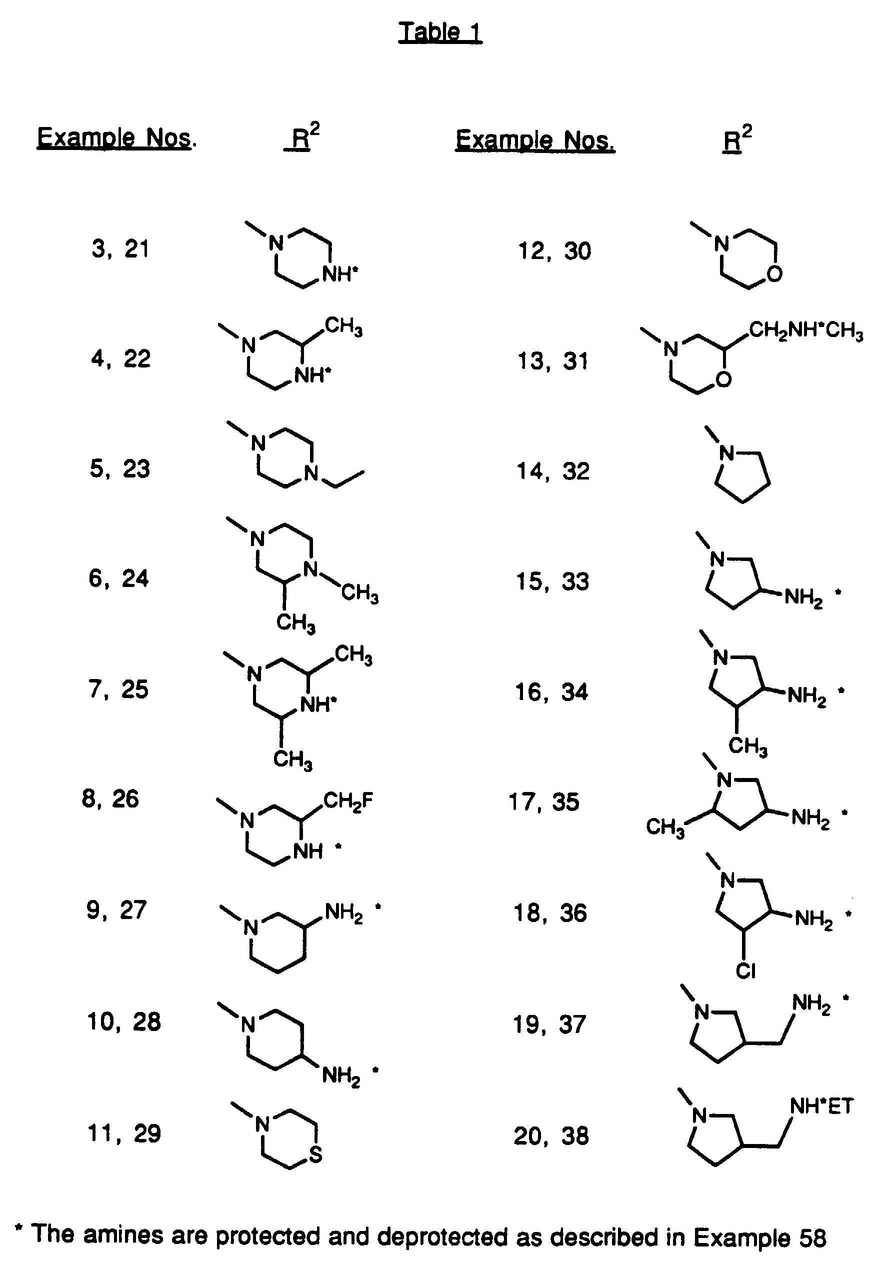

### Example 39

### 9-Cyclopropyl-3-fluoro-2-(4-methylpiperazin-1-yl)-6H-6-oxo-pyrido[1,2-a]pyrimidine-7-carboxylic acid

### Step 1: 2-Cyclopropyl-3-hydroxyacrylic acid

A 1.1 M solution of diethylzinc (350 mL) in an oven-dried system under positive nitrogen atmosphere is coled in an ice bath. Vinyl acetic acid (17 mL, 200 mmol) is added dropwise with stirring, followed by 24 mL (300 mmol) of diiodomethane. The reaction mixture is stirred overnight at ambient temperature. The reaction mixture is then cautiously poured into 500 mL of 1 N aqueous hydrochloric acid solution and the aqueous mixture is extracted with diethyl ether. The organic layer is dried over anhydrous sodium sulfate, filtered and concentrated. The residue is vacuum distilled to give cyclopropylacetic acid.

The cyclopropylacetic acid (15 g, 150 mmol) in a flask protected from moisture is cooled in an ice bath and 13.2 mL (180 mmol) of thionyl chloride is added dropwise with stirring. After the addition is complete, the reaction mixture is warmed to ambient temperature and then to 50°C. The reaction mixture is heated at 50°C for 1 h and then cooled in an ice bath. Absolute ethanol (26 mL, 450 mmol) is added dropwise with stirring to the reaction mixture. After the addition is complete, the reaction mixture is stirred at ambient temperature overnight. The reaction mixture is diluted with 500 mL of methylene chloride and then washed with 200 mL of 5% aqueous sodium bicarbonate solution. The organic layer is dried over anhydrous sodium sulfate, filtered and the ethyl ester of cyclopropylacetic acid is obtained by distillation.

2-Cyclopropyl-3-hydroxyacrylic acid (12.8 g, 100 mmol), from Step 1, is dissolved in 150 mL of dry dimethoxyethane in an oven-dried system under positive nitrogen atmosphere. The resultant solution is cooled in an ice bath and 4.4 g of 60% sodium hydride in mineral oil is added. The mixture is stirred for several h at approximately 0°C and then for several hours at ambient temperature. The reaction mixture is cooled in an ice bath and 8.9 mL (110 mmol) of ethyl formate in 90 mL of dry dimethoxyethane is added dropwise with stirring. After the addition is complete, the reaction mixture is stirred overnight at ambient temperature. The reaction mixture is then cautiously poured into 300 mL of saturated aqueous ammonium chloride solution and extracted with ethyl acetate. The ethyl acetate solution is dried over anhydrous sodium sulfate, filtered and concentrated *in vacuo* to afford the title compound.

### Step 2: Ethyl 5-Cyclopropyl-2,6-dihydroxy-nicotinic acid

A solution of 11.5 (88 mmol) of monoethyl malonate monoamide in 25 mL of dry THF is cooled in an ice bath and is treated with 10.7 g (95 mmol) of potassium t-butoxide. The reaction mixture is stirred at 0-5°C for 1 h. A solution of 12.5 g (80 mmol) of 2-cyclopropyl-3-hydroxyacryllic acid, from Step 1, in 20 mL of dry THF is added dropwise with stirring. The reaction mixture is then warmed to ambient temperature and then heated at reflux overnight. The reaction mixture is poured into brine and is extracted with ethyl acetate. The organic layer is dried over anhydrous sodium sulfate, filtered and concentrated *in vacuo* to afford the title compound.

### Step 3: Ethyl 5-Cyclopropyl-2,6-dichloro-nicotinic acid

Ethyl 5-Cyclopropyl-2,6-dihydroxy-nicotinic acid (15.6 g , 70 mmol) from Step 2, 1,2-dichloroethane (25 mL), anhydrous DMF (2 mL) and phosphoryl chloride (14.3 mL, 150 mmol) are combined in a system under positive nitrogen atmosphere, The reaction mixture is stirred at ambient temperature for 24 h then diluted with 1,2-dichloroethane. The reaction mixture is then washed with 5% aqueous sodium bicarbonate solution and brine. The organic layer is dried over anhydrous sodium sulfate, filtered and concentrated *in vacuo* to afford the this compound.

### Step 4: 2-Chloro-5-cyclopropyl-6-N-((4,5dimethoxy-2-nitrophenyl)methoxycarbonyl)amino-nicotinic acid

Ethyl 5-Cyclopropyl-2,6-dichloro-nicotinic acid (11.2 g, 50 mmol) from Step 3 is dissolved in 15 mL of anhydrous DMF. To this solution is added 25 mL of concentrated ammonium hydroxide and the reaction mixture is heated at reflux overnight. The reaction mixture is cooled to ambient temperature, diluted with water and extracted with 1,2-dichloroethane. The organic layer is dried over anhydrous sodium sulfate, filtered and concentrated *in vacuo*. The residue is dissolved in 250 mL of 1,2-dichloroethane and 200 mL of 10% aqueous sodium carbonate solution. The reaction mixture is cooled in an ice bath and 16.5 g (60 mmol) of 3,4-dimethoxy-6-nitrobenzylchloroformate is added. The reaction mixture is stirred at 0-5°C for 1 h. The layers are separated and the aqueous layer is extracted with 1,2-dichloroethane. The combined organic layers are dried over anhydrous sodium sulfate, filtered and concentrated *in vacuo*.

### Step 5: 2-Chloro-5-cyclopropyl-6- N-((4,5dimethoxy-2-nitro-phenyl)methoxycarbonyl)-N-(2-fluoroacetyl)amino-nicotinic acid

2-Chloro-5-cyclopropyl-6- N-((4,5dimethoxy-2-nitrophenyl)methoxycarbonyl)amino-nicotinic acid (14.4 g, 30 mmol) from Step 4 is dissolved in 20 mL of dry THF in an oven-dried system under positive nitrogen atmosphere. The reaction mixture is cooled in an ice bath and 1.3 g of 60% sodium hydride in mineral oil is added. The reaction mixture is stired at 0-5°C for 1 h. and 3.2 g (33 mmol) of α-fluoroacetyl chloride in 5 mL of dry THF is added dropwise with stirring. After the addition is complete, the reaction mixture is slowly warmed to ambient temperature and stirred overnight at ambient temperature. The reaction mixture is then poured into brine and extracted with ethyl acetate. The ethyl acetate solution is dried over anhydrous sodium sulfate, filtered and concentrated *in vacuo*. to afford the title compound.

### Step 6: 2-Chloro-5-cyclopropyl-6- N-((4,5dimethoxy-2-nitrophenyl)methoxycarbonyl)-N-(2-fluoro-3-hydroxy-1-oxo-1-prop-2-enyl)amino-nicotinic acid

Sodium hydride ()880 mg of 60% NaH in mineral oil) is suspended in 10 mL of dry THF. The suspension is cooled in an ice bath and 10.7 g (20 mmol) of 2-chloro-5-cyclopropyl-6- N-((4,5dimethoxy-2-nitro-phenyl)methoxycarbonyl)-N-(2-fluoroacetyl)amino-nicotinic acid, from Step 5, in 150 mL of dry THF is added dropwise with stirring. After the addition is complete, the reaction mixture is stirred at 0-5°C for 1 h. Ethyl formate (1.78 mL, 22 mmol) in 25 mL of dry THF is added dropwise with stirring. After the addition is complete, the reaction is stirred overnight at ambient temperature and then poured into 10% aqueous ammonium chloride solution. The aqueous mixture is extracted with ethyl acetate. The organic layer is dried over anhydrous sodium sulfate, filtered and concentrated *in vacuo* to afford the title compound.

### Step 7: Ethyl 9-cyclopropyl-1-((4,5dimethoxy-2-nitro-phenyl)methoxycarbonyl)3-fluoro-2-hydroxy-6H-6-oxo-pyrido[1,2-a]pyrimidine-7-carboxylate

A solution of 8.5 g (15 mmol) of 2-Chroro-5-cyclopropyl-6- N-((4,5dimethoxy-2-nitro-phenyl)methoxycarbonyl)-N-(2-fluoro-3-hydroxy-1-oxo-1-prop-2- enyl)amino-nicotinic acid, from Step 6, is dissolved in 200 mL of dioxane/water (1:1). To this solution is added 4.1 g (30 mmol) of potassium carbonate. The reaction mixture is heated at reflux with stirring overnight and then cooled to ambient temperature. The reaction mixture is then diluted with water and extracted with ethyl acetate. The organic layer is dried over anhydrous sodium sulfate, filtered and concentrated *in vacuo* to afford the title compound.

### Step 8: Ethyl 9-cyclopropyl-3-fluoro-2-chloro-6H-6-oxo-pyrido[1,2-a]pyrimidine-7-carboxylate

Ethyl 9-cyclopropyl-1-((4,5dimethoxy-2-nitro-phenyl) methoxy-carbonyl)3-fluoro-2-hydroxy-6H-6-oxo-pyrido[1,2-a]pyrimidine-7-carboxylate (5.3 g, 10 mmol) from Step 7 is dissolved in 75 mL of 2:1 dioxane:water and the resultant solution is illuminated with 320 nm light for 30 min. The reaction mixture is extracted with ethyl acetate. The organic layer is dried over anhydrous sodium sulfate, filtered and concentrated *in vacuo*. The residue is purified by silica gel chromatography to afford the product of Step 7 with the nitrogen protecting group removed. This product is dissolved in 1,2-dichloroethane and tretaed with phosphorous oxychloride at ambient temperature for 18 h. The reaction mixture is diluted with 1,2-dichloroethane and is washed with saturated aqueous sodium bicarbonate solution and brine. The organic layer is dried over anhydrous sodium sulfate, filtered and concentrated *in vacuo* to afford crude title compound which is purified by recrystallization from ethyl alcohol.

### Step 9: Ethyl 9-cyclopropyl-3-fluoro-2-(4-methylpiperazin-1-yl)-6H-6-oxo-pyrido[1,2-a]pyrimidine-7-carboxylic acid

Following the procedures described in Step 3 of Example 1, ethyl 9-cyclopropyl-3-fluoro-2-chloro-6H-6-oxo-pyrido[1,2-a]pyrimidine-7-carboxylate from Step 8 is reacted with 4-methylpiperazine to afford the title compound.

### Step 10: 9-Cyclopropyl-3-fluoro-2-(4-methylpiperazin-1-yl)-6H-6-oxo-pyrido[1,2-a]pyrimidine-7-carboxylic acid

Following the procedures described in Steps 5 - 7 of Example 1, Ethyl 9-cyclopropyl-3-fluoro-2-(4-methylpiperazin-1-yl)-6H-6-oxo-pyrido[1,2-a]pyrimidine-7-carboxylic acid is converted to the title compound.

### Examples 40-57

By following the procedures described in Example 39 and replacing 4-methylpiperazine in Step 4 with the appropriate amine, Examples 40 - 57 may be prepared as disclosed in Table 2 wherein the compounds have the general formula

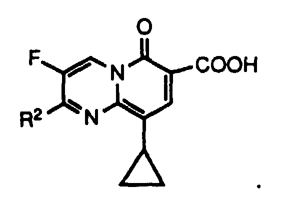

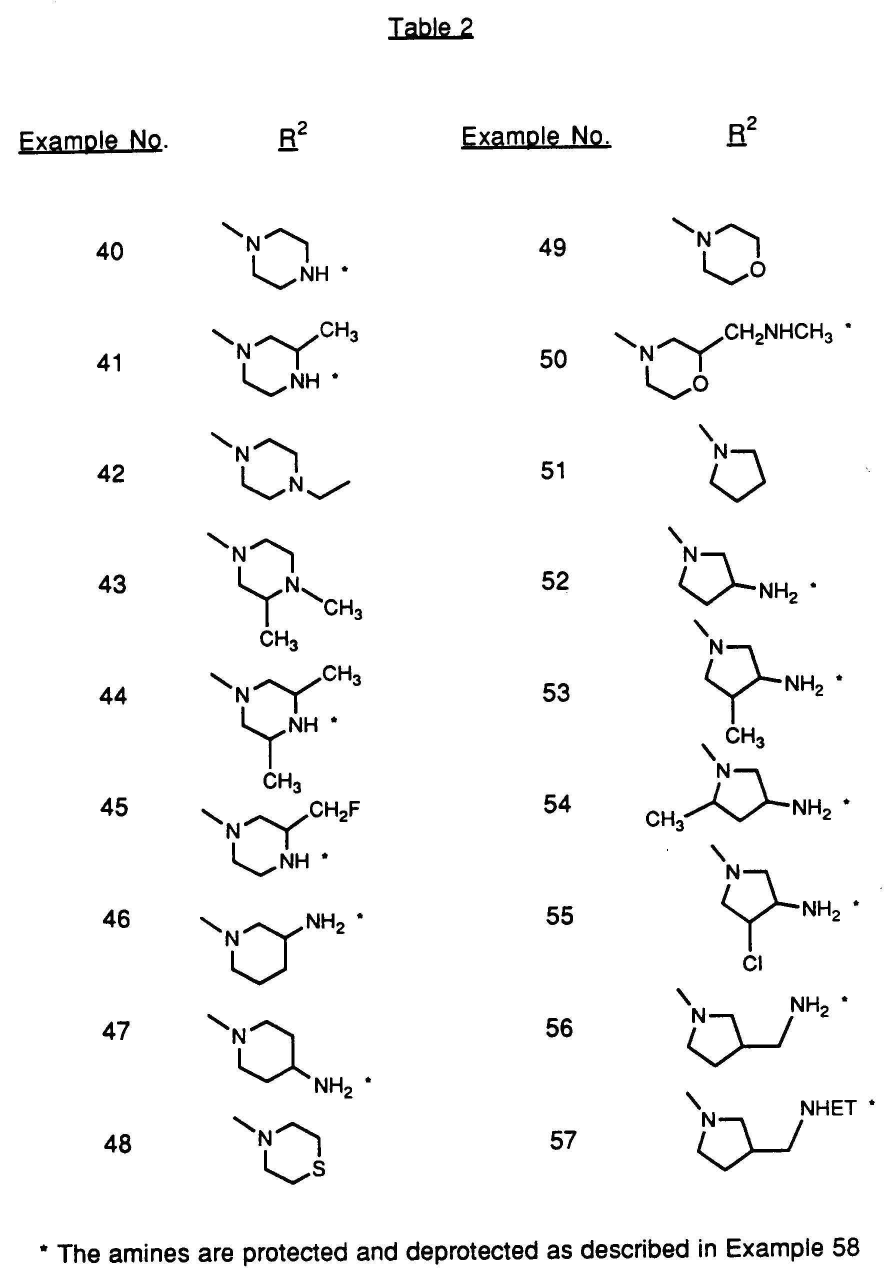

### Example 58 (reference)

### 8-(3-Amino-1-pyrrolidinyl)-4H-quinolizin-4-one-3-carboxylic acid hydrochloride

### Step 1: 4-Chloro-2-picoline

To 34.5 mL (0.37 mol) of phosphorus oxychloride, under a nitrogen atmosphere, was added 20.0 g (0.19 mol) of 2-picoline-N-oxide (commercially available from Aldrich Chemical Company) in small portions. The reaction temperature slowly increased during the addition to ∼60°C. After the addition was complete, the reaction mixture was a homogeneous dark red solution and the reaction temperature was 80°C. This solution was heated at 120°C for 1.5 h. The reaction mixture was concentrated under reduced pressure in order to remove most of the phosphorus oxychloride and the concentrate was poured into ice water. The aqueous mixture was allowed to stand for 2 h at ambient temperature and then was extracted with diethyl ether. The ether extract was discarded. The aqueous layer was adjusted to pH 8.0 with potassium carbonate and then extracted with ethyl acetate. The organic extract was dried over anhydrous sodium sulfate, filtered and concentrated under reduced pressure. The liquid concentrate was distilled to afford 8.737 g of a mixture of the title compound and the isomeric 6-chloro-2-picoline as a clear colorless liquid, b.p. 70°C (25 mm Hg). This product was combined with another sample of the same mixture prepared separately by the same procedure. The isomeric products were inseparable by distillation. The combined products (12.905 g) were dissolved in 750 mL of ethyl alcohol. To the resultant solution was added, dropwise, concentrated nitric acid solution until a white precipitate formed and the pH of the supernatant solution was 1. The precipitate was removed by filtration and dIssolved in water. The resultant aqueous solution was adjusted to neutral pH with sodium bicarbonate and then extracted with methylene chloride. The organic extract was dried over anhydrous sodium sulfate, filtered and concentrated under reduced pressure to afford 7.487 g of the title compound.
¹H NMR (CDCl₃) δ 2.55 (s, 3H), 7.12 (dd, 1H, J=3 Hz, 6 Hz), 7.18 (d, 1H, J=3 Hz), 8.40 (d, 1H, J=6 Hz).

### Step 2: Diethyl 2-ethoxy-3-(5-fluoropyridin-2-yl)-propane-1,1-dicarboxylate

Lithium diisopropylamide (LDA: 16 mL of a 1.5 M solution in hexane) was added to 8 mL of dry THF, under a nitrogen atmosphere, and the resultant solution was cooled to -70°C in a isopropyl alcohol/dry ice bath. To the cooled solution of LDA, was added dropwise, over a 30 minute period, a solution of 2.5 g (19.6 mmol) of 4-chloro-2-picoline, from Step 1, in 20 mL of dry THF. The solution turned a very dark red color. After stirring the dark red solution for 0.5 h at -70°C, a solution of 4.04 mL (19.6 mmol) of ethoxymethylenemalonate in 18 mL of dry THF was added dropwise over a 30 minute period. The reaction solution turned from dark red to orange. After stirring for 0.5 h at -70°C, the reaction solution was allowed to warm to -20°C and was stirred at -20°C for 1 h. The reaction was quenched at -20°C by the addition of 1.3 mL of glacial acetic acid and the cooling bath was removed. After 20 minutes the reaction solution was poured into 5% aqueous sodium bicarbonate solution. The aqueous mixture was extracted with methylene chloride and the organic extract was dried over anhydrous sodium sulfate, filtered and concentrated under reduced pressure. The residue (8.03 g) was purified by chromatography on a silica gel column (∼120 g of SiO₂) eluted with 0.5% methanol in methylene chloride to afford 4.59 g (68% yield) of the title compound.

### Step 3: Ethyl 8-chloro-4H-quinolizin-4-one-3-carboxylate

80 mL of Dowtherm A® in a 3-neck flask equipped with a thermometer, an addition funnel and an air-cooled condenser was heated to 235°C, under nitrogen, using a heating mantel. A solution of 4.26 g (12.4 mmol) of diethyl 2-ethoxy-3-(5-fluoropyridin-2-yl)-propane-1,1-dicarboxylate, from Step 2, in 45 mL of Dowtherm A® was added, dropwise over a 1.5 h period, through the addition funnel to the heated stirring Dowtherm A®. After the addition was complete, the resultant solution was heated at ∼200°C for 1 h and then was cooled to ambient temperature. The black-green-colored solution was then poured into 500 mL of hexane and a precipitate formed. The precipitate was collected by filtration, washed with 5 X 100 mL of hexane and dried to afford 1.487 g (48% yield) of the title compound.

### Step 4: Ethyl 8-(3-(N-t-butoxycarbonyl)amino-1-pyrrolidinyl)-4H-quinolizin-4-one-3-carboxylate

Ethyl 8-chloro-4H-quinolizin-4-one-3-carboxylate (1.0 g, 3.97 mmol), from Step 3, was dissolved in 20 mL of dry pyridine under a nitrogen atmosphere. To the resultant solution was added a solution of 1.85 g (9.92 mmol) of 3-(N-t-butoxycarbonylamino)pyrrolidine in 5 mL of dry pyridine and the reaction mixture was heated at 70°C for 4.5 h. The reaction mixture was then concentrated *in vacuo* In order to remove all of the pyridine. The dry residue (3.124 g) was purified by chromatography on silica gel eluted with 2% methanol in methylene chloride to afford 0.889 g (56% yield) of the title compound.

### Step 5: 8-(3-Amino-1-pyrrolidinyl)-4H-quinolizin-4-one-3-carboxylic acid hydrochloride

A solution of 0.889 g (2.2 mmol) of ethyl 8-(3-(N-t-butoxycarbonyl)amino-1-pyrrolidinyl)-4H-quinolizin-4-one-3-carboxylate, from Step 4, in 20 mL of trifluoroacetic acid (TFA) was stirred for 2 h at ambient temperature. The TFA was evaporated *in vacuo* and the residue was dissolved in 200 mL of methanol. To the resultant solution was added 4.5 g of strongly basic ion exchange resin and the mixture was stirred at ambient temperature for 1 h. The mixture was filtered and the filtrate was concentrated under reduced pressure to afford crude ethyl 8-(3-amino-1-pyrrolidinyl)-4H-quinolizin-4-one-3-carboxylate as a residue. The residue was dissolved in 5 mL of THF and 11 mL of a 1 M aqueous solution of sodium hydroxide was added. The reaction mixture was heated at 60°C for 1 h and then the reaction temperature was increased to 85°C in order to evaporate the THF. The concentrated reaction solution was diluted with 20 mL of water and the pH of the resultant solution was adjusted to 1 - 2 with concentrated hydrochloric acid. The aqueous solution was concentrated *in vacuo*. The residue was crystallized from ethyl alcohol:isopropyl alcohol:water (4:4:1 v/v/v) and recrystallized from ethyl alcohol/water to afford 0.388 g (57% yield) of the title compound, m.p.225-230°C; MS DCl-NH₃: 274 (M-Cl)⁺ 90%, 230 ((M-Cl)-CO₂H)⁺ base; IR (KBr): 3420 (OH), 1650(C=O) cm⁻¹; ¹H NMR (TFA) δ 2.8-3.1 (m, 6H), 4.62 (m, 1H), 7.06 (s, 1H), 7.4 (d, 2H, J=9 Hz), 8.14 (d, 1H, J=9 Hz), 9.06 (d, 1H, J=9 Hz). Analysis calculated for C₁₄H₁₆ClN₃O₃+1/3H₂O: C, 53.21; H, 5.10; N, 13.30. Found: C, 53.58; H, 5.38; N, 13.30.

### Example 59 (reference)

### 8-(3-(N-Norvalyl)amino-pyrrolidinyl)-4H-quinolizin-4-one-3-carboxylic acid

3-Amino-1-benzylpyrrolidine (I. Sumio and T. Matsuo, Japanese Kokai JP 5328161, published March 16, 1978) is coupled to N-t-butoxycarbonyl norvaline (Boc-nVal) using conventional N-hydroxysuccinimide coupling procedures. The 1-benzyl group is removed by hydrogenolysis in methanol using palladium on carbon catalyst. The 3-(N-Boc-norvalyl)aminopyrrolidine is then reacted with ethyl 8-chloro-4H-quinolizin-4-one-3-carboxylate, the product of Step 3 of Example 58, as described in Step 4 of Example 58, replacing 3-(N-t-butoxycarbonylamino)pyrrolidine with 3-(N-Boc-norvalyl)aminopyrrolidine, to give 8-(3-(N-norvalyl)amino-pyrrolidinyl)-4H-quinolizin-4-one-3-carboxylic acid with the nitrogen of the amino acid protected with a Boc group. The Boc protecting group is removed by standard hydrolysis using trifluoroacetic acid and dilute aqueous hydrochloric acid.

Using the procedure outlined in Example 59, or any of the other conventional condensation methods listed above, other amino acid derivatives of compounds having an amino group can be prepared. Examples of amino acids which can be coupled, either alone or in combination with one and other, include naturally occurring amino acids such as glycine, alanine, leucine, isoleucine, methionine, phenylalanine, valine, and the like, as well as synthetic amino acids such as cyclohexylalanine, cyclohexylglycine, aminopentanoic acid, and the like.

### Example 60 (reference)

### 8-Chloro-4-H-quinolizin-4-one-3-carboxylic acid

### Step 1: Ethyl 8-chloro-4H-quinolizin-4-one-3-carboxylate

35 mL of Dowtherm A® in a 3-neck flask equipped with a thermometer, an addition funnel and an air-cooled condenser was heated to 230-235°C, under positive nitrogen pressure, using a heating mantel. A solution of 2.7 g (7.85 mmol) of diethyl 2-ethoxy-3-(5-fluoropyridin-2-yl)-propane-1,1-dicarboxylate, the product of Step 2 of Example 58, in 45 mL of Dowtherm A® was added, dropwise over a 1.5 h period, through the addition funnel to the heated stirring Dowtherm A®. After the addition was complete, the resultant solution was heated at ∼200°C for 40 minutes and then was cooled to ambient temperature. The black-green-colored solution was then poured into 600 mL of hexane and a precipitate formed. The precipitate was collected by filtration, washed with 2 X 150 mL of hexane and dried to afford 1.15 g (58% yield) of the title compound, m.p. 153-154°C.

### Step 2: 8-Chloro-4-H-quinolizin-4-one-3-carboxylic acid

Ethyl 8-chloro-4H-quinolizin-4-one-3-carboxylate (125 mg, 0.5 mmol) was suspended in 5 ml of 0.5 N aqueous sodium hydroxide solution. The reaction mixture was heated to 65°C and 2 mL of THF was added. After the reaction mixture was stirred at 65°C for 1 h, the THF was distilled from the mixture. Stirring was continued for 2 h at 65°C and then the reaction mixture was allowed to cool to ambient temperature. The aqueous mixture was adjusted to pH 2 with 3 ml of 1.0 N aqueous hydrochloric acid solution and diluted with 10 ml of water. The precipitate was collected by filtration, washed with 2 X 15 ml of water and dried *in vacuo* to afford 100 mg (89% yield) of the title compound, m.p. 229-230°C. The product was recrystallized from ethyl alcohol and dried *in vacuo* to afford 50 mg (44.5% yield) of the title compound, m.p. 237-238°C; MS DCl-NH₃: 224 (M+H)⁺, 241 (M+NH₄)⁺; IR (KBr): 3430 (OH), 1740 (C=O) cm⁻¹; ¹H NMR (CDCl₃) δ 6.89 (d, 1H, J=6.9 Hz), 7.30 (dd, 1H, J=2.1 Hz, J=6.6 Hz), 7.71 (d, 1H, J=2.1 Hz), 8.64 (d, 1H, J=6.9 Hz), 9.25 (d, 1H, J=6.6 Hz). Analysis calculated for C₁₀H₆ClNO₃: C, 53.71; H, 2.70; N, 6.26. Found: C, 54.27; H, 2.86; N, 6.23.

### Example 61 (reference)

### 8-(4-methylpiperazin-1-yl)-4H-quinolizin-4-one-3-carboxylic acid hydrochloride

### Step 1: Ethyl 8-(4-methylpiperazin-1-yl)-4H-quinolizin-4-one-3-carboxylate

Ethyl 8-chloro-4H-quinolizin-4-one-3-carboxylate (755 mg, 3.0 mmol), the product of Step 3 of Example 58, was suspended in 12 ml of dry pyridine under a nitrogen atmosphere. To the resultant solution was added 6.0 mL (6.0 mmol) of N-methylpiperazine and the reaction mixture was heated at 70°C for 8 h. The reaction mixture was then concentrated *in vacuo* in order to remove all of the pyridine. The dry residue (3.124 g) was dissolved in 125 ml of methylene chloride and the methylene chloride solution was washed with 125 ml of saturated sodium chloride solution (brine). The aqueous layer was extracted with 125 ml of methylene chloride and the combined methylene chloride solutions were dried over anhydrous sodium sulfate, filtered and concentrated and dried *in vacuo* to afford 1.01 g of the title compound.

### Step 2: 8-(4-methylpiperazin-1-yl)-4H-quinolizin-4-one-3-carboxylic acid hydrochloride

A mixture of 0.865 g (2.75 mmol) of ethyl 8-(4-methylpiperazin-1-yl)-4H-quinolizin-4-one-3-carboxylate, from Step 1, in 12 mL of THF and 16.5 mL of a 0.5 N aqueous solution of sodium hydroxide was heated, with stirring, at 75°C for 8 h. The THF was removed from the reaction mixture by distillation during the reaction. The concentrated reaction mixture was cooled to ambient temperature and adjusted to pH 2.0 with 10.5 mL of 1 N aqueous hydrochloric add solution. The aqueous solution was concentrated *in vacuo* to remove ∼80% of the water and the concentrate was diluted with 50 ml of 95% ethyl alcohol. The solid was collected by filtration, washed with 2 X 5 ml of ethyl alcohol and dried *in vacuo* to afford the desired product.The product was recrystallized from ethyl alcohol/water (3:1 v/v) to afford 0.332 g (37% yield) of the title compound, m.p.257-258°C; MS DCl-NH₃: 288 (M-Cl)⁺ 90%, 244 ((M-Cl)-CO₂H)⁺ base, 270 (M-Cl-H₂O)⁺; IR (KBr): 3420 (OH), 1645 (C=O) cm⁻¹; ¹H NMR (TFA) δ 3.20 (m, 3H), 3.52 (dd, 2H, J=10 Hz), 4.02 (m, 4H), 4.63 (d, 2H, J=12 Hz), 7.41 (m, 2H), 7.65 (d, 1H, J=7.5 Hz), 8.26 (d, 1H, J=9 Hz), 9.18 (d, 1H, J=7.5 Hz). Analysis calculated for C₁₅H₁₈ClN₃O₃+0.5H₂O: C, 54.14; H, 5.75; N, 12.62. Found: C,54.23; H, 5.54; N, 12.64.

### Example 62 (reference)

### 8-(3-Amino-1-pyrrolidinyl)-1-ethyl-4H-quinolizin-4-one-3-carboxylic acid hydrochloride

### Step 1: 4-Chloro-2-propyl-pyridine

A 1.5 M solution of LDA in hexane (100 mL, 150 mmol) was cooled to -60°C in an isopropyl alcohol/dry ice bath. To the stirred LDA solution, under nitrogen, was added, dropwise over a 0.5 h period, a solution of 17.466 g (137 mmol) of 4-chloro-2-picoline (the product of Step 1 of Example 58) in 80 mL of dry THF. The reaction mixture was stirred for 0.5 h at -60°C and then a solution of 10.95 ml (137 mmol) of ethyl iodide in 30 ml of dry THF was added, dropwise over a 20 minute period. After the reaction mixture was stirred at -60°C for 0.5 h, the cooling bath was allowed to slowly (1.5 h) warm to -30°C. According to TLC analysis on silica gel eluted with 5% methanol in methylene chloride, the reaction had gone to completion. The reaction mixture was poured into cold brine and the aqueous mixture was extracted with methylene chloride. The organic extract was dried over anhydrous sodium sulfate, filtered and concentrated *in vacuo*. The residue was distilled to afford 12.667 g (60% yield of the title compound, b.p. 77-80°C (10 mm Hg).

### Step 2: Diethyl 2-ethoxy-3-[4-chloro-2-pyridyl]-pentane-1,1-dicarboxylate

A solution of 12.6 mL (89.9 mmol) of diisopropylamine in 20 mL of anhydrous tetrahydrofuran (THF) was prepared under a nitrogen atmosphere and cooled in an ice/water bath. To this solution was added, dropwise over a 30 minute period, 36 mL of a 2.5 M solution of n-butyllithium (90 mmol) in hexane. The solution was stirred for 30 minutes at 0°C and then cooled to -60°C. To the amine solution at -60°C, was added, dropwise over a 30 minute period, a solution of 12.66 g (81.9 mmol) of 4-chloro-2-propyl-pyridine, from Step 1, in 100 mL of anhydrous THF and a dark red-colored solution was formed. The solution was stirred at -60°C for 0.5 h and then 16.55 mL (81.9 mmol) of ethyl 2-carboethoxy-3-ethoxy-2-propenecarboxylate was added, dropwise over a 30 minute period. Stirring was continued at -60°C for 0.5 h and at -20°C for 1.5 h. The reaction mixture was poured into cold brine and the aqueous mixture was extracted with methylene chloride. The combined organic extract was dried over anhydrous sodium sulfate, filtered and concentrated *in vacuo* to afford 35.48 g of the title compound. The product was carried on to the next step without purification.

### Step 3: Ethyl 8-chloro-1-ethyl-4-H-quinolizin-4-one-3-carboxylate

A solution of 35.48 g (99.2 mmol) of diethyl 2-ethoxy-3-[4-chloro-2-pyridyl]-pentane-1 ,1-dicarboxylate, from Step 2, in 1 L of xylene was heated at 150°C, with stirring, for 24 h and then concentrated *in vacuo*. The residue was washed with a mixture of hexane and cyclohexane to afford 14.867 g (54% yield) of the title compound as a green solid; MS DCl-NH₃ M/Z: 280 (M+H)⁺, 246 (M-Cl)⁺, 217 (M-Cl-Et)⁺; ¹H NMR (CDCl₃) δ 1.31 (t, 3H, J=7.5 Hz), 1.43 (t, 3H, J=7.2 Hz), 2.78 (q, 2H, J=7.5 Hz), 4.43 (q, 2H, J=7.2 Hz), 7.10 (dd, 1H, J=2.4 Hz, 8.1 Hz), 7.70 (d, 1H, J=2.4 Hz), 8.32 (s, 1H), 9.40 (d, 1H, 8.1Hz).

### Step 4: Ethyl 8-(3-(N-t-butoxycarbonyl)amino-1-pyrrolidinyl)-1-ethyl-4H-quinolizin-4-one-3-carboxylate

Ethyl 8-chloro-1-ethyl-4H-quinolizin-4-one-3-carboxylate (1.20 g, 4.3 mmol), from Step 3, was dissolved, under a nitrogen atmosphere, in 15 mL of dry pyridine. To the resultant solution was added 1.04 g (5.59 mmol) of 3-(N-t-butoxycarbonylaminopyrrolidine) and 1.8 mL (12.9 mmol) of dry triethylamine and the reaction mixture was heated at 60°C for 12 h. The reaction mixture was then concentrated *in vacuo* in order to remove all of the pyridine. Ethyl alcohol (4 mL) was added to the dry residue. The mixture was filtered to give 0.421 g of the desired product as a solid. The filtrate was concentrated and the residue purified by flash chromatography on silica gel eluted with 2% methanol in methylene chloride, followed by 5% methanol in methylene chloride to afford an additional 1.273 g of the desired product. The title compound was obtained in 92% yield (1.694 g) as a yellow solid and taken on to the next step.

### Step 5: 8-(3-Amino-1-pyrrolidinyl)-1-ethyl-4H-quinolizin-4-one-3-carboxylic acid hydrochloride

A solution of 1.694 g (3.94 mmol) of ethyl 8-(3-(N-t-butoxycarbonyl)-amino-1-pyrrolidinyl)-1-ethyl-4H-quinolizin-4-one-3-carboxylate, from Step 4, in 25 mL of trifluoroacetic acid (TFA) was stirred for 2 h at ambient temperature. The TFA was evaporated *in vacuo* and the residue was dissolved in 200 mL of methanol. To the resultant solution was added 25 g of strongly basic ion exchange resin and the mixture was stirred at ambient temperature for 2 h. The mixture was filtered and the filtrate was concentrated under reduced pressure to afford 1.146 g (88% yield) of ethyl 8-(3-amino-1-pyrrolidinyl)-1-ethyl-4H-quinolizin-4-one-3-carboxylate as a residue. The residue was dissolved in 6 mL of THF and 10.5 mL of a 1 M aqueous solution of sodium hydroxide was added. The reaction mixture was heated at 60°C for 2 h and then the reaction temperature was increased to 90°C for 2 h, in order to evaporate the THF. The concentrated reaction solution was poured into water and the pH of the resultant solution was adjusted to ∼2 with concentrated hydrochloric acid. The solid was filtered to afford 0.365 g (31% yield) of the title compound, m.p.196-198°C; MS DCl-NH₃: 302 (M-Cl)⁺ base, 258 ((M-Cl)-CO₂H)⁺ 25%; IR (KBr): 3440 (OH), 2960, 1650 (C=O), 1500, 1360, 1280 cm⁻¹; ¹H NMR (TFA) δ 1.41 (t, 3H, J=7.5 Hz), 2.39 (q, 2H, J=7.5), 2.70 (m, 3H), 4.0 (m, 3H), 4.53 (m, 1H), 6.93 (d, 1H, J=1.5 Hz), 7.33 (dd, 1H, J=9 Hz, 1.5 Hz), 7.93 (s, 1H), 9.08 (d, 1H, J=9 Hz). Analysis calculated for C₁₆H₂₀ClN₃O₃: C, 56.98; H, 5.97; N, 12.44. Found: C, 56.83; H, 6.00; N, 11.93.

### Example 63 (reference)

### 8-(3-(Alanyl)amino-pyrrolidinyl)-1-ethyl-4H-quinolizin-4-one-3-carboxylic acid

3-Amino-1-benzylpyrrolidine (I. Sumio and T. Matsuo, Japanese Kokai JP 5328161, published March 16, 1978) is coupled to N-t-butoxycarbonyl alanine (Boc-Ala) using conventional N-hydroxysuccinimide coupling procedures. The 1-benzyl group is removed by hydrogenolysis in methanol using palladium on carbon catalyst. The 3-(N-Boc-alanyl)aminopyrrolidine is then reacted with ethyl 8-chloro-1-ethyl-4H-quinolizin-4-one-3-carboxylate, the product of Step 3 of Example 62, as described in Step 4 of Example 62 replacing 3-(N-t-butoxycarbonylaminopyrrolidine) with 3-(N-Boc-alanyl)aminopyrrolidine, to give 8-(3-(N-alanyl)amino-pyrrolidinyl)-4H-quinolizin-4-one-3-carboxylic acid with the nitrogen of the amino acid protected with a Boc group. The Boc protecting group is removed by standard hydrolysis using trifluoroacetic acid and dilute aqueous hydrochloric acid.

Using the procedure outlined in Example 63, or any of the other conventional condensation methods listed above, other amino acid derivatives of compounds having an amino group can be prepared. Examples of amino acids which can be coupled, either alone or in combination with one and other, include naturally occurring amino acids such as glycine, alanine, leucine, isoleucine, methionine, phenylalanine, valine, and the like, as well as synthetic amino acids such as cyclohexylalanine, cyclohexylglycine, aminopentanoic acid, and the like.

### Example 64 (reference)

### 1-Ethyl-8-(3-methyl-1-piperazinyl)-4H-quinolizin-4-one-3-carboxylic acid hydrochloride

### Step 1: Ethyl 1-ethyl-8-(3-methyl-1-piperazinyl)-4H-quinolizin-4-one-3-carboxylate

Ethyl 8-chloro-1-ethyl-4H-quinolizin-4-one-3-carboxylate (558 mg, 2.0 mmol), the product of Step 3 of Example 62, was dissolved in 10 mL of dry pyridine under a nitrogen atmosphere. To the resultant solution was added 600 mg (6.0 mmol) of 2-methylpiperazine and the stirred reaction mixture was heated at 65°C for 3 h. The reaction mixture was allowed to cool to ambient temperature and then concentrated *in vacuo* in order to remove all of the pyridine. The residue was dissolved in 60 mL of methylene chloride and the methylene chloride solution was washed with 60 mL of water. The aqueous layer was extracted with 2 X 60 mL of methylene chloride and the combined methylene chloride solutions were dried aver anhydrous sodium sulfate, filtered and concentrated and dried *in vacuo* to afford 690 mg of the title compound. The product was carried on to the next step without purification.

### Step 2: 1-Ethyl-8-(3-methyl-1-piperazinyl)-4H-quinolizin-4-one-3-carboxylic acid hydrochloride

To a suspension of 0.686 g (2 mmol) of ethyl 1-ethyl-8-(3-methyl-1-piperazinyl)-4H-quinolizin-4-one-3-carboxylate, from Step 1, in 8 mL of THF was added 8.0 mL of a 1.0 N aqueous sodium hydroxide solution and the reaction mixture was heated , with stirring, at 65°C for 3 h. The THF was removed from the reaction mixture by distillation during the reaction. The concentrated reaction mixture was cooled to ambient temperature and adjusted to pH 1-2 with 16 mL of 1 N aqueous hydrochloric acid solution. The aqueous solution was concentrated *in vacuo* to remove the water and the residue was suspended in 10 mL of water. The solid was collected by filtration and dried *in vacuo* to afford the 385 mg (55% yield) of the title compound, m.p.>295°C; MS DCl-NH₃: 316 (M-Cl)⁺ : IR (KBr): 3420 (OH). 1720 (C=O) cm⁻¹; ¹H NMR (TFA) δ 1.50 (t, 3H, J=7.5 Hz), 1.70 (d, 3H, J=6 Hz), 3.00 (q, 2H, J=7.5 Hz), 3.70-4.10 (m, 6H), 4.55 (m, 1H), 4.60 (m, 1H), 7.40 (d, 1H, J=3.0 Hz), 7.68 (dd, 1H, J=3.0 Hz, 8.4 Hz), 8.18 (s, 1H), 9.19 (d, 1H, J=8.4 Hz). Analysis calculated for C₁₇H₂₂ClN₃O₃+H₂O: C, 55.21; H, 6.54; N, 11.36. Found: C, 55.19; H, 6.07; N, 11.34.

### Example 65 (reference)

### 1-Ethyl-8-(4-methylpiperazin-1-yl)-4H-quinolizin-4-one-3-carboxylic acid hydrochloride

### Step 1: Ethyl 1-ethyl-8-(4-methylpiperazin-1-yl)-4H-quinolizin-4-one-3-carboxylate

Ethyl 8-chloro-1-ethyl-4H-quinolizin-4-one-3-carboxylate (279 mg, 1.0 mmol), the product of Step 3 of Example 62, was dissolved in 5 ml of dry pyridine under a nitrogen atmosphere. To the resultant solution was added 2 mL (2.0 mmol) of N-methylpiperazine and the stirred reaction mixture was heated at 85°C for 2.5 h. The reaction mixture was allowed to cool to ambient temperature and then concentrated *in vacuo* in order to remove all of the pyridine. The residue was dissolved in 50 mL of methylene chloride and the methylene chloride solution was washed with 50 mL of 5% aqueous sodium bicarbonate solution. The aqueous layer was extracted with 3 X 50 mL of methylene chloride and the combined methylene chloride solutions were dried over anhydrous sodium sulfate, filtered and concentrated and dried *in vacuo* to afford 343 mg of the title compound, m.p. 94-96°C; MS DCl-NH₃: 344 (M+H)⁺.

### Step 2: 1-Ethyl-8-(4-methylpiperazin-1-yl)-4H-quinolizin-4-one-3-carboxylic acid hydrochloride

To a solution of 171 mg (0.5 mmol) of ethyl 1-ethyl-8-(4-methylpiperazin-1-yl)-4H-quinolizin-4-one-3-carboxylate, from Step 1, in 4 mL of THF was added 4.0 mL of a 1.0 N aqueous sodium hydroxide solution and the reaction mixture was heated, with stirring, at 75°C for 4.5 h. The reaction mixture was cooled to ambient temperature and adjusted to pH 2 with 5 mL of 1 N aqueous hydrochloric acid solution. The aqueous solution was concentrated *in vacuo* to ∼5 mL and the solid was collected by filtration and dried *in vacuo* to afford 120 mg (68% yield) of the title compound, m.p. 293-294°C (dec); MS DCl-NH₃: 316 (M-Cl)⁺ 90%, 272 ((M-Cl)-CO₂H)⁺ base; IR (KBr): 3420 (OH), 1695 (C=O), 1640 (C=O) cm⁻¹; ¹H NMR (TFA) δ 1.47 (t, 3H, J=7.5 Hz), 3.00 (q, 2H, J=7.5 Hz), 3.23 (s, 3H), 3.55 (dd, 2H, J=9 Hz), 4.12 (m, 4H), 4.65 (d, 2H, J=15 Hz), 7.40 (s, 1H), 7.67 (d, 1H, J=9 Hz), 8.18 (s, 1H), 9.20 (d, 1H, J=7.5 Hz). Analysis calculated for C₁₇H₂₂ClN₃O₃: C, 56.59; H, 6.42; N, 11.64. Found: C,56.86; H, 6.19; N, 11.60.

### Example 66

### 4-Chloro-5-fluoro-2-picoline

### Step 1: 2-(5-Nitro-2-pyridyl)-1,3-propanedicarboxylate

Sodium hydride (20.2 g of NaH suspended in hexane, 0.504 mol) was suspended, under a nitrogen atmosphere, in 600 mL of anhydrous THF in a 3-neck 2 L round-bottom flask equiped with an addition funnel and a mechanical stirrer. The suspension was cooled to 0°C in an ice bath. A solution of 71.8 mL (0.473 mol) of diethyl malonate in 60 mL of anhydrous THF was added dropwise to the sodium hydride suspension over a 1 h period. After the addition and the evolution of hydrogen gas were complete, the reaction mixture was stirred for 20 min at 0°C. A solution of 50 g (0.315 mol) of 2-chloro-5-nitropyridine in 150 mL of anhydrous THF was added dropwise to the mixture, over a 25 min period. The ice bath was removed and the deep red-colored solution was stirred at ambient temperature for 48 h. These procedures were repeated on the same scale. The two solutions containing the product were concentrated to ∼ 500 mL and poured into a mixture of 1 L of 10% aqueous sodium bicarbonate solution and 1 L of brine. The aqueous mixture was extracted with 3 X 500 mL of methylene chloride. The combined organic extract was dried over anhydrous sodium sulfate, filtered and concentrated *in vacuo* to a solid residue. The residue was crystallized from ethyl alcohol and the crystals were washed with hexane to yield 140 g (79% yield) of the title compound as a bright yellow solid; MS DCl-NH₃ M/Z: 283 (M+H)⁺ base, 253 ((M+H)-C₂H₅)⁺ base; ¹H NMR (CDCl₃) δ 1.30 (t, 6H, J=7.5 Hz), 4.26 (q, 2H, J=6.0 Hz), 4.29 (q, 2H, J=6.0 Hz), 5.08 (s, 1H), 7.77 (dd, 1H, J=9.0 Hz, 0.6 Hz), 8.49 (dd, 1H, J=3.0 Hz, 9.0 Hz), 9.38 (dd, 1H, J=3.0 Hz, 9.0 Hz).

### Step 2: 5-Nitro-2-picoline

A suspension of 102.0 g (0.361 mol) of 2-(5-nitro-2-pyridyl)-1,3-propanedicarboxylate, from Step 1, in 600 mL of 20% aqueous sulfuric acid solution was heated at 95°C for 24 h. The resultant solution was poured onto 1 kg of ice and the aqueous mixture was adjusted to a pH within the range pH 10 - 12 with 50% aqueous sodium hydroxide solution. The precipitate was filtered and dissolved in ethyl acetate. The ethyl acetate solution was dried over anhydrous sodium sulfate, filtered and concentrated to a solid residue. The residue was washed with hexane. The hexane was removed by filtration and the solid was dried to afford 45.86 g (92% yield) of the title compound; ¹H NMR (CDCl₃) δ 2.71 (s, 3H), 7.36 (d, 1H, J=9.0 Hz), 8.37 (dd, 1H, J=3.0 Hz, 9.0 Hz), 9.33 (d, 1H, J=3.0 Hz).

### Step 3: 5-Amino-2-picoline

The product of Step 2, 5-nitro-2-picoline (45.86, 0.332 mol), was dissolved in 200 mL of methanol and 1.15 g of 10% palladium on carbon was added to the resultant solution. The reaction mixture was hydrogenated at ambient temperature under 4 atmospheres of hydrogen. The palladium catalyst was removed by filtration through a 45 µ Millipore® filter and the filtrate was concentrated *in vacuo* to afford 33.96 g (95% yield) of the title compound as a tan solid; ¹H NMR (CDCl₃) δ 2.42 (s, 3H), 3.54 (brs, 2H), 6.91 (m, 2H), 8.00 (m, 1H).

### Step 4: 5-Fluoro-2-picoline

A solution of 5-amino-2-picoline (20 g, 0.185 mol), from Step 3, in 105 mL of ethyl alcohol was cooled to 0°C. Tetrafluoroboric acid (55 mL of a 48% solution in water) was added to the cold 5-aminopicoline solution and the flask containing the resultant solution was weighed. Ethyl nitrite was bubbled through the cold solution until 13.88 g (0.185 mol) had been added. The addition took place over a 1.25 h period. After the addition was complete the reaction solution was allowed to sit at 0°C for 15 min, during which time, the excess ethyl nitrite evaporated from the solution. Diethyl ether (120 mL) was added to the reaction mixture to ensure complete precipitation of the tetrafluoroborate salt. After 30 minutes at 0°C, the mixture was filtered. The filter cake was washed with 200 mL of diethyl ether, followed by 300 mL of hexane. The solid was transferred to a 1 L beaker containing approximately 300 mL of hexane and 10.75 g (0.185 mol) of potassium fluoride. The mixture was heated to 40°C over a 4.5 h period. The orange-colored solid was convened to a black oily solid. The hexane was decanted and the residue was cooled to 0°C. The cold residue was triturated with approximately 200 mL of 50% sodium hydroxide. The mixture was combined with material obtained from duplicate runs of the preceeding procedures and the combined aqueous mixtures were steam distilled. The aqueous distillate collected between 92°C and 100°C was extracted with two portions of methylene chloride. The combined methylene chloride extract was dried over anhydrous sodium sulfate, filtered and added to the (hexane) distillate which was collected between 62°C and 65°C. The product was carried on to the next step in solution.

### Step 5: 5-Fluoro-2-picoline-N-oxide

To the solution of 5-fluoro-2-picoline obtained in Step 4, at 0°C, was added, with vigorous stirring, a cold solution of 40% peracetic acid (prepared by carefully adding 50 mL of 30% hydrogen peroxide solution to 150 mL of glacial acetic acid). The reaction mixture was heated at reflux temperature (50°C) for 4 days and then poured into 600mL of ice water. The aqueous mixture was adjusted to pH 9 by the addition of potassium carbonate and then was stirred at ambient temperature for 4 h. The aqueous solution was continuously extracted with methylene chloride for 24 h and the methylene chloride extract was dried over anhydrous sodium sulfate, filtered and concentrated *in vacuo* to afford 30.8 g (22% yield) of the title compound: MS DCl-NH₃ M/Z: 128 (M+H)⁺ base; ¹H NMR (CDCl₃) δ 2.48 (s, 3H), 7.00 (ddd, 1H), 7.22 (dd, 1H), 8.22 (dd, 1H).

### Step 6: 5-Fluoro-4-nitro-2-picoline-N-oxide

The reaction was carried out in a flask vented to a gas scrubber containing aqueous sodium hydroxide solution. The product of Step 5, 5-fluoro-2-picoline-N-oxide (1.0 g, 7.86 mmol) was cooled to 0°C and concentrated sulfuric acid (4.2 mL) was slowly added, with stirring. Solid potassium nitrate (1.27 g, 12.5 mmol) was then added to this mixture at 0°C, in small portions over a 45 minute period. The reaction mixture was allowed to warm to ambient temperature and was stirred at ambient temperature for 1 h. Not all of the potassium nitrate had dissolved and the reaction mixture was heated at 50°C for 0.5 h and then at 100°C for 18 h. The homogeneous reaction solution was poured over ice and the resultant aqueous solution was adjusted to pH 9 with solid potassium carbonate. The aqueous solution was then extracted with 3 X 80 mL of methylene chloride. The combined organic extract was dried over anhydrous sodium sulfate, filtered and concentrated *in vacuo* to give 1.084 g (80% yield) of the title compound as a yellow solid, m.p. 107-108°C; MS DCl-NH₃ M/Z: 190 (M+NH₄)⁺ 10%, 173 (M+H)⁺ 30%, 157 (M-O)⁺ 50%; ¹H NMR (CDCl₃) δ 2.48 (s, 3H), 8.05 (d, 1H, J=9.0 Hz), 8.31 (d, 1H, J=6.0 Hz).

### Step 7: 4-Chloro-5-fluoro-2-picoline-N-oxide

The product of Step 6, 5-fluoro-4-nitro-2-picoline-N-oxide (3.56 g, 20.6 mmol) was dissolved in 30 mL of concentrated (37.5%) aqueous hydrochloric acid. The resultant solution was heated, with stirring, at 110°C for 48 h and then concentrated *in vacuo*. Water (30 mL) was added to the residue and the resultant aqueous solution was adjusted to pH 9-10 with sodium carbonate. The aqueous solution was then extracted with 3 X 50 mL of methylene chloride and the combined organic extract was dried over anhydrous sodium sulfate, filtered and concentrated *in vacuo*. The product was crystallized from hexane to afford 1.8 g (55% yield) of the title compound, m.p. 92-93°C; MS DCl-NH₃ M/Z: 179 (M+NH₄)⁺ 30%, 162 (M+H)⁺ base, 146 (M-O)⁺ 60%; ¹H NMR (CDCl₃) δ 2.46 (s, 3H), 7.30 (d, 1H, J=9.0 Hz), 8.26 (d, 1H, J=4.5 Hz); IR (chloroform solution) 1605 (N-O), 1180 (C-F) cm⁻¹. Analysis calculated for C₆H₅ClFNO: C, 44.61; H, 3.12; N, 8.62. Found: C, 44.89; H, 3.25; N, 9.40.

### Step 8: 4-Chloro-5-fluoro-2-picoline

4-Chloro-5-fluoro-2-picoline-N-oxide (12.43 g, 76.93 mmol), from Step 7, was dissolved in 52 mL of glacial acetic acid in a 3-necked flask equiped with a mechanical stirrer, a condenser and a thermometer. Iron powder (6.45 g, 115.5 mmol) was added to the solution at ambient temperature and the reaction mixture was carefully heated to 35-40°C. After 10 min at 30°C, an exothermic reaction took place which caused the reaction temperature to rise to 120°C and the reaction mixture became a very dark brown-colored solution. The flask was transferred to a cold water bath and the temperature of the solution brought down to ambient. The reaction mixture was then poured over ice. The resultant aqueous mixture was adjusted to pH 9 with potassium carbonate and steam distilled. The aqueous distillate collected at 92-96°C was extracted with three portions of methylene chloride. The combined organic extract was dried over anhydrous sodium sulfate, filtered and distilled to afford 15.91 g (71% yield) of the title compound, b.p. 138-140°C; MS GC-MS M/Z:146 (M+H)⁺; ¹H NMR (CDCl₃) δ 2.53 (s, 3H), 7.23 (d, 1H, J=6.0 Hz), 8.37 (s, 1H).

### Example 67

### 3,4-Dichloro-5-fluoro-2-picoline

To 0.87 g (6 mmol) of 4-chloro-5-fluoro-2-picoline, the product of Example 66, in 20 mL of chloroform cooled to -45°C, is added 0.75 mL of t-butylhypochlorite. The reaction mixture is stirred at -45°C for 2 h and at 0°C for 2 h. The reaction mixture is then poured into water and the resultant aqueous mixture is extracted with methylene chloride. The organic solution is dried over anhydrous magnesium sulfate, filtered, concentrated under reduced pressure and distilled to afford the title compound.

### Example 68

### 3-Bromo-4-chloro-5-fluoro-2-picoline

4-Chloro-5-fluoro-2-picoline, the product of Example 66, is treated with bromine in fuming sulfuric acid containing 65% sulfur trioxide for 7 h at 80°C as described by L. van der Does and H.J. Hertog in Rec Tray Chim *81*: 864 (1965) to afford the title compound.

### Example 69

### 4-Chloro-3,5-difluoro-2-picoline

4-Chloro-5-fluoro-2-picoline is treated with 1.1 equivalents of acetyl hypofluorite as described by O. Lerman, *et al*. J Org Chem, *49*: 806-813 (1984) to afford the title compound.

### Example 70

### 4-Chloro-5-fluoro-2-propyl-pyridine

Diisopropylamine (924 µL, 6.59 mmol) was dissolved in 9 mL of dry THF and the resultant solution was cooled to 0°C in an ice bath. n-Butyllithium (3.07 mL of a 2.05 M solution in THF, 6.29 mmol) was added via syringe to the amine solution and the resultant solution was stirred for 30 minutes at 0°C. The lithium diisopropylamide (LDA) solution was then cooled to -50°C in an isopropyl alcohol/dry ice bath. To the cold LDA solution was added, dropwise from an addition funnel, over a 15 min period, a solution of 4-chloro-5-fluoro-2-picoline (435 µL, 3.0 mmol), the product of Example 64, in 9 mL of THF. The reaction solution turned dark orange-brown in color. The reaction solution was stirred at a temperature in the range -50°C to -45°C for 5 h and then was cooled over a 15 min period to -78°C. Ethyl iodide (792 µL, 9.9 mmol) was added in one portion and the reaction solution was stirred at -78°C for 20 min. The reaction was then quenched by pouring the reaction solution into 60 mL of 10% aqueous ammonium chloride solution. The aqueous mixture was extracted with 2 X 50 mL of methylene chloride. The combined organic extract was dried over anhydrous sodium sulfate, filtered and concentrated *in vacuo* and the residue was distilledto afford the title compound, b.p. 80-82°C (12 mm Hg); MS DCl-NH₃ M/Z: 174 (M+H)⁺ 40%; ¹H NMR (CDCl₃) δ 0.96 (t, 3H, J=7.5 Hz), 1.73 (spt, 2H, J=7.5 Hz), 2.73 (t, 2H, J=7.5 Hz), 7.21 (d, 1H, J=6.0 Hz), 8.38 (s, 1H).

### Example 71

### 3,4-Dichloro-5-fluoro-2-propyl-pyridine

By following the procedures described in Example 67 and replacing 4-chloro-5-fluoro-2-picoline (the product of Example 66) with 4-chloro-5-fluoro-2-propyl-pyridine (the product of Example 70), the title compound can be prepared.

### Example 72

### 3-Bromo-4-chloro-5-fluoro-2-propyl-pyridine

By following the procedures described in Example 68 and replacing 4-chloro-5-fluoro-2-picoline (the product of Example 66) with 4-chloro-5-fluoro-2-propyl-pyridine (the product of Example 70), the title compound can be prepared.

### Example 73

### 4-Chloro-3,5-difluoro-2-propyl-pyridine

By following the procedures described in Example 69 and replacing 4-chloro-5-fluoro-2-picoline (the product of Example 66) with 4-chloro-5-fluoro-2-propyl-pyridine (the product of Example 70), the title compound can be prepared.

### Example 74 (reference)

### 1-Ethyl-7-fluoro-8-(4-methylpiperazin-1-yl)-4H-quinolizin-4-one-3-carboxylic acid hydrochloride

By following the procedures described in Step 2 of Example 62 and in Example 65 and replacing 4-chloropicoline with 4-chloro-5-fluoro-picoline (the product of Example 66), the title compound can be prepared.

### Example 75 (reference)

### 1-Ethyl-7-fluoro-8-(3-methyl-1-piperazinyl)-4H-quinolizin-4-one-3-carboxylic acid hydrochloride

By following the procedures described in Step 2 of Example 62 and in Example 65 and replacing 4-chloropicoline with 4-chloro-5-fluoro-picoline (the product of Example 66), and replacing N-methylpiperazine with 2-methylpiperazine, the title compound can be prepared.

### Example 76 (reference)

### 8-(3-Amino-1-pyrrolidinyl)-1-ethyl-7-fluoro-4H-quinolizin-4-one-3-carboxylic acid hydrochloride

Following the procedures described in Example 62, replacing 4-chloropicoline with 4-chloro-5-fluoro-picoline (the product of Example 66), the title compound is prepared.

### Example 77

### 9-Chloro-1-ethyl-7-fluoro-8-(4-methylpiperazin-1-yl)-4H-quinolizin-4-one-3-carboxylic acid hydrochloride

Following the procedures described in Step 2 of Example 62 and in Example 65, replacing 4-chloropicoline with 3,4-dichloro-5-fluoro-picoline (the product of Example 67), the title compound is prepared.

### Example 78

### 9-Chloro-1-ethyl-7-fluoro-8-(3-methyl-1-piperazinyl)-4H-quinolizin-4-one-3-carboxylic acid hydrochloride

Following the procedures described in Step 2 of Example 62 and in Example 65, replacing 4-chloropicoline with 3,4-dichloro-5-fluoropicoline (the product of Example 67), and replacing N-methylpiperazine with 2-methylpiperazine, the title compound is prepared.

### Example 79

### 8-(3-Amino-1-pyrrolidinyl)-9-chloro-1-ethyl-7-fluoro-4H-quinolizin-4-one-3-carboxylic acid hydrochloride

Following the procedures described in Example 62, replacing 4-chloropicoline with 3,4-dichloro-5-fluoropicoline (the product of Example 67), the title compound is prepared.

### Example 80

### 9-Bromo-1-ethyl-7-fluoro-8-(4-methylpiperazin-1-yl)-4H-quinolizin-4-one-3-carboxylic acid hydrochloride

Following the procedures described in Step 2 of Example 62 and in Example 65, replacing 4-chloropicoline with 3-bromo-4-chloro-5-fluoropicoline (the product of Example 68, the title compound is prepared.

### Example 81

### 9-Bromo-1-ethyl-7-fluoro-8-(3-methyl-1-piperazinyl)-4H-quinolizin-4-one-3-carboxylic acid hydrochloride

Following the procedures described in Step 2 of Example 62 and in Example 65, replacing 4-chloropicoline with 3-bromo-4-chloro-5-fluoro-picoline (the product of Example 68), and replacing N-methylpiperazine with 2-methylpiperazine, the title compound is prepared.

### Example 82

### 8-(3-Amino-1-pyrrolidinyl)-9-bromo-1-ethyl-7-fluoro-4H-quinolizin-4-one-3-carboxylic acid hydrochloride

Following the procedures described in Example 62, replacing 4-chloropicoline with 3-bromo-4-chloro-5-fluoro-picoline (the product of Example 68), the title compound is prepared.

### Example 83

### 7,9-Difluoro-1-ethyl-8-(4-methylpiperazin-1-yl)-4H-quinolizin-4-one-3-carboxylic acid hydrochloride

Following the procedures described in Step 2 of Example 62 and in Example 65, replacing 4-chloropicoline with 4-chloro-3,5-difluoropicoline (the product of Example 69), the title compound is prepared.

### Example 84

### 7,9-Difluoro-1-ethyl-8-(3-methyl-1-piperazinyl)-4H-quinolizin-4-one-3-carboxylic acid hydrochloride

Following the procedures described in Step 2 of Example 62 and in Example 65, replacing 4-chloropicoline with 4-chloro-3,5-difluoropicoline (the product of Example 69), and replacing N-methylpiperazine with 2-methylpiperazine, the title compound is prepared.

### Example 85

### 8-(3-Amino-1-pyrrolidinyl)-7,9-difluoro-1-ethyl-4H-quinolizin-4-one-3-carboxylic acid hydrochloride

Following the procedures described in Example 62, replacing 4-chloropicoline with 4-chloro-3,5-difluoropicoline (the product of Example 69), the title compound is prepared.

### Example 86 (reference)

### 1-Cyclopropyl-7-fluoro-8-(4-methylpiperazin-1-yl)-4H-4-quinolizin-4-one-3-carboxylic acid hydrochloride

Following the procedures described in Steps 1 and 2 of Example 62 and in Example 65, replacing 4-chloropicoline with 4-chloro-5-fluoropicoline (the product of Example 66), and replacing ethyl iodide with cyclopropyl iodide, the title compound is prepared.

### Example 87 (reference)

### 1-Cyclopropyl-7-fluoro-8-(3-methyl-1-piperazinyl)-4H-quinolizin-4-one-3-carboxylic acid hydrochloride

Following the procedures described in Steps 1 and 2 of Example 62, replacing 4-chloropicoline with 4-chloro-5-fluoropicoline (the product of Example 66) and replacing ethyl iodide with cyclopropyl iodide, and the procedures described in Example 65, replacing N-methylpiperazine with 2-methylpiperazine, the title compound is prepared.

### Example 88 (reference)

### 8-(3-Amino-1-pyrrolidinyl)-1-cyclopropyl-7-fluoro-4H-quinolizin-4-one-3-carboxylic acid hydrochloride

Following the procedures described in Example 62, replacing 4-chloropicoline with 4-chloro-5-fluoropicoline (the product of Example 66), and replacing ethyl iodide with cyclopropyl iodide, the title compound is prepared.

### Example 89

### 9-Chloro-1-cyclopropyl-7-fluoro-8-(4-methylpiperazin-1-yl)-4H-quinolizin-4-one-3-carboxylic acid hydrochloride

Following the procedures described in Steps 1 and 2 of Example 62, replacing 4-chloropicoline with 3,4-dichloro-5-fluoropicoline (the product of Example 67) and replacing ethyl iodide with cyclopropyl iodide, and the procedures described in Example 65, the title compound is prepared.

### Example 90

### 9-Chloro-1-cyclopropyl-7-fluoro-8-(3-methyl-1-piperazinyl)-4H-quinolizin-4-one-3-carboxylic acid hydrochloride

Following the procedures described in Steps 1 and 2 of Example 62, replacing 4-chloropicoline with 3,4-dichloro-5-fluoropicoline (the product of Example 67) and replacing ethyl iodide with cyclopropyl iodide, and the procedures described in Example 65, replacing N-methylpiperazine with 2-methylpiperazine, the title compound is prepared.

### Example 91

### 8-(3-Amino-1-pyrrolidinyl)-9-chloro-1-cyclopropyl-7-fluoro-4H-quinolizin-4-one-3-carboxylic acid hydrochloride

Following the procedures described in Example 62, replacing 4-chloropicoline with 3,4-dichloro-5-fluoropicoline (the product of Example 67) and replacing ethyl iodide with cyclopropyl iodide, the title compound is prepared.

### Example 92

### 9-Bromo-1-cyclopropyl-7-fluoro-8-(4-methylpiperazin-1-yl)-4H-quinolizin-4-one-3-carboxylic acid hydrochloride

Following the procedures described in Steps 1 and 2 of Example 62, replacing 4-chloropicoline with 3-bromo-4-chloro-5-fluoropicoline (the product of Example 68) and replacing ethyl iodide with cyclopropyl iodide, and the procedures described in Example 65, the title compound is prepared.

### Example 93

### 9-Bromo-1-cyclopropyl-7-fluoro-8-(3-methyl-1-piperazinyl)-4H-quinolizin-4-one-3-carboxylic acid hydrochloride

Following the procedures described in Steps 1 and 2 of Example 62, replacing 4-chloropicoline with 3-bromo-4-chloro-5-fluoropicoline (the product of Example 68) and replacing ethyl iodide with cyclopropyl iodide, and the procedures described in Example 65, replacing N-methylpiperazine with 2-methylpiperazine, the title compound is prepared.

### Example 94

### 8-(3-Amino-1-pyrrolidinyl)-9-bromo-1-cyclopropyl-7-fluoro-4H-quinolizin-4-one-3-carboxylic acid hydrochloride

Following the procedures described in Example 62, replacing 4-chloropicoline with 3-bromo-4-chloro-5-fluoropicoline (the product of Example 68) and replacing ethyl iodide with cyclopropyl iodide, the title compound is prepared.

### Example 95

### 1-Cyclopropyl-7,9-difluoro-8-(4-methylpiperazin-1-yl)-4H-quinolizin-4-one-3-carboxylic acid hydrochloride

Following the procedures described in Steps 1 and 2 of Example 62, replacing 4-chloropicoline with 4-chloro-3,5-difluoropicoline (the product of Example 69) and replacing ethyl iodide with cyclopropyl iodide, and the procedures described in Example 65, the title compound is prepared.

### Example 96

### 1-Cyclopropyl-7,9-difluoro-8-(3-methyl-1-piperazinyl)-4H-quinolizin-4-one-3-carboxylic acid hydrochloride

Following the procedures described in Steps 1 and 2 of Example 62, replacing 4-chloropicoline with 4-chloro-3,5-difluoropicoline (the product of Example 69) and replacing ethyl iodide with cyclopropyl iodide, and the procedures described in Example 65, replacing N-methylpiperazine with 2-methylpiperazine, the title compound is prepared.

### Example 97

### 8-(3-Amino-1-pyrrolindinyl)-1-cyclopropyl-7,9-difluoro-4H-quinolizin-4-one-3-carboxylic acid hydrochloride

Following the procedures described in Example 62, replacing 4-chloropicoline with 4-chloro-3,5-difluoropicoline (the product of Example 69) and replacing ethyl iodide with cyclopropyl iodide, the title compound is prepared.

### Example 98 (reference)

### 7-Fluoro-1-methylamino-8-(4-methylpiperazin-1-yl)-4H-quinolizin-4-one-3-carboxylic acid hydrochloride

### Step 1: 4-Chloro-5-fluoro-α-bromo-2-picoline

4-Chloro-5-fluoro-2-picoline (2.9 g, 20 mmol), the product of Example 66, was dissolved in 50 mL of 1,2-dichloroethane in a dry flask. The resultant solution was heated, with stirring, to 75°C and 4.09 (23 mmol) of N-bromosuccinimide was added, followed by 100 mG (0.7 mmol) of 2,2-azobisisobutyronitrile (AIBN), a free radical initiator. After the reaction mixture was stirred at 75°C for 24 h, it was diluted with 450 mL of methylene chloride and washed with 3 X 400 mL of water. The organic layer was separated and dried over anhydrous sodium sulfate, filtered and concentrated under reduced pressure. The residue was dried *in vacuo* to give 3.5 g (69% yield) of the title compound as an amber oil; ¹H NMR (ODCl₃) δ 4.50 (s, 2H), 7.54 (d, 1H), 8.44 (s, 1H).

### Step 2: 4-Chloro-5-fluoro-2-(N-methylaminomethyl)-pyridine

4-Chloro-5-fluoro-α-bromo-2-picoline (1.37 g, 6.1 mmol), from Step 1 was dissolved in 15 mL of methanol in a pressure tube. Methylamine (3 mL of 40% aqueous solution) was added to the tube and the tube was sealed. The reaction mixture was stirred at ambient temperature for 26 h and then the solvent was removed under reduced pressure. To the residue was added 50 mL of 10% aqueous sodium carbonate solution and the resultant aqueous mixture was extracted with 3 X 50 mL of methylene chloride. The organic combined extract was dried over anhydrous sodium sulfate, filtered and concentrated under reduced pressur. The residue was dried *in vacuo* to give 754 mg g (70% yield) of the title compound; MS DCl-NH₃ M/Z: 175 (M+H)⁺ base; ¹H NMR (CDCl₃) δ 2.50 (s, 3H), 3.90 (s, 2H), 7.47 (d, 1H), 8.42 (s, 1H).

### Step 3: N-(4-chloro-5-fluoro-2-pyridyl)methyl-N-methyl-N-(2,2-dimethylethyl)-formamidine

4-Chloro-5-fluoro-2-(N-methylaminomethyl)-pyridine (650 mg, 3.72 mmol), from Step 2 was dissolved in 15 mL of toluene. To the resultant solution was added 2.3 mL (15 mmol) of N,N-dimethyl-N-(2,2-dimethylethyl)-formamide, followed by 40 mg (0.3 mmol) of ammonium sulfate. The reaction mixture was heated at reflux temperature, with stirring, for 28 h and then allowed to cool to ambient temperature. The solvent was removed under reduced pressure and the residue dried in v acuo to give 560 mg (59% yield) of the title compound; MS DCl-NH₃ M/Z: 175 (M+H)⁺ 73%, 203 ((M+H)-Cl-F)⁺ base; ¹H NMR (ODCl₃) δ 1.17 (s, 3H), 1.19 (s, 9H), 2.83 (d, 2H), 4.47 (s, 1H), 7.43 (d, 1H, J=3 Hz), 8.40 (dd, 1H), J=3 Hz, 1.5 Hz).

### Step 4: Diethyl 2-ethoxy-3-(5-fluoropyridin-2-yl)-3-[N-methyl-N-(2'',2''-dimethylethyl)methylaminol-propane-1,1-dicarboxylate

Lithium diisopropylamide (LDA: 16 mL of a 1.5 M solution in hexane) is added to 8 mL of dry THF, under a nitrogen atmosphere, and the resultant solution is cooled to -70°C in a isopropyl alcohol/dry ice bath. To the cooled solution of LDA, is added dropwise, over a 30 minute period, a solution of 3.41 g (19.6 mmol) of N-(4-chloro-5-fluoro-2-pyridyl) methyl-N-methyl-N-(2,2-dimethylethyl)-formamidine, from Step 3, in 25 mL of dry THF. After stirring the solution for 0.5 h at -70°C, a solution of 4.04 mL (19.6 mmol) of ethoxymethylenemalonate in 18 mL of dry THF is added dropwise over a 30 minute period. The reaction solution turns from dark red to orange. After stirring for 0.5 h at -70°C, the reaction solution is allowed to warm to -20°C and is stirred at -20°C for 1 h. The reaction is quenched at -20°C by the addition of 1.3 mL of glacial acetic acid and the cooling bath is removed. After 20 minutes the reaction solution is poured into 5% aqueous sodium bicarbonate solution. The aqueous mixture is extracted with methylene chloride and the organic extract is dried over anhydrous sodium sulfate, filtered and concentrated under reduced pressure. The residue is purified by chromatography on a silica gel column to afford the title compound.

### Step 5: Diethyl 2-ethoxy-3-(5-fluoropyridin-2-yl)-3-methylamino-propane-1,1-

A solution of 2 mmol (0.8 g) of diethyl 2-ethoxy-3-(5-fluoropyridin-2-yl)-3-[N-methyl-N-(2'',2''-dimethylethyl)methylamino]-propane-1,1-dicarboxylate, from Step 4, 16 mmol of hydrazine and 6 mml of glacial acetic acid in 20 mL of 95% ethyl alcohol is heated at 50°C under nitrogen for approximately 15 h. Upon cooling, the solvent is removed *in vacuo* and the residue extracted with diethyl ether. The ether solution is washed with saturated aqueous sodium bicarbonate solution, dried over anhydrous sodium sulfate, and concentrated *in vacuo* to afford the title compound.

### Step 6: Ethyl 8-chloro-7-fluoro-1-methylamino-4H-quinolizin-4-one-3-carboxylate

80 mL of Dowtherm A® in a 3-neck flask equipped with a thermometer, an addition funnel and an air-cooled condenser is heated to 235°C, under nitrogen, using a heating mantel. A solution of 3.9 g (12.4 mmol) of diethyl 2-ethoxy-3-(5-fluoropyridin-2-yl)-3-methylamino-propane-1,1-dicarboxylate, from Step 5, in 45 mL of Dowtherm A® is added, dropwise over a 1.5 h period, through the addition funnel to the heated stirring Dowtherm A®. After the addition is complete, the resultant solution is heated at ∼200°C for 1 h and then is cooled to ambient temperature. The solution is then poured into 500 mL of hexane and a precipitate forms. The precipitate is collected by filtration, washed with 5 X 100 mL of hexane and dried to afford the title compound.

### Step 7: Ethyl 7-fluoro-1-methylamino-8-(4-methylpiperazin-1-yl)-4H-quinolizin-4-one-3-carboxylate

Ethyl 8-chloro-7-fluoro-1-methylamino-4H-quinolizin-4-one-3-carboxylate (899 mg, 3.0 mmol), the product of Step 6, is suspended in 12 mL of dry pyridine under a nitrogen atmosphere. To the resultant solution is added 6.0 mL (6.0 mmol) of N-methylpiperazine and the reaction mixture is heated at 70°C for 8 h. The reaction mixture is then concentrated *in vacuo* in order to remove all of the pyridine. The dry residue is dissolved in 125 mL of methylene chloride and the methylene chloride solution is washed with 125 mL of brine. The aqueous layer is extracted with 125 mL of methylene chloride and the combined methylene chloride solutions are dried over anhydrous sodium sulfate, filtered and concentrated and dried *in vacuo* to afford the title compound.

### Stop 8: 8-(4-methylpiparazin-1-yl)-4H-quinolizin-4-one-3-carboxylic acid hydrochloride

A mixture of 1 g (2.75 mmol) of ethyl 7-fluoro-1-methylamino-8-(4-methylpiperazin-1-yl)-4H-quinolizin-4-one-3-carboxylate, from Step 7, in 12 mL of THF and 16.5 mL of a 0.5 N aqueous solution of sodium hydroxide is heated, with stirring, at 75°C for 8 h. The THF is removed from the reaction mixture by distillation during the reaction. The concentrated reaction mixture is cooled to ambient temperature and adjusted to pH 2.0 with 10.5 mL of 1 N aqueous hydrochloric acid solution. The aqueous solution is concentrated *in vacuo* to remove ∼80% of the water and the concentrate is diluted with 50 mL of 95% ethyl alcohol. The solid is collected by filtration, washed with 2 X 5 mL of ethyl alcohol and dried *in vacuo* to afford the desired product.

### Examples 99-116 (reference)

By following the procedures described in Example 98 and replacing N-methylpiperazine in Step 7 with the appropriate amine as shown, Examples 99-116 are prepared as disclosed in Table 3 wherein the compounds have the general formula

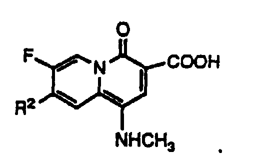

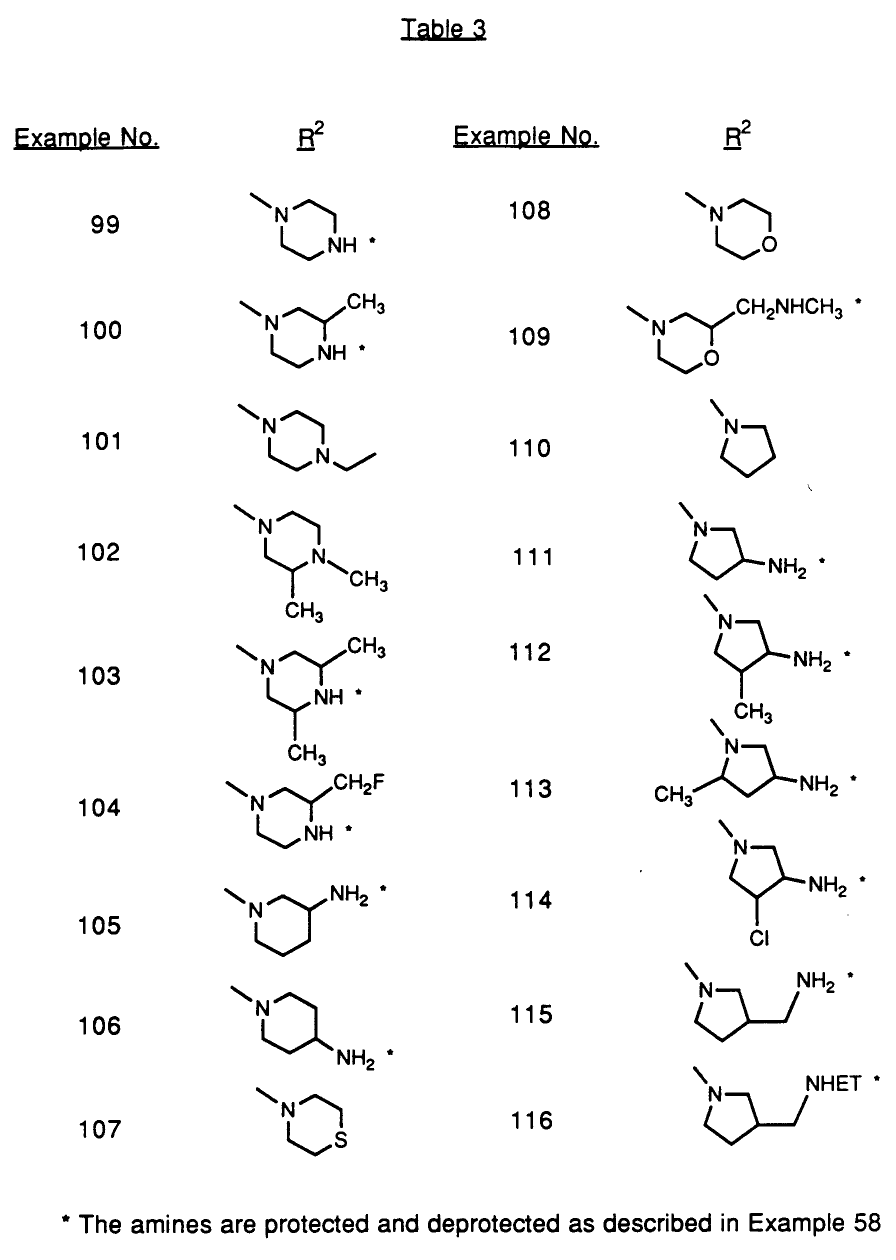

### Example 117

### 7,9-Difluoro-1-methylamino-8-(4-methylpiperazin-1-yl)-4H-quinolizin-4-one-3-carboxylic acid hydrochloride

By following the procedures described in Example 98 and replacing 4-chloro-5-fluoro-2-picoline (the product of Example 66) with 4-chloro-3,5-difluoro-2-picoline (the product of Example 69), the title compound is prepared.

### Examples 118-135

By following the procedures described in Example 98, replacing 4-chloro-5-fluoro-2-picoline (the product of Example 66) with 4-chloro-3,5-difluoro-2-picoline (the product of Example 69) and replacing N-methylpiperazine with the appropriate amine as shown, Examples 118-135 are prepared as disclosed in Table 4 wherein the compounds have the general formula

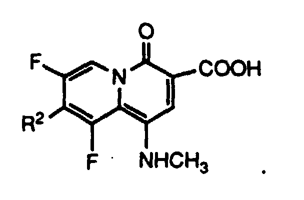

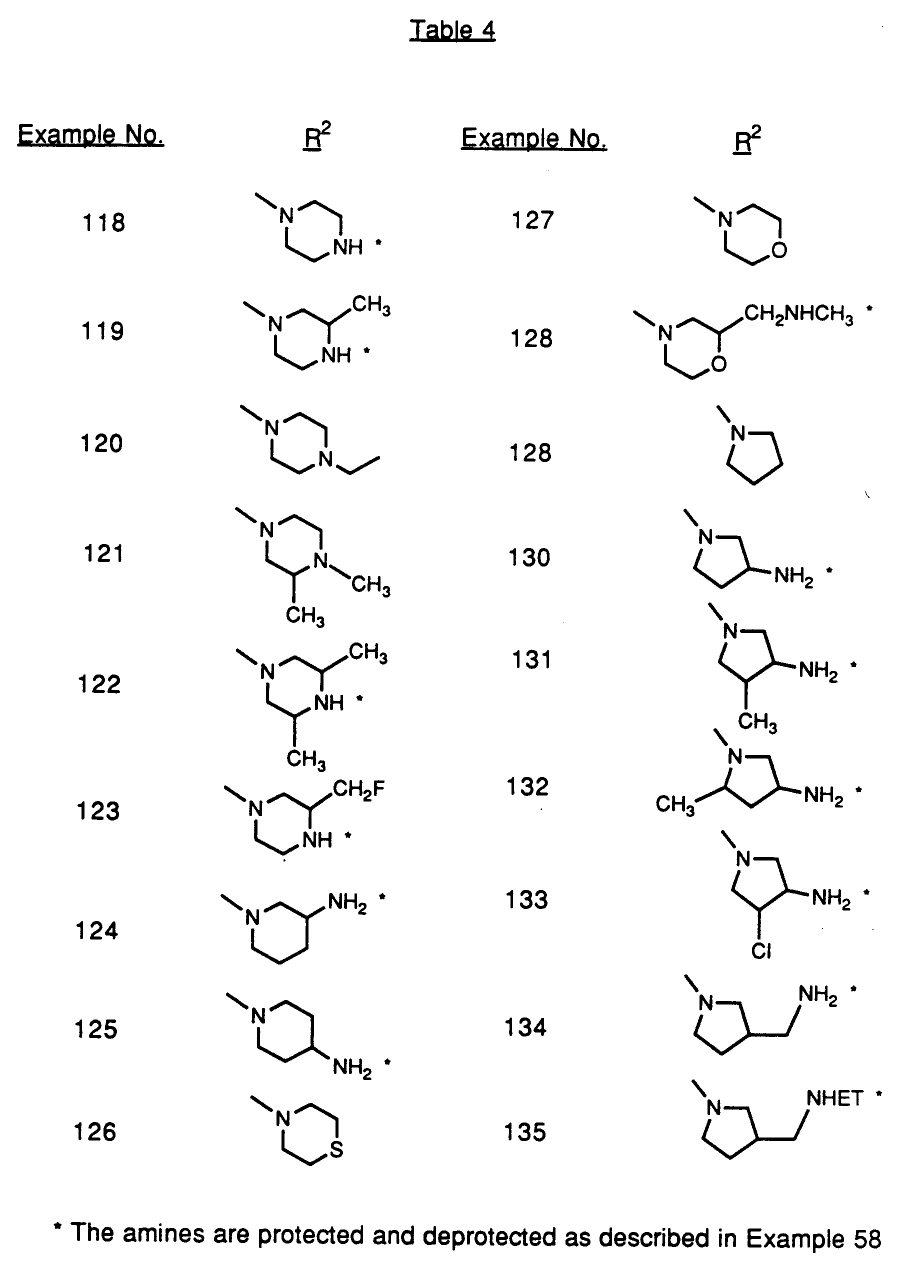

### Example 136

### 1-Ethyl-8-(4-methylpiperazin-1-yl)-6,7,9-trifluoro-4H-quinolizin-4-one-3-carboxylic acid hydrochloride

### Step 1: 3,4,5,6-Tertrafluoro-2-picoline

2,3,4,5,6-Pentafluoropyridine (commercially available from Aldich Chemical Co.) is oxidized to the corresponding N-oxide following the procedures described in Step 6 of Example 66. The 2,3,4,5,6-pentafluoropyridine N-oxide is treated at ambient temperature with one equivalent of methylmagnesium iodide in diethyl ether as described by F. Binns and H. Suschitsky in Chemical Communications, 750-751 (1970) and J Chem Soc (C), 1223-1231 (1771). The reaction mixture is treated with aqueous ammonium chloride and extracted with diethyl ether. The ether solution is dried over anhydrous magnesium sulfate, filtered and concentated under reduced pressure and the crude product is chromatographed on silica gel to afford 2-methyl-3,4,5,6-tetrafluoropyridine N-oxide (3,4,5,6-tetrafluoro-2-picoline). The N-oxide is then reduced to afford the title compound by the procedures described in Step 8 of Example 66.

### Step 2: 2-Propyl-3,4,5,6-tetrafluoropyridine

A 1.5 M solution of LDA in hexane (100 mL, 150 mmol) is cooled to -60°C in an isopropyl alcohol/dry ice bath. To the stirred LDA solution, under nitrogen, is added, dropwise over a 0.5 h period, a solution of 22.617 g (137 mmol) of 3,4,5,6-tetrafluoro-2-picoline, the product of Step 1, in 80 mL of dry THF. The reaction mixture is stirred for 0.5 h at -60°C and then a solution of 10.95 mL (137 mmol) of ethyl iodide in 30 mL of dry THF is added, dropwise over a 20 minute period. After the reaction mixture is stirred at -60°C for 0.5 h, the cooling bath is allowed to slowly (1.5 h) warm to -30°C. The reaction mixture is poured into cold brine and the aqueous mixture is extracted with methylene chloride. The organic extract is dried over anhydrous sodium sulfate, filtered and concentrated *in vacuo*. The residue is distilled to afford the title compound.

### Step 3: Diethyl 2-ethoxy-3-[3,4,5,6-tetrafluoro-2-pyridyl]-pentane-1,1-dicarboxylate

A solution of 12.6 mL (89.9 mmol) of diisopropylamine in 20 mL of anhydrous tetrahydrofuran (THF) is prepared under a nitrogen atmosphere and cooled in an ice/water bath. To this solution is added, dropwise over a 30 minute period, 36 mL of a 2.5 M solution of n-butyllithium (90 mmol) in hexane. The solution is stirred for 30 minutes at 0°C and then cooled to -60°C. To the amine solution at -60°C, is added, dropwise over a 30 minute period, a solution of 15.82 g (81.9 mmol) of 2-propyl-3,4,5,6-tetrafluoropyridine, from Step 2, in 100 mL of anhydrous THF. The resultant solution is stirred at -60°C for 0.5 h and then 16.55 mL (81.9 mmol) of ethyl 2-carboethoxy-3-ethoxy-2-propenecarboxylate is added, dropwise over a 30 minute period. Stirring is continued at -60°C for 0.5 h and at -20°C for 1.5 h. The reaction mixture is poured into cold brine and the aqueous mixture is extracted with methylene chloride. The combined organic extract is dried over anhydrous sodium sulfate, filtered and concentrated *in vacuo* to afford 35.48 g of the title compound. The product is carried on to the next step without purification.

### Step 4: Ethyl 1-ethyl-6,7,8,9-tetrafluoro-4-H-quinolizin-4-one-3-carboxylate

A solution of 40.61 g (99.2 mmol) of diethyl 2-ethoxy-3-[4-chloro-2-pyridyl]-pentane-1,1-dicarboxylate, from Step 3, in 1 L of xylene is heated at 150°C, with stirring, for 24 h and then concentrated *in vacuo*. The residue is washed with a mixture of hexane and cyclohexane to afford the title compound.

### Step 5: Ethyl 1-ethyl-8-(4-methylpiperazin-1-yl)-6,7,9-trifluoro-4H-quinolizin-4-one-3-carboxylate

Ethyl 8-chloro-1-ethyl-6,7,8,9-tetrafluoro-4H-quinolizin-4-one-3-carboxylate (317 mg, 1.0 mmol), from Step 4, is dissolved in 5 mL of dry pyridine under a nitrogen atmosphere. To the resultant solution is added 2 mL (2.0 mmol) of N-methylpiperazine and the stirred reaction mixture is heated at 85°C for 2.5 h. The reaction mixture is allowed to cool to ambient temperature and then concentrated *in vacuo* in order to remove all of the pyridine. The residue is dissolved in 50 mL of methylene chloride and the methylene chloride solution is washed with 50 mL of 5% aqueous sodium bicarbonate solution. The aqueous layer is extracted with 3 X 50 mL of methylene chloride and the combined methylene chloride solutions are dried over anhydrous sodium sulfate, filtered and concentrated and dried *in vacuo* to afford the title compound.

### Step 6: 1-Ethyl-8-(4-methylpiperazin-1-yl)-6,7,9-trifluoro-4H-quinolizin-4-one-3-carboxylic acid hydrochloride

To a solution of 199 mg (0.5 mmol) of ethyl 1-ethyl-8-(4-methylpiperazin-1-yl)-6,7,9-trifluoro-4H-quinolizin-4-one-3-carboxylate, from Step 5, in 4 mL of THF is added 4.0 mL of a 1.0 N aqueous sodium hydroxide solution and the reaction mixture is heated, with stirring, at 75°C for 4.5 h. The reaction mixture is cooled to ambient temperature and adjusted to pH 2 with 5 mL of 1 N aqueous hydrochloric acid solution. The aqueous solution is concentrated *in vacuo* to ∼5 mL and the solid is collected by filtration and dried *in vacuo* to afford the title compound.

### Example 137

### 8-(3-Amino-1-1-pyrrolidinyl)-1-ethyl-8,7,9-trifluoro-4H-quinolizin-4-one-3-carboxylic acid hydrochloride

### Step 1: Ethyl 8-(3-(N-t-butoxycarbonyl)amino-1-pyrrolidinyl)-1-ethyl-6,7,9-trifluoro-4H-quinolizin-4-one-3-carboxylate

Ethyl 6,7,8,9-tetrafluoro-1-ethyl-4H-quinolizin-4-one-3-carboxylate (1.26 g, 3.97 mmol), from Step 3 of Example 136, is dissolved in 20 mL of dry pyridine under a nitrogen atmosphere. To the resultant solution is added a solution of 1.85 g (9.92 mmol) of 3-(N-t-butoxycarbonylamino)pyrrolidine in 5 mL of dry pyridine and the reaction mixture is heated at 70°C for 4.5 h. The reaction mixture is then concentrated *in vacuo* in order to remove all of the pyridine. The dry residue (3.124 g) is purified by chromatography on silica gel to afford the title compound.

### Step 2: 8-(3-Amino-1-pyrrolidinyl)-1-ethyl-6,7,9-trifluoro-4H-quinolizin-4-one-3-carboxylic acid hydrochloride

A solution of 1.11 g (2.2 mmol) of ethyl 8-(3-(N-t-butoxycarbonyl)amino-1-pyrrolidinyl)-1-ethyl-6,7,9-trifluoro-4H-quinolizin-4-one-3-carboxylate, from Step 1, in 20 mL of trifluoroacetic acid (TFA) is stirred for 2 h at ambient temperature. The TFA is evaporated *in vacuo* and the residue is dissolved in 200 mL of methanol. To the resultant solution is added 4.5 g of strongly basic ion exchange resin and the mixture is stirred at ambient temperature for 1 h. The mixture is filtered and the filtrate is concentrated under reduced pressure to afford crude ethyl 8-(3-amino-1-pyrrolidinyl)-1-ethyl-6,7,9-trifluoro-4H-quinolizin-4-one-3-carboxylate as a residue. The residue is dissolved in 5 mL of THF and 11 mL of a 1 M aqueous solution of sodium hydroxide is added. The reaction mixture is heated at 60° for 1 h and then the reaction temperature is increased to 85°C in order to evaporate the THF. The concentrated reaction solution is diluted with 20 mL of water and the pH of the resultant solution is adjusted to 0 with concentrated hydrochloric acid. The aqueous solution is concentrated *in vacuo*. The residue is crystallized from ethyl alcohol:isopropyl alcohol:water (4:4:1 v/v/v) and recrystallized from ethyl alcohol/water to afford the title compound.

### Example 138 (reference)

### 1-Ethyl-8-(3-(N-norvalyl)amino-pyrrolidinyl)-4H-quinolizin-4-one-3-carboxylic acid

3-Amino-1-benzylpyrrolidine (I. Sumio and T. Matsuo, Japanese Kokai JP 5328161, published March 16, 1978) is coupled to N-t-butoxycarbonyl norvaline (Boc-nVal) using conventional N-hydroxysuccinimide coupling procedures. The 1-benzyl group is removed by hydrogenolysis in methanol using palladium on carbon catalyst The 3-(N-Boc-norvalyl)aminopyrrolidine is then reacted with ethyl 6,7,8,9-tetrafluoro-1-ethyl-4H-quinolizin-4-one-3-carboxylate, as described in Step 1 of Example 137, replacing 3-(N-t-butoxycarbonylamino)pyrrolidine with 3-(N-Boc-norvalyl)aminopyrrolidine, to give 1-ethyl-(3-(N-norvalyl)amino-pyrrolidinyl)-4H-quinolizin-4-one-3-carboxylic acid with the nitrogen of the amino acid protected with a Boc group. The Boc protecting group is removed by standard hydrolysis using trifluoroacetic acid and dilute aqueous hydrochloric acid.

Using the procedure outlined in Example 138, or any of the other conventional condensation methods listed above, other amino acid derivatives of compounds having an amino group can be prepared. Examples of amino acids which can be coupled, either alone or in combination with one and other, include naturally occurring amino acids such as glycine, alanine, leucine, isoleucine, methionine, phenylalanine, valine, and the like, as well as synthetic amino acids such as cyclohexylalanine, cyclohexylglycine, aminopentanoic acid, and the like.

### Examples 139-155 (reference)

By following the procedures described in Example 136 or Example 137 and replacing N-methylpiprazine or 3-(N-t-butoxycarbonylamino)pyrrolidine with the appropriate amine as shown, Examples 139-155 are prepared as disclosed in Table 5 in which the compounds have the general formula

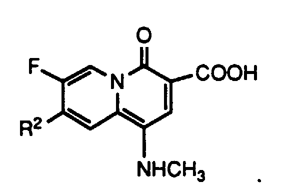

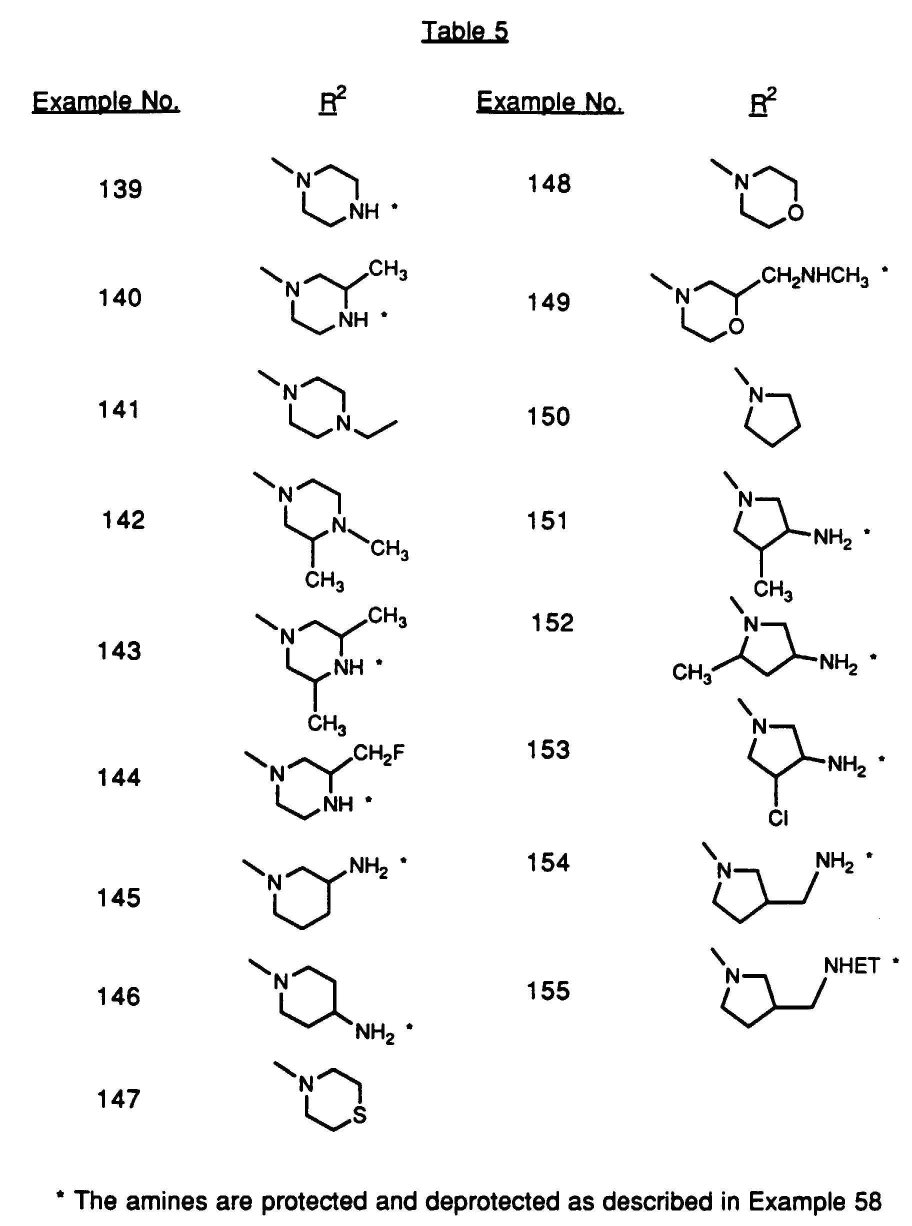

### Example 156

### 11,12-Dihydro-7-fluoro-12-methyl-8-(4-methyl-1-piperazinyl)-4H-pyrano[i,j]quinolizin-4-one-3-carboxylic acid

### Step 1: 4-Chloro-3,5-difluoro-2-(1-(2-tetrahydropyranyl)oxy-2-propyl)pyridine

A solution of 12.8 g (150 mmol) of 2-chloro-1-propanol is dissolved in 200 mL of acetone. To the resultant solution are added 40 g of anhydrous ferric chloride and 30 g (200 mmcl) of sodium iodide. The reaction mixture is stirred at room temperature for 24 h and then filtered to remove sodium chloride. The solvent is evaporated to afford the corresponding 2-iodo-1-propanol. The iodo alcohol is dissolved in 200 mL of methylene chloride and is treated with 20.5 mL (225 mmol) of 3,4-dihydro-2H-pyran and 50 mg of p-toluenesulfonic acid. The reaction mixture is stirtred at room temperature for several hours and then poured into 200 mL of 5% aqueous sodium bicarbonate solution. The aqueous mixture is extracted with methylene chloride. The methylene chloride solution is dried over anhydrous sodium sulfate, filtered and concentrated under reduced pressure to afford the THP-protected 2-iodo-1-propanol.

A solution of 4-chloro-3,5-difluoro-2-methylpyridine (16.5 g, 100 mmol) in 150 mL of dry THF under a positive nitrogen atmosphere is treated with 73 mL of 1.5 M lithium diisopropylamine (LDA) at -78°C. After stirring at -78°C for 30 minutes, a solution of 27.0 g (100 mmol) of the THP-protected 1-iodo-2-propanol in 150 mL of THF is added dropwise with stirring. The reaction mixture is stirred at -78°C for several hours and then is slowly warmed to -20°C. The reaction is quenched by pouring the reaction mixture into 400 mL of saturated aqueous ammonium chloride solution. The aqueous layer is separated and extracted with methylene chloride. The combined organic layers are dried over anhydrous sodium sulfate, filtered and concentrated under *in vacuo* to afford the title compound.

### Step 2: 4-Chloro-3,5-difluoro-2-(1-hydroxy-2-propyl)pyridine

The product of Step 1 is dissolved in 200 mL of 2:1 THF:water and to this solution is added 6 mL of acetic acid. The reaction mixture is heated at 45°C for approximately 5 h. The THF is removed under reduced pressure and the aqueous reaction mixture is adjusted to a pH in the range of 8 to 9 with 10% sodium carbonate and is then extracted with methylene chloride. The organic layer is dried over anhydrous sodium sulfate, filtered and concentrated *in vacuo* to afford the title compound.

### Step 3: 8-Chloro-3,4-dihydro-7-fluoro-3-methyl-2H-pyrano[3,2-b]pyridine

The product of Step 2 (15.5 g, 75 mmol) is dissolved in 100 mL of dry THF in an oven-dried system under positive nitrogen atmosphere. The reaction mixture is cooled in ice and 3.2 g (80 mmol) of 60% sodium hydride is added. The reaction mixture is warmed to room temperature and then heated at reflux temperature overnight with stirring. The reaction mixturte is cooled to room temperature and poured into brine. The aqueous mixture is extracted with ethyl acetate. The organic layer is dried over anhydrous magnesium sulfate, filtered and concentrated in vacuo to afford the title compound.

### Step 4: Diethyl 2-(8-chloro-3,4-dihydro-7-fluoro-3-methyl-2H-pyrano[3 2-b]pyridin-4-yl)-2-ethoxy-1,1-ethanedicarboxylate

Following the procedure described in Step 2 of Example 62, the product of Step 3 is treated with ethyl 2-carboethoxy-3-ethoxy-2-propenecarboxylate and LDA to afford the title compound.

### Step 5: Ethyl 8-chloro-11,12-dihydro-7-fluoro-12-methyl-4H-pyrano[i,j]quin-olizin-4-one-3-carboxylate

Following the procedures described in Step 3 of Example 62, the product of Step 4 is heated in refluxing Dowtherm A® to afford the desired cyclized product.

### Step 6: Ethyl 11,12-dihydro-7-fluoro-12-methyl-8-(4-methyl-1-piperazinyl)-4H-pyrano[i,j]quin-olizin-4-one-3-carboxylate

Following the procedures described in Step 1 of Example 65, the product of Step 5 is reacted with N-methylpiperazine to afford the title compound.

### Step 7: 11,12-Dihydro-7-fluoro-12-methyl-8-(4-methyl-1-piperazinyl)-4H-pyrano[i,j]quin-olizin-4-one-3-carboxylic acid

Following the procedures described in Step 2 of Example 65, the tile compound is prepared.

### Example 157

### 2-(3-Aminopyrrolidin-1-yl)-9-cyclopropyl-3-fluoro-6H-6-oxo-pyrido[1,2-a]pyrimidine-7-carboxylic acid hydrochloride salt

### Step 1. 2-Cyclopropyl-2-ethoxycarbonylacetamidine hydrochloride

Into a stirred solution of 38.72 g (0.253 mol) of ethyl 2-cyano-2-cyclopropylacetate (preparation described by R.W.J. Carney and J. Wojtkunski, *Org. Prep. Proced. Int.*, 5, 25 (1973)) in 17.7 mL (0.303 mol) of anhydrous ethanol under a dry N₂ atmosphere was introduced 10.0 g (0.274 mol) of gaseous hydrogen chloride with ice cooling. The mixture was allowed to warm to room temperature and stand for 72 hours. The reaction was diluted with 100 mL of anhydrous ethanol, 70 mL of ammonia in ethanol (4.17 M) was added slowly at room temperature and the reaction was stirred for 3 hours. The reaction mixture was filtered to remove the ammonium chloride, and the solvent was removed to afford the title compound as a viscous off-white oil, which was taken directly to the next step.

### Step 2. 2-Cyclopropyl-2-(5-fluoro-4-hydroxypyrimidin-2-yl)acetic acid methyl ester and 2-cyclopropyl-2-(5-fluoro-4-hydroxypyrimidin-2-yl)acetic acid ethyl ester

A mixture of 0.253 mol of the compound from Step 1, 0.254 mol of the sodium salt of ethyl 2-fluoro-3-hydroxy-2-propenoate (prepared as described by E. Elkik and M. Imbeaux-Oudotte, *Bull. Soc. Chim. Fr.*, 5-6 pt 2, 1165 (1975)) and 37.0 ml (0.265 mol) of triethylamine in 250 mL of anhydrous methanol was heated at reflux under a dry N₂ atmosphere for 17 hours. The solvent was removed, 200 mL of water added and the residue acidified to pH 5 with acetic acid. This mixture was then extracted with methylene chloride. The extract was washed with water, dried over anhydrous magnesium sulfate, and the solvent was removed by evaporation under vacuum to give a dark brown oil. The product was purified by column chromatography on silica gel eluting with 1:1 ethyl acetate:hexane to afford 22.8 g of the methyl ester title compound as a pale yellow viscous oil and 6.45 g of the ethyl ester title compound as a pale yellow viscous oil.
Methyl ester: MS M/Z: 227 (M+H). NMR (CDCl₃): δ 0.43 (1H, m), 0.52 (1H, m), 0.65 (1H, m), 0.77 (1H, m), 1.42 (1H, m), 2.97 (1H, d, J=10 Hz), 3.80 (3H, s), 7.88 (1H, d, J=3 Hz), 11.8 (1H, b). IR: (neat) 1740, 1690, 1615 cm⁻¹. Analysis calculated for C₁₀H₁₁FN₂O₃·1/4 H₂O: C, 52.06; H, 5.02; N, 12.14. Found: C, 52.45; H, 4.94; N, 11.76.
Ethyl ester: MS M/Z: 258 (M+NH₄). NMR (CDCl₃): δ 0.47 (1H, m), 0.54 (1H, m), 0.66 (1H, m), 0.74 (1H, m), 1.31 (3H, t, J=7 Hz), 1.34 (1H, m), 2.96 (1H, d, J=10 Hz), 4.27 (2H, m), 7.83 (1H, d, J=3 Hz), 11.0 (1H, b): IR: (neat) 1735, 1682, 1605 cm⁻¹.
Analysis calculated for C₁₁H₁₃FN₂O₃·0.3 H₂O: C, 53.78 H, 5.58; N, 11.40.
Found: C, 54.05; H, 5.59; N, 11.11.

### Step 3. 2-Cyclopropyl-2-(5-fluoro-4-hydroxypyrimidin-2-yl)acetaldehyde

To a solution of 4.960 g (21.9 mmol) of the methyl ester compound from Step 2 in 40 mL of toluene stirred at -70°C under a dry N₂ atmosphere was added 46.0 mL of 1N diisobutylaluminum hydride in toluene (46 mmol). The reaction was stirred for 40 min and then quenched by the addition of 5 mL of acetic acid. The mixture was allowed to warm to room temperature, and the reaction was extracted with ethyl acetate. The extract was washed with water (3x), dried over anhydrous magnesium sulfate and concentrated under vacuum to afford 2.230 g of the title compound as a white solid. This compound was used directly in the next step. MS M/Z 214 (M+NH₄). NMR:(CDCl₃) δ 0.48 (m, 2H), 0.91 (m, 2H), 1.35 (m, 1H0, 7.40 (d, 1H, J=10 Hz), 7.75 (d, 1H, J=4 Hz), 9.61 (br s, 1H), 13.64 (d, 1H, J=10 Hz). IR (KBr) 1695, 1660, 1635 cm⁻¹.

### Step 4. 9-Cyclopropyl-3-fluoro-2-hydroxy-6H-6-oxo-pyrido[1,2-a]pyrimidine-7-carboxylic acid benzyl ester

A 2.230 g (11.37 mmol) sample of the compound from Step 3 was dissolved in 100 mL of anhydrous ethanol. To this was added 3.5 mL (14.00 mmol) of dibenzyl malonate, 2.5 mL of piperidine and 0.25 mL of acetic acid. This reaction mixture under a dry N₂ atmosphere was heated under reflux conditions for 3 hours and stirred at room temperature overnight. The solvent was removed by evaporation, the residue was dissolved in methylene chloride which was washed with water and dried over anhydrous magnesium sulfate. The solvent was removed by evaporation under vacuum to give a yellow oil, which was purified by column chromatography on silica gel, eluting with 1:5:100 acetic acid:methanol:methylene chloride. Removal of the solvent afforded 1.800 g of the title compound as a pale yellow solid, mp 225.5-226.5°C. MS M/Z 355 (M+H). NMR:(CDCl₃) δ 0.64 (m, 2H), 1.08 (m, 2H) 1.62 (m, 1H), 5.37 (s, 2H), 7.35-7.48 (m, 5H), 8.28 (s, 1H), 9.00 (d, 1H, J=6 Hz). IR (KBr) 1720, 1700, 1690 cm⁻¹.
Analysis calculated for C₁₉H₁₅FN₂O₄·1/4 H₂O: C, 63.60; H, 4.35; N, 7.81.
Found: C, 63.54; H, 4.08; N, 7.78.

### Step 5. 2-Chloro-9-cyclopropyl-3-fluoro-6H-6-oxo-pyrido[1,2-a]pyrimidine-7-carboxylic acid benzyl ester

A mixture of 0.200 g (0.564 mmol) of the compound from Step 4, 0.50 mL of DMF, 0.60 mL of phosphorous oxychloride and 10 mL of methylene chloride was stirred under a dry N₂ atmosphere at room temperature for 4 hours. Ice was added to react with the excess phosphorous oxychloride. The mixture was extracted with methylene chloride, which was washed with water, then the solvent was dried over anhydrous magnesium sulfate and the solvent was removed by evaporation under vacuum to yield the title compound as an orange residue. This compound was taken directly to the next step.

### Step 6. 2-(3-(N-t-butoxycarbonyl)aminopyrrolidin-1-yl)-9-cyclopropyl-3-fluoro-6H-6-oxo-pyrido[1,2-a]pyrimidine-7-carboxylic acid benzyl ester

The 0.564 mmol sample of the compound from the previous step was dissolved in 5 mL of dry methylene chloride and cooled to 0°C. To this solution was added 0.45 g of 3-(N-*t*-butoxycarbonyl)aminopyrrolidine, and the reaction mixture was stirred at room temperature overnight. The solvent was removed by evaporation under vacuum, and the product was purified by column chromatography on silica gel, eluting with 10% methanol in methylene chloride to afford 0.295 g of the title compound as a yellow solid, mp 159-160°C. MS M/Z 523 (M+H). NMR:(CDCl₃) δ 0.60 (m, 2H), 0.87 (m, 2H), 1.46 (s, 9H), 1.90-2.40 (m, 2H), 3.70-4.45 (m, 5H), 4.94 (br s, 1H), 5.37 (s, 2H), 7.29 (m, 1H), 7.37 (m, 2H), 7.50 (m, 2H), 7.99 (br s, 1H), 9.10 (d 1H, J=10 Hz). IR (KBr) 1715, 1685, 1660 cm⁻¹.
Analysis calculated for C₂₈H₃₁FN4O₅·1/2 H₂O: C, 63.44; H, 6.08; N, 10.57.
Found: C, 63.39; H, 6.13; N, 10.83.

### Step 7. 2-(3-(N-t-butoxycarbonyl)aminopyrrolidin-1-yl)-9-cyclopropyl-3-fluoro-6H-6-oxo-pyrido[1,2-a]pyrimidine-7-carboxylic acid

To a 0.135 g (0.259 mmol) sample of the benzyl ester from Step 6 in 20 mL of methanol and 2 mL of THF was added 2.0 mL of 98% formic acid and 0.05 g of 10% Pd/C. This mixture was stirred under a dry N₂ atmosphere at room temperature for 37 min. The catalyst was removed by filtration, and the solvent was removed under vacuum. The crude product was purified by column chromatography on silica gel, eluting with 1:5:100 acetic acid:methanol:methylene chloride to afford the title compound as a yellow solid after removal of the solvent. This product was taken directly to the next step.

### Step 8. 2-(3-Aminopyrrolidin-1-yl)-9-cyclopropyl-3-fluoro-6H-6-oxo-pyridol[1,2-a]pyrimidine-7-carboxylic acid hydrochloride salt

The sample of the compound from the previous step was reacted with 10 mL of 4N HCl in dioxane under a dry N₂ atmosphere at room temperature 3 hours. The solvent was removed, the yellow solid was dissolved in distilled water. The yellow solution was filtered and freeze-dried to afford 0.0681 g of the title compound as a yellow solid, mp 234°C, (dec.). MS M/Z 333 (M-Cl). NMR: (CDCl₃) δ 0.64 (m, 2H), 0.96 (m, 2H), 2.20-2.65 (m, 3H), 3.58-4.35 (m, 5H), 7.80 (d, 1H, J=10 Hz), 9.05 (br s, 1H), IR (KBr) 1665, 1620 cm⁻¹.

### Example 158

### 2-(3-Aminopyrrolidin-1-yl)-9-cyclopropyl-3-fluoro-6H-6-oxo-pyrido[1,2-a]pyrimidine-7-carboxylic acid

### Step 1 9-Cyclopropyl-3-fluoro-2-hydroxy-6H-6-oxo-pyrido[1,2-a]pyrimidine-7-carboxylic acid t-butyl ester

A 0.247 g (1.262 mmol) sample of 2-cyclopropyl-2-(5-fluoro-4-hydroxypyrimidin-2-yl)acetaldehyde, from Example 157 Step 3 above, was dissolved in 20 mL of ethanol, and 0.290 mL of ethyl *t*-butyl malonate, 0.5 mL of piperidine and 0.05 mL of acetic acid were added. The reaction was heated under a dry N₂ atmosphere at reflux for 25 hours, the solvents were removed by evaporation and the product was purified by column chromatography on silica gel, eluting with 1:10:100 acetic acid:methanol:methylene chloride. Removal of the solvent afforded 0.287 g of the title compound as a pale yellow solid, mp >265°C. MS M/Z 321 (M+H). NMR: (CDCl₃ + CD₃OD) δ 0.61 (m, 2H), 1.06 (m, 2H), 1.58 (s, 9H), 1.72 (m, 1H), 8.07 (s, 1H), 8.93 (d, 1H, J=6 Hz). IR (KBr)1720, 1525 cm-1.

### Step 2. 2-Chloro-9-cyclopropyl-3-fluoro-6H-6-oxo-pyrido[1,2-a]pyrimidine-7-carboxylic acid t-butyl ester

A mixture of 0.100 g (0.312 mmol) of the compound from Step 1, 0.29 mL of DMF, 0.33 mL of phosphorous oxychloride and 10 mL of methylene chloride was stirred under a dry N₂ atmosphere at room temperature for 1 hour. After workup as described in Example 157 Step 5, the title compound was obtained as a orange solution in methylene chloride. This compound was taken directly to the next step.

### Step 3. 2-(3-(N-t-butoxycarbonyl)aminopyrrolidin-1-yl)-9-cyclopropyl-3-fluoro-6H-6-oxo-pyrido[1,2-a]pyrimidine-7-carboxylic acid t-butyl ester

To the 0.312 mmol sample in methylene chloride from the previous step at room temperature was added several small portions of 3-(N-*t*-butoxycarbonyl)aminopyrrolidine until the color of the reaction turned from orange to light yellow. The solution was concentrated to leave a yellow residue. The product was purified by column chromatography on silica gel, eluting with 10:100 methanol: methylene chloride to afford 0.132g of the title compound as a yellow solid after removal of the solvent. This compound was taken directly to the next step.

### Step 4. 2-(3-aminopyrrolidin-1-yl)-9-cyclopropyl-3-fluoro-6H-6-oxo-pyrido[1,2-a]pyrimidine-7-carboxylic acid

The boc-protected *t*-butyl ester from Step 4 was hydrolyzed by reacting the 0.132 g sample with 1 mL of 4N HCl in dioxane under a dry N₂ atmosphere. The solvent was removed, the yellow solid was dissolved in water and the solution adjusted to pH 7-8, and extracted with methylene chloride. The reaction was incomplete at this point, so the solid was redissolved in 5 mL of trifluoroacetic acid and the reaction stirred at room temperature overnight. The solvent was removed by evaporation. The residue was redissolved and extracted as above, then the product was purified by column chromatography on silica gel, eluting with 2:5:20:100 water:acetic acid:methanol:methylene chloride to afford 0.0515 g of the title compound as a yellow solid.

### Example 159

### 9-(2,4-Difluorophenyl)-3-fluoro-2-(4-methylpiperazin-1-yl)-6H-6-oxopyridol[1,2-a]pyrimidine-7-carboxylic acid

### Step 1. 2-(2,4-Difluorophenyl)-acetamidine hydrochloride

Into a solution of 49.44 g (0.323 mol) of 2,4-difluorophenylacetonitrile (commercially available) in 20.8 mL (0.354 mol) of ethanol cooled to 0°C in an ice bath and stirred under a dry N₂ atmosphere was added 14.61 g (0.400 mol) of gaseous HCl. After 20 min the reaction mixture solidified, this was then allowed to warm to room temperature and held at this temperature for 72 hours. To the mixture was then added 140 mL of ethanol, followed by 150 mL (0.42 mol) of 4.2 M ammonia in ethanol. This mixture was stirred for an additional 3 hours at room temperature and filtered. The solvent was removed from the filtrate by evaporation to afford 65.7 g of the title compound as a white solid, mp 163-164°C. NMR: (DMSO-d6) δ 3.72 (s, 2H), 7.16 (m, 1H), 7.33 (m, 1H), 7.50 (m, 1H), 8.95 (broad, 4H). This compound was taken directly to the next step.

### Step 2. 2-(2,4-Difluorobenzyl)-5-fluoro-4-hydroxypyrimidine

A mixture of 68.0 g (0.33 mol) of the compound from Step 1, 0.34 mol of the sodium salt of ethyl 2-fluoro-3-hydroxy-2-propenoate (prepared as described by E. Elkik and M. Imbeaux-Oudotte, *Bull Soc. Chim. Fr.*, 5-6 pt 2, 1165 (1975)), 300 mL of anhydrous methanol and 50 mL of triethylamine was heated at reflux under a dry N₂ atmosphere for 23 hours. The solvent was removed by evaporation under vacuum, 200 mL of water added and the mixture acidified to pH 3-4 with 10% HCl. This mixture was then extracted with methylene chloride. The solvent was washed with water, dried over anhydrous magnesium sulfate, and the solvent was removed by evaporation under vacuum to give a dark oil which solidified upon standing. The solid was washed with ethyl acetate, ethyl acetate/hexane and hexane to afford 29.8 g of the title compound as a white solid, mp 155-156°C. A second crop of 10.2 g of product was obtained from the filtrates after chromatography on silica gel, eluting with 2.5% methanol in methylene chloride. MS M/Z: 258 (M=NH₄), 241 (M+H). NMR: (CDCl₃) δ 4.02 (s, 2H), 6.88 (m, 2H), 7.33 (m, 1H), 7.89 (d, 1H, J=3 Hz). IR (KBr): 1690, 1605 cm⁻¹. Analysis calculated for C₁₁H₇F₃N₂O: C, 55.00; H, 2.94; N, 11.67. Found: C, 54.63; H, 2.98; N, 11.50.

### Step 3. 4-Chloro-2-(2,4-difluorobenzyl)-5-fluoropyrimidine

A mixture of 1.000 g (4.16 mmol) of the compound from Step 2, 3.40 mL (43.7 mmol) of DMF and 3.90 mL (43.7 mmol) of phosphorous oxychloride in 15 mL of methylene chloride was stirred under a dry N₂ atmosphere at ambient temperature for 2 hours, then quenched with water and ice. The mixture was then extracted with methylene chloride, which was washed with water, dried, filtered and concentrated to yield the title compound as a yellow oil. MS M/Z: 259 (M+H). NMR: (CDCl₃) δ 4.27 (s, 2H), 6.83 (m, 2H), 7.27 (m, 1H), 8.48 (s, 1H). This compound was taken directly to the next step.

### Step 4. 2-(2,4-Difluorobenzyl)-5-fluoro-4-(4-methylpiperazin-1-yl)pyrimidine

To the 4.16 mmol of the compound from Step 3 in 10 mL of methylene chloride was added 3 mL of N-methylpiperidine and the mixture was stirred under a dry N₂ atmosphere at room temperature for 1 hour. The solvent was removed by evaporation, and the product was purified by column chromatography on silica gel eluting with 5% methanol in methylene chloride. The solvent was removed by evaporation to afford 1.229 g of the title compound as a pale yellow oil, MS M/Z: 323 (M+H). NMR: (CDCl₃) δ 2.32 (s, 3H), 2.46 (t, 4H, J+7 Hz), 3.75 (t, 4H, J=7 Hz), 4.05 (s, 2H), 6.80 (m, 2H), 7.25 (m, 1H), 7.99 (d, 1H, J=7 Hz). Analysis calculated for C₁₆H₁₇F₃N₄: C, 59.61; H, 5.32; N, 17.38. Found: C, 59.63; H, 5.31; N, 17.31.

### Step 5. 3-(2,4-Difluorophenyl)-2-ethoxy-3-(5-fluoro-4-(4-methylpiperidin-1-yl)pyrimidin-2-yl)propane-1,1-dicarboxylic acid diethyl ester

Following the procedure of Step 4 Example 1 the compound from Step 4 above (0.74 g, 2.3 mmol), 1.0 mL (2.5 mmol) of a 2.5 M solution of n-butyllithiuin in hexane and 0.35 mL of diisopropylamine was reacted with 0.46 mL ethyl 2-carboethoxy-3-ethoxy-2-propenecarboxylate, to afford after work-up 1.22 g of the title compound as an oil. This material was further purified by column chromatography over silica gel, eluting with 5% ethanol in ethyl acetate to give 0.774 g of an oil; MS M/Z: 539 (M+H). NMR: (CDCl₃) δ 0.87 (m, 3H), 1.22 (m, 6H), 2.34 (s, 3H), 2.50 (m, 4H), 3.52 (m, 2H), 3.81 (m, 4H), 4.16 (m, 5H), 4.82 (m, 1H), 4.99 (m, 1H), 6.78 (m, 2H), 7.59 (m, 1H), 8.01 (m, 1H).

### Step 6. 9-(2,4-Difluorophenyl)-3-fluoro-2-(4-methylpiperazin-1-yl)-6H-6-oxopyrido[1,2-a]pyrimidine-7-carboxylic acid ethyl ester

To a 1.847 g (3.43 mmol) sample of the compound from Step 5 dissolved in 40 mL of anhydrous ethanol was added 1.5 mL of piperidine and 0.05 mL of acetic acid, and the reaction was heated at reflux conditions under a dry N₂ atmosphere for 3 hours. The solvent was removed by evaporation to leave a yellow solid which was purified by column chromatography over silica gel, eluting with 0.5:10:100 28% aq. NH₄OH:methanol:methylene chloride to afford after removal of the solvent 1.282 g of the title compound as a yellow solid, mp 193-195°C. MS M/Z: 447 (M+H). NMR: (CDCl₃) δ 1.40 (t, 3H, J=7 Hz), 2.33 (s, 3H), 2.50 (m, 4H), 3.89 (m, 4H), 4.39 (q, 2H, J=7 Hz), 6.91 (m, 2H), 7.33 (m, 1H), 8.37 (s, 1H), 9.16 (d, 1H, J=10 Hz). IR (KBr): 1725, 1685, 1660 cm⁻¹. Analysis calculated for C₂₂H₂₁F₃N₄O₃·0.5 H₂O: C, 58.02; H, 4.87; N, 12.30. Found: C, 58.15; H, 4.70; N, 12.15.

### Step 7. 9-(2,4-Difluorophenyl)-3-fluoro-2-(4-methylpiperazin-1-yl)-6H-6-oxopyrido[1,2-a]pyrimidine-7-carboxylic acid benzyl ester

A mixture of a 1.166 g (2.61 mmol) sample of the ethyl ester compound from Step 1, 150 mL of dry benzyl alcohol and 0.5 mL of titanium tetramethoxide was heated under a dry N₂ atmosphere with stirring at reflux conditions for 17 hours. The solvent was removed by distillation at 100°C under reduced pressure in a kugelrohr apparatus. The product was purified by column chromatography on silica gel, eluting with 0.5:10:100 28% aq. NH₄OH:methanol:methylene chloride to afford after removal of the solvent 0.895 g of the title compound as a yellow solid, mp 207-208°C. MS M/Z: 509 (M+H). NMR: (CDCl₃) δ 2.33 (s, 3H), 2.50 (m, 4H), 3.88 (m, 4H), 5.38 (s, 2H), 6.90 (m, 2H), 7.30-7.50 (m, 6H), 8.37 (s, 1H), 9.17 (d, 1H, J=10 Hz). IR (KBr): 1730, 1685, 1660 cm⁻¹.

### Step 8. 9-(2,4-Difluorophenyl)-3-fluoro-2-(4-methylpiperazin-1-yl)-6H-6-oxopyrido[1,2-a]pyrimidine-7-carboxylic acid

A 0.300 g (0.590 mmol) sample of the benzyl ester from Step 7 was dissolved in 40 mL of dry methanol and 0.1 g of 10% Palladium on carbon was added. Four mL of 98% formic acid was added and the mixture stirred under a dry N₂ atmosphere for 20 min. The catalyst was removed by filtration through diatomaceous earth, and the solvent was removed under vacuum. The product was purified by column chromatography on silica gel, eluting with 1:10:100 acetic acid:methanol:methylene chloride give a yellow solid. This material was washed with pH 7.5 sodium bicarbonate solution, followed by water rinse to afford 0.178 g of the title compound as a yellow solid, mp 246-248°C (dec.). MS M/Z: 419 (M+H). NMR: (CDCl₃ + CD₃OD) δ 2.34 (s, 3H), 2.53 (m, 4H), 3.85-4.00 (m, 4H), 6.90 (m, 2H), 7.32 (m, 1H), 8.49 (s, 1H), 9.07 (d, 1H, J=9 Hz). IR (KBr): 1720, 1660 cm⁻¹. Analysis calculated for C₂₀H₁₇F₃N₄O₃: C, 57.42; H, 4.10; N, 13.39. Found: C, 57.21; H, 4.08; N, 13.21.

### Example 160

### 2-(3-(N-t-butoxycarbonyl)aminopyrrolidin-1-yl)-9-(2,4-difluorophenyl)-3-fluoro-6H-6-oxopyrido[1,2-a]pyrimidine-7-carboxylic acid

### Step 1. 3-(2,4-Difluorophenyl)-2-ethoxy-3-(5-fluoro-4-hydroxypyrimidin-2-yl)propane-1,1-dicarboxylic acid diethyl ester

A 4.804 g (20.0 mmol) sample of 2-(2,4-Difluorobenzyl)-5-fluoro-4-hydroxypyrimidine (prepared as described in Step 2 Example 159 above) was dissolved in 150 mL of dry THF and cooled to -78°C with stirring under a dry N₂ atmosphere. To this was slowly added 16.40 mL of 2.5 N n-butyllithium in hexane, and the mixture was stirred for 30 min. Then 4.85 mL (24 mmol) of diethyl ethoxymethylenemalonate was added and the mixture stirred for an additional 30 min at -78°C. The reaction mixture was quenched with 10% HCl until the mixture was at pH 3, whereupon it was then extracted with ethyl acetate. This was dried over anhydrous magnesium sulfate and the solvent was removed by evaporation under vacuum to afford the title compound as a yellow oil. This material was taken directly to the next step.

### Step 2. 9-(2,4-Difluorophenyl)-3-fluoro-2-hydroxy-6H-6-oxopyrido[1,2-a]pyrimidine-7-carboxylic acid ethyl ester

The compound from Step 1 was dissolved in 80 ml of ethanol, 2 mL of piperidine and 0.2 mL of acetic acid was added and the mixture heated at reflux (bath temperature at 90°C) for 16 hours under a dry N₂ atmosphere. The solvent was removed by evaporation, and the residue was washed with methanol and methylene chloride to give 4.794 g of a pale yellow solid. The washings were concentrated and the residue was purified by column chromatography on silica gel, eluting with 2:10:100 acetic acid:methanol:methylene chloride to afford an additional 2.220 g of the title compound as a pale yellow solid, mp 239-240°C. MS M/Z: 382 (M+NH₄), 365 (M+H). NMR:(DMSO-d₆) δ 1.23 (t, 3H, J=7 Hz), 4.14 (q, 2H, J=7 Hz), 7.08 (m, 1H), 7.21 (m, 1H), 7.40 (m, 1H), 7.83 (s, 1H), 8.74 (d, 1H, J=8 Hz). IR (KBr) 1710, 1675, 1620 cm⁻¹.

### Step 3. 9-(2,4-Difluorophenyl)-3-fluoro-2-hydroxy-6H-6-oxopyrido[1,2-a]pyrimidine-7-carboxylic acid benzyl ester

To a 7.000 g sample of the ethyl ester compound from Step 2 dissolved in 200 mL of benzyl alcohol was added 0.70 mL of titanium tetraethoxide and the mixture heated with stirring at 100°C for 2.5 hours under a dry N₂ atmosphere. The reaction was diluted with methylene chloride, then washed once with 1 N HCl and three times with water, and the solvent was dried over anhydrous magnesium sulfate and removed by evaporation under vacuum to leave a yellow solid. This material was washed with ether and dried under vacuum to afford 6.655 g of the title compound as a yellow solid, mp 218-219°C. MS M/Z 427 (M+H). NMR:(DMSO-d₆) δ 5.26 (s, 2H), 7.15-7.45 (m, 8H), 8.00 (s, 1H), 9.00 (d, 1H, J=7 Hz). IR (KBr) 1710, 1675, 1620 cm⁻¹.

### Step 4. 2-(3-(N-t-butoxycarbonyl)aminopyrrolidin-1-yl)-9-(2,4-difluorophenyl)-3-fluoro-6H-6-oxopyrido[1,2-a]pyrimidine-7-carboxylic acid benzyl ester

A 1.200 g (2.815 mmol) sample of the compound from Step 3 was dissolved in 45 mL of methylene chloride and 2.50 mL of DMF and 2.95 mL of POCl₃ were added. The reaction was stirred under a dry N₂ atmosphere at room temperature for 2.5 hours, then quenched with ice and water. The mixture was extracted with methylene chloride, and the solvent was washed with water until the acidity of the rinse water was above pH 3. The solvent was then dried with magnesium sulfate and an excess of 2-(N-*t*-butoxycarbonylamino)pyrrolidine was added and allowed to react. The solution was then concentrated and the product was purified by column chromatography over silica gel eluting with 0.5:5:100 conc. ammonium hydroxide:methanol:methylene chloride. The solvent was removed to afford 1.579 g of the title compound as a light yellow crystalline solid, mp 103-104°C. MS M/Z: 595 (M+H). NMR: (CDCl₃) δ 1.45 (s, 9H), 1.85-2.30 (m, 2H), 3.42-4.35 (m, 5H), 4.65 (br s, 1H), 5.38 (s, 2H), 6.89 (m, 2H), 7.30-7.50 (m, 6H), 8.35 (s, 1H), 9.15 (d, 1H, J=9 Hz), 9.16 (d, 1H, J=9 Hz). IR (KBr): 1735, 1710, 1660 cm⁻¹.

### Step 5. 2-(3-(N-t-butoxycarbonyl)aminopyrrolidin-1-yl)-9-(2,4-difluorophenyl)-3-fluoro-6H-8-oxopyrido[1,2-a]pyrimidine-7-carboxylic acid

A 1.769 g sample of the compound from Example 160 Step 4 was dissolved in 80 mL of dry methanol, and the benzyl ester was removed by reacting with 4.0 mL of 98% formic acid in the presence of 0.200 g of 10% Pd/C under a dry N₂ atmosphere. After filtration and evaporation of the solvent, the product was purified by column chromatography on silica gel, eluting with 1:10:100 acetic acid:methanol:methylene chloride to afford, after removal of the solvent, 1.125 g of the title compound as a yellow solid, mp 209.5-210.5°C. MS M/Z: 505 (M+H). NMR: (CDCl₃/CD₃OH) δ 1.45 (s, 9H), 1.90-2.30 (m, 2H), 3.50-4.35 (m, 5H), 6.91 (m, 2H), 7.32 (m, 1H), 8.44 (s, 1H), 9.03 (d, 1H, J=8 Hz), 9.04 (d, 1H, J=8 Hz). IR (KBr): 1714, 1662, 1620 cm⁻¹.

### Example 161

### 2-(3-Aminopyrrolidin-1-yl)-9-(2,4-difluorophenyl)-3-fluoro-6H-6-oxopyrido[1,2-a]pyrimidine-7-carboxylic acid

A 0.100 g, (0.198 mmol) sample of 2-(3-(N-*t*-butoxycarbonyl)-aminopyrrolidin-1-yl)-9-(2,4-difluorophenyl)-3-fluoro-6H-6-oxopyrido[1,2-a]pyrimidine-7-carboxylic acid , from Example 160 Step 5, was dissolved in a small volume of 4 N HCl in dioxane and stirred at room temperature for 3 hours under a dry N₂ atmosphere. The solvent was removed by evaporation under vacuum to yield a yellow solid, which was dissolved in water and neutralized to pH 7 with 5% sodium bicarbonate solution,. The resulting precipitate was filtered off, washed with water and dried to afford 0.075 g of the title compound as a yellow solid, mp >250°C. MS M/Z: 405 (M+H). NMR: (DMSO) δ 1.90-2.30 (m, 2H), 3.00-4.10 (m, 5H), 7.16 (m, 2H), 7.30 (m, 1H), 8.18 (s, 1H), 9.17 (d, 1H, J=8 Hz), 9.18 (d, 1H, J=8 Hz). IR (KBr): 1715, 1660 cm⁻¹. Analysis calculated for C₁₉H₁₅F₃N₄O₃·1.25 H₂O: C, 53.46; H, 4.07; N, 13.12. Found: C, 53.64; H, 3.70; N, 12.80.

### 2-(3-Aminopyrrolidin-1-yl)-9-(2,4-difluorophenyl)-3-fluoro-6H-6-oxopyrido[1,2-a]pyrimidine-7-carboxylic acid trifluoroacetic acid salt

A 0.879 g (2.174 mmol) sample of 2-(3-aminopyrrolidin-1-yl)-9-(2,4-difluorophenyl)-3-fluoro-6H-6-oxopyrido[1,2-a]pyrimidine-7-carboxylic acid, from Example 161, was dissolved in 10 mL of trifluoroacetic acid, then the excess acid was removed by evaporation under vacuum. The yellow residue was dissolved in 600 mL of water with containing 1 mL of trifluoroacetic acid, the solution was filtered through sintered glass and freeze dried to afford 0.876 g of the title compound as a light yellow solid: mp 191-192°C (dec.);. MS M/Z: 405 (M+H). NMR (CD₃OH): δ 2.102.55 (m, 2H), 3.75-4.20 (m, 5H), 7.05 (m, 2H), 7.50 (m, 1H), 8.30 (s, 1H), 9.19 (d, 1H), J=8 Hz). IR (KBr): 1720, 1660, 1620 cm⁻¹. Analysis calculated for C₂₁H₁₆F₆N₄O₅·H₂O: C, 47.02; H, 3.38; N, 10.45. Found: C, 47.36; H, 3.07; N, 10.36.

### Example 163

### 9-Cyclopropyl-3-fluoro-2-(4-methylpiperazin-1-yl)-6H-6-oxo-pyrido[1,2-a]pyrimidine-7-carboxylic acid

### Step 1. 2-Chloro-9-cyclopropyl-3-fluoro-6H-6-oxo-pyrido[1,2-a]pyrimidine-7-carboxylic acid benzyl ester

To a 0.100 g (0.282 mmol) sample of 9-cyclopropyl-3-fluoro-2-hydroxy-6H-6-oxo-pyrido[1,2-a]pyrimidine-7-carboxylic acid benzyl ester, prepared as described in Example 157 Step 4, was added 5 mL of methylene chloride, 0.275 mL of DMF and 0.33 mL of phosphorous oxychloride, and the reaction was stirred 5 hours at room temperature under a dry N₂ atmosphere. The solution was cooled to 0°C, and ice was added to destroy the excess phosphorous oxychloride. This mixture was then extracted with methylene chloride which was dried over anhydrous magnesium sulfate The solvent was removed by evaporation under vacuum to afford the title compound as an orange solid. NMR (CDCl₃): δ 4.27 (s, 2H), 6.83 (m, 2H), 7.27 (m, 2H), 8.48 (s, 1H). This material was taken directly to the next step.

### Step 2. 9-Cyclopropyl-3-fluoro-2-(4-methylpiperazin-1-yl)-6H-6-oxo-pyrido[1,2-a]pyrimidine-7-carboxylic acid benzyl ester

The compound from the previous step was dissolved in 2.5 mL of methylene chloride and 0.5 mL of N-methylpiperazine was added with cooling. The reaction was stirred at room temperature overnight. The solvent was removed by evaporation and the product was purified by column chromatography on silica gel, eluting with 10% methanol in methylene chloride. The solvent was removed to afford 0.107 g of the title compound as a yellow solid. Recrystallization from methanol gave yellow needles, mp 194-195°C. MS M/Z 437 (M+H). NMR:(CDCl₃) δ 0.62 (m, 2H), 0.88 (m, 2H), 2.12 (m, 1H), 2.57 (s, 3H), 2.59 (t, 4H, J=7 Hz), 4.07 (t, 4H, J=7 Hz), 5.38 (s, 2H), 7.28 (m, 1H), 7.36 (m, 2H), 7.51 (m, 2H), 8.04 (s, 1H), 9.16 (d, 1H, J=10 Hz). IR (KBr): 1715, 1685, 1660 cm⁻¹. Analysis calculated for C₂₄H₂₅FN₄O₃·1/4 H₂O: C, 65.37; H, 5.83; N, 12.70. Found: C, 65.21; H, 5.53; N, 12.59.

### Step 3. 9-Cyclopropyl-3-fluoro-2-(4-methylpiperazin-1-yl)-6H-6-oxo-pyrido[1,2-a]pyrimidine-7-carboxylic acid

To a 0.050 g (0.115 mmol) sample of the benzyl ester compound from the previous step was added 10 mL of methanol, 1 mL of 98% formic acid and 0.04 g of 10% Pd/C, and the mixture was stirred under Argon for 30 min at room temperature. The solution was diluted with methylene chloride, filtered through diatomaceous earth and the solvent was removed to leave a yellow residue. The product was purified by column chromatography on silica gel, eluting with 1:10:100 acetic acid:methanol:methylene chloride. After removal of the solvent, 0.0345 g of the title compound was obtained as a yellow solid, mp 219-220°C. MS M/Z 347 (M+H). NMR:(CDCl₃) δ 0.67 (m, 2H), 0.95 (m, 2H), 2.18 (m, 1H), 2.39 (s, 3H), 2.65 (t, 4H, J=6 Hz), 4.13 (m, 4H), 8.11 (s, 1H), 9.02 (d, 1H), J=10 Hz). IR (KBr): 1720, 1660, 1620 cm⁻¹. Analysis calculated for C₁₇H₁₉FN₄O₃·0.6 CH₃COOH: C, 57.17; H, 5.64; N, 14.65. Found: C, 57.60; H, 5.79; N, 14.13.

### Example 164

### 9-Cyclopropyl-3-fluoro-2-(piperazin-1-yl)-6H-6-oxo-pyrido[1,2-a]pyrimidine-7-carboxylic acid

### Step 1. 2-Chloro-9-cyclopropyl-3-fluoro-6H-6-oxo-pyrido[1,2-a]pyrimidine-7-carboxylic acid t-butyl ester

A mixture of 0.100 g (0.312 mmol) of 9-cyclopropyl-3-fluoro-2-hydroxy-6H-6-oxo-pyrido[1,2-a]pyrimidine-7-carboxylic acid *t*-butyl ester from Example 158 Step 1, 0.29 mL of DMF, 0.33 mL of phosphorous oxychloride and 10 mL of methylene chloride was stirred under a dry N₂ atmosphere at room temperature for 1 hour. After workup as described in Example 157 Step 5, the title compound was obtained as a orange solution in methylene chloride, which was taken directly to the next step.

### Step 2. 9-Cyclopropyl-3-fluoro-2-(piperazin-1-yl)-6H-8-oxo-pyrido[1,2-a]pyrimidine-7-carboxylic acid t-butyl ester

The sample from Step 1 in 5 mL of methylene chloride was added dropwise to a solution of 0.289 g piperazine in 10 mL of methylene chloride stirred under a dry N₂ atmosphere. The resulting yellow solution was concentrated to give a yellow residue, which was purified by column chromatography on silica gel, eluting with 0.5:10:100 conc. ammonium hydroxide:methanol:methylene chloride, to afford after removal of the solvent 0.068 g of the title compound as a yellow solid. This material was taken directly to the next step.

### Step 3. 9-Cyclopropyl-3-fluoro-2-(piperazin-1-yl)-6H-6-oxo-pyrido[1,2-a]pyrimidine-7-carboxylic acid

The sample of the compound from the previous step was reacted with 10 mL of 4N HCl in dioxane under a dry N₂ atmosphere at room temperature overnight. The solvent was removed, the yellow solid was dissolved in distilled water, adjusted to pH 7-8 with saturated sodium carbonate solution, and the solution extracted with methylene chloride. The extracts were washed with water, dried, concentrated, and chromatographed on silica gel to afford 0.043 g of the title compound as a yellow solid, mp 198-199°C. MS M/Z 333 (M+H). NMR:(CDCl₃) δ 0.67 (m, 2H), 0.94 (m, 2H), 2.19 (m, 1H), 3.08 (t, 4H, J=6 Hz), 4.08 (m, 4H), 8.11 (s, 1H), 9.01 (d, 1H, J=10 Hz). IR (KBr): 1710, 1660 cm⁻¹. Analysis calculated for C₁₆H₁₇FN₄O₃·0.1 H₂O: C, 57.36; H, 5.20; N, 16.72. Found: C, 57.69; H, 5.22; N, 16.31.

### Example 165

### 9-Cyclopropyl-3-fluoro-2-(morpholin-1-yl)-6H-6-oxo-pyrido[1,2-a]pyrimidine-7-carboxylic acid

### Step 1. 9-Cyclopropyl-3-fluoro-2-(morpholin-1-yl)-6H-6-oxo-pyrido[1,2-a]pyrimidine-7-carboxylic acid benzyl ester

To a 0.150 g (0.396 mmol) sample of 2-chloro-9-cyclopropyl-3-fluoro-6H-6-oxo-pyrido[1,2-a]pyrimidine-7-carboxylic acid benzyl ester, prepared as in Example 164 Step 1, dissolved in anhydrous methylene chloride and cooled to 0°C and stirred under a dry N₂ atmosphere was added 0.042 mL (0.483 mmol) of morpholine dropwise. The color changed from orange to yellow, and the reaction was complete in 15 min. The solvent was removed by evaporation, and the product was purified by column chromatography on silica gel, eluting with 2:10:100 acetic acid:methanol:methylene chloride. The solvent was removed to afford the title compound as a yellow solid. This was taken directly to the next step.
NMR:(CDCl₃) δ 0.62 (m, 2H), 0.89 (m, 2H), 2.11 (m, 1H), 3.87 (t, 4H), J=6 Hz), 4.07 (t, 4H, J=6 Hz), 5.39 (s, 2H), 7.29 (m, 1H), 7.37 (m, 2H), 7.51 (m, 2H), 8.07 (s, 1H), 9.19 (d, 1H, J=10 Hz).

### Step 2. 9-Cyclopropyl-3-fluoro-2-(morpholin-1-yl)-6H-6-oxo-pyrido[1,2-a]pyrimidine-7-carboxylic acid

The benzyl ester product from the previous step was dissolved in 20 mL of anhydrous methanol and stirred with 0.020 g of 10% Pd/C catalyst under 1 atm. Hydrogen at room temperature for 5 hours. The catalyst was removed by filtration, and the solvent was removed under vacuum to afford 0.100 g of the title compound as a yellow solid, mp >260°C. MS M/Z 334 (M+H). NMR:(CDCl₃) δ 0.68 (m, 2H), 0.95 (m, 2H), 2.19 (m, 1H), 3.90 (t, 4H, J=6 Hz), 4.10 (t, 4H, J=6 Hz)., 8.15 (s, 1H), 9.06 (d, 1H, J=10 Hz). IR 1720, 1660, 1620 cm⁻¹. Analysis calculated for C₁₆H₁₆FN₃O₄·H₂O: C, 54.70; H, 5.16; N, 11.96. Found: C, 55.01; H, 4.71; N, 11.62.

### Example 166

### 9-(2,4-Difluorophenyl)-3-fluoro-2-(3-(N-(S)-norvalyl)aminopyrrolidin-1-yl)-6H-6-oxopyrido[1,2-a]pyrimidine-7-carboxylic acid hydrochloride salt

### Step 1. 2-(3-Aminopyrrolidin-1-yl)-9-(2,4-difluorophenyl)-3-fluoro-6H-6-oxopyrido[1,2-a]pyrimidine-7-carboxylic acid benzyl ester

A 1.579 g (2.655 mmol) sample of the 9-(2,4-difluorophenyl)-3-fluoro-2-(3-(N-*t*-butoxycarbonyl)aminopyrrolidin-1-yl)-6H-6-oxopyrido[1,2-a]pyrimidine-7-carboxylic acid benzyl ester, from Example 160 Step 4, was dissolved in 5 mL of trifluoroacetic acid and stirred at room temperature for 1 hour under a dry N₂ atmosphere. The solvent was removed by evaporation under vacuum to yield the deprotected title product as a yellow solid, which was taken directly to the next step. Mp 185-186°C. NMR (CDCl₃): δ 1.75-2.19 (m, 2H), 3.33-4.07 (m, 5H), 5.38 (s, 2H), 6.87 (m, 2H), 7.32 (m, 4H), 7.48 (m, 2H), 8.33 (s, 1H), 9.13 (apparent d, 1H, J=9 Hz).

### Step 2. 2-(3-(N-(N-Benzyloxycarbonyl)norvalyl)aminopyrroildin-1-yl)-9-(2,4-difluorophenyl)-3-fluoro-6H-6-oxopyrido[1,2-a]pyrimidine-7-carboxylic acid benzyl ester

The sample from the previous step was suspended in 50 mL of THF and diisopropylethylamine was added with stirring at room temperature until a homogeneous solution resulted. Then 0.885 g (2.66 mmol) of the N-benzyloxycarbonyl protected (s)-norvaline succinamide was added and stirred at room temperature for 1 hour under a dry N₂ atmosphere. Another 0.050 g of the protected norvaline was added, and the solution was stirred for another 0.5 hours. The reaction was diluted with methylene chloride, washed with water (4x), and the organic solvent dried over anhydrous magnesium sulfate and removed by evaporation under vacuum. This product was purified by column chromatography on silica gel, eluting with 5% methanol in methylene chloride, to afford 1.678 g of the title compound as a yellow crystalline solid after removal of the solvent. Mp 103-105°C. MS M/Z: 728 (M+H). NMR: (CDCl₃) δ 0.90 (t, 3H, J=7 Hz), 1.39-2.30 (m, 6H), 3.30-4.40 (m, 5H), 4.85-5.40 (m, 5H), 6.75-7.40 (m, 13 H), 8.15-8.80 (m, 2H). IR (KBr): 1700, 1660 cm⁻¹. Analysis calculated for C₃₉H₃₆F₃N₅O₆·0.25 H₂O: C, 63.97; H, 5.02; N, 9.56. Found: C, 64.19; H, 5.11; N, 9.50.

### Step 3. 9-(2,4-Difluorophenyl)-3-fluoro-2-(3-(N-(S)-norvalyl)aminopyrrolidin-1-yl)-6H-6-oxopyrido[1,2-a]pyrimidine-7-carboxylic acid hydrochloride salt

A 1.515 g sample (2.0822 mmol) sample of the compound from the previous step was dissolved in 80 mL of methanol, and 4.0 mL of 98% formic acid and 0.2 g of 10% Pd/C was added. The mixture was stirred at room temperature for 1.7 hours under a dry N₂ atmosphere, filtered and concentrated to leave a yellow solid residue. This solid was dissolved in methanol and filtered through sintered glass, then the solvent was removed to leave a yellow solid. This solid was dissolved in 50 mL of methanol, 3 mL of conc. HCl was added and the solvent evaporated off. The residue was dissolved in 200 mL of water, filtered again through sintered glass, and the solution was freeze-dried to afford 0.969 g of the title product as a yellow solid, mp 192-194°C. MS M/Z: 504 (M+H). NMR: (CD₃OD) δ 0.96 (m, 3H), 1.90-2.35 (m, 6H), 3.50-4.60 (m, 5H), 7.02 (m, 2H), 7.48 (m, 1H), 8.22 (br s, 1H), 8.35 (br s, 2H), 9.09 (m, 1H). IR (KBr): 1710, 1665, 1610 cm⁻¹. Analysis calculated for C₂₄H₂₅F₃N₅O₄·2 H₂O: C, 50.05; H, 5.07; N, 12.16. Found: C, 50.00; H, 4.56; N, 12.03.

### Example 167

### 2-(3-(N-(S)-Alanyl)aminopyrrolidin-1-yl)-9-(2,4-difluorophenyl)-3-fluoro-6H-6-oxopyrido[1,2-a]pyrimidine-7-carboxylic acid hydrochloride

### Step 1. 2-(3-(N-(N-Benzyloxycarbonyl)alanyl)aminopyrrolidin-1-yl)-9-(2,4-difluorophenyl)-3-fluoro-6H-6-oxopyrido[1,2-a]pyrimidine-7-carboxylic acid benzyl

A 0.982 g (1.986 mmol) sample of 9-(2,4-difluorophenyl)-3-fluoro-2-(3-aminopyrrolidin-1-yl)-6H-6-oxopyrido[1,2-a]pyriinidine-7-carboxylic acid benzyl ester, prepared as described in Example 166 Step 1, was suspended in 40 mL of THF and 0.700 g (2.196 mmol) of the N-benzyloxycarbonyl protected (S)-alanine succinamide was added.The mixture was stirred at room temperature for 2 hour under a dry N₂ atmosphere. The reaction solvent was evaporated off, then the residue was dissolved in methylene chloride, which was washed with water (3x). The organic solvent was dried over anhydrous magnesium sulfate and removed by evaporation under vacuum. This product was purified by column chromatography on silica gel, eluting with 5% methanol in methylene chloride, to afford, after removal of the solvent, 1.318 g of the title compound as a yellow crystalline solid, mp 104-107°C. MS M/Z 700 (M+H). NMR: (CDCl₃) δ 1.43 (m, 3H), 1.95-2.30 (m, 2H), 3.40-4.40 (m, 5H), 4.75-5.35 (M, 5H), 6.77 (m, 2H), 7.10-7.40 (m, 1H), 8.18-8.40 (m, 2H). IR (KBr): 1720, 1660 cm⁻¹. Analysis calculated for C₃₇H₃₂F₃N₅O₆·1/2 H₂O: C, 62.71; H, 4.69; N, 9.88. Found: C, 63.04; H, 4.49; N, 9.92

### Step 2. 2-(3-(N-(S)-Alanyl)aminopyrrolidin-1-yl)-9-(2,4-difluorophenyl)-3-fluoro-6H-6-oxopyrido[1,2-a]pyrimidine-7-carboxylic acid hydrochloride

A 1.262 g (1.804 mmol) sample of the compound from the previous step was suspended in 80 mL of methanol and 4.0 mL of 98% formic acid and 0.200 g of 10% Pd/C was added with stirring. The mixture was stirred at room temperature for 1.7 hours, then 40 ml of THF was added and the mixture stirred for 0.3 hours longer under a dry N₂ atmosphere, filtered and concentrated to leave a yellow solid residue. This was dissolved in 500 mL of water and 4 mL of conc. HCl was added, then the solution was filtered through sintered glass and freeze-dried to afford 0.877 g of the title compound as a yellow solid, mp 198-200° C (dec). MS M/Z 476 (M-Cl). NMR: (DMSO-d₆) δ 1.33 (apparent t, 3H, J=7 Hz), 1.90-2.30 (m, 2H), 3.35-4.40 (m, 6H), 7.17 (m, 1H), 7.32 (m, 1H), 7.58 (m, 1H), 8.20 (d, 1H), 9.19 (m, 1H), 13.45 (br, 1H). IR (KBr): 1715, 1665, 1620 cm⁻¹. Analysis calculated for C₂₂H₂₁ClF₃N₅O₄·1.5 H₂O: C, 49.03; H, 4.48; N, 12.99. Found: C, 49.18; H, 4.17; N, 12.53.

### Example 168

### 2-(3-(N-(S)-Alanyl-(S)-alanyl)aminopyrrolidin-1-yl)-9-(2,4-difluorophenyl)-3-fluoro-6H-6-oxopyrido[1,2-a]pyrimidine-7-carboxylic acid hydrochloride

### Step 1. 2-(3-(N-(N-Benzyloxycarbonyl)-(S)-alanyl-(S)-alanyl)aminopyrrolidin-1-yl)-9-(2,4-difluorophenyl)-3-fluoro-6H-6-oxopyrido[1,2-a]pyrimidine-7-carboxylic acid benzyl ester

A 0.905 g (1.830 mmol) sample of 9-(2,4-difluorophenyl)-3-fluoro-2-(3-aminopyrrolidin-1-yl)-6H-6-oxopyrido[1,2-a]pyrimidine-7-carboxylic acid benzyl ester, prepared as described in Example 166 Step 1, was suspended in 10 mL of DMF and 0.700 g (2.196 mmol) of the N-benzyloxycarbonyl protected (S)-alanyl-(S)-alanine. The mixture was stirred at 0°C and 0.530 g of 1-ethyl-3-[3-dimethylaminopropyl]carbodiimide hydrochloride (EDAC) and 0.370 g of 1-hydroxybenzotriazole hydrate (HOBT) was added. The mixture was stirred for 30 mm at 0°C, then at room temperature for 2 hours. The solvent was removed in a kugelrohr apparatus, then the residue was dissolved in methylene chloride, washed 2x with water, washed 2x with saturated sodium bicarbonate solution, then 2x again with water and dried over magnesium sulfate. The solvent was removed by evaporation, and the product was purified by column chromatography on silica gel, eluting with 10% methanol in methylene chloride to afford 1.187 g of the title product as yellow crystals, mp 123-126°C. MS M/Z 771 (M+H). NMR: (CDCl₃) δ 1.37 (m, 6H), 1.92-2.18 (m, 2H), 3.58-4.48 (m, 5H), 4.76-5.00 (m, 2H), 5.30 (s, 2H), 5.32 (s, 2H), 6.80 (m, 2H), 7.10-7.45 (m, 1H), 8.23 and 8.30 (two s, 1H), 8.87 and 8.93 (two d, 1H, J=8 Hz). IR (KBr): 1720, 1660 cm⁻¹. Analysis calculated for C₄₀H₃₇F₃N₆O₇·1/2 H₂O: C, 61.62; H, 4.91; N, 10.78. Found: C, 61.51; H, 4.71; N, 10.75.

### Step 2. 2-(3-(N-(S)-Alanyl-(S)-alanyl)aminopyrrolidin-1-yl)-9-(2,4-difluorophenyl)-3-fluoro-6H-6-oxopyrido[1,2-a]pyrimidine-7-carboxylic acid hydrochloride

A 1.131 g (1.467 mmol) sample of the compound from Step 1 was dissolved in 80 mL of methanol and 4.0 mL of 98% formic acid and 0.2 g of 10% Pd/C was added. The mixture was stirred 1 hour at room temperature under a dry N₂ atmosphere, filtered, and concentrated to leave a yellow residue. This was dissolved in 500 mL of distilled water and 3 mL of conc. HCl was added, then the solution was filtered though sintered glass, and freeze-dried to afford 0.729 g of the title compound as a pale yellow solid, mp 217-219°C (dec). MS M/Z 547 (M-Cl). NMR: (DMSO-d₆) δ 1.24 (m, 3H), 1.32 (d, 3H, J=7 Hz), 1.80-2.20 (m, 2H), 3.40-4.50 (m, 7H), 7.17 (m, 1H), 7.31 (m, 1H), 7.57 (m, 1H), 8.20 (br, 4H), 8.47 (m, 1H), 8.66 (m, 1H), 9.19 (m, 1H), 13.45 (br, 1H). IR (KBr): 1710, 1660, 1630 cm⁻¹.

### Example 169

### 2-((2S,4S)-4-Acetamido-2-methylpyrrolidin-1-yl)-9-(2,4-difluorophenyl)-3-fluoro-6H-6-oxopyrido[1,2-a]pyrimidine-7-carboxylic acid

### Step 1. 2-((2S,4S)-4-Acetamido-2-methylpyrrolidin-1-yl)-9-(2,4-D difluorophenyl)-3-fluoro-6H-6-oxopyrido[1,2-a]pyrimidine-7-carboxylic acid benzyl ester

A 0.200 g (0.469 mmol) sample of 9-(2,4-difluorophenyl)-3-fluoro-2-hydroxy-6H-6-oxopyrido[1,2-a]pyrimidine-7-carboxylic acid benzyl ester, from Example 160 Step 3, was dissolved in 5 mL of methylene chloride and 0.42 mL of DMF and 0.49 mL of POCl₃ were added. The reaction was stirred under a dry N₂ atmosphere at room temperature for 3.5 hours, then quenched with ice and water. The mixture was extracted with methylene chloride, and the solvent was washed with water until the acidity of the rinse water was above pH 3. The solvent was then dried with magnesium sulfate and 0.120 g (0.656 mmol) of (2S,4S)-4-acetamido-2-methylpyrrolidine (prepared as described by Rosen, T., *et al, J. Med. Chem.*, 31, 1598-1611 (1988)) in 10 mL of methylene chloride and 2 mL of triethylamine was added and allowed to react. The solution was then concentrated and the product was purified by column chromatography over silica gel eluting with 1:10:100 acetic acid:methanol:methylene chloride. The solvent was removed to afford 0.205 g of the title compound as yellow crystals, mp 117-119°C. [α]=-122.6° (25°C, D, c=0.05, CHCl₃). MS M/Z 551 (M+H). NMR: (CDCl₃) δ 1.10 (d, 3H, J=7 Hz), 1.85-2.25 (m, 2H), 2.10 (s, 3H), 4.05 (m, 2H), 4.23 (m, 1H), 4.80 (m, 1H), 5.06 (d, 1H, J=13 Hz), 5.27 (d, 1H, J=13 Hz), 6.79 (m, 2H), 7.20-7.40 (m, 6H), 7.76 (br, 1H), 8.21 (s, 1H), 8.80 (d, 1H, J=9 Hz). IR (KBr): 1725, 1660 cm⁻¹. Analysis calculated for C₂₉H₂₅F₃N₄O₄·H₂O: C, 61.26; H, 4.79; N, 9.85. Found: C, 61.59; H, 4.37; N, 9.72.

### Step 2. 2-((2S,4S)-4-Acetamido-2-methylpyrrolidin-1-yl)-9-(2,4-difluorophenyl)-3-fluoro-6H-6-oxopyrido[1,2-a]pyrimidine-7-carboxylic acid

To a 0.198 g (0.359 mmol) sample of the compound from Step 1 in 20 mL of methanol was added 1 mL of 98% formic acid and 0.1 g of 10% Pd/C. The mixture was stirred at room temperature under a dry N₂ atmosphere for 1.25 hours. The mixture was filtered, and the filtrate concentrated to leave a yellow residue. The product was purified by column chromatography on silica gel, eluting with 1:10:100 acetic acid:methanol:methylene chloride to afford 0.126 g of the title compound as a yellow solid, after removal of the solvent, mp 163-164°C. [α]=-50.2° (23°C, D, c=0.5, CHCl₃). MS M/Z 461 (M+H). NMR: (CDCl₃ + CD₃OD) d 1.09 and 1.39 (two d, 3H, J=6 Hz), 1.92-2.15 (m, 2H), 2.00 (s, 3H), 3.97 (m, 1H), 4.16 (m, 1H), 4.32 (m, 1H), 4.72 (m, 1H), 6.90 (m, 2H), 7.25 (m, 1H), 8.17 and 8.31 (two s, 1H), 8.93 and 8.97 (two d, 1H, J=8 Hz). IR (KBr): 1720, 1660, 1035 cm⁻¹. Analysis calculated for C₂₂H₁₉F₃N₄O₄·H₂O: C, 55.23; H, 4.42; N, 11.71. Found: C, 55.25; H, 4.20; N, 11.21.

### Example 170

### 9-(2,4-Difluorophenyl)-3-fluoro-2-(3-hydroxypyrrolidin-1-yl)-6H-6-oxopyrido[1,2-a]pyrimidine-7-carboxylic acid

### Step 1. 9-(2,4-Difluorophenyl)-3-fluoro-2-(3-hydroxypyrrolidin-1-yl)-6H-6-oxopyrido[1,2-alpyrimidine-7-carboxylic acid benzyl ester

A 0.200 g (0.469 mmol) sample of 9-(2,4-difluorophenyl)-3-fluoro-2-hydroxy-6H-6-oxopyrido[1,2-a]pyrimidine-7-carboxylic acid benzyl ester, from Example 160 Step 3, was dissolved in 5 mL of methylene chloride and 0.42 mL of DMF and 0.49 mL of POCl₃ were added. The reaction was stirred under a dry N₂ atmosphere at room temperature for 3.5 hours, then quenched with ice and water. The mixture was extracted with methylene chloride, and the solvent was washed with water until the acidity of the rinse water was above pH 3. The solvent was then dried with magnesium sulfate and 0.1 mL of 3-pyrrolidinol was added and allowed to react. The solution was then concentrated and the product was purified by column chromatography over silica gel eluting with 1:10:100 acetic acid:methanol:methylene chloride. The solvent was removed to afford 0.183 g of the title compound as yellow crystals, mp 105-107°C. MS M/Z 496 (M+H). NMR: (CDCl₃) δ 2.00-2.16 (m, 2H), 3.55-3.68 (m, 2H), 3.96-4.16 (m, 2H), 4.18 and 4.55 (m, 1H), 5.36 and 5.38 (two s, 2H), 6.90 (m, 2H), 7.30-7.48 (m, 6H, 8.33 (s, 1H), 9.08 and 9.14 (two d, 1H, J=6 Hz). IR (KBr): 1725, 1690, 1660 cm⁻¹. Analysis calculated for C₂₆H₂₀F₃N₃O₄·3/4 H₂O: C, 61.36; H, 4.26; N, 8.26. Found: C, 60.97; H, 3.67; N, 7.98.

### Step 2. 9-(2,4-Difluorophenyl)-3-fluoro-2-(3-hydroxypyrrolidin-1-yl)-6H-6-oxopyrido[1,2-a]pyrimidine-7-carboxylic acid

To a 0.166 g (0.334 mmol) sample of the compound from Step 1 in 20 mL of methanol and 15 mL of DMF was added 2 mL of 98% formic acid and 0.12 g of 10% Pd/C. The mixture was stirred at room temperature under a dry N₂ atmosphere for 1.33 hours. The mixture was filtered, and the filtrate concentrated, removing the DMF in a kugelrohr apparatus, to leave a yellow residue. The product was purified by column chromatography on silica gel, eluting with 1:10:100 acetic acid:methanol:methylene chloride to afford 0.088 g of the title compound as a yellow solid, after removal of the solvent, mp 168-170°C (dec). MS M/Z 406 (M+H). NMR: δ 2.00-2.15 (m, 2H), 3.55-3.70 (m, 2H), 3.97-4.12 (m, 2H), 4.50-4.60 (m, 1H), 6.93 (m, 2H), 7.35 (m, 1H), 8.43 (s, 1H), 9.01 and 9.04 (two d, 1H, J=4 Hz). IR (KBr): 1715, 1665, 1625 cm⁻¹. Analysis calculated for C₁₉H₁₄F₃N₃O₄·1/2 H₂O: C, 55.08; H, 3.65; N, 10.14. Found: C, 55.10; H, 3.53; N, 10.04.

### Example 171

### 2-((2S,4S)-4-Amino-2-methylpyrrolidin-1-yl)-9-(2,4-difluorophenyl)-3-fluoro-6H-6-oxopyrido[1,2-a]pyrimidine-7-carboxylic acid hydrochloride

### Step 1 (2S,4S)-4-acetamido-2-methylpyrrolidine

A 6.000 g (24.760 mmol) sample of (2S, 4S)-4-acetamido-1-(*t*-butoxycarbonyl)-2-methylpyrrolidine, prepared as described by Rosen, T., *et al., J. Med. Chem.*, 31, 1598-1611 (1988), was dissolved in 30 mL of 4N HCl in dioxane and stirred at room temperature for 24 hours to remove the *boc* group.. The solvent was removed by evaporation to give the hydrochloride salt of this compound as a white solid, which was taken directly to the next step.

### Step 2. (2S, 4S)-4-acetamido-1-benzyl-2-methylpyrrolidine

This salt from the previous step was suspended in 27 mL of methylene chloride ,8.4 mL of triethylamine was added and the mixture stirred for 10 min. Next was added 3.2 mL (26.9 mmol) of benzyl bromide and the mixture heated at reflux for 5 hours. The mixture was diluted with methylene chloride, which was washed 3x with water, dried over magnesium sulfate, and evaporated to leave the 1-benzyl protected compound as a white solid, which was taken directly to the next step.

### Step 3. (2S, 4S)-4-amino-1-benzyl-2-methylpyrrolidine hydrochloride

The acetyl group was removed from the compound from the previous step by heating at reflux for 6 hours in 6N HCl. Removal of the solvent gave the solid product which was taken directly to the next step.

### Step 4. (2S, 4S)-1-benzyl-4-t-butoxycarbonylamino-2-methylpyrrolidine

The sample from the previous step was dissolved in 10 mL of water and 35 mL of methanol. To this solution stirred at 0°C was added 5.2 mL of triethylamine and 4.21 g of di-*t*-butyl dicarbonate. The reaction was stirred for 2 hours at 0°C and then at room temperature for 19 hours. The solvent was removed by evaporation, the residue dissolved in methylene chloride, which was washed with water and concentrated. The product was purified by column chromatography on silica gel, eluting with 0.5:5:100 conc. ammonium hydroxide:methanol:methylene chloride to give the title compound as a white solid after removal of the solvent. This material was taken directly to the next step.

### Step 5. (2S, 4S)-4-t-butoxycarbonylamino-2-methylpyrrolidine

The sample from the previous step was dissolved in 150 mL of methanol, 0.90 g of 10% Pd/C was added and the mixture shaken under 4 atm of hydrogen at room temperature for 13 hours. The mixture was concentrated, the catalyst was removed by filtration, and the solvent removed to afford 3.081 g of the title compound as a white solid. MS M/Z 201 (M+H). NMR (CDCl₃): δ 1.15 (d, 3H, J=6 Hz), 1.44 (s, (H), 1.54-1.63 (m, 2H), 1.75 (m, 1H), 2.64 (dd, 1H, J=5, J=12 Hz), 3.26 (m, 1H), 3.38 (dd, 1H, J=7, J=12 Hz), 4.12 (br, 1H), 4.63 (br, 1H). IR (KBr): 1685 cm⁻¹.

### Step 6. 2-((2S,4S)-4-t-butoxycarbonylamino-2-methylpyrrolidin-1-yl)-9-(2,4-difluorophenyl)-3-fluoro-6H-6-oxopyrido[1,2-a]pyrimidine-7-carboxylic acid benzyl ester

A 1.500 (3.518 mmol) sample of 9-(2,4-difluorophenyl)-3-fluoro-2-hydroxy-6H-6-oxopyrido[1,2-a]pyrimidine-7-carboxylic acid benzyl ester, from Example 160 Step 3, was dissolved in 40 mL of methylene chloride and 3.20 mL of DMF and 3.70 mL of POCl₃ were added. The reaction was stirred under a dry N₂ atmosphere at room temperature for 2.25 hours, then quenched with ice and water. The mixture was extracted with methylene chloride, and the solvent was washed with water until the acidity of the rinse water was above pH 3. The solvent was then dried with magnesium sulfate and 1.06 g (0.656 mmol) of (2S,4S)-4-*t*-butoxycarbonylamino-2-methylpyrrolidine, from Step 5 above, in 50 mL of methylene chloride and 7 mL of triethylamine was added and allowed to react. The solution was then concentrated and the product was purified by column chromatography over silica gel eluting with 0.5:10:100 conc. ammonium hydroxide:methanol:methylene chloride. The solvent was removed to afford 1.856 g of the title compound as yellow crystals, mp 106-107°C. [α]=+13.4 (23°, D, c=0.5, CHCl₃). MS M/Z 609 (M+H). NMR: (CDCl₃) δ 1.11 (two d, 3H, J=7 Hz), 1.45 and 1.55 (two s, 9H), 1.90-2.10 (m, 2H), 3.60-4.60 (m, 5H), 5.39 (s, 1H), 6.89 (m, 2H), 7.34-7.50 (m, 6H), 8.34 and 8.36 (two s, 1H), 9.16 and 9.19 (two d, 1H, J=9 Hz). IR (KBr): 1715, 1690, 1660 cm⁻¹. Analysis calculated for C₃₂H₃₁F₃N₄O₅·1/2 H₂O: C, 62.23; H, 5.22; N, 9.07. Found: C, 62.44; H, 5.20; N, 9.16.

### Step 7. 2-((2S,4S)-4-Amino-2-methylpyrrolidin-1-yl)-9-(2,4-difluorophenyl)-3-fluoro-6H-6-oxopyrido[1,2-a]pyrimidine-7-carboxylic acid

To a 1.814 g (2.981 mmol) sample of the compound from Step 6 dissolved in 80 mL of methanol and 10 mL of THF was added 8 mL of 98% formic acid and 1 g of 10% Pd/C. The mixture was stirred at room temperature under a dry N₂ atmosphere for 2.3 hours. The mixture was filtered, and the filtrate concentrated to leave a yellow residue. The product was purified by column chromatography on silica gel, eluting with 1:10:100 acetic acid:methanol:methylene chloride to afford 1.513 g of the title compound as a yellow solid, after removal of the solvent. The compound was taken directly to the next step.

### Step 8. 2-((2S,4S)-4-Amino-2-methylpyrrolidin-1-yl)-9-(2,4-difluorophenyl)-3-fluoro-6H-6-oxopyrido[1,2-a]pyrimidine-7-carboxylic acid hydrochloride

The 1.528 g sample of the compound from the previous step was dissolved in 20 mL of 4N HCl in dioxane and stirred at room temperature for 3.5 hours. The solvent was removed, the residue redissolved in 500 mL of water, 0.5. mL of conc. HCl was added, and the solution freeze-dried to afford 1.147 g of the title compound as a yellow solid, mp 204°C (dec). [α]=+35.4° (22°C, D, c=0.5, CH₃OH). MS M/Z 419 (M-Cl). NMR: (CD₃OD) δ 1.16 and 1.41 (two d, 3H, J=7 Hz), 2.15-2.31 (m, 2H), 3.75-4.40 (m, 4H), 7.04 (m, 2H), 7.46 (m, 1H), 8.25 and 8.30 (two s, 1H), 9.11 and 9.21 (two d, 1H, J=9 Hz). IR(KBr): 1710, 1660, 1630 cm⁻¹. Analysis calculated for C₂₀H₁₈F_{3Cl}N₄O₃·H₂O: C, 50.80; H, 4.26; N, 11.85. Found: C, 50.98; H, 4.10; N, 11.85.

### Example 172

### 2-(3-Aminopyrrolidin-1-yl)-3-fluoro-9-(2,3,4,5,6-pentafluorophenyl)-6H-6-oxopyrido[1,2-a]pyrimidine-7-carboxylic acid hydrochloride salt

### Step 1. 2-(2,3,4,5,6-Pentafluorophenyl)-acetamidine hydrochloride

Into a solution of 26.72 g (0.129 mol) of pentafluoroacetonitrile (commercially available) in 8.30 mL of anhydrous ethanol cooled to 0°C and stirred under a dry N₂ atmosphere was introduced gaseous HCl, until the mixture solidified. The reaction was allowed to stand for 96 hours, then 60 mL of ethanol and 30.7 mL of 4.2 N HCl in ethanol (0.124 M) was added, and the slurry was stirred at room temperature for 2 hours. The mixture was filtered through sintered glass, and the filtrate was concentrated under vacuum to afford the title compound as a brownish solid, which was taken directly to the next step.

### Step 2. 5-Fluoro-4-hydroxy-2-(2,3,4,5,6-pentafluorobenzyl)pyrimidine

A mixture of the compound (0.129 mol) from Step 1, 0.135 mol of the sodium salt of ethyl 2-fluoro-3-hydroxy-2-propenoate (prepared as described by E. Elkik and M. Imbeaux-Oudotte, *Bull. Soc. Chim. Fr.*, 5-6 pt 2, 1165 (1975)), 150 mL of anhydrous methanol and 25 mL of triethylamine was stirred under a dry N₂ atmosphere for 24 hours. The solvent was removed by evaporation under vacuum and the residue was dissolved in methylene chloride and washed (1x) with 10% HCl and (1x) with water, then dried over anhydrous magnesium sulfate, and the solvent was removed by evaporation under vacuum to give a dark oil which solidified upon standing. This solid was washed with 1:2 ethyl acetate:hexane to afford 4.843 g of the title compound as a white solid, mp 161-162°C. The filtrate was concentrated and extracted with 1:4 ethyl acetate:hexane to leave a second crop of 4.454 g of product. Additional product was obtained by chromatography of the residue, for a total yield of 19.20 g of product. MS M/Z 312 (M+NH₄). NMR (CDCl₃): δ 4.15 (apparent s, 2H), 7.80 (d, 1H, J=3 Hz), 13.38 (br s, 1H). IR (KBr): 3440, 1685, 1660, 1610 cm⁻¹.

### Step 3. 2-Ethoxy-3-(5-fluoro-4-hydroxy-3-(2,3,4,5,6-pentafluorophenyl)propane-1,1-dicarboxylic acid diethyl ester

The compound from Step 2 above (0.294 g, 1.00 mmol) was dissolved in 10 mL of THF and cooled to -78°C with stirring, then 0.82 mL (2.05 mmol) of a 2.5 M solution of n-butyllithium in hexane was added and the resulting yellow solution was stirred for 30 mm. To this was added 0.243 mL (1.2 mmol) of ethyl 2-carboethoxy-3-ethoxy-2-propenecarboxylate with stirring for 15 mm. The reaction was quenched with 10% HCl, allowed to warm to room temperature and extracted with ethyl acetate. The extract was washed (2x) with brine, and the solvent dried over magnesium sulfate and concentrated to afford the title compound as an oil, which was taken directly to the next step.

### Step 4. 9-(2,3,4,5,6-pentafluorophenyl)-3-fluoro-2-hydroxy-6H-6-oxopyrido[1,2-a]pyrimidine-7-carboxylic acid ethyl ester

The compound from Step 3 above was dissolved in 10 mL of ethanol, 0.2 mL of conc. sulfuric acid was added and the solution was heated at reflux for 18 hours. The solvent was removed and the residue washed with ether to afford 0.222 g of the title compound as a yellow solid, mp 235-236C. MS M/Z 419 (M+H), 436 (M+NH₄). IR (KBr): 3440 (br), 1710, 1680, 1615 cm⁻¹. NMR (CDCl₃) δ 1.38 (t, 3H), J=7 Hz), 4.37 (q, 2H, J=7 Hz), 8.23 (s, 1H), 9.05 (d, 1H, J=6 Hz).

### Step 5. 3-Fluoro-2-hydroxy-9-(2,3,4,5,6-pentafluorophenyl)-6H-6-oxopyrido[1,2-a]pyrimidine-7-carboxylic acid benzyl ester

A 1.000 g (2.391 mmol) sample of the compound from Step 4 was dissolved in 25 mL of benzyl alcohol, 0.09 mL of titanium tetraethoxide was added and the mixture was stirred at 90°C for 20 hours. The reaction was diluted with methylene chloride, washed (1x) with 10% HCl and concentrated in a rotary evaporator. The crude product was purified in a kugelrohr apparatus to yield a yellow solid, which was washed with ether and dried to afford 0.457 g of the title compound, which was taken directly to the next step.

### Step 6. 2-(3-(N-t-Butoxycarbonyl)aminopyrrolidin-1-yl)-3-fluoro-9-(2,3,4,5,6-pentafluorophenyl)-6H-6-oxopyrido[1,2-a]pyrimidine-7-carboxylic acid benzyl ester

A 0.400 g (0.833 mmol) sample of the compound from Step 5 was dissolved in 10 mL of methylene chloride and 0.746 mL of DMF, and 0.870 mL of POCl₃ were added and stirred under a dry N₂ atmosphere at room temperature for 1.7 hours. The reaction was quenched with ice and the mixture extracted with methylene chloride which was washed (2x) with water. The organic layer was added to a stirred solution of 0.235 g (1.2 mmol) of 2-(N-*t*-butoxycarbonylamino)pyrrolidine in 4 mL of triethylamine. The solvent was removed by evaporation, and the product was purified by column chromatography on silica gel, eluting with 2.5:100 methanol:methylene chloride. Removal of the solvent afforded 0.353 g of the title product as a yellow crystalline solid, mp 107-108°C. MZ M/Z 649 (M+H). NMR (CDCl₃) δ 1.44 (s, 9H), 1.90-2.30 (m, 2H), 3.40-4.65 (m, 5H), 5.38 (s, 2H), 7.35 (m, 3H), 7.48 (m, 2H), 8.34 (s, 1H), 9.14 and 9.15 (two d, 1H, J=9 Hz).

### Step 7. 2-(3-(N-t-Butoxycarbonyl)aminopyrrolidin-1-yl)-3-fluoro-9-(2,3,4,5,6-pentafluorophenyl)-6H-6-oxopyrido[1,2-a]pyrimidine-7-carboxylic acid

A 0.335 g (0.516 mmol) sample of the compound from Step 6 was dissolved in 40 mL of dry methanol, and the benzyl ester was removed by reacting with 2.0 mL of 98% formic acid in the presence of 0.100 g of 10% Pd/C, stirring under a dry N₂ atmosphere for .0.25 hours. After filtration and evaporation of the solvent, the product was purified by column chromatography on silica gel, eluting with 1:15:100 acetic acid:methanol:methylene chloride to afford, after removal of the solvent, the title compound as a yellow solid, which was taken directly to the next step.

### Step 8. 2-(3-Aminopyrrolidin-1-yl)-3-fluoro-9-(2,3,4,5,6-pentafluorophenyl)-6H-6-oxopyrido[1,2-a]pyrimidine-7-carboxylic acid hydrochloride salt

The compound from the previous step was dissolved in 10 mL of 4 N HCl in dioxane and stirred at room temperature for 0.7 hours, after which the solvent was removed under vacuum. The residue was dissolved in water which was filtered through sintered glass and freeze-dried to afford 0.232 g of the title compound as a yellow solid, mp 202-204°C. MS M/Z 459 (M-Cl). NMR (CD₃OD): δ 2.12-2.54 (m, 2H), 3.70-4.36 (m, 5H), 8.42 (s, 1H), 9.21 (d, 1H, J=9 Hz). IR (KBr): 1715, 1660, 1630 cm⁻¹. Analysis calculated for C₁₉H₁₂F₆N₄O₃·HCl·0.5H₂O: C, 45.30; H, 2.80; N, 11.12. Found: C, 45.46; H, 2.39; N, 10.57.

### Example 173

### 2-((2S, 4S)-4-(N-(S)-Alanyl-(S)-alanyl)amino-2-methylpyrrolidin-1-yl)-9-(2,4-difluorophenyl)-3-fluoro-6H-6-oxopyrido[1,2-a]pyrimidine-7-carboxylic acid hydrochloride

### Step 1. 2-((2S,4S)-4-amino-2-methylpyrrolidin-1-yl)-9-(2,4-difluorophenyl)-3-fluoro-6H-6-oxopyrido[1,2-a]pyrimidine-7-carboxylic acid benzyl ester

Following the procedure described in Example 166 Step 1, replacing the boc-protected benzyl ester compound with a 2.345 mmol sample of 2-((2S,4S)-4-*t*-butoxycarbonylamino-2-methylpyrrolidin-1-yl)-9-(2,4-difluorophenyl)-3-fluoro-6H-6-oxopyrido[1,2-a]pyrimidine-7-carboxylic acid benzyl ester, from Example 171 Step 6, the boc protecting group was removed to afford 1.06 g of the title compound.

### Step 2. 2-((2S, 4S)-4-(N-(N_Benzoyloxycarbonyl)-(S)-alanyl-(S)-alanyl)amino-2-methylpyrrolidin-1-yl)-9-(2,4-difluorophenyl)-3-fluoro-6H-6-oxopyrido[1,2-a]pyrimidine-7-carboxylic acid benzyl ester

Following the procedure of Example 168 Step 1, replacing the benzyl ester compound of that example with 1.06 g of the compound from Step 1 above, 0.98 g of the title compound was prepared.

### Step 3. 2-((2S, 4S)-4-(N-(S)-Alanyl-(S)-alanyl)amino-2-methylpyrrolidin-1-yl)-9-(2,4-difluorophenyl)-3-fluoro-6H-6-oxopyrido[1,2-a]pyrimidine-7-carboxylic acid hydrochloride

Following the procedure of Example 168 Step 2, replacing the boc-protected benzyl ester compound of that example with the compound from Step 2 above, 0.66 g of the title compound was prepared. Mp 198-200°C. MS M/Z 561 (M-Cl). NMR (CD₃OD): δ 1.14 and 1.40 (two d, 3H, J=7 Hz), 1.34 and 1.35 (two d, 3H, J=7 Hz), 1.50 and 1.51 (two d, 3H, J=7 Hz), 1.96-2.11 (m, 2H), 3.50-4.60 (m, 6H), 7.40 (m, 2H), 7.47 (m, 1H), 8.26 and 8.29 (two s, 1H), 9.12 and 9.16 (two d, 1H, J=9 Hz).

### Example 174

### 9-(2,4-Difluorophenyl)-3-fluoro-2-hydroxy-4-methyl-6H-6-oxopyrido[1,2-a]pyrimdine-7-carboxylic acid ethyl ester

### Step 1. 2-(2,4-Difluorobenzyl)-5-fluoro-4-hydroxy-6-methylpyrimidine

A mixture of 8.6 g (0.0445 mmol) of 2-(2,4-difluorophenyl)-acetamidine hydrochloride, prepared as in Example 159 Step 1, and 6.1 g (0.0405 mmol) of ethyl 2-fluoro-3-oxo-butanoate (prepared as described by E. O. Bergmann, S. Cohen, and I. Shahak, *J. Chem .Soc..*, 3278₋ (1959)), in 30 mL of anhydrous methanol and 10.1 mL of a 2.5% solution of sodium methoxide was heated at reflux under a dry N₂ atmosphere for 16 hours. The solvent was removed by evaporation under vacuum, and the residue was washed with water, then 200 mL of water added and the mixture was acidified and the resulting precipitate was filtered off. The aqueous solution was then extracted (3x) with methylene chloride. The solvent was washed with water, dried over anhydrous magnesium sulfate, and the solvent was removed by evaporation under vacuum to give a dark solid. The solid was washed with ethyl ether and dried, then combined with the earlier precipitate which was recrystallized from methanol:ether to afford 4.51 g of the title compound. MS M/Z 272 (M+NH₄). NMR: (CDCl₃) δ 2.22 (d, 3H, J=4 Hz), 3.92 (s, 2H), 6.92 (m, 2H), 7.30 (m, 1H).

### Step 2. 3-(2,4-Difluorophenyl)-2-ethoxy-3-(5-fluoro-4-hydroxy-6-methylpyrimidin-2-yl)propane-1,1-dicarboxylic acid diethyl ester

A 0.615 g (2.42 mmol) sample of the compound from Step 1 above was dissolved in THF and cooled to -78°C with stirring under a dry N₂ atmosphere. To this was slowly added 1.98 mL of 2.5 N n-butyllithium in hexane, and the mixture was stirred for 30 min. Then 0.586 mL (2.9 mmol) of diethyl ethoxymethylenemalonate was added at -78°C and the mixture stirred for an additional 15 min at room temperature. The reaction mixture was quenched with 10% HCl until the mixture was about pH 3, whereupon it was then extracted with ethyl acetate. This was dried over anhydrous magnesium sulfate, and the solvent was removed by evaporation under vacuum to afford 1.6 g of the title compound as a yellow oil. This material was taken directly to the next step.

### Step 3. 9-(2,4-Difluorophenyl)-3-fluoro-2-hydroxy-4-methyl-6H-6-oxopyrido[1,2-a]pyrimidine-7-carboxylic acid ethyl ester

The compound from Step 2 was dissolved in toluene, 0.62 mL of DBU was added and the mixture heated at reflux in a flask equipped with a Dean-Stark condenser for 16 hours under a dry N₂ atmosphere. The mixture was removed from the heat and stirred with 70 mL of water for 2 hours. After separation, the organic phase was dried over magnesioum sulfate, and the solvent was removed by evaporation. The residue was purified by column chromatography on silica gel, eluting with 1:5:100 acetic acid:methanol:methylene chloride to afford 0.175 g of the title compound as a yellow solid. MS M/Z: 379 (M+H). NMR:(DMSO-d₆) δ 1.21 (t, 3H, J=7 Hz), 2.07 (d, 3H, J=4 Hz), 4.10 (q, 2H, J=7 Hz), 7.03 (m, 1H), 7.16 (m, 1H), 7.38 (m, 1H), 7.66 (s, 1H).

### Examples 175-178

By following the procedures described in Example 174 and substituting the appropriate ester for ethyl 2-fluoro-3-oxobutyrate, Examples 175-178 may be prepared as disclosed in Table 6 (where R = ethyl and R¹ = 2,4-difluorophenyl).

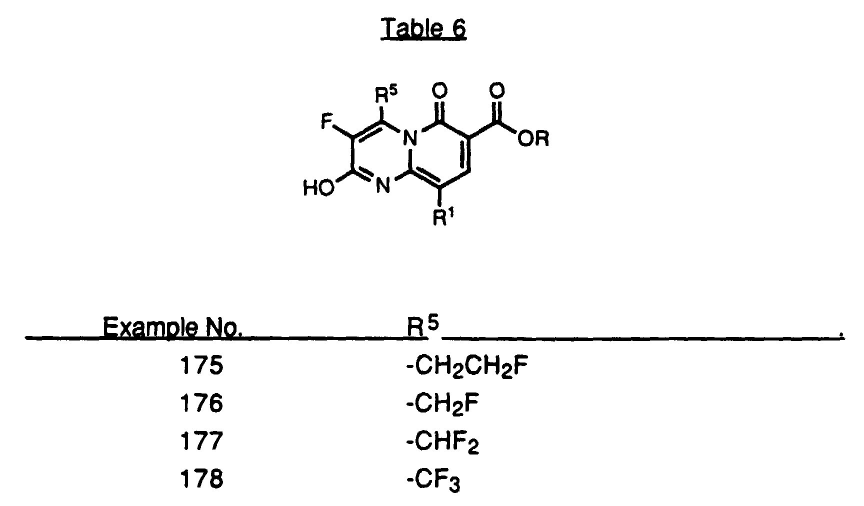

### Examples 179-195

By following the procedures described in Example 160 Steps 3, 4 and 5 and Example 161, and replacing 2-(N-t-butoxycarbonylamino)pyrrolidine in Step 4 with the appropriate N-methyl- or boc-protected amine, Examples 179-195 may be prepared as disclosed in Table 7 (where R¹ = 2.4-difluorophenyl).

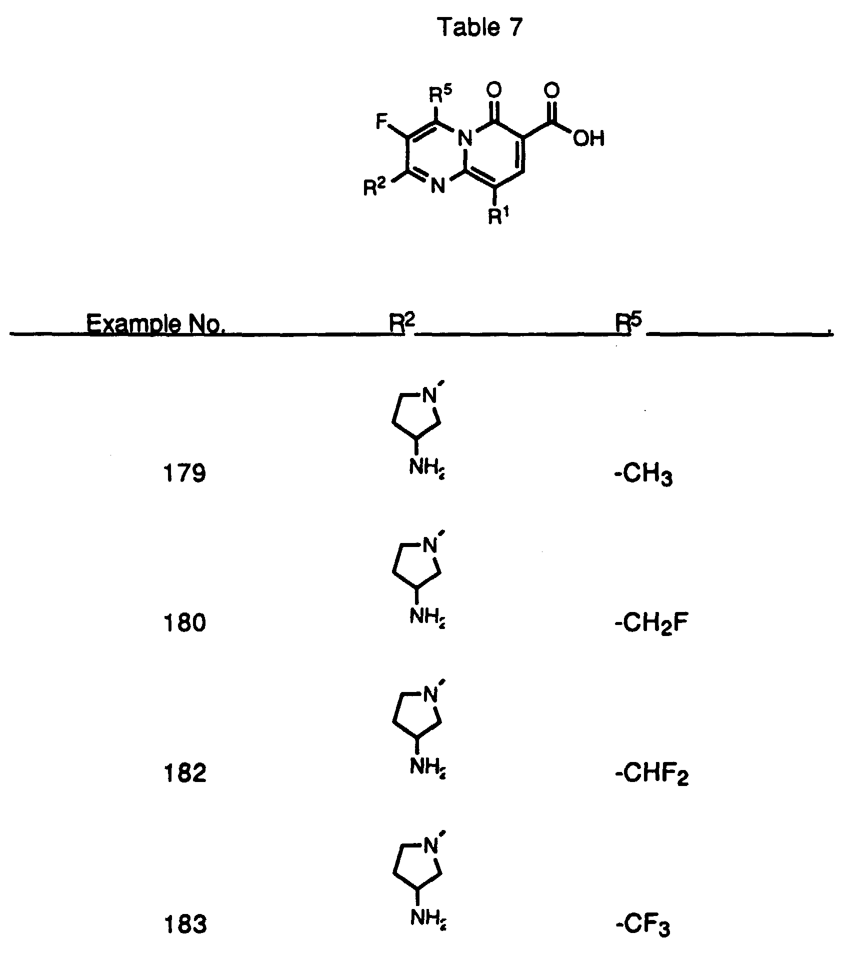

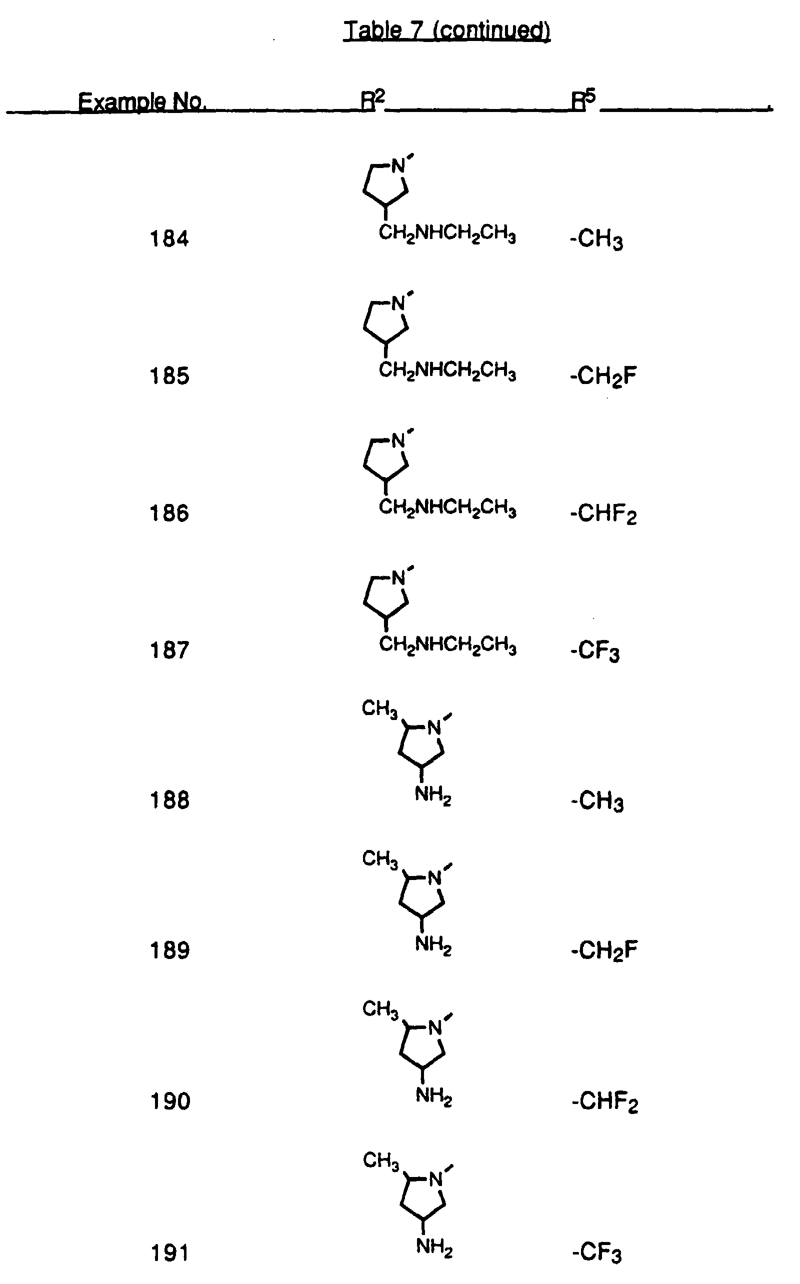

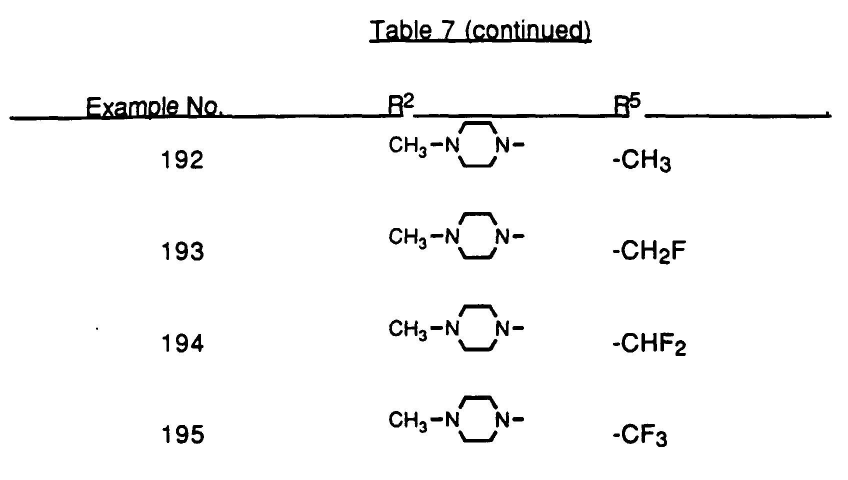

### Examples 196-240

By following the procedures described in Example 160 Steps 3, 4 and 5 and Example 161, replacing 2-(N-t-butoxycarbonylamino)pyrrolidine in Step 4 with the appropriate substituted or boc-protected amine and replacing 9-(2,4-difluorophenyl)-3-fluoro-2-hydroxy-6H-6-oxopyrido[1,2-a]pyrimidine-7-carboxylic acid benzyl ester with the compound containing the appropriate R¹ group (as described in Examples 2 and 39), Examples 196-240 may be prepared as disclosed in Table 8.

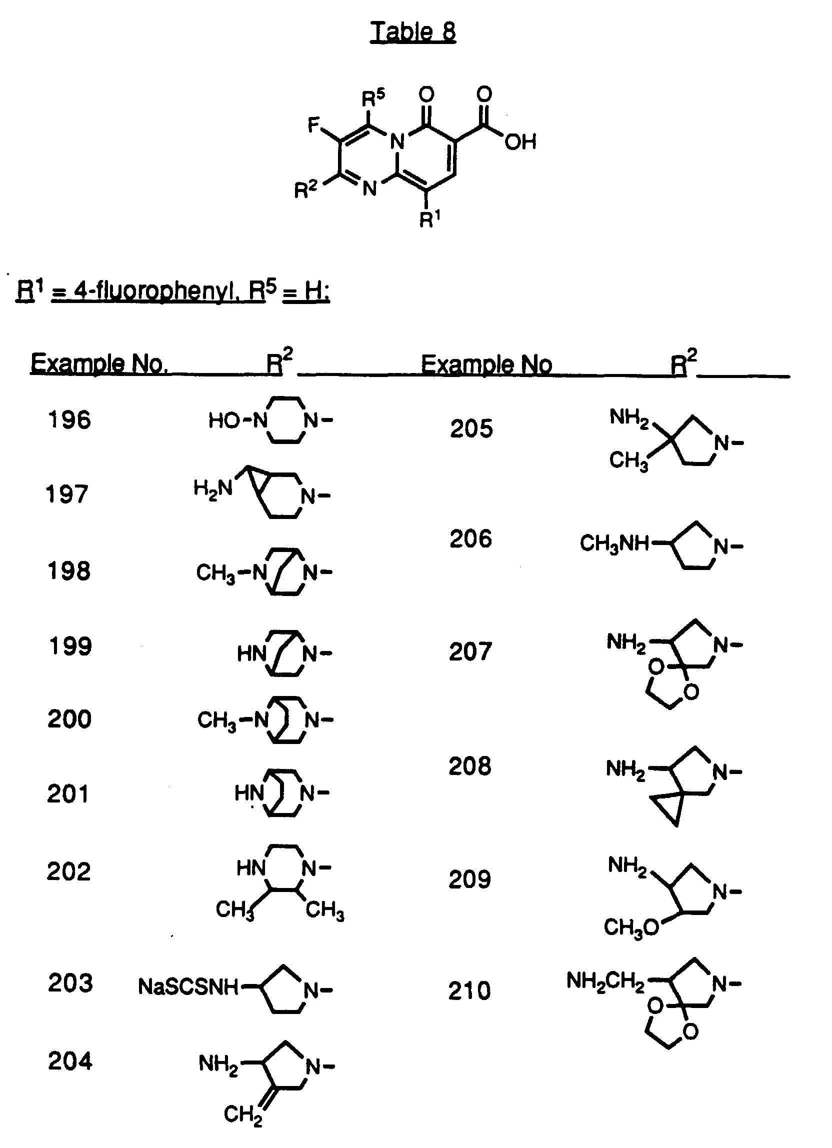

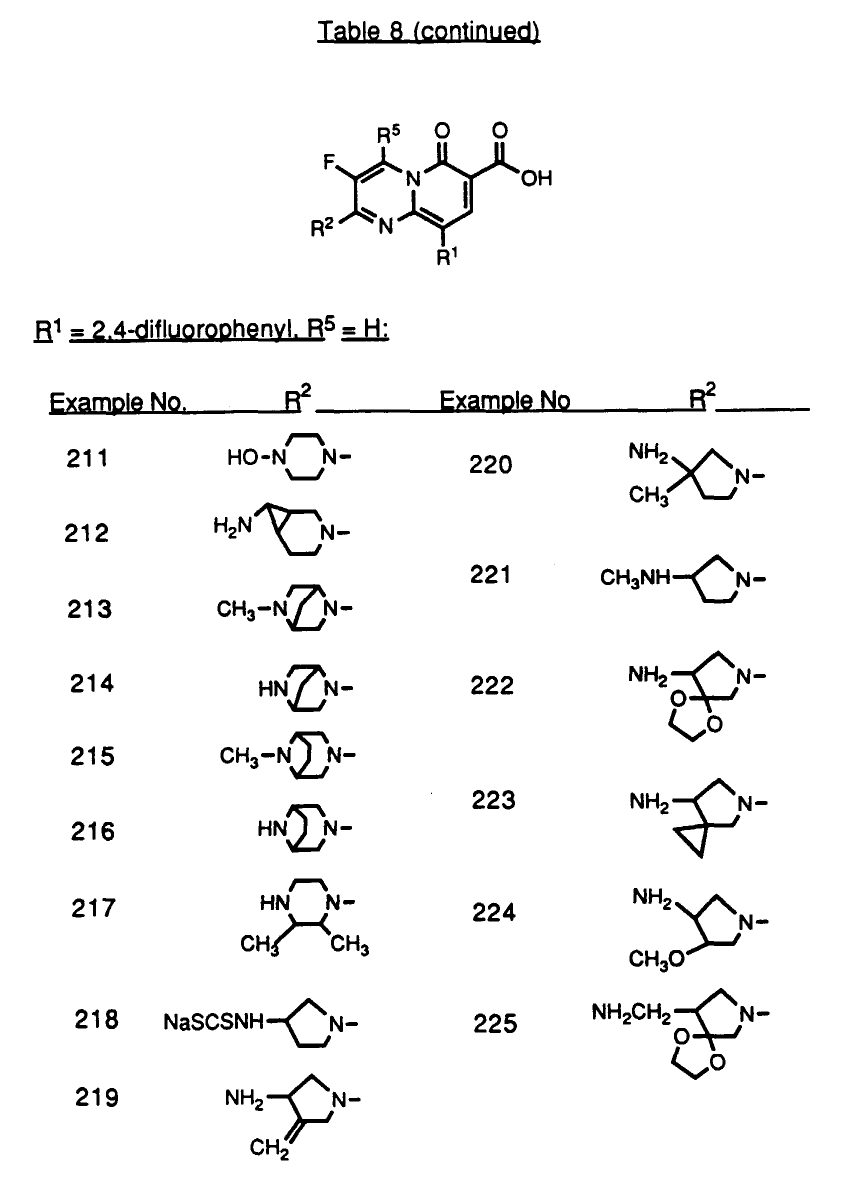

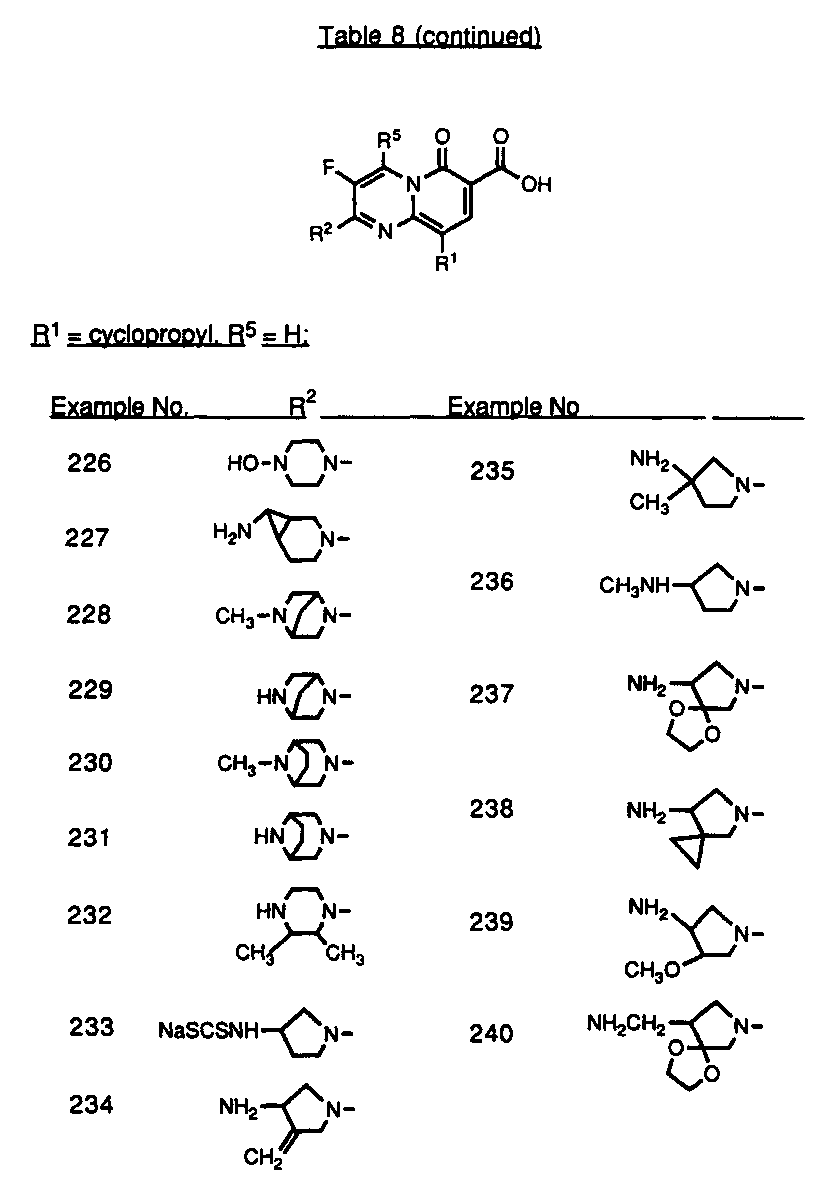

### Examples 241-250

By following the procedures of Example 157 Steps 2-8, replacing 2-cyclopropyl-2-ethoxycarbonylacetamidine hydrochloride in Step 2 with the compound containing the appropriate R¹ group (refer to compound 6B in Scheme II), and replacing the 3-(N-*t*-butoxycarbonyl)aminopyrrolidine in Step 6 with the appropriately protected amine, Examples 241-250 may be prepared as disclosed in Table 9 (in which R⁵ is H).

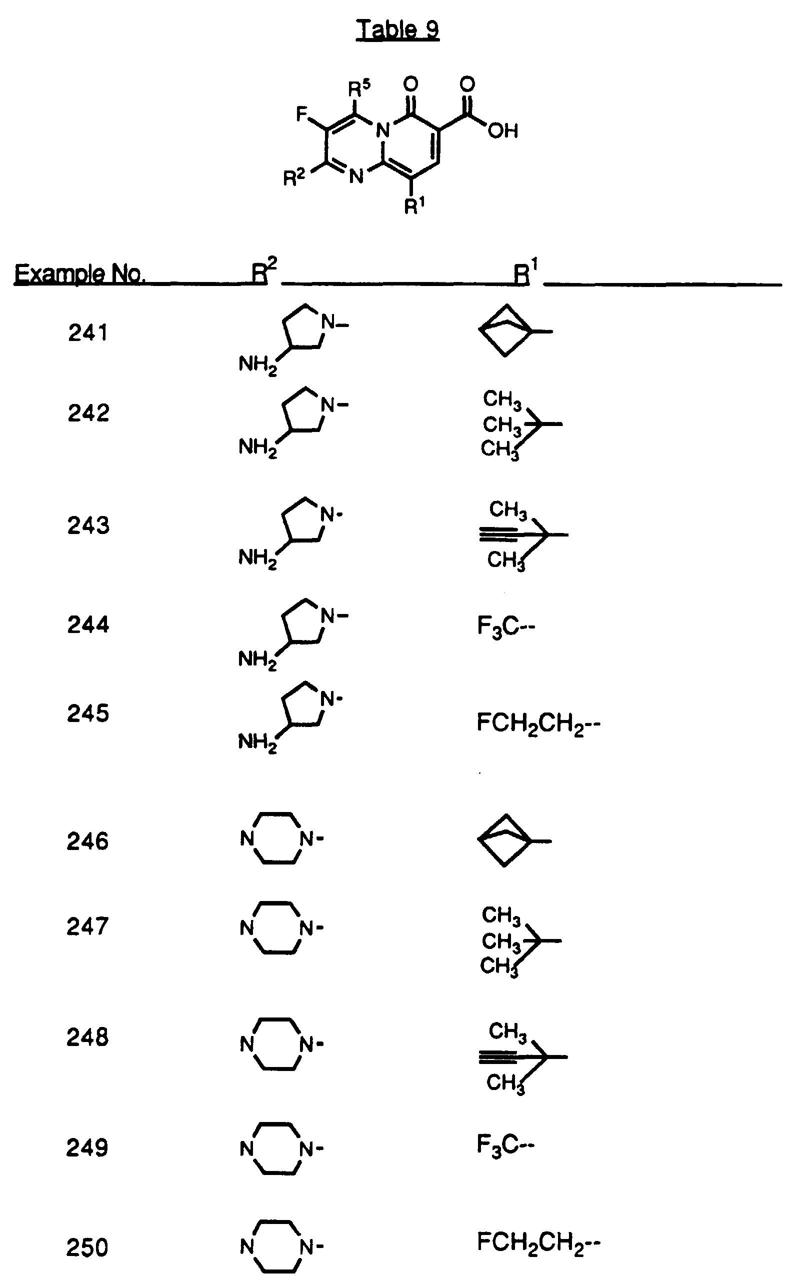

### Examples 251-252

By following the procedures of Example 157, steps 2-8, replacing replacing 2-cyclopropyl-2-ethoxycarbonylacetamidine hydrochloride in Step 2 with 2-(N-benzoyloxycarbonyl-N-methylamino)-2-ethoxycarbonylacetamidine hydrochloride , and replacing the 3-(N-*t*-butoxycarbonyl)aminopyrrolidine in Step 6 with the appropriately protected amine, Examples 251-252 may be prepared as disclosed in Table 10 (in which R⁵= H).

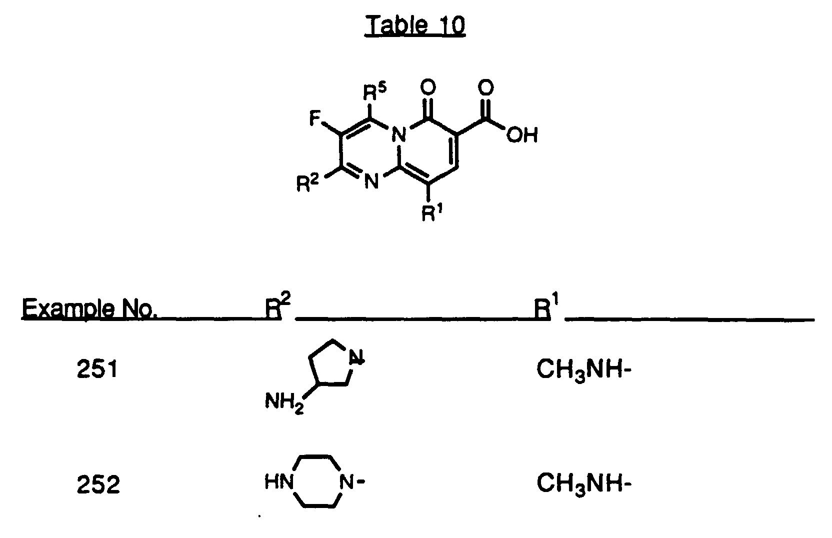

### Example 253

### In Vitro Assay of Antibacterial Activity

The *in vitro* antibacterial activity of the compounds of the present invention was demonstrated as follows: Minimum inhibitory concentrations (MICs) were determined by the agar dilution method, in which twelve petri dishes were prepared, each containing successive aqueous 2-fold dilutions of the test compounds mixed with 10 mL of sterilized Brain Heart Infusion (BHI) agar. Each plate was inoculated with 1:100 (or 1:10 for slow growing strains, primarily *Micrococcus* and *Streptococcus*) dilutions of up to 32 different microorganisms, using a Steers replicator block calibrated to deliver approximately 10⁴ colony forming units (CFUs). The inoculated plates were incubated at from about 35°C to about 37°C for approximately 20-24 hours. In addition, a control plate using BHI agar containing no test compound was prepared and incubated at the beginning and at the end of each test. Ciprofloxacin was used as the control antibiotic.

After incubation, each petri dish was observed for the presence or absence of microorganism growth. The MIC was defined as the lowest concentration of test compound yielding no growth (a slight haze or sparsely isolated colonies at the inoculum spot) as compared to the growth control containing no test compound.

The results of the above tests, shown in Table 11 below, demonstrate that the compounds of the present invention are effective in combating bacterial growth.

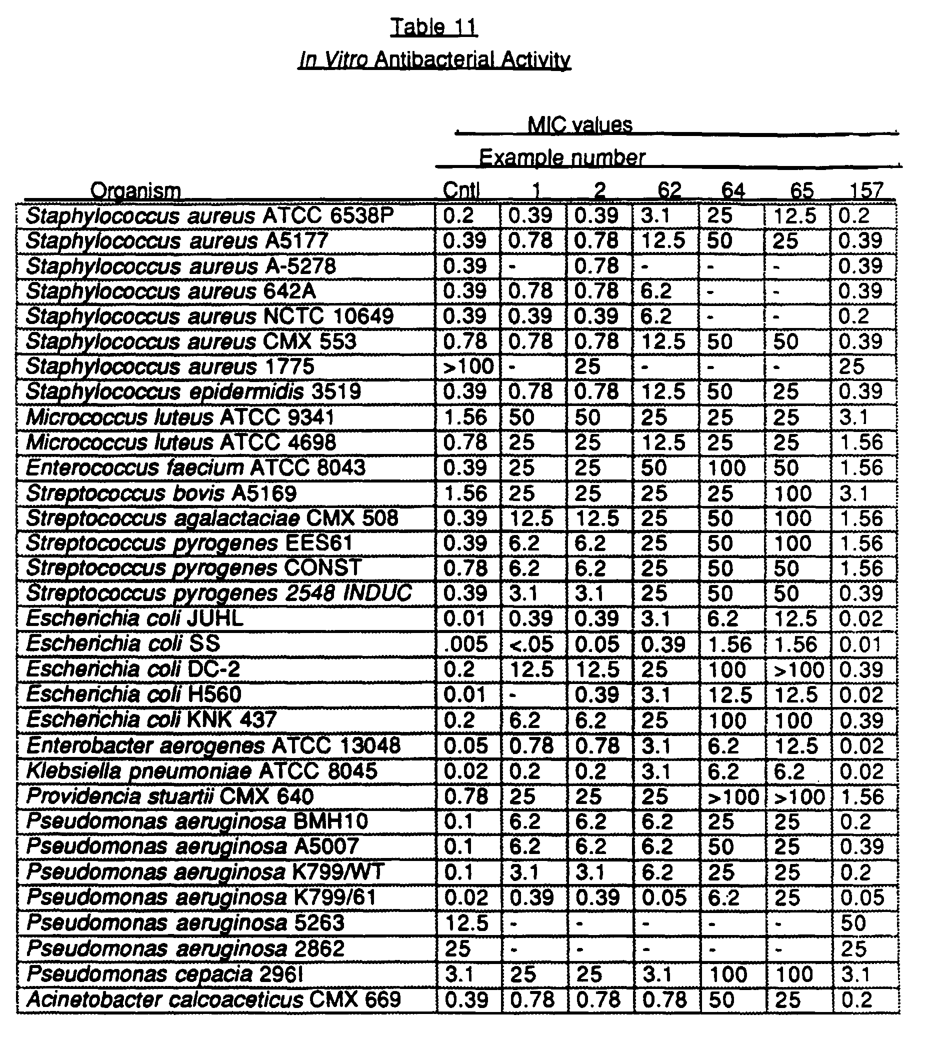

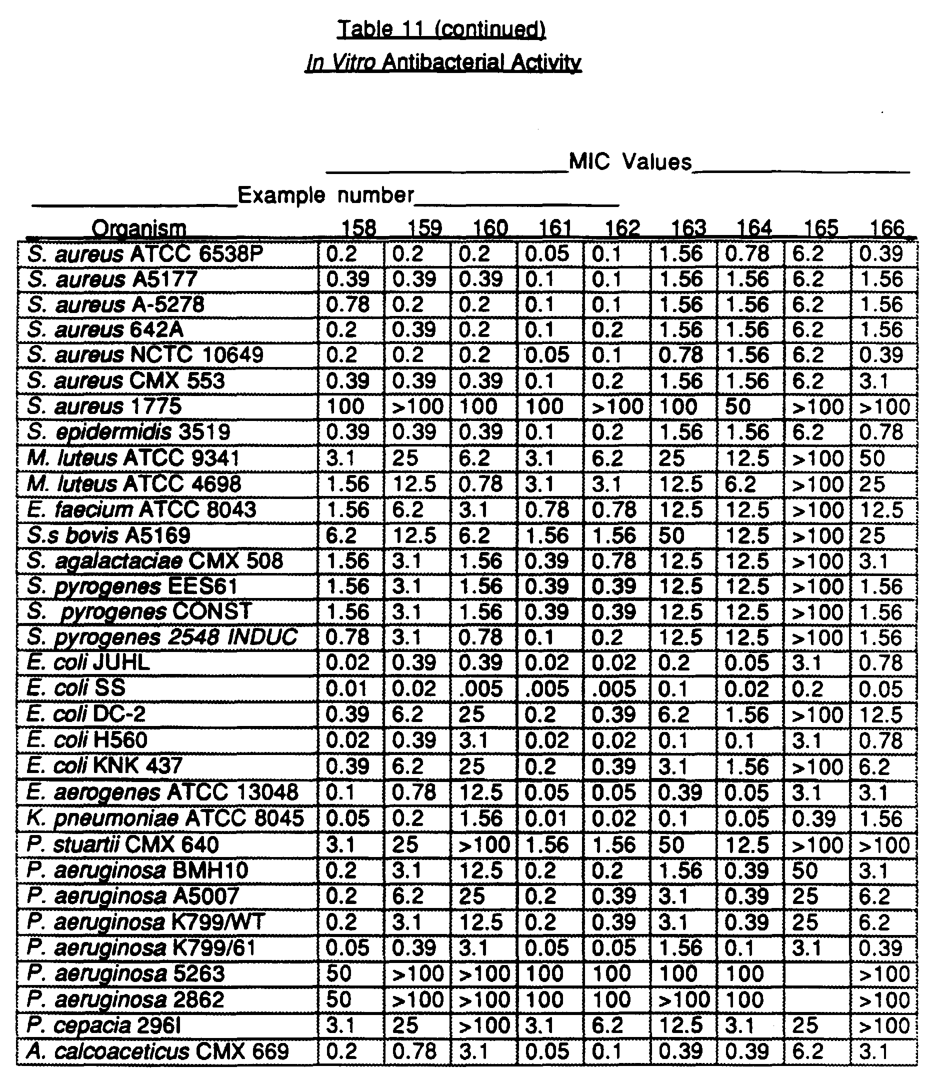

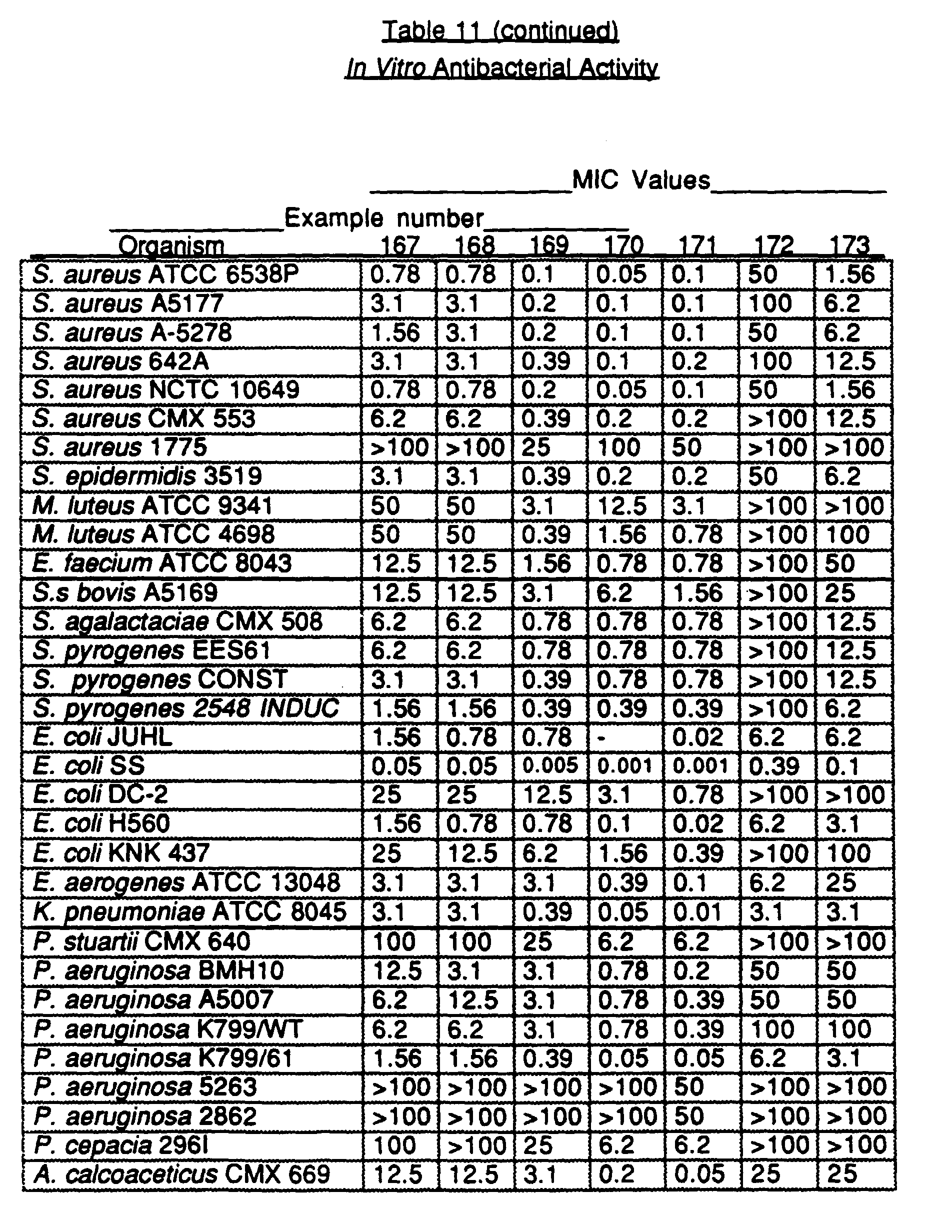

### Example 254

### In vivo Assay of Antibacterial Activity in Mice

The *in vivo* antibacterial activity of the compounds of the present invention was determined as follows: Aqueous solutions of the test compounds were prepared by dissolving the compound in distilled water, except for the compounds of examples 169 and 170 which were dissolved in a solution of 2% DMSO in distilled water.

After 18 hr incubation, a culture of *Staphylococcus aureus* NCTC 10649 was serially diluted by using 10-fold dilutions in 5% (w/v) hog gastric mucin. Cultures (0.5 mL) in a dilution from 10⁻¹ to 10⁻⁸ were injected intraperitoneally into CF-1 female mice weighing approximately 20g. The LD₅₀ (median lethal dose) for the test organism was calculated from the cumulative mortalities on the sixth day by the using the Reed and Muench procedure (Reed, L.J., and H. Muench, *Amer. J. Hygiene*, 27, 493 (1938)).

The above 18 hr culture was diluted in 5% (w/v) hog gastric mucin to obtain 100 times the LD₅₀, and 0.5 mL was injected intraperitoneally into mice. The mice were treated subcutaneously (sc) or orally (po) with a specific amount of the test compound divided equally to be administered at 1 and 5 hr after infection. A group of 10 animals each for at least three dose levels was thus treated, and the deaths were recorded daily for 6 days. Ten mice were left untreated as infection control. ED₅₀ values were calculated from the cumulative mortalities on the sixth day after infection by using the trimmed version of the Logit method ( Hamilton, M.A., R.C. Russo, and R.V. Thurston, *Environ. Sci. Technol.* 11, 714 (1977)).

The LD₅₀ for *Escherichia coli* JUHL was determined in a manner similar to that above for *S. aureus*. The 18 hr cultures of this organism were also prepared for po and sc testing in mice, from which ED₅₀ values were calculated. Positive controls were run with temafloxacin as the test compound, for which ED₅₀ values were also calculated.

The results of these tests, shown below in Table 12, demonstrate that the compounds of the present invention are active *in vivo* against bacterial pathogens.

**Table 12**

| *In Vivo* Activity Against Bacteria | | | | |
|---|---|---|---|---|
| | ED₅₀ (mg/kg/day) | | | |
| | po | | sc | |
| Example # | *S. aureus* | *E. coli* JUHL | *S. aureus* | *E. coli* JUHL |
| 162 | 12 | 5.6 | 4.4 | 1.0 |
| 166 | >24.0 | 25.0 | 8.0 | <2.5 |
| 167 | 14.4 | 16.6 | 2.7 | <2.5 |
| 168 | 3.0 | 14.3 | 2.8 | <2.5 |
| 169 | >48 | nt | 7.5 | nt |
| 171 | 12.8 | >20.0 | >8.0 | 4.8 |
| temafloxacin | 1.5 | 1.5 | 1.3 | 0.5 |

| | | | | |
|---|---|---|---|---|
| nt = not tested | | | | |

It is understood that the foregoing detailed description and accompanying examples are merely illustrative and are not to be taken as limitations upon the scope of the invention, which is defined solely by the appended claims and their equivalents. Various changes and modifications to the disclosed embodiments will be apparent to those skilled in the art. Such changes and modifications, including without limitation those relating to the chemical structures, substituents, derivatives, intermediates, syntheses, formulations and/or methods of use of the invention, may be made without departing from the spirit and scope thereof. Accordingly, it is intended that all such changes and modifications be covered by the appended claims and their equivalents.

## Claims (Claims for the following Contracting State(s): AT, BE, CH, LI, DE, DK, FR, GB, IT, LU, NL, SE)

1. A compound of the formula 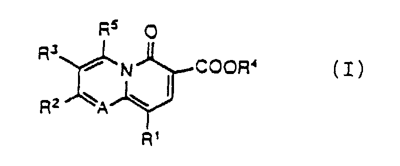 or a pharmaceutically acceptable salt, ester, or amide thereof, wherein
R¹ is selected from the group consisting of (a) C₁-C₆ alkyl, (b) C₂-C₆ alkenyl, (c) C₁-C₆ haloalkyl, (d) C₁-C₆ alkoxy, (e) C₃-C₈ cycloalkyl, (f) phenyl, (g) halo, (h) cyano, (i) nitro, (j) bicycloalkyl having from 4 to 9 carbon atoms in the ring system where non-adjacent atoms are bridged by 1-3 additional carbon atoms, (k) C₂-C₆ alkynyl, (l) (C₁-C₆ alkoxy)carbonyl, (m) nitrogen-containing aromatic heterocycle having from 5 to 7 ring atoms and (n) a group of the formula -NR⁷R⁸ wherein R⁷ and R⁸ are independently selected from the group consisting of hydrogen, C₁-C₆ alkyl and C₁-C₈ alkanoyl or, taken together with the nitrogen atom to which they are attached, R⁷ and R⁸ form a 5-, 6- or 7-membered saturated heterocycle optionally substituted with a C₆-C₁₀ carbocyclic aryl-C₁-C₆ alkyl, (C₁-C₆ alkoxy)carbonyl, C₁-C₆ alkyl, C₁-C₆ haloalkyl, amino-substituted C₁-C₆ alkyl wherein the amino groups may have one or two C₁-C₆ alkyl substituents or one alpha amino acid or polypeptide residue substituent, hydroxy-substituted C₁-C₆ alkyl, hydroxy, C₁-C₆ alkoxy or halo, or an amino, mono- or di-(C₁-C₆ alkyl)amino, or C₁-C₈ alkanoylamino, where the amino group may be substituted with C₁-C₈ alkanoyl, an alpha-amino acid or a polypeptide of from two to five amino acids;
R² is selected from the group consisting of halo, C₁-C₆ alkoxy, C₆-C₁₀ carbocyclic aryloxy, C₆-C₁₀ carbocyclic aryl-C₁-C₆ alkoxy, C₁-C₆ alkyl, C₂-C₆ alkenyl, C₃-C₈ cycloalkyl, C₄-C₈ cycloalkenyl, C₆-C₁₀ carbocyclic aryl-C₁-C₆ alkyl, C₃-C₈ cycloalkyl-C₁-C₆ alkyl, phenyl, amino, mono- or di-(C₁-C₆ alkyl)amino, C₆-C₁₀ carbocyclic aryl-C₁-C₆ alkylamino, (hydroxy-substituted C₁-C₆ alkyl)amino, bicyclic nitrogen-containing heterocyclic group selected from one of the formulae 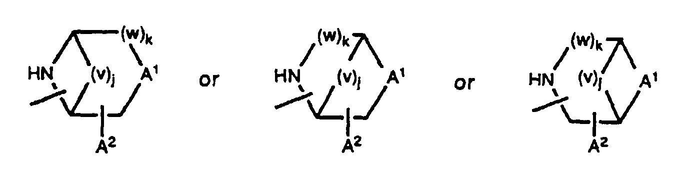 wherein v and w are CH₂; j and k are independently 1, 2 or 3; A¹ is a carbon atom or a heteroatom selected from S, O and N; and A² is one or more non-hydrogen substituents selected independently from the group consisting of C₁-C₆ alkyl, C₁-C₆ haloalkyl, hydroxy-substituted C₁-C₆ alkyl, hydroxy, halo, amino-substituted C₁-C₆ alkyl wherein the amino groups may have one or two C₁-C₆ alkyl substituents or one alpha amino acid or polypeptide residue substituent, C₁-C₈ alkanoylamino, phenyl and a group having the formula -NR⁷R⁸ wherein R⁷ and R⁸ are independently selected from hydrogen and C₁-C₆ alkyl or, when one is hydrogen, the other is an alpha-amino acid or a polypeptide residue of from 2 to 5 amino acids or R² can be a nitrogen-containing heterocycle having the formula 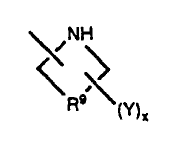 wherein x is 0 to 3; R⁹ is selected from the group consisting of (a) -(CH₂)ₘ-wherein m is 1, 2 or 3, and (b) -(CH₂)ₙR¹⁰(CH₂)ₚ- wherein R¹⁰ is S, O or N, n is 1 or 2, and p is 1 or 2; and Y is a non-hydrogen substituent independently selected from the group consisting of C₁-C₆ alkyl, C₁-C₆ haloalkyl, C₁-C₆ alkoxy, hydroxy-substituted C₁-C₆ alkyl, hydroxy, amino-substituted C₁-C₆ alkyl wherein the amino groups may have one or two C₁-C₆ alkyl substituents or one alpha amino acid or polypeptide residue substituent, halogen and a group having the formula -NR¹¹R¹² wherein R¹¹ and R¹² are independently selected from the group consisting of hydrogen and C₁-C₆ alkyl or, when one is hydrogen, the other is selected from the group consisting of a C₁-C₈ alkanoyl, an alpha-amino acid and a polypeptide residue of from 2 to 5 amino acids;
R³ is selected from the group consisting of hydrogen, halo and C₁-C₆ alkoxy;
R⁴ is selected from the group consisting of hydrogen, C₁-C₆ alkyl, a pharmaceutically acceptable cation and a prodrug ester group, said prodrug ester group being an ester group which is hydrolyzable under physiological conditions;
R⁵ is selected from the group consisting of hydrogen, halo, hydroxy, C₁-C₆ alkyl, C₁-C₆ haloalkyl, C₁-C₆ alkoxy, and a group having the formula -NR¹³R¹⁴ wherein R¹³ and R¹⁴ are independently selected from the group consisting of hydrogen, C₁-C₆ alkyl, hydroxy-substituted C₁-C₆ alkyl, C₁-C₆ alkoxy-substituted C₁-C₆ alkyl and C₁-C₈ alkanoyl; and
A is N or CR⁶, wherein R⁶ is selected from the group consisting of halogen, C₁-C₆ alkyl, C₁-C₆ haloalkyl, hydroxy-substituted C₁-C₆ alkyl, C₁-C₆ alkoxy-C₁-C₆ alkyl, C₁-C₆ alkoxy and amino-substituted C₁-C₆ alkyl wherein the amino groups may have one or two C₁-C₆ alkyl substituents or one alpha amino acid or polypeptide residue substituent or wherein R1 and R6, taken together with the atoms to which they are attached, form a 6-membered saturated ring which may contain an oxygen or a sulfur atom and which may be substituted with C₁-C₆ alkyl;
wherein cycloalkyl by itself or as part of another group, cycloalkenyl or bicycloalkyl may be substituted with a C₆-C₁₀ carbocyclic aryl-C₁-C₆ alkyl, (C₁-C₆ alkoxy)carbonyl, C₁-C₆ alkyl, C₁-C₆ haloalkyl, amino-substituted C₁-C₆ alkyl wherein the amino groups may have one or two C₁-C₆ alkyl substituents or one alpha amino acid or polypeptide residue substituent, hydroxy-substituted C₁-C₆ alkyl, hydroxy, C₁-C₆ alkoxy or halo, or an amino, mono- or di-(C₁-C₆ alkyl)amino, or C₁-C₈ alkanoylamino, where the amino group may be substituted with C₁-C₈ alkanoyl, an alpha-amino acid or a polypeptide of from two to five amino acids.

2. A compound according to Claim 1 wherein A is N or CR⁶ and R⁶ is selected from the group consisting of halo, C₁-C₆ alkyl, C₁-C₆ haloalkyl, hydroxy-substituted C₁-C₆ alkyl, C₁-C₆ alkoxy-C₁-C₆ alkyl, C₁-C₆ alkoxy and amino-substituted C₁-C₆ alkyl wherein the amino groups may have one or two C₁-C₆ alkyl substituents or one alpha amino acid or polypeptide residue substituent.

3. A compound according to Claim 2 wherein R² is a nitrogen-containing heterocycle having the formula 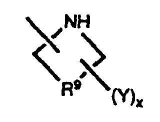 wherein R9, Y and x are as defined in Claim 1.

4. A compound according to Claim 1 having the formula 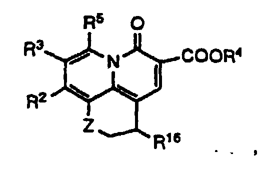 wherein
Z is selected from the group consisting of CH₂, O and S, and R¹⁶ is C₁-C₆ alkyl.

5. A compound according to Claim 4 wherein Z is O and R² is a nitrogen-containing heterocycle having the formula 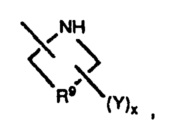 wherein R⁹, Y and x are as defined in Claim 1.

6. A compound selected from the group consisting of:
3-fluoro-9-(4-fluorophenyl)-2-(4-methylpiperazin-1-yl)-6(H)-6-oxo-pyrido-[1,2-a]pyrimidine-7-carboxylic acid;
9-(2,4-difluorophenyl)-3-fluoro-2-(4-methylpiperazin-1-yl)-6(H)-6-oxo-pyrido[1,2-a]pyrimidine-7-carboxyilc acid;
3-fluoro-9-cyclopropyl-2-(4-methylpiperazin-1-yl)-6(H)-6-oxo-pyrido-[1,2-a]pyrimidine-7-carboxylic acid;
2-(3-aminopyrrolidin-1-yl)-9-cyclopropyl-3-fluoro-6H-6-oxo-pyrido-[1,2-a]pyrimidine-7-carboxylic acid hydrochloride salt;
2-(3-aminopyrrolidin-1-yl)-9-cyclopropyl-3-fluoro-6H-6-oxo-pyrido-[1,2-a]pyrimidine-7-carboxylic acid;
9-(2,4-difluorophenyl)-3-fluoro-2-(4-methylpiperazin-1-yl)-6H-6-oxo-pyrido[1,2-a]pyrimidine-7-carboxylic acid;
2-(3-aminopyrrolidin-1-yl)-9-(2,4-difluorophenyl)-3-fluoro-6H-6-oxo-pyrido[1,2-a]pyrimidine-7-carboxylic acid;
2-(3-(N-*t*-butoxycarbonyl)aminopyrrolidin-1-yl)-9-(2,4-difluorophenyl)-3-fluoro-6H-6-oxopyrido[1,2-a]pyrimidine-7-carboxyiic acid;
2-(3-aminopyrrolidin-1-yl)-9-(2,4-difluorophenyl)-3-fluoro-6H-6-oxo-pyrido[1,2-a]pyrimidine-7-carboxylic acid;
9-cyclopropyl-3-fluoro-2-(4-methylpiperazin-1-yl)-6H-6-oxo-pyrido-[1,2-a]pyrimidine-7-carboxylic acid;
9-cyclopropyl-3-fluoro-2-(piperazin-1-yl)-6H-6-oxo-pyrido[1,2-a]pyrimidine-7-carboxylic acid;
9-cyclopropyl-3-fluoro-2-(morpholin-1-yl)-6H-6-oxo-pyrido[1,2-a]pyrimidine-7-carboxylic acid;
9-(2,4-difluorophenyl)-3-fluoro-2-(3-(N-(S)-norvalyl)aminopyrrolidin-1-yl)-6H-6-oxopyrido[1,2-a]pyrimidine-7-carboxylic acid hydrochloride salt;
2-(3-(N-(S)-alanyl)aminopyrrolidin-1-yl)-9-(2,4-difluorophenyl)-3-fluoro-6H-6-oxopyrido[1,2-a]pyrimidine-7-carboxylic acid hydrochloride;
2-(3-(N-(S)-alanyl-(S)-alanyl)aminopyrrolidin-1-yl)-9-(2,4-difluorophenyl)-3-fluoro-6H-6-oxopyrido[1,2-a]pyrimidino-7-carboxylic acid hydrochloride;
2-((2S,4S)-4-acetamido-2-methylpyrrolidin-1-yl)-9-(2,4-difluorophenyl)-3-fluoro-6H-6-oxopyrido[1,2-a]pyrimidine-7-carboxylic acid;
9-(2,4-difluorophenyl)-3-fluoro-2-(3-hydroxypyrrolin-1-yl)-6H-6-oxo-pyrido[1,2-a]pyrimidine-7-carboxylic acid; and
2-((2S,4S)-4-amino-2-methylpyrrolidin-1-yl)-9-(2,4-difluorophenyl)-3-fluoro-6H-6-oxopyrido[1,2-a]pyrimidine-7-carboxylic acid hydrochloride.

7. A pharmaceutical composition for treating or preventing a bacterial infection comprising a therapeutically effective amount of a compound according to Claim 1 and a pharmaceutically acceptable carrier.

8. A pharmaceutical composition for treating or preventing a bacterial infection comprising a therapeutically effective amount of a compound according to Claim 6 and a pharmaceutically acceptable carrier.

9. A compound according to Claim 1 or 6 for use as a therapeutic agent.

10. Use of a compound according to Claim 1 for manufacturing a medicament for treating or preventing a bacterial infection in a human or other animal host.

11. Use of a compound according to Claim 6 for manufacturing a medicament for treating or preventing a bacterial infection in a human or other animal host.

## Claims (Claims for the following Contracting State(s): ES)

1. A process for preparing a compound of the formula 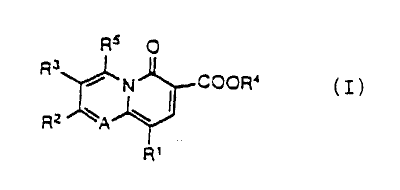 or a pharmaceutically acceptable salt, ester, or amide thereof, wherein
R¹ is selected from the group consisting of (a) C₁-C₆ alkyl, (b) C₂-C₆ alkenyl, (c) C₁-C₆ haloalkyl, (d) C₁-C₆ alkoxy, (e) C₃-C₈ cycloalkyl, (f) phenyl, (g) halo, (h) cyano, (i) nitro, (j) bicycloalkyl having from 4 to 9 carbon atoms in the ring system where non-adjacent atoms are bridged by 1-3 additional carbon atoms, (k) C₂-C₆ alkynyl, (l) (C₁-C₆ alkoxy)carbonyl, (m) nitrogen-containing aromatic heterocycle having from 5 to 7 ring atoms and (n) a group of the formula -NR⁷R⁸ wherein R⁷ and R⁸ are independently selected from the group consisting of hydrogen, C₁-C₆ alkyl and C₁-C₈ alkanoyl or, taken together with the nitrogen atom to which they are attached, R⁷ and R⁸ form a 5-, 6- or 7-membered saturated heterocycle optionally substituted with a C₆-C₁₀ carbocyclic aryl-C₁-C₆ alkyl, (C₁-C₆ alkoxy)carbonyl, C₁-C₆ alkyl, C₁-C₆ haloalkyl, amino-substituted C₁-C₆ alkyl wherein the amino groups may have one or two C₁-C₆ alkyl substituents or one alpha amino acid or polypeptide residue substituent, hydroxy-substituted C₁-C₆ alkyl, hydroxy, C₁-C₆ alkoxy or halo, or an amino, mono- or di-(C₁-C₆ alkyl)amino, or C₁-C₈ alkanoylamino, where the amino group may be substituted with C₁-C₈ alkanoyl, an alpha-amino acid or a polypeptide of from two to five amino acids;
R² is selected from the group consisting of halo, C₁-C₆ alkoxy, C₆-C₁₀ carbocyclic aryloxy, C₆-C₁₀ carbocyclic aryl-C₁-C₆ alkoxy, C₁-C₆ alkyl, C₂-C₆ alkenyl, C₃-C₈ cycloalkyl, C₄-C₈ cycloalkenyl, C₆-C₁₀ carbocyclic aryl-C₁-C₆ alkyl, C₃-C₈ cycloalkyl-C₁-C₆ alkyl, phenyl, amino, mono- or di-(C₁-C₆ alkyl) amino, C₆-C₁₀ carbocyclic aryl-C₁-C₆ alkylamino, (hydroxy-substituted C₁-C₆ alkyl)amino, bicyclic nitrogen-containing heterocyclic group selected from one of the formulae 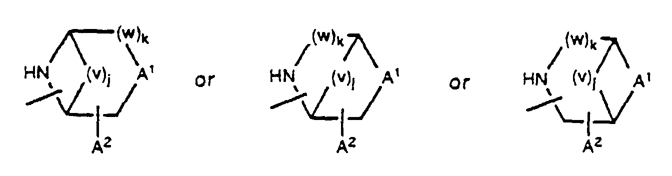 wherein v and w are CH₂; j and k are independently 1, 2 or 3; A¹ is a carbon atom or a heteroatom selected from S, O and N; and A² is one or more non-hydrogen substituents selected independently from the group consisting of C₁-C₆ alkyl, C₁-C₆ haloalkyl, hydroxy-substituted C₁-C₆ alkyl, hydroxy, halo, amino-substituted C₁-C₆ alkyl wherein the amino groups may have one or two C₁-C₆ alkyl substituents or one alpha amino acid or polypeptide residue substituent, C₁-C₈ alkanoylamino, phenyl and a group having the formula -NR⁷R⁸ wherein R⁷ and R⁸ are independently selected from hydrogen and C₁-C₆ alkyl or, when one is hydrogen, the other is an alpha-amino acid or a polypeptide residue of from 2 to 5 amino acids or R² can be a nitrogen-containing heterocycle having the formula 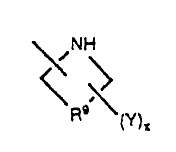 wherein x is 0 to 3; R⁹ is selected from the group consisting of (a) -(CH₂)ₘ-wherein m is 1, 2 or 3, and (b) -(CH₂)ₙR¹⁰(CH₂)ₚ- wherein R¹⁰ is S, O or N, n is 1 or 2, and p is 1 or 2; and Y is a non-hydrogen substituent independently selected from the group consisting of C₁-C₆ alkyl, C₁-C₆ haloalkyl, C₁-C₆ alkoxy, hydroxy-substituted C₁-C₆ alkyl, hydroxy, amino-substituted C₁-C₆ alkyl wherein the amino groups may have one or two C₁-C₆ alkyl substituents or one alpha amino acid or polypeptide residue substituent, halogen and a group having the formula -NR¹¹R¹² wherein R¹¹ and R¹² are independently selected from the group consisting of hydrogen and C₁-C₆ alkyl or, when one is hydrogen, the other is selected from the group consisting of a C₁-C₈ alkanoyl, an alpha-amino acid and a polypeptide residue of from 2 to 5 amino acids;
R³ is selected from the group consisting of hydrogen, halo and C₁-C₆ alkoxy;
R⁴ is selected from the group consisting of hydrogen, C₁-C₆ alkyl, a pharmaceutically acceptable cation and a prodrug ester group, said prodrug ester group being an ester group which is hydrolyzable under physiological conditions;
R⁵ is selected from the group consisting of hydrogen, halo, hydroxy, C₁-C₆ alkyl, C₁-C₆ haloalkyl, C₁-C₆ alkoxy, and a group having the formula -NR¹³R¹⁴ wherein R¹³ and R¹⁴ are independently selected from the group consisting of hydrogen, C₁-C₆ alkyl, hydroxy-substituted C₁-C₆ alkyl, C₁-C₆ alkoxy-substituted C₁-C₆ alkyl and C₁-C₈ alkanoyl; and
A is N or CR⁶, wherein R⁶ is selected from the group consisting of halogen, C₁-C₆ alkyl, C₁-C₆ haloalkyl, hydroxy-substituted C₁-C₆ alkyl, C₁-C₆ alkoxy-C₁-C₆ alkyl, C₁-C₆ alkoxy and amino-substituted C₁-C₆ alkyl wherein the amino groups may have one or two C₁-C₆ alkyl substituents or one alpha amino acid or polypeptide residue substituent or wherein R1 and R6, taken together with the atoms to which they are attached, form a 6-membered saturated ring which may contain an oxygen or a sulfur atom and which may be substituted with C₁-C₆ alkyl;
wherein cycloalkyl by itself or as part of another group, cycloalkenyl or bicycloalkyl may be substituted with a C₆-C₁₀ carbocyclic aryl-C₁-C₆ alkyl, (C₁-C₆ alkoxy)carbonyl, C₁-C₆ alkyl, C₁-C₆ haloalkyl, amino-substituted C₁-C₆ alkyl wherein the amino groups may have one or two C₁-C₆ alkyl substituents or one alpha amino acid or polypeptide residue substituent, hydroxy-substituted C₁-C₆ alkyl, hydroxy, C₁-C₆ alkoxy or halo, or an amino, mono- or di-(C₁-C₆ alkyl) amino, or C₁-C₈ alkanoylamino, where the amino group may be substituted with C₁-C₈ alkanoyl, an alpha-amino acid or a polypeptide of from two to five amino acids, said process comprising the step of hydrolyzing an ester of the carboxylic acid compound of formula I.

2. A process according to Claim 1 wherein A is N or CR⁶ and R⁶ is selected from the group consisting of halo, C₁-C₆ alkyl, C₁-C₆ haloalkyl, hydroxy-substituted C₁-C₆ alkyl, C₁-C₆ alkoxy-C₁-C₆ alkyl, C₁-C₆ alkoxy and amino-substituted C₁-C₆ alkyl wherein the amino groups may have one or two C₁-C₆ alkyl substituents or one alpha amino acid or polypeptide residue substituent.

3. A process according to Claim 2 wherein R² is a nitrogen-containing heterocycle having the formula 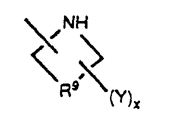 wherein R⁹, Y and x are as defined in Claim 1.

4. A process according to Claim 1 for preparing a compound having the formula 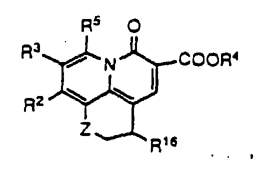 wherein
Z is selected from the group consisting of CH₂, O and S, and R¹⁶ is C₁-C₆ alkyl.

5. A process according to Claim 4 wherein Z is O and R² is a nitrogen-containing heterocycle having the formula 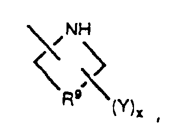 wherein R⁹, Y and x are as defined in Claim 1.

6. A process according to Claim 1 for preparing a compound selected from the group consisting of:
3-fluoro-9-(4-fluorophenyl)-2-(4-methylpiperazin-1-yl)-6(H)-6-oxo-pyrido-[1,2-a]pyrimidine-7-carboxylic acid;
9-(2,4-difluorophenyl)-3-fluoro-2-(4-methylpiperazin-1-yl)-6(H)-6-oxo-pyrido[1,2-a]pyrimidine-7-carboxylic acid;
3-fluoro-9-cyclopropyl-2-(4-methylpiperazin-1-yl)-6(H)-6-oxo-pyrido-[1,2-a]pyrimidine-7-carboxylic acid;
2-(3-aminopyrrolidin-1-yl)-9-cyclopropyl-3-fluoro-6H-6-oxo-pyrido-[1,2-a]pyrimidine-7-carboxylic acid hydrochloride salt;
2-(3-aminopyrrolidin-1-yl)-9-cyclopropyl-3-fluoro-6H-6-oxo-pyrido-[1,2-a]pyrimidine-7-carboxylic acid:
9-(2,4-difluorophenyl)-3-fluoro-2-(4-methylpiperazin-1-yl)-6H-6-oxo-pyrido[1,2-a]pyrimidine-7-carboxylic acid;
2-(3-aminopyrrolidin-1-yl)-9-(2,4-difluorophenyl)-3-fluoro-6H-6-oxo-pyrido[1,2-a]pyrimidine-7-carboxylic acid;
2-(3-(N-*t*-butoxycarbonyl)aminopyrrolidin-1-yl)-9-(2,4-difluorophenyl)-3-fluoro-6H-6-oxopyrido[1,2-a]pyrimidine-7-carboxylic acid;
2-(3-aminopyrrolidin-1-yl)-9-(2,4-difluorophenyl)-3-fluoro-6H-6-oxo-pyrido[1,2-a]pyrimidine-7-carboxylic acid;
9-cyclopropyl-3-fluoro-2-(4-methylpiperazin-1-yl)-6H-6-oxo-pyrido-[1,2-a]pyrimidine-7-carboxylic acid;
9-cyclopropyl-3-fluoro-2-(piperazin-1-yl)-6H-6-oxo-pyrido[1,2-a]pyrimidin-7-carboxylic acid;
9-cyclopropyl-3-fluoro-2-(morpholin-1-yl)-6H-6-oxo-pyrido[1,2-a]pyrimidine-7-carboxylic acid;
9-(2,4-difluorophenyl)-3-fluoro-2-(3-(N-(S)-norvalyl)aminopyrrolidin-1-yl)-6H-6-oxopyrido[1,2-a]pyrimidine-7-carboxylic acid hydrochloride salt;
2-(3-(N-(S)-alanyl)aminopyrrolidin-1-yl)-9-(2,4-difluorophenyl)-3-fluoro-6H-6-oxopyrido[1,2-a]pyrimidine-7-carboxylic acid hydrochloride;
2-(3-(N-(S)-alanyl-(S)-alanyl)aminopyrrolidin-1-yl)-9-(2,4-difluorophenyl)-3-fluoro-6H-6-oxopyrido[1,2-a]pyrimidine-7-carboxylic acid hydrochloride;
2-((2S,4S)-4-acetamido-2-methylpyrrolidin-1-yl)-9-(2,4-difluorophenyl)-3-fluoro-6H-6-oxopyrido[1,2-a]pyrimidine-7-carboxylic acid;
9-(2,4-difluorophenyl)-3-fluoro-2-(3-hydroxypyrrolin-1-yl)-6H-6-oxo-pyrido[1,2-a]pyrimidine-7-carboxylic acid; and
2-((2S,4S)-4-amino-2-methylpyrrolidin-1-yl)-9-(2,4-difluorophenyl)-3-fluoro-6H-6-oxopyrido[1,2-a]pyrimidine-7-carboxylic acid hydrochloride.

## Claims (Claims for the following Contracting State(s): GR)

1. A process for preparing a compound of the formula 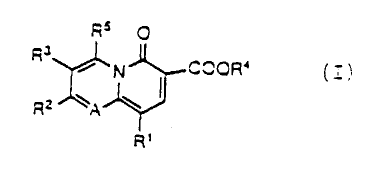 or a pharmaceutically acceptable salt, ester, or amide thereof, wherein
R¹ is selected from the group consisting of (a) C₁-C₆ alkyl, (b) C₂-C₆ alkenyl, (c) C₁-C₆ haloalkyl, (d) C₁-C₆ alkoxy, (e) C₃-C₈ cycloalkyl, (f) phenyl, (g) halo, (h) cyano, (i) nitro, (j) bicycloalkyl having from 4 to 9 carbon atoms in the ring system where non-adjacent atoms are bridged by 1-3 additional carbon atoms, (k) C₂-C₆ alkynyl, (l) (C₁-C₆ alkoxy)carbonyl, (m) nitrogen-containing aromatic heterocycle having from 5 to 7 ring atoms and (n) a group of the formula -NR⁷R⁸ wherein R⁷ and R⁸ are independently selected from the group consisting of hydrogen, C₁-C₆ alkyl and C₁-C₈ alkanoyl or, taken together with the nitrogen atom to which they are attached, R⁷ and R⁸ form a 5-, 6- or 7-membered saturated heterocycle optionally substituted with a C₆-C₁₀ carbocyclic aryl-C₁-C₆ alkyl, (C₁-C₆ alkoxy) carbonyl, C₁-C₆ alkyl, C₁-C₆ haloalkyl, amino-substituted C₁-C₆ alkyl wherein the amino groups may have one or two C₁-C₆ alkyl substituents or one alpha amino acid or polypeptide residue substituent, hydroxy-substituted C₁-C₆ alkyl, hydroxy, C₁-C₆ alkoxy or halo, or an amino, mono- or di-(C₁-C₆ alkyl)amino, or C₁-C₆ alkanoylamino, where the amino group may be substituted with C₁-C₆ alkanoyl, an alpha-amino acid or a polypeptide of from two to five amino acids;
R² is selected from the group consisting of halo, C₁-C₆ alkoxy, C₆-C₁₀ carbocyclic aryloxy, C₆-C₁₀ carbocyclic aryl-C₁-C₆ alkoxy, C₁-C₆ alkyl, C₂-C₆ alkenyl, C₃-C₈ cycloalkyl, C₄-C₈ cycloalkenyl, C₆-C₁₀ carbocyclic aryl-C₁-C₆ alkyl, C₃-C₈ cycloalkyl-C₁-C₆ alkyl, phenyl, amino, mono- or di-(C₁-C₆ alkyl)amino, C₆-C₁₀ carbocyclic aryl-C₁-C₆ alkylamino, (hydroxy-substituted C₁-C₆ alkyl)amino, bicyclic nitrogen-containing heterocyclic group selected from one of the formulae 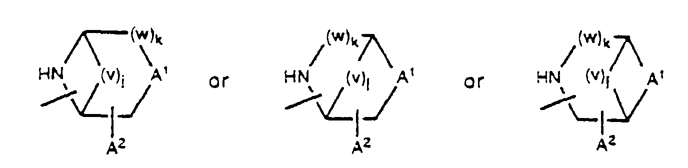 wherein v and w are CH₂; j and k are independently 1, 2 or 3; A¹ is a carbon atom or a heteroatom selected from S, O and N; and A² is one or more non-hydrogen substituents selected independently from the group consisting of C₁-C₆ alkyl, C₁-C₆ haloalkyl, hydroxy-substituted C₁-C₆ alkyl, hydroxy, halo, amino-substituted C₁-C₆ alkyl wherein the amino groups may have one or two C₁-C₆ alkyl substituents or one alpha amino acid or polypeptide residue substituent, C₁-C₈ alkanoylamino, phenyl and a group having the formula -NR⁷R⁸ wherein R⁷ and R⁸ are independently selected from hydrogen and C₁-C₆ alkyl or, when one is hydrogen, the other is an alpha-amino acid or a polypeptide residue of from 2 to 5 amino acids or R² can be a nitrogen-containing heterocycle having the formula 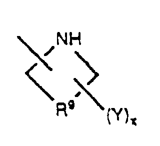 wherein x is 0 to 3; R⁹ is selected from the group consisting of (a) -(CH₂)ₘ-wherein m is 1, 2 or 3, and (b) -(CH₂)ₙR¹⁰(CH₂)ₚ- wherein R¹⁰ is S, O or N, n is 1 or 2, and p is 1 or 2; and Y is a non-hydrogen substituent independently selected from the group consisting of C₁-C₆ alkyl, C₁-C₆ haloalkyl, C₁-C₆ alkoxy, hydroxy-substituted C₁-C₆ alkyl, hydroxy, amino-substituted C₁-C₆ alkyl wherein the amino groups may have one or two C₁-C₆ alkyl substituents or one alpha amino acid or polypeptide residue substituent, halogen and a group having the formula -NR¹¹R¹² wherein R¹¹ and R¹² are independently selected from the group consisting of hydrogen and C₁-C₆ alkyl or, when one is hydrogen, the other is selected from the group consisting of a C₁-C₆ alkanoyl, an alpha-amino acid and a polypeptide residue of from 2 to 5 amino acids;
R³ is selected from the group consisting of hydrogen, halo and C₁-C₆ alkoxy;
R⁴ is selected from the group consisting of hydrogen, C₁-C₆ alkyl, a pharmaceutically acceptable cation and a prodrug ester group, said prodrug ester group being an ester group which is hydrolyzable under physiological conditions;
R⁵ is selected from the group consisting of hydrogen, halo, hydroxy, C₁-C₆ alkyl, C₁-C₆ haloalkyl, C₁-C₆ alkoxy, and a group having the formula -NR¹³R¹⁴ wherein R¹³ and R¹⁴ are independently selected from the group consisting of hydrogen, C₁-C₆ alkyl, hydroxy-substituted C₁-C₆ alkyl, C₁-C₆ alkoxy-substituted C₁-C₆ alkyl and C₁-C₈ alkanoyl; and
A is N or CR⁶, wherein R⁶ is selected from the group consisting of halogen, C₁-C₆ alkyl, C₁-C₆ haloalkyl, hydroxy-substituted C₁-C₆ alkyl, C₁-C₆ alkoxy-C₁-C₆ alkyl, C₁-C₆ alkoxy and amino-substituted C₁-C₆ alkyl wherein the amino groups may have one or two C₁-C₆ alkyl substituents or one alpha amino acid or polypeptide residue substituent or wherein R1 and R6, taken together with the atoms to which they are attached, form a 6-membered saturated ring which may contain an oxygen or a sulfur atom and which may be substituted with C₁-C₆ alkyl;
wherein cycloalkyl by itself or as part of another group, cycloalkenyl or bicycloalkyl may be substituted with a C₆-C₁₀ carbocyclic aryl-C₁-C₆ alkyl, (C₁-C₆ alkoxy)carbonyl, C₁-C₆ alkyl, C₁-C₆ haloalkyl, amino-substituted C₁-C₆ alkyl wherein the amino groups may have one or two C₁-C₆ alkyl substituents or one alpha amino acid or polypeptide residue substituent, hydroxy-substituted C₁-C₆ alkyl, hydroxy, C₁-C₆ alkoxy or halo, or an amino, mono- or di-(C₁-C₆ alkyl)amino, or C₁-C₈ alkanoylamino, where the amino group may be substituted with C₁-C₈ alkanoyl, an alpha-amino acid or a polypeptide of from two to five amino acids, said process comprising the step of hydrolyzing an ester of the carboxylic acid compound of formula I.

2. A process according to Claim 1 wherein A is N or CR⁶ and R⁶ is selected from the group consisting of halo, C₁-C₆ alkyl, C₁-C₆ haloalkyl, hydroxy-substituted C₁-C₆ alkyl, C₁-C₆ alkoxy-C₁-C₆ alkyl, C₁-C₆ alkoxy and amino-substituted C₁-C₆ alkyl wherein the amino groups may have one or two C₁-C₆ alkyl substituents or one alpha amino acid or polypeptide residue substituent.

3. A process according to Claim 2 wherein R² is a nitrogen-containing heterocycle having the formula 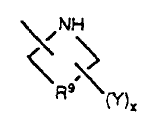 wherein R⁹, Y and x are as defined in Claim 1.

4. A process according to Claim 1 for preparing a compound having the formula 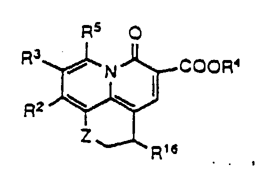 wherein
Z is selected from the group consisting of CH₂, O and S, and R¹⁶ is C₁-C₆ alkyl.

5. A process according to Claim 4 wherein Z is O and R² is a nitrogen-containing heterocycle having the formula 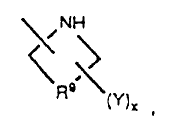 wherein R⁹, Y and x are as defined in Claim 1.

6. A process according to Claim 1 for preparing a compound selected from the group consisting of:
3-fluoro-9-(4-fluorophenyl)-2-(4-methylpiperazin-1-yl)-6(H)-6-oxo-pyrido-[1,2-a]pyrimidine-7-carboxylic acid;
9-(2,4-difluorophenyl)-3-fluoro-2-(4-methylpiperazin-1-yl)-6(H)-6-oxo-pyrido[1,2-a]pyrimidine-7-carboxylic acid;
3-fluoro-9-cyclopropyl-2-(4-methylpiperazin-1-yl)-6(H)-6-oxo-pyrido-[1,2-a]pyrimidine-7-carboxylic acid;
2-(3-aminopyrrolidin-1-yl)-9-cyclopropyl-3-fluoro-6H-6-oxo-pyrido-[1,2-a]pyrimidine-7-carboxylic acid hydrochloride salt;
2-(3-aminopyrrolidin-1-yl)-9-cyclopropyl-3-fluoro-6H-6-oxo-pyrido-[1,2-a]pyrimidine-7-carboxylic acid;
9-(2,4-difluorophenyl)-3-fluoro-2-(4-methylpiperazin-1-yl)-6H-6-oxo-pyrido[1,2-a]pyrimidine-7-carboxylic acid;
2-(3-aminopyrrolidin-1-yl)-9-(2,4-difluorophenyl)-3-fluoro-6H-6-oxo-pyrido[1,2-a]pyrimidine-7-carboxylic acid;
2-(3-(N-*t*-butoxycarbonyl)aminopyrrolidin-1-yl)-9-(2,4-difluorophenyl)-3-fluoro-6H-6-oxopyrido[1,2-a]pyrimidine-7-carboxylic acid;
2-(3-aminopyrrolidin-1-yl)-9-(2,4-difluorophenyl)-3-fluoro-6H-6-oxo-pyrido[1,2-a]pyrimidine-7-carboxylic acid;
9-cyclopropyl-3-fluoro-2-(4-methylpiperazin-1-yl)-6H-6-oxo-pyrido-[1,2-a]pyrimidine-7-carboxylic acid;
9-cyclopropyl-3-fluoro-2-(piperazin-1-yl)-6H-6-oxo-pyrido[1,2-a]pyrimidine-7-carboxylic acid;
9-cyclopropyl-3-fluoro-2-(morpholin-1-yl)-6H-6-oxo-pyrido[1,2-a]pyrimidine-7-carboxylic acid;
9-(2,4-difluorophenyl)-3-fluoro-2-(3-(N-(S)-norvalyl)aminopyrrolidin-1-yl)-6H-6-oxopyrido[1,2-a]pyrimidine-7-carboxylic acid hydrochloride salt;
2-(3-(N-(S)-alanyl)aminopyrrolidin-1-yl)-9-(2,4-difluorophenyl)-3-fluoro-6H-6-oxopyrido[1,2-a]pyrimidine-7-carboxylic acid hydrochloride;
2-(3-(N-(S)-alanyl-(S)-alanyl)aminopyrrolidin-1-yl)-9-(2,4-difluorophenyl)-3-fluoro-6H-6-oxopyrido[1,2-a]pyrimidine-7-carboxylic acid hydrochloride;
2-((2S,4S)-4-acetamido-2-methylpyrrolidin-1-yl)-9-(2,4-difluorophenyl)-3-fluoro-6H-6-oxopyrido[1,2-a]pyrimidine-7-carboxylic acid;
9-(2,4-difluorophenyl)-3-fluoro-2-(3-hydroxypyrrolin-1-yl)-6H-6-oxo-pyrido[1,2-a]pyrimidine-7-carboxylic acid; and
2-((2S,4S)-4-amino-2-methylpyrrolidin-1-yl)-9-(2,4-difluorophenyl)-3-fluoro-6H-6-oxopyrido[1,2-a]pyrimidine-7-carboxylic acid hydrochloride.

7. Use of a compound of the formula 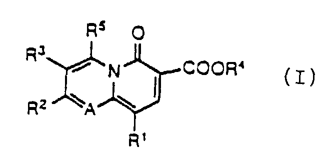 or a pharmaceutically acceptable salt, ester, or amide thereof, wherein
R¹ is selected from the group consisting of (a) C₁-C₆ alkyl, (b) C₂-C₆ alkenyl, (c) C₁-C₆ haloalkyl, (d) C₁-C₆ alkoxy, (e) C₃-C₈ cycloalkyl, (f) phenyl, (g) halo, (h) cyano, (i) nitro, (j) bicycloalkyl having from 4 to 9 carbon atoms in the ring system where non-adjacent atoms are bridged by 1-3 additional carbon atoms, (k) C₂-C₆ alkynyl, (l) (C₁-C₆ alkoxy)carbonyl, (m) nitrogen-containing aromatic heterocycle having from 5 to 7 ring atoms and (n) a group of the formula -NR⁷R⁸ wherein R⁷ and R⁸ are independently selected from the group consisting of hydrogen, C₁-C₆ alkyl and C₁-C₈ alkanoyl or, taken together with the nitrogen atom to which they are attached, R⁷ and R⁸ form a 5-, 6- or 7-membered saturated heterocycle optionally substituted with a C₆-C₁₀ carbocyclic aryl-C₁-C₆ alkyl, (C₁-C₆ alkoxy)carbonyl, C₁-C₆ alkyl, C₁-C₆ haloalkyl, amino-substituted C₁-C₆ alkyl wherein the amino groups may have one or two C₁-C₆ alkyl substituents or one alpha amino acid or polypeptide residue substituent, hydroxy-substituted C₁-C₆ alkyl, hydroxy, C₁-C₆ alkoxy or halo, or an amino, mono- or di-(C₁-C₆ alkyl)amino, or C₁-C₈ alkanoylamino, where the amino group may be substituted with C₁-C₈ alkanoyl, an alpha-amino acid or a polypeptide of from two to five amino acids;
R² is selected from the group consisting of halo, C₁-C₆ alkoxy, C₆-C₁₀ carbocyclic aryloxy, C₆-C₁₀ carbocyclic aryl-C₁-C₆ alkoxy, C₁-C₆ alkyl, C₂-C₆ alkenyl, C₃-C₈ cycloalkyl, C₄-C₈ cycloalkenyl, C₆-C₁₀ carbocyclic aryl-C₁-C₆ alkyl, C₃-C₈ cycloalkyl-C₁-C₆ alkyl, phenyl, amino, mono- or di-(C₁-C₆ alkyl)amino, C₆-C₁₀ carbocyclic aryl-C₁-C₆ alkylamino, (hydroxy-substituted C₁-C₆ alkyl)amino, bicyclic nitrogen-containing heterocyclic group selected from one of the formulae 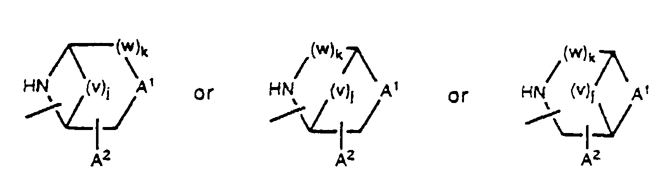 wherein v and w are CH₂; j and k are independently 1, 2 or 3; A¹ is a carbon atom or a heteroatom selected from S, O and N; and A² is one or more non-hydrogen substituents selected independently from the group consisting of C₁-C₆ alkyl, C₁-C₆ haloalkyl, hydroxy-substituted C₁-C₆ alkyl, hydroxy, halo, amino-substituted C₁-C₆ alkyl wherein the amino groups may have one or two C₁-C₆ alkyl substituents or one alpha amino acid or polypeptide residue substituent, C₁-C₈ alkanoylamino, phenyl and a group having the formula -NR⁷R⁸ wherein R⁷ and R⁸ are independently selected from hydrogen and C₁-C₆ alkyl or, when one is hydrogen, the other is an alpha-amino acid or a polypeptide residue of from 2 to 5 amino acids or R² can be a nitrogen-containing heterocycle having the formula 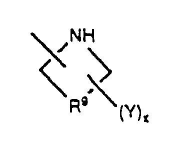 wherein x is 0 to 3; R⁹ is selected from the group consisting of (a) -(CH₂)ₘ-wherein m is 1, 2 or 3, and (b) -(CH₂)ₙR¹⁰(CH₂)ₚ- wherein R¹⁰ is S, O or N, n is 1 or 2, and p is 1 or 2; and Y is a non-hydrogen substituent independently selected from the group consisting of C₁-C₆ alkyl, C₁-C₆ haloalkyl, C₁-C₆ alkoxy, hydroxy-substituted C₁-C₆ alkyl, hydroxy, amino-substituted C₁-C₆ alkyl wherein the amino groups may have one or two C₁-C₆ alkyl substituents or one alpha amino acid or polypeptide residue substituent, halogen and a group having the formula -NR¹¹R¹² wherein R¹¹ and R¹² are independently selected from the group consisting of hydrogen and C₁-C₆ alkyl or, when one is hydrogen, the other is selected from the group consisting of a C₁-C₈ alkanoyl, an alpha-amino acid and a polypeptide residue of from 2 to 5 amino acids;
R³ is selected from the group consisting of hydrogen, halo and C₁-C₆ alkoxy;
R⁴ is selected from the group consisting of hydrogen, C₁-C₆ alkyl, a pharmaceutically acceptable cation and a prodrug ester group, said prodrug ester group being an ester group which is hydrolyzable under physiological conditions;
R⁵ is selected from the group consisting of hydrogen, halo, hydroxy, C₁-C₆ alkyl, C₁-C₆ haloalkyl, C₁-C₆ alkoxy, and a group having the formula -NR¹³R¹⁴ wherein R¹³ and R¹⁴ are independently selected from the group consisting of hydrogen, C₁-C₆ alkyl, hydroxy-substituted C₁-C₆ alkyl, C₁-C₆ alkoxy-substituted C₁-C₆ alkyl and C₁-C₈ alkanoyl; and
A is N or CR⁶, wherein R⁶ is selected from the group consisting of halogen, C₁-C₆ alkyl, C₁-C₆ haloalkyl, hydroxy-substituted C₁-C₆ alkyl, C₁-C₆ alkoxy-C₁-C₆ alkyl, C₁-C₆ alkoxy and amino-substituted C₁-C₆ alkyl wherein the amino groups may have one or two C₁-C₆ alkyl substituents or one alpha amino acid or polypeptide residue substituent or wherein R1 and R6, taken together with the atoms to which they are attached, form a 6-membered saturated ring which may contain an oxygen or a sulfur atom and which may be substituted with C₁-C₆ alkyl;
wherein cycloalkyl by itself or as part of another group, cycloalkenyl or bicycloalkyl may be substituted with a C₆-C₁₀ carbocyclic aryl-C₁-C₆ alkyl, (C₁-C₆ alkoxy)carbonyl, C₁-C₆ alkyl, C₁-C₆ haloalkyl, amino-substituted C₁-C₆ alkyl wherein the amino groups may have one or two C₁-C₆ alkyl substituents or one alpha amino acid or polypeptide residue substituent, hydroxy-substituted C₁-C₆ alkyl, hydroxy, C₁-C₆ alkoxy or halo, or an amino, mono- or di-(C₁-C₆ alkyl)amino, or C₁-C₈ alkanoylamino, where the amino group may be substituted with C₁-C₈ alkanoyl, an alpha-amino acid or a polypeptide of from two to five amino acids, for manufacturing a medicament for treating or preventing a bacterial infection in a human or other animal host.

8. Use of a compound selected from the group consisting of:
3-fluoro-9-(4-fluorophenyl)-2-(4-methylpiperazin-1-yl)-6(H)-6-axo-pyrido-[1,2-a]pyrimidine-7-carboxylic acid;
9-(2,4-difluorophenyl)-3-fluoro-2-(4-methylpiperazin-1-yl)-6(H)-6-oxo-pyrido[1,2-a]pyrimidine-7-carboxylic acid;
3-fluoro-9-cyclopropyl-2-(4-methylpiperazin-1-yl)-6(H)-6-oxo-pyrido-[1,2-a]pyrimidine-7-carboxylic acid;
2-(3-aminopyrrolidin-1-yl)-9-cyclopropyl-3-fluoro-6H-6-oxo-pyrido-[1,2-a]pyrimidine-7-carboxylic acid hydrochloride salt;
2-(3-aminopyrrolidin-1-yl)-9-cyclopropyl-3-fluoro-6H-6-oxo-pyrido-[1,2-a]pyrimidine-7-carboxylic acid;
9-(2,4-difluorophenyl)-3-fluoro-2-(4-methylpiperazin-1-yl)-6H-6-oxo-pyrido[1,2-a]pyrimidine-7-carboxylic acid;
2-(3-aminopyrrolidin-1-yl)-9-(2,4-difluorophenyl)-3-fluoro-6H-6-oxo-pyrido[1,2-a]pyrimidine-7-carboxylic acid;
2-(3-(N-*t*-butoxycarbonyl)aminopyrrolidin-1-yl)-9-(2,4-difluorophenyl)-3-fluoro-6H-6-oxopyrido[1,2-a]pyrimidine-7-carboxylic acid;
2-(3-aminopyrrolidin-1-yl)-9-(2,4-difluorophenyl)-3-fluoro-6H-6-oxo-pyrido[1,2-a]pyrimidine-7-carboxylic acid;
9-cyclopropyl-3-fluoro-2-(4-methylpiperazin-1-yl)-6H-6-oxo-pyrido-[1,2-a]pyrimidine-7-carboxylic acid;
9-cyclopropyl-3-fluoro-2-(piperazin-1-yl)-6H-6-oxo-pyrido[1,2-a]pyrimidine-7-carboxylic acid;
9-cyclopropyl-3-fluoro-2-(morpholin-1-yl)-6H-6-oxo-pyrido[1,2-a]pyrimidine-7-carboxylic acid;
9-(2,4-difluorophenyl)-3-fluoro-2-(3-(N-(S)-norvalyl)aminopyrrolidin-1-yl)-6H-6-oxopyrido[1,2-a]pyrimidine-7-carboxylic acid hydrochloride salt;
2-(3-(N-(S)-alanyl)aminopyrrolldin-1-yl)-9-(2,4-difluorophenyl)-3-fluoro-6H-6-oxopyrido[1,2-a]pyrimidine-7-carboxylic acid hydrochloride;
2-(3-(N-(S)-alanyl-(S)-alanyl)aminopyrrolidin-1-yl)-9-(2,4-difluorophenyl)-3-fluoro-6H-6-oxopyrido[1,2-a]pyrimidine-7-carboxylic acid hydrochloride;
2-((2S,4S)-4-acetamido-2-methylpyrrolidin-1-yl)-9-(2,4-difluorophenyl)-3-fluoro-6H-6-oxopyrido[1,2-a]pyrimidine-7-carboxylic acid;
9-(2,4-difluorophenyl)-3-fluoro-2-(3-hydroxypyrrolin-1-yl)-6H-6-oxo-pyrido[1,2-a]pyrimidine-7-carboxylic acid; and
2-((2S,4S)-4-amino-2-methylpyrrolidin-1-yl)-9-(2,4-difluorophenyl)-3-fluoro-6H-6-oxopyrido[1,2-a]pyrimidine-7-carboxylic acid hydrochloride, for manufacturing a medicament for treating or preventing a bacterial infection in a human or other animal host.

## Patentansprüche (Patentansprüche für folgende(n) Vertragsstaat(en): AT, BE, CH, LI, DE, DK, FR, GB, IT, LU, NL, SE)

1. Eine Verbindung mit der Formel 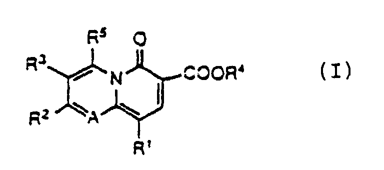 oder ein pharmazeutisch verträgliches Salz, Ester oder Amid davon, worin
R¹ aus der Gruppe gewählt ist, die aus (a) C₁-C₆-Alkyl, (b) C₂-C₆-Alkenyl, (c) C₁-C₆-Haloalkyl, (d) C₁-C₆-Alkoxy, (e) C₃-C₈-Cycloalkyl, (f) Phenyl, (g) Halo, (h) Cyano, (i) Nitro, (j) Bicycloalkyl mit 4 bis 9 Kohlenstoffatomen im Ringsystem, wobei nicht-benachbarte Atome durch 1-3 weitere Kohlenstoffatome miteinander verbunden sind, (k) C₂-C₆-Alkynyl, (l) (C₁-C₆-Alkoxy)carbonyl, (m) stickstoffhaltigem aromatischen Heterozyklus mit 5 bis 7 Ringatomen und (n) einer Gruppe mit der Formel -NR⁷R⁸ besteht, worin R⁷ und R⁸ unabhängig voneinander aus der Gruppe gewählt sind, die aus Wasserstoff, C₁-C₆-Alkyl und C₁-C₈-Alkanoyl besteht, oder zusammengenommen mit dem Stickstoffatom, an das sie gebunden sind, R⁷ und R⁸ einen 5-, 6- oder 7-gliedrigen gesättigten Heterozyklus bilden, der wahlweise substituiert ist mit einem C₆-C₁₀-carbocyclischen Aryl-C₁-C₆-Alkyl, (C₁-C₆-Alkoxy)carbonyl, C₁-C₆-Alkyl, C₁-C₆-Haloalkyl, aminosubstituiertem C₁-C₆-Alkyl, worin die Aminogruppen einen oder zwei C₁-C₆-Alkylsubstituenten oder einen Alphaaminosäure- oder Polypeptidrestsubstituenten, einen hydroxysubstituierten C₁-C₆-Alkyl, Hydroxy, C₁-C₆-Alkoxy oder Halo, oder einen Amino, mono- oder di-(C₁-C₆-Alkyl)amino, oder C₁-C₈-Alkanoylamino enthalten können, wobei die Aminogruppe mit C₁-C₈-Alkanoyl, einer Alphaaminosäure oder einem Polypeptid mit zwei bis fünf Aminosäuren substituiert sein kann;
R² ist gewählt aus der Gruppe bestehend aus Halo, C₁-C₆-Alkoxy, C₆-C₁₀-carbocyclischem Aryloxy, C₆-C₁₀-carbocyclischem Aryl-C₁-C₆-Alkoxy, C₁-C₆-Alkyl, C₂-C₆-Alkenyl, C₃-C₈-Cycloalkyl, C₄-C₈-Cycloalkenyl, C₆-C₁₀-carbocyclischem Aryl-C₁-C₆-Alkyl, C₃-C₈-Cycloalkyl-C₁-C₆-Alkyl, Phenyl, Amino, mono- oder di-(C₁-C₆-Alkyl)amino, C₆-C₁₀-carbocyclischem Aryl-C₁-C₆-Alkylamino, (hydroxysubstituiertem C₁-C₆-Alkyl)amino, bicyclischer stickstoffhaltiger heterocyclischer Gruppe, die gewählt ist aus einer der Formeln 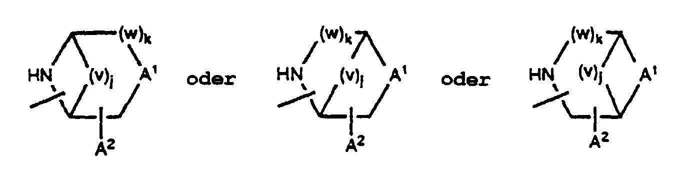 worin v und w gleich CH₂ sind; j und k sind unabhängig voneinander 1, 2 oder 3; A¹ ist ein Kohlenstoffatom oder ein Heteroatom, das aus S, O und N gewählt ist; und A² sind einer oder mehrere Nicht-Wasserstoff-Substituenten, die unabhängig voneinander gewählt sind aus der Gruppe, die aus C₁-C₆-Alkyl, C₁-C₆-Haloalkyl, hydroxysubstituiertem C₁-C₆-Alkyl, Hydroxy, Halo, aminosubstituiertem C₁-C₆-Alkyl, worin die Aminogruppen einen oder zwei C₁-C₆-Alkylsubstituenten oder einen Alphaaminosäure- oder Polypeptidrestsubstituenten enthalten können, C₁-C₈-Alkanoylamino, Phenyl und einer Gruppe mit der Formel -NR⁷R⁸ besteht, worin R⁷ und R⁸ unabhängig voneinander aus Wasserstoff und C₁-C₆-Alkyl gewählt sind, oder wenn ein Rest Wasserstoff ist, ist der andere eine Alphaaminosäure oder ein Polypeptidrest mit 2 bis 5 Aminosäuren, oder R² kann eine stickstoffhaltige heterocyclische Verbindung sein mit der Formel 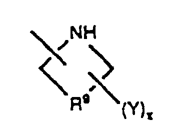 worin x gleich 0 bis 3 ist; R⁹ ist gewählt aus der Gruppe, die aus (a) -(CH₂)ₘ-, worin m gleich 1, 2 oder 3 ist, und (b) -(CH₂)ₙR¹⁰(CH₂)ₚ- besteht, worin R¹⁰ gleich S, O oder N ist, n ist 1 oder 2, und p ist 1 oder 2; und Y ist ein Nicht-Wasserstoff-Substituent, der unabhängig gewählt ist aus der Gruppe bestehend aus C₁-C₆-Alkyl, C₁-C₆-Haloalkyl, C₁-C₆-Alkoxy, hydroxysubstituiertem C₁-C₆-Alkyl, Hydroxy, aminosubstituiertem C₁-C₆-Alkyl, worin die Aminogruppen einen oder zwei C₁-C₆-Alkylsubstituenten oder eine Alphaaminosäure- oder Polypeptidrestsubstituenten enthalten können, Halogen und einer Gruppe mit der Formel -NR¹¹R¹², worin R¹¹ und R¹² unabhängig voneinander gewählt sind aus der Gruppe, die aus Wasserstoff und C₁-C₆-Alkyl besteht, oder wenn ein Rest Wasserstoff ist, ist der andere gewählt aus der Gruppe, die aus C₁-C₈-Alkanoyl, einer Alphaaminosäure und einem Polypeptidrest mit 2 bis 5 Aminosäuren besteht;
R³ ist gewählt aus der Gruppe, die aus Wasserstoff, Halo und C₂-C₆-Alkoxy besteht;
R⁴ ist gewählt aus der Gruppe, die aus Wasserstoff, C₁-C₆-Alkyl, einem pharmazeutisch verträglichen Kation und einer Prodrug-Estergruppe besteht, wobei die Prodrug-Estergruppe eine Estergruppe ist, die unter physiologischen Bedingungen hydrolysierbar ist;
R⁵ ist gewählt aus der Gruppe, die aus Wasserstoff, Halo, Hydroxy, C₁-C₆-Alkyl, C₁-C₆-Haloalkyl, C₁-C₆-Alkoxy und einer Gruppe mit der Formel NR¹³R¹⁴ besteht, worin R¹³ und R¹⁴ unabhängig voneinander gewählt sind aus der Gruppe, die aus Wasserstoff, C₁-C₆-Alkyl, hydroxysubstituiertem C₁-C₆-Alkyl, C₁-C₆-Alkoxysubstituiertem C₁-C₆-Alkyl und C₁-C₈-Alkanoyl besteht; und
A ist N oder CR⁶, worin R⁶ gewählt ist aus der Gruppe bestehend aus Halogen, C₁-C₆-Alkyl, C₁-C₆-Haloalkyl, hydroxysubstituiertem C₁-C₆-Alkyl, C₁-C₆-Alkoxy-C₁-C₆-Alkyl, C₁-C₆-Alkoxy und aminosubstituiertem C₁-C₆-Alkyl, worin die Aminogruppen einen oder zwei C₁-C₆-Alkylsubstituenten einen Alphaaminosäure- oder Polypeptidrestsubstituenten enthalten können, oder worin R¹ und R⁶, zusammengenommen mit den Atomen, an die sie gebunden sind, einen 6-gliedrigen gesättigten Ring bilden, der ein Sauerstoff- oder ein Schwefelatom enthalten kann und der mit einem C₁-C₆-Alkyl substituiert sein kann;
worin Cycloalkyl allein oder als Teil einer anderen Gruppe, Cycloalkenyl oder Bicycloalkyl substituiert sein können mit einem C₆-C₁₀-carbocyclischem Aryl-C₁-C₆-Alkyl, (C₁-C₆-Alkoxy)carbonyl, C₁-C₆-Alkyl, C₁-C₆-Haloalkyl, aminosubstituiertem C₁-C₆-Alkyl, worin die Aminogruppen einen oder zwei C₁-C₆-Alkylsubstituenten oder einen Alphaaminosäure- oder Polypeptidrestsubstituenten enthalten können, hydroxysubstituiertem C₁-C₆-Alkyl, Hydroxy, C₁-C₆-Alkoxy oder Hab, oder einem Amino, mono- oder di-(C₁-C₆-Alkyl)amino, oder C₁-C₈-Alkanoylamino, worin die Aminogruppe mit einem C₁-C₈-Alkanoyl, einer Aminosäure oder einem Polypeptid mit zwei bis fünf Aminosäuren substituiert sein kann.

2. Eine Verbindung nach Anspruch 1, worin A gleich N oder CR⁶ ist und R⁶ aus der Gruppe gewählt ist, die aus Halo, C₁-C₆-Alkyl, C₁-C₆-Haloalkyl, hydroxysubstituiertem C₁-C₆-Alkyl, C₁-C₆-Alkoxy-C₁-C₆-Alkyl, C₁-C₆-Alkoxy und aminosubstituiertem C₁-C₆-Alkyl besteht, worin die Aminogruppen einen oder zwei C₁-C₆-Alkylsubstituenten oder einen Alphaaminosäure- oder Polypeptidrestsubstituenten enthalten können.

3. Eine Verbindung nach Anspruch 2, worin R² ein stickstoffhaltiger Heterozyklus ist mit der Formel 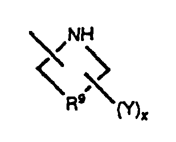 worin R⁹, Y und x wie in Anspruch 1 definiert sind.

4. Eine Verbindung nach Anspruch 1 mit der Formel 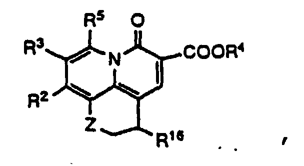 worin worin Z aus der Gruppe gewählt ist, die aus CH₂, O und S besteht, und R¹⁶ ein C₁-C₆- Alkyl ist.

5. Eine Verbindung nach Anspruch 4, worin Z gleich O und R² ein stickstoffhaltiger Heterozyklus ist mit der Formel 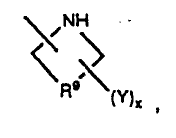 worin R⁹, Y und x wie in Anspruch 1 definiert sind.

6. Eine Verbindung, die aus der Gruppe gewählt ist, die sich wie folgt zusammensetzt:
3-Fluor-9-(4-fluorphenyl)-2-(4-methylpiperazin-1-yl)-6(H)-6-oxo-pyrido-[1,2-a]pyrimidin-7-carbonsäure;
9-(2,4-Difluorphenyl)-3-fluor-2-(4-methylpiperazin-1-yl)-6(H)-6-oxo-pyrido[1,2-a]pyrimidin-7-carbonsäure;
3-Fluor-9-cyclopropyl-2-(4-methylpiperazin-1-yl)-6(H)-6-oxo-pyrido[1,2-a]pyrimidin-7-carbonsäure;
2-(3-Aminopyrrolidin-1-yl)-9-cyclopropyl-3-fluor-6H-6-oxo-pyrido[1,2-a]pyrimidin-7-carbonsäurehydrochloridsalz;
2-(3-Aminopyrrolidin-1-yl)-9-cyclopropyl-3-fluor-6H-6-oxo-pyrido[1,2-a]pyrimidin-7-carbonsäure;
9-(2,4-Difluorphenyl)-3-fluor-2-(4-methylpiperazin-1-yl)-6H-6-oxo-pyrido[1,2-a]pyrimidin-7-carbonsäure;
2-(3-Aminopyrrolidin-1-yl)-9-(2,4-difluorphenyl)-3-fluor-6H-6-oxo-pyrido[1,2-a]pyrimidin-7-carbonsäure;
2-(3-(N-*t*-Butoxycarbonyl)aminopyrrolidin-1-yl)-9-(2,4-difluorphenyl)-3-fluor-6H-6-oxopyrido[1,2-a]pyrimidin-7- carbonsäure;
2-(3-Aminopyrrolidin-1-yl)-9-(2,4-difluorphenyl)-3-fluor-6H-6-oxo-pyrido[1,2-a]pyrimidin-7-carbonsäure;
9-Cyclopropyl-3-fluor-2-(4-methylpiperazin-1-yl)-6H-6-oxo-pyrido-[1,2-a]pyrimidin-7-carbonsäure;
9-Cyclopropyl-3-fluor-2-(piperazin-1-yl)-6H-6-oxo-pyrido-[1,2-a]pyrimidin-7-carbonsäure;
9-Cyclopropyl-3-fluor-2-(morpholin-1-yl)-6H-6-oxo-pyrido-[1,2-a]pyrimidin-7-carbonsäure;
9-(2,4-Difluorphenyl)-3-fluor-2-(3-(N-(S)-norvalyl)aminopyrrolidin-1-yl)-6H-6-oxopyrido[1,2-a]pyrimidin-7-carbonsäurehydrochloridsalz;
2-(3-(N-(S)-Alanyl)aminopyrrolidin-1-yl)-9-(2,4-difluorphenyl)-3-fluor-6H-6-oxopyrido[1,2-a]pyrimidin-7-carbonsäurehydrochlorid;
2-(3-(N-(S)-Alanyl-(S)-alanyl)aminopyrrolidin-1-yl)-9-(2,4-difluorphenyl)-3-fluor-6H-6-oxopyrido[1,2-a]pyrimidin-7-carbonsäurehydrochlorid;
2-((2S,4S)-4-Acetamido-2-methylpyrrolidin-1-yl)-9-(2,4-difluorphenyl)-3-fluor-6H-6-oxopyrido[1,2-a]pyrimidin-7-carbonsäure;
9-(2,4-Difluorphenyl)-3-fluor-2-(3-hydroxypyrrolin-1-yl)-6H-6-oxopyrido[1,2-a]pyrimidin-7-carbonsäure; und
2-((2S,4S)-4-Amino-2-methylpyrrolidin-1-yl)-9-(2,4-difluorphenyl)-3-fluor-6H-6-oxopyrido[1,2-a]pyrimidin-7-carbonsäurehydrochlorid.

7. Eine pharmazeutische Zusammensetzung zur Behandlung oder Prävention einer bakteriellen Infektion, die eine therapeutisch wirksame Menge einer Verbindung nach Anspruch 1 und einen pharmazeutisch verträglichen Träger enthält.

8. Eine pharmazeutische Zusammensetzung zur Behandlung oder Prävention einer bakteriellen Infektion, die eine therapeutisch wirksame Menge einer Verbindung nach Anspruch 6 und einen pharmazeutisch verträglichen Träger enthält.

9. Eine Verbindung nach Anspruch 1 oder 6 für die Verwendung als therapeutischer Wirkstoff.

10. Die Verwendung einer Verbindung nach Anspruch 1 für die Herstellung eines Medikaments zur Behandlung oder Prävention einer bakteriellen Infektion bei einem Menschen oder einem Wirtstier.

11. Die Verwendung einer Verbindung nach Anspruch 6 für die Herstellung eines Medikaments zur Behandlung oder Prävention einer bakteriellen Infektion bei einem Menschen oder einem Wirtstier.

## Patentansprüche (Patentansprüche für folgende(n) Vertragsstaat(en): ES)

1. Ein Verfahren zur Darstellung einer Verbindung mit der Formel 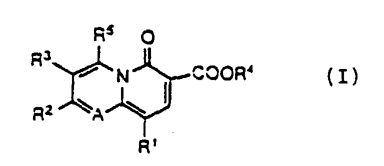 oder eines pharmazeutisch verträglichen Salzes, Esters oder Amids davon, worin
R¹ aus der Gruppe gewählt ist, die aus (a) C₁-C₆-Alkyl, (b) C₂-C₆-Alkenyl, (c) C₁-C₆-Haloalkyl, (d) C₁-C₆-Alkoxy, (e) C₃-C₈-Cycloalkyl, (f) Phenyl, (g) Halo, (h) Cyano, (i) Nitro, (j) Bicycloalkyl mit 4 bis 9 Kohlenstoffatomen im Ringsystem, wobei nicht-benachbarte Atome durch 1-3 weitere Kohlenstoffatome miteinander verbunden sind, (k) C₂-C₆-Alkynyl, (l) (C₁-C₆-Alkoxy)carbonyl, (m) stickstoffhaltiger aromatische heterocyclischer Verbindung mit 5 bis 7 Ringatomen und (n) einer Gruppe mit der Formel -NR⁷R⁸ besteht, worin R⁷ und R⁸ unabhängig voneinander aus der Gruppe gewählt sind, die aus Wasserstoff, C₁-C₆ -Alkyl und C₁-C₈-Alkanoyl besteht, oder zusammengenommen mit dem Stickstoffatom, an das sie gebunden sind, bilden R⁷ und R⁸ einen 5-, 6- oder 7-gliedrigen gesättigten Heterozyklus, der wahlweise substituiert ist mit einem C₆-C₁₀-carbocyclischen Aryl-C₁-C₆-Alkyl, (C₁-C₆-Alkoxy)carbonyl, C₁-C₆-Alkyl, C₁-C₆-Haloalkyl, aminosubstituierten C₁-C₆-Alkyl, worin die Aminogruppen einen oder zwei C₁-C₆-Alkylsubstituenten oder einen Alphaaminosäure- oder Polypeptidrestsubstituenten enthalten können, hydroxysubstituierten C₁-C₆-Alkyl, Hydroxy, C₁-C₆-Alkoxy oder Halo, oder einem Amino, mono- oder di-(C₁-C₆-Alkyl)amino, oder C₁-C₈-Alkanoylamino, wobei die Aminogruppe mit C₁-C₈-Alkanoyl, einer Alphaaminosäure oder einem Polypeptid mit zwei bis fünf Aminosäuren substituiert sein kann;
R² ist gewählt aus der Gruppe bestehend aus Halo, C₁-C₆-Alkoxy, C₁-C₆-carbocyclischem Aryloxy, C₆-C₁₀-carbocyclischem Aryl-C₁-C₆-Alkoxy, C₁-C₆-Alkyl, C₂-C₆-Alkenyl, C₃-C₈-Cycloalkyl, C₄-C₈-Cycloalkenyl, C₆-C₁₀-carbocyclischem Aryl-C₁-C₆-Alkyl, C₃-C₈-Cycloalkyl-C₁-C₆-Alkyl, Phenyl, Amino, mono- oder di-(C₁-C₆-Alkyl)amino, C₆-C₁₀-carbocyclischem Aryl-C₁-C₆-Alkylamino, (hydroxysubstituiertem C₁-C₆-Alkyl)amino, bicyclischer stickstoffhaltiger heterocyclischer Gruppe, die gewählt ist aus einer der Formeln 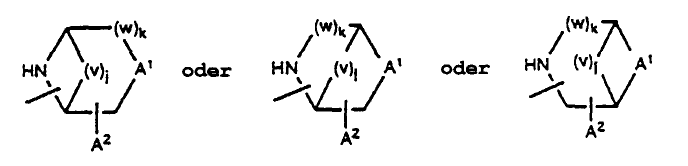 worin v und w gleich CH₂ sind; j und k sind unabhängig voneinander 1, 2 oder 3; A¹ ist ein Kohlenstoffatom oder ein Heteroatom, das aus S, O und N gewählt ist; und A² sind einer oder mehrere Nicht-Wasserstoff-Substituenten, die unabhängig voneinander aus der Gruppe gewählt sind, die aus C₁-C₆-Alkyl, C₁-C₆-Haloalkyl, hydroxysubstituiertem C₁-C₆-Alkyl, Hydroxy, Halo, aminosubstituiertem C₁-C₆-Alkyl, worin die Aminogruppen einen oder zwei C₁-C₆-Alkylsubstituenten oder einen Alphaaminosäure- oder Polypeptidrestsubstituenten enthalten können, C₁-C₈-Alkanoylamino, Phenyl und einer Gruppe mit der Formel -NR⁷R⁸ besteht, worin R⁷ und R⁸ unabhängig voneinander aus Wasserstoff und C₁-C₆-Alkyl gewählt sind, oder wenn ein Rest Wasserstoff ist, ist der andere eine Alphaaminosäure oder ein Polypeptidrest mit 2 bis 5 Aminosäuren, oder R² kann ein stickstoffhaltiger Heterozyklus sein mit der Formel 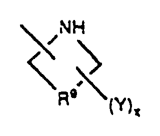 worin x gleich 0 bis 3 ist; R⁹ ist gewählt aus der Gruppe, die aus (a) -(CH₂)ₘ-, worin m gleich 1, 2 oder 3 ist, und (b) -(CH₂)ₙR¹⁰(CH₂)ₚ- besteht, worin R¹⁰ gleich S, O oder N ist, n ist 1 oder 2, und p ist 1 oder 2; und Y ist ein Nicht-Wasserstoff-Substituent, der unabhängig gewählt ist aus der Gruppe bestehend aus C₁-C₆-Alkyl, C₁-C₆-Haloalkyl, C₁-C₆-Alkoxy, hydroxysubstituiertem C₁-C₆-Alkyl, Hydroxy, aminosubstituiertem C₁-C₆-Alkyl, worin die Aminogruppen einen oder zwei C₁-C₆-Alkylsubstituenten oder einen Alphaaminosäure- oder Polypeptidrestsubstituenten enthalten können, Halogen und einer Gruppe mit der Formel -NR¹¹R¹², worin R¹¹ und R¹² unabhängig voneinander gewählt sind aus der Gruppe, die aus Wasserstoff und C₁-C₆-Alkyl besteht, oder wenn ein Rest Wasserstoff ist, ist der andere gewählt aus der Gruppe, die aus C₁-C₈-Alkanoyl, einer Alphaaminosäure und einem Polypeptidrest mit 2 bis 5 Aminosäuren besteht;
R³ ist gewählt aus der Gruppe, die aus Wasserstoff, Halo und C₁-C₆-Alkoxy besteht;
R⁴ ist gewählt aus der Gruppe, die aus Wasserstoff, C₁-C₆-Alkyl, einem pharmazeutisch verträglichen Kation und einer Prodrug-Estergruppe besteht, wobei die Prodrug-Estergruppe eine Estergruppe ist, die unter physiologischen Bedingungen hydrolysierbar ist;
R⁵ ist gewählt aus der Gruppe, die aus Wasserstoff, Halo, Hydroxy, C₁-C₆-Alkyl, C₁-C₆-Haloalkyl, C₁-C₆-Alkoxy und einer Gruppe mit der Formel NR¹³R¹⁴ besteht, worin R¹³ und R¹⁴ unabhängig voneinander gewählt sind aus der Gruppe, die aus Wasserstoff, C₁-C₆-Alkyl, hydroxysubstituiertem C₁-C₆-Alkyl, C₁-C₆-Alkoxysubstituiertem C₁-C₆-Alkyl und C₁-C₈-Alkanoyl besteht; und
A ist N oder CR⁶, worin R⁶ gewählt ist aus der Gruppe bestehend aus Halogen, C₁-C₆-Alkyl, C₁-C₆-Haloalkyl, hydroxysubstituiertem C₁-C₆-Alkyl, C₁-C₆-Alkoxy-C₁-C₆-Alkyl, C₁-C₆-Alkoxy und aminosubstituiertem C₁-C₆-Alkyl, worin die Aminogruppen einen oder zwei C₁-C₆-Alkylsubstituenten oder einen Alphaaminosäure- oder Polypeptidrestsubstituenten enthalten können, oder worin R¹ und R⁶, zusammengenommen mit den Atomen, an die sie gebunden sind, einen 6-gliedrigen gesättigten Ring bilden, der ein Sauerstoff- oder ein Schwefelatom enthalten kann und der mit einem C₁-C₆-Alkyl substituiert sein kann;
worin Cycloalkyl allein oder als Teil einer anderen Gruppe, Cycloalkenyl oder Bicycloalkyl substituiert sein können mit einem C₆-C₁₀-carbocyclischem Aryl-C₁-C₆-Alkyl, (C₁-C₆ -Alkoxy)carbonyl, C₁-C₆-Alkyl, C₁-C₆-Haloalkyl, aminosubstituiertem C₁-C₆-Alkyl, worin die Aminogruppen einen oder zwei C₁-C₆-Alkylsubstituenten oder einen Alphaaminosäure- oder Polypeptidrestsubstituenten enthalten können, hydroxysubstituiertem C₁-C₆-Alkyl, Hydroxy, C₁-C₆-Alkoxy oder Halo, oder einem Amino, mono- oder di-(C₁-C₆-Alkyl)amino, oder C₁ -C₆-Alkanoylamino, worin die Aminogruppe mit einem C₁-C₈-Alkanoyl, einer Aminosäure oder einem Polypeptid mit zwei bis fünf Aminosäuren substituiert sein kann, wobei das Verfahren den Schritt Hydrolysieren eines Esters der Carbonsäureverbindung nach Formel I beinhaltet.

2. Ein Verfahren nach Anspruch 1, worin A gleich N oder CR⁶ ist und R⁶ aus der Gruppe gewählt ist, die aus Halo, C₁-C₆-Alkyl, C₁-C₆-Haloalkyl, hydroxysubstituiertem C₁-C₆-Alkyl, C₁-C₆-Alkoxy-C₁-C₆-Alkyl, C₁-C₆-Alkoxy und aminosubstituiertem C₁-C₆-Alkyl besteht, worin die Aminogruppen einen oder zwei C₁-C₆-Alkylsubstituenten oder einen Alphaaminosäure- oder Polypeptidrestsubstituenten enthalten können.

3. Ein Verfahren nach Anspruch 2, worin R² ein stickstoffhaltiger Heterozyklus ist mit der Formel 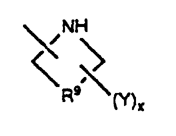 worin R⁹, Y und x wie in Anspruch 1 definiert sind.

4. Ein Verfahren nach Anspruch 1 zur Darstellung einer Verbindung mit der Formel 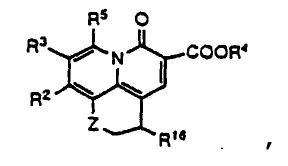 worin
worin Z aus der Gruppe gewählt ist, die aus CH₂, O und S besteht, und R¹⁶ ein C₁-C₆- Alkyl ist.

5. Ein Verfahren nach Anspruch 4, worin Z gleich O und R² ein stickstoffhaltiger Heterozyklus ist mit der Formel 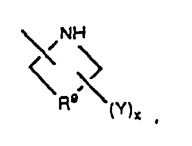 worin R⁹, Y und x wie in Anspruch 1 definiert sind.

6. Ein Verfahren nach Anspruch 1 zur Darstellung einer Verbindung, die aus der Gruppe gewählt ist, die sich wie folgt zusammensetzt:
3-Fluor-9-(4-fluorphenyl)-2-(4-methylpiperazin-1-yl)-6(H)-6-oxo-pyrido-[1,2-a]pyrimidin-7-carbonsäure;
9-(2,4-Difluorphenyl)-3-fluor-2-(4-methylpiperazin-1-yl)-6(H)-6-oxo-pyrido[1,2-a]pyrimidin-7-carbonsäure;
3-Fluor-9-cyclopropyl-2-(4-methylpiperazin-1-yl)-6(H)-6-oxo-pyrido[1,2-a]pyrimidin-7-carbonsäure;
2-(3-Aminopyrrolidin-1-yl)-9-cyclopropyl-3-fluor-6H-6-oxo-pyrido[1,2-a]pyrimidin-7-carbonsäurehydrochloridsalz;
2-(3-Aminopyrrolidin-1-yl)-9-cyclopropyl-3-fluor-6H-6-oxo-pyrido[1,2-a]pyrimidin-7-carbonsäure;
9-(2,4-Difluorphenyl)-3-fluor-2-(4-methylpiperazin-1-yl)-6H-6-oxo-pyrido[1,2-a]pyrimidin-7-carbonsäure;
2-(3-Aminopyrrolidin-1-yl)-9-(2,4-difluorphenyl)-3-fluor-6H-6-oxo-pyrido[1,2-a]pyrimidin-7-carbonsäure;
2-(3-(N-*t*-Butoxycarbonyl)aminopyrrolidin-1-yl)-9-(2,4-difluorphenyl)-3-fluor-6H-6-oxopyrido[1,2-a]pyrimidin-7-carbonsäure;
2-(3-Aminopyrrolidin-1-yl)-9-(2,4-difluorphenyl)-3-fluor-6H-6-oxo-pyrido[1,2-a]pyrimidin-7-carbonsäure;
9-Cyclopropyl-3-fluor-2-(4-methylpiperazin-1-yl)-6H-6-oxo-pyrido-[1,2-a]pyrimidin-7-carbonsäure;
9-Cyclopropyl-3-fluor-2-(piperazin-1-yl)-6H-6-oxo-pyrido-[1,2-a]pyrimidin-7-carbonsäure;
9-Cyclopropyl-3-fluor-2-(morpholin-1-yl)-6H-6-oxo-pyrido-[1,2-a]pyrimidin-7-carbonsäure;
9-(2,4-Difluorphenyl)-3-fluor-2-(3-(N-(S)-norvalyl)aminopyrrolidin-1-yl)-6H-6-oxopyrido[1,2-a]pyrimidin-7-carbonsäurehydrochloridsalz;
2-(3-(N-(S)-Alanyl)aminopyrrolidin-1-yl)-9-(2,4-difluorphenyl)-3-fluor-6H-6-oxopyrido[1,2-a]pyrimidin-7-carbonsäurehydrochlorid;
2-(3-(N-(S)-Alanyl-(S)-alanyl)aminopyrrolidin-1-yl)-9-(2,4-difluorphenyl)-3-fluor-6H-6-oxopyrido[1,2-a]pyrimidin-7-carbonsäurehydrochlorid;
2-((2S,4S)-4-Acetamido-2-methylpyrrolidin-1-yl)-9-(2,4-difluorphenyl)-3-fluor-6H-6-oxopyrido[1,2-a]pyrimidin-7-carbonsäure;
9-(2,4-Difluorphenyl)-3-fluor-2-(3-hydroxypyrrolin-1-yl)-6H-6-oxopyrido[1,2-a]pyrimidin-7-carbonsäure; und
2-((2S,4S)-4-Amino-2-methylpyrrolidin-1-yl)-9-(2,4-difluorphenyl)-3-fluor-6H-6-oxopyrido[1,2-a]pyrimidin-7-carbonsäurehydrochlorid.

## Patentansprüche (Patentansprüche für folgende(n) Vertragsstaat(en): GR)

1. Ein Verfahren zur Darstellung einer Verbindung der Formel 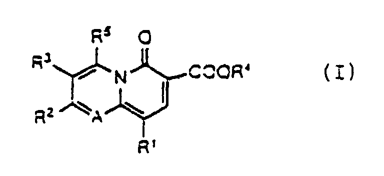 oder eines pharmazeutisch verträglichen Salzes, Esters oder Amids davon, worin
R¹ aus der Gruppe gewählt ist, die aus (a) C₁-C₆-Alkyl, (b) C₂-C₆-Alkenyl, (c) C₁-C₆-Haloalkyl, (d) C₁-C₆-Alkoxy, (e) C₃-C₈-Cycloalkyl, (f) Phenyl, (g) Halo, (h) Cyano, (i) Nitro, (j) Bicycloalkyl mit 4 bis 9 Kohlenstoffatomen im Ringsystem, wobei nicht-benachbarte Atome durch 1-3 weitere Kohlenstoffatome miteinander verbunden sind, (k) C₂-C₆-Alkynyl, (l) (C₁-C₆-Alkoxy)carbonyl, (m) stickstoffhaltigem aromatischen Heterozyklus mit 5 bis 7 Ringatomen und (n) einer Gruppe mit der Formel -NR⁷R⁸ besteht, worin R⁷ und R⁸ unabhängig voneinander aus der Gruppe gewählt sind, die aus Wasserstoff, C₁-C₆-Alkyl und C₁-C₈-Alkanoyl besteht, oder zusammengenommen mit dem Stickstoffatom, an das sie gebunden sind, bilden R⁷ und R⁸ einen 5-, 6- oder 7-gliedrigen gesättigten Heterozyklus, der wahlweise substituiert ist mit einem C₆-C₁₀-carbocyclischen Aryl-C₁-C₆-Alkyl, (C₁-C₆-Alkoxy)carbonyl, C₁-C₆-Alkyl, C₁-C₆-Haloalkyl, aminosubstituiertem C₁-C₆-Alkyl, worin die Aminogruppen einen oder zwei C₁-C₆-Alkylsubstituenten oder einen Alphaaminosäure- oder Polypeptidrestsubstituenten enthalten können, hydroxysubstituierten C₁-C₆-Alkyl, Hydroxy, C₁-C₆-Alkoxy oder Halo, oder einem Amino, mono- oder di-(C₁-C₆-Alkyl)amino, oder C₁-C₈-Alkanoylamino, wobei die Aminogruppe mit C₁-C₈-Alkanoyl, einer Alphaaminosäure oder einem Polypeptid mit zwei bis fünf Aminosäuren substituiert sein kann;
R² ist gewählt aus der Gruppe bestehend aus Halo, C₁-C₆-Alkoxy, C₆-C₁₀-carbocyclischem Aryloxy, C₆-C₁₀-carbocyclischem Aryl-C₁-C₆-Alkoxy, C₁-C₆-Alkyl, C₂-C₆-Alkenyl, C₃-C₈-Cycloalkyl, C₄-C₈ -Cycloalkenyl, C₆-C₁₀-carbocyclischem Aryl-C₁-C₆-Alkyl, C₃-C₈-Cycloalkyl-C₁-C₆-Alkyl, Phenyl, Amino, mono- oder di-(C₁-C₆-Alkyl)amino, C₆-C₁₀-carbocyclischem Aryl-C₁-C₆-Alkylamino, (hydroxysubstituiertem C₁-C₆-Alkyl)amino, bicyclischer stickstotfhaltiger heterocyclischer Gruppe, die gewählt ist aus einer der Formeln 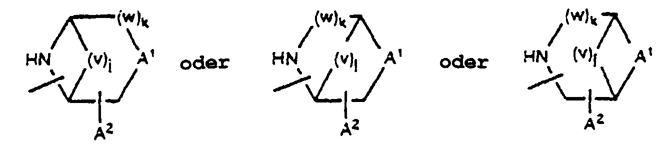 worin v und w gleich CH₂ sind; j und k sind unabhängig voneinander 1, 2 oder 3; A¹ ist ein Kohlenstoffatom oder ein Heteroatom, das aus S, O und N gewählt ist; und A² sind einer oder mehrere Nicht-Wasserstoff-Substituenten, die unabhängig gewählt sind aus der Gruppe, die aus C₁-C₆-Alkyl, C₁-C₆-Haloalkyl, hydroxysubstituiertem C₁-C₆-Alkyl, Hydroxy, Halo, aminosubstituiertem C₁-C₆-Alkyl, worin die Aminogruppen einen oder zwei C₁-C₆-Alkylsubstituenten oder einen Alphaaminosäure- oder Polypeptidrestsubstituenten enthalten können, C₁-C₈-Alkanoylamino, Phenyl und einer Gruppe mit der Formel -NR⁷R⁸ besteht, worin R⁷ und R⁸ unabhängig voneinander aus Wasserstoff und C₁-C₆-Alkyl gewählt sind, oder wenn ein Rest Wasserstoff ist, ist der andere eine Alphaaminosäure oder ein Polypeptidrest mit 2 bis 5 Aminosäuren, oder R² kann ein stickstoffhaltiger Heterozyklus sein mit der Formel 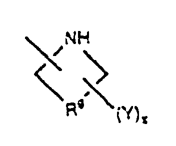 worin x gleich 0 bis 3 ist; R⁹ ist gewählt aus der Gruppe, die aus (a) -(CH₂)ₘ-, worin m gleich 1, 2 oder 3 ist, und (b) -(CH₂)ₙR¹⁰(CH₂)ₚ- besteht, worin R¹⁰ gleich S, O oder N ist, n ist 1 oder 2, und p ist 1 oder 2; und Y ist ein Nicht-Wasserstoff-Substituent, der unabhängig gewählt ist aus der Gruppe bestehend aus C₁-C₆-Alkyl, C₁-C₆-Haloalkyl, C₁-C₆-Alkoxy, hydroxysubstituiertem C₁-C₆-Alkyl, Hydroxy, aminosubstituiertem C₁-C₆-Alkyl, worin die Aminogruppen einen oder zwei C₁-C₆-Alkylsubstituenten oder einen Alphaaminosäure- oder Polypeptidrestsubstituenten enthalten können, Halogen und einer Gruppe mit der Formel -NR¹¹R¹², worin R¹¹ und R¹² unabhängig voneinander gewählt sind aus der Gruppe, die aus Wasserstoff und C₁-C₆-Alkyl besteht, oder wenn ein Rest Wasserstoff ist, ist der andere gewählt aus der Gruppe, die aus C₁-C₈-Alkanoyl, einer Alphaaminosäure und einem Polypeptidrest mit 2 bis 5 Aminosäuren besteht;
R³ ist gewählt aus der Gruppe, die aus Wasserstoff, Halo und C₁-C₆-Alkoxy besteht;
R⁴ ist gewählt aus der Gruppe, die aus Wasserstoff, C₁-C₆-Alkyl, einem pharmazeutisch verträglichen Kation und einer Prodrug-Estergruppe besteht, wobei die Prodrug-Estergruppe eine Estergruppe ist, die unter physiologischen Bedingungen hydrolysierbar ist;
R⁵ ist gewählt aus der Gruppe, die aus Wasserstoff, Halo, Hydroxy, C₁-C₆-Alkyl, C₁-C₆-Haloalkyl, C₁-C₆-Alkoxy und einer Gruppe mit der Formel NR¹³R¹⁴ besteht, worin R¹³ und R¹⁴ unabhängig voneinander gewählt sind aus der Gruppe, die aus Wasserstoff, C₁-C₆-Alkyl, hydroxysubstituiertem C₁-C₆-Alkyl, C₁-C₆-Alkoxysubstituiertem C₁-C₆-Alkyl und C₂-C₈-Alkanoyl besteht; und
A ist N oder CR⁶, worin R⁶ gewählt ist aus der Gruppe bestehend aus Halogen, C₁-C₆-Alkyl, C₁-C₆-Haloalkyl, hydroxysubstituiertem C₁-C₆-Alkyl, C₁-C₆-Alkoxy-C₁-C₆-Alkyl, C₁-C₆-Alkoxy und aminosubstituiertem C₁-C₆-Alkyl, worin die Aminogruppen einen oder zwei C₁-C₆-Alkylsubstituenten oder einen Alphaaminosäure- oder Polypeptidrestsubstituenten enthalten können, oder worin R¹ und R⁶, zusammengenommen mit den Atomen, an die sie gebunden sind, einen 6-gliedrigen gesättigten Ring bilden, der ein Sauerstoff- oder ein Schwefelatom enthalten kann und der mit einem C₁-C₆-Alkyl substituiert sein kann;
worin Cycloalkyl allein oder als Teil einer anderen Gruppe, Cycloalkenyl oder Bicycloalkyl substituiert sein können mit einem C₆-C₁₀-carbocyclischem Aryl-C₁-C₆-Alkyl, (C₁-C₆-Alkoxy)carbonyl, C₁-C₆-Alkyl, C₁-C₆-Haloalkyl, aminosubstituiertem C₁-C₆-Alkyl, worin die Aminogruppen einen oder zwei C₁-C₆-Alkylsubstituenten oder einen Alphaaminosäure- oder Polypeptidrestsubstituenten enthalten können, hydroxysubstituiertem C₁-C₆-Alkyl, Hydroxy, C₁-C₆-Alkoxy oder Halo, oder einem Amino, mono- oder di-(C₁-C₆-Alkyl)amino, oder C₁-C₈-Alkanoylamino, worin die Aminogruppe mit einem C₁-C₈-Alkanoyl, einer Aminosäure oder einem Polypeptid mit zwei bis fünf Aminosäuren substituiert sein kann, wobei das Verfahren den Schritt Hydrolysieren eines Esters der Carbonsäureverbindung nach Formel I beinhaltet.

2. Ein Verfahren nach Anspruch 1, worin A gleich N oder CR⁶ ist und R⁶ aus der Gruppe gewählt ist, die aus Halo, C₁-C₆-Alkyl, C₁-C₆-Haloalkyl, hydroxysubstituiertem C₁-C₆-Alkyl, C₁-C₆-Alkoxy-C₁-C₆-Alkyl, C₁-C₆-Alkoxy und aminosubstituiertem C₁-C₆-Alkyl besteht, worin die Aminogruppen einen oder zwei C₁-C₆-Alkylsubstituenten oder einen Alphaaminosäure- oder Polypeptidrestsubstituenten enthalten können.

3. Ein Verfahren nach Anspruch 2, worin R² ein stickstoffhaltiger Heterozyklus ist mit der Formel 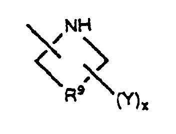 worin R⁹, Y und x wie in Anspruch 1 definiert sind.

4. Ein Verfahren nach Anspruch 1 zur Darstellung einer Verbindung mit der Formel 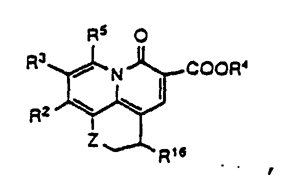 worin
worin Z aus der Gruppe gewählt ist, die aus CH₂, O und S besteht, und R¹⁶ ein C₁-C₆- Alkyl ist.

5. Ein Verfahren nach Anspruch 4, worin Z gleich O und R² ein stickstoffhaltiger Heterozyklus ist mit der Formel 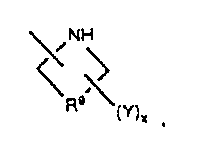 worin R⁹, Y und x wie in Anspruch 1 definiert sind.

6. Ein Verfahren nach Anspruch 1 zur Darstellung einer Verbindung, die aus der Gruppe gewählt ist, die sich wie folgt zusammensetzt:
3-Fluor-9-(4-fluorphenyl)-2-(4-methylpiperazin-1-yl)-6(H)-6-oxo-pyrido-[1,2-a]pyrimidin-7-carbonsäure;
9-(2,4-Difluorphenyl)-3-fluor-2-(4-methylpiperazin-1-yl)-6(H)-6-oxo-pyrido[1,2-a]pyrimidin-7-carbonsäure;
3-Fluor-9-cyclopropyl-2-(4-methylpiperazin-1-yl)-6(H)-6-oxo-pyrido[1,2-a]pyrimidin-7-carbonsäure;
2-(3-Aminopyrrolidin-1-yl)-9-cyclopropyl-3-fluor-6H-6-oxo-pyrido[1,2-a]pyrimidin-7-carbonsäurehydrochloridsalz;
2-(3-Aminopyrrolidin-1-yl)-9-cyclopropyl-3-fluor-6H-6-oxo-pyrido[1,2-a]pyrimidin-7-carbonsäure;
9-(2,4-Difluorphenyl)-3-fluor-2-(4-methylpiperazin-1-yl)-6H-6-oxo-pyrido[1,2-a]pyrimidin-7-carbonsäure;
2-(3-Aminopyrrolidin-1-yl)-9-(2,4-difluorphenyl)-3-fluor-6H-6-oxo-pyrido[1,2-a]pyrimidin-7-carbonsäure;
2-(3-(N-*t*-Butoxycarbonyl)aminopyrrolidin-1-yl)-9-(2,4-difluorphenyl)-3-fluor-6H-6-oxopyrido[1,2-a]pyrimidin-7-carbonsäure;
2-(3-Aminopyrrolidin-1-yl)-9-(2,4-difluorphenyl)-3-fluor-6H-6-oxo-pyrido[1,2-a]pyrimidin-7-carbonsäure;
9-Cyclopropyl-3-fluor-2-(4-methylpiperazin-1-yl)-6H-6-oxo-pyrido-[1,2-a]pyrimidin-7-carbonsäure;
9-Cyclopropyl-3-fluor-2-(piperazin-1-yl)-6H-6-oxo-pyrido-[1,2-a]pyrimidin-7-carbonsäure;
9-Cyclopropyl-3-fluor-2-(morpholin-1-yl)-6H-6-oxo-pyrido-[1,2-a]pyrimidin-7-carbonsäure;
9-(2,4-Difluorphenyl)-3-fluor-2-(3-(N-(S)-norvalyl)aminopyrrolidin-1-yl)-6H-6-oxopyrido[1,2-a]pyrimidin-7-carbonsäurehydrochloridsalz;
2-(3-(N-(S)-Alanyl)aminopyrrolidin-1-yl)-9-(2,4-difluorphenyl)-3-fluor-6H-6-oxopyrido[1,2-a]pyrimidin-7-carbonsäurehydrochlorid;
2-(3-(N-(S)-Alanyl-(S)-alanyl)aminopyrrolidin-1-yl)-9-(2,4-difluorphenyl)-3-fluor-6H-6-oxopyrido[1,2-a]pyrimidin-7-carbonsäurehydrochlorid;
2-((2S,4S)-4-Acetamido-2-methylpyrrolidin-1-yl)-9-(2,4-difluorphenyl)-3-fluor-6H-6-oxopyrido[1,2-a]pyrimidin-7-carbonsäure;
9-(2,4-Difluorphenyl)-3-fluor-2-(3-hydroxypyrrolin-1-yl)-6H-6-oxopyrido[1,2-a]pyrimidin-7-carbonsäure; und
2-((2S,4S)-4-Amino-2-methylpyrrolidin-1-yl)-9-(2,4-difluorphenyl)-3-fluor-6H-6-oxopyrido[1,2-a]pyrimidin-7-carbonsäurehydrochlorid.

7. Die Verwendung einer Verbindung mit der Formel 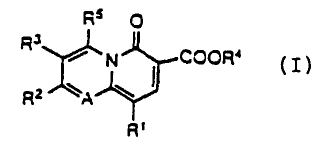 oder eines pharmazeutisch verträglichen Salzes, Esters oder Amids davon, worin
R¹ aus der Gruppe gewählt ist, die aus (a) C₁-C₆-Alkyl, (b) C₂-C₆-Alkenyl, (c) C₁-C₆-Haloalkyl, (d) C₁-C₆-Alkoxy, (e) C₃-C₈-Cycloalkyl, (f) Phenyl, (g) Halo, (h) Cyano, (i) Nitro, (j) Bicycloalkyl mit 4 bis 9 Kohlenstoffatomen im Ringsystem, wobei nicht-benachbarte Atome durch 1-3 weitere Kohlenstoffatome miteinander verbunden sind, (k) C₂-C₆-Alkynyl, (l) (C₁-C₆-Alkoxy)carbonyl, (m) stickstoffhaltigem aromatischen Heterozyklus mit 5 bis 7 Ringatomen und (n) einer Gruppe mit der Formel -NR⁷R⁸ besteht, worin R⁷ und R⁸ unabhängig voneinander aus der Gruppe gewählt sind, die aus Wasserstoff, C₁-C₆-Alkyl und C₁-C₈-Alkanoyl besteht, oder zusammengenommen mit dem Stickstoffatom, an das sie gebunden sind, bilden R⁷ und R⁸ einen 5-, 6- oder 7-gliedrigen gesättigten Heterozyklus, der substituiert ist mit einem C₆-C₁₀-carbocyclischen Aryl-C₁-C₆-Alkyl, (C₁-C₆-Alkoxy)carbonyl, C₁-C₆-Alkyl, C₁-C₆-Haloalkyl, aminosubstituierten C₁-C₆-Alkyl, worin die Aminogruppen einen oder zwei C₁-C₆-Alkylsubstituenten oder einen Alphaaminosäure- oder Polypeptidrestsubstituenten enthalten können, hydroxysubstituierten C₁-C₆-Alkyl, Hydroxy, C₁-C₆-Alkoxy oder Halo, oder einem Amino, mono- oder di-(C₁-C₆-Alkyl)amino, oder C₁-C₈-Alkanoylamino, wobei die Aminogruppe mit C₁-C₈-Alkanoyl, einer Alphaaminosäure oder einem Polypeptid mit zwei bis fünf Aminosäuren substituiert sein kann;
R² ist gewählt aus der Gruppe bestehend aus Halo, C₁-C₆-Alkoxy, C₆-C₁₀-carbocyclischem Aryloxy, C₆-C₁₀-carbocyclischem Aryl-C₁-C₆-Alkoxy, C₁-C₆-Alkyl, C₂-C₆-Alkenyl, C₃-C₆-Cycloalkyl, C₄-C₈-Cycloalkenyl, C₆-C₁₀-carbocyclischem Aryl-C₁-C₆-Alkyl, C₃-C₈-Cycloalkyl-C₁-C₆-Alkyl, Phenyl, Amino, mono- oder di-(C₁-C₆-Alkyl)amino, C₆-C₁₀-carbocyclischem Aryl-C₁-C₆-Alkylamino, (hydroxysubstituiertem C₁-C₆-Alkyl)amino, bicyclischer stickstoffhaltiger heterocyclischer Gruppe, die gewählt ist aus einer der Formeln 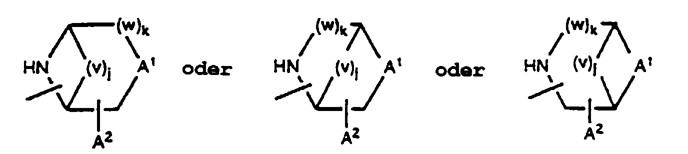 worin v und w gleich CH₂ sind; j und k sind unabhängig voneinander 1, 2 oder 3; A¹ ist ein Kohlenstoffatom oder ein Heteroatom, das aus S, O und N gewählt ist; und A² sind einer oder mehrere Nicht-Wasserstoff-Substituenten, die unabhängig gewählt sind aus der Gruppe, die aus C₁-C₆-Alkyl, C₁-C₆-Haloalkyl, hydroxysubstituiertem C₁-C₆-Alkyl, Hydroxy, Halo, aminosubstituiertem C₁-C₆-Alkyl, worin die Aminogruppen einen oder zwei C₁-C₆-Alkylsubstituenten oder einen Alphaaminosäure- oder Polypeptidrestsubstituenten enthalten können, C₁-C₈-Alkanoylamino, Phenyl und einer Gruppe mit der Formel -NR⁷R⁸ besteht, worin R⁷ und R⁸ unabhängig voneinander aus Wasserstoff und C₁-C₆-Alkyl gewählt sind, oder wenn ein Rest Wasserstoff ist, ist der andere eine Alphaaminosäure oder ein Polypeptidrest mit 2 bis 5 Aminosäuren, oder R² kann ein stickstoffhaltiger Heterozyklus sein mit der Formel 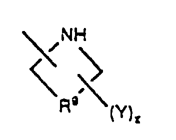 worin x gleich 0 bis 3 ist; R⁹ ist gewählt aus der Gruppe, die aus (a) -(CH₂)ₘ-, worin m gleich 1, 2 oder 3 ist, und (b) -(CH₂)ₙR¹⁰(CH₂)ₚ- besteht, worin R¹⁰ gleich S, O oder N ist, n ist 1 oder 2, und p ist 1 oder 2; und Y ist ein Nicht-Wasserstoff-Substituent, der unabhängig gewählt ist aus der Gruppe bestehend aus C₁-C₆-Alkyl, C₁-C₆-Haloalkyl, C₁-C₆-Alkoxy, hydroxysubstituiertem C₁-C₆-Alkyl, Hydroxy, aminosubstituiertem C₁-C₆-Alkyl, worin die Aminogruppen einen oder zwei C₁-C₆-Alkylsubstituenten oder einen Alphaaminosäure- oder Polypeptidrestsubstituenten enthalten können, Halogen und einer Gruppe mit der Formel -NR¹¹R¹², worin R¹¹ und R¹² unabhängig voneinander gewählt sind aus der Gruppe, die aus Wasserstoff und C₁-C₆-Alkyl besteht, oder wenn ein Rest Wasserstoff ist, ist der andere gewählt aus der Gruppe, die aus C₁-C₈-Alkanoyl, einer Alphaaminosäure und einem Polypeptidrest mit 2 bis 5 Aminosäuren besteht;
R³ ist gewählt aus der Gruppe, die aus Wasserstoff, Halo und C₁-C₆-Alkoxy besteht;
R⁴ ist gewählt aus der Gruppe, die aus Wasserstoff, C₁-C₆-Alkyl, einem pharmazeutisch verträglichen Kation und einer Prodrug-Estergruppe besteht, wobei die Prodrug-Estergruppe eine Estergruppe ist, die unter physiologischen Bedingungen hydrolysierbar ist;
R⁵ ist gewählt aus der Gruppe, die aus Wasserstoff, Halo, Hydroxy, C₁-C₆-Alkyl, C₁-C₆-Haloalkyl, C₁-C₆-Alkoxy und einer Gruppe mit der Formel NR¹³R¹⁴ besteht, worin R¹³ und R¹⁴ unabhängig voneinander gewählt sind aus der Gruppe, die aus Wasserstoff, C₁-C₆-Alkyl, hydroxysubstituiertem C₁-C₆-Alkyl, C₁-C₆-Alkoxysubstituiertem C₁-C₆-Alkyl und C₁-C₈-Alkanoyl besteht; und
A ist N oder CR⁶, worin R⁶ gewählt ist aus der Gruppe bestehend aus Halogen, C₁-C₆-Alkyl, C₁-C₆-Haloalkyl, hydroxysubstituiertem C₁-C₆-Alkyl, C₁-C₆-Alkoxy-C₁-C₆-Alkyl, C₁-C₆-Alkoxy und aminosubstituiertem C₁-C₆-Alkyl, worin die Aminogruppen einen oder zwei C₁-C₆-Alkylsubstituenten oder einen Alphaaminosäure- oder Polypeptidrestsubstituenten enthalten können, oder worin R¹ und R⁶, zusammengenommen mit den Atomen, an die sie gebunden sind, einen 6-gliedrigen gesättigten Ring bilden, der ein Sauerstoff- oder ein Schwefelatom enthalten kann und der mit einem C₁-C₆-Alkyl substituiert sein kann;
worin Cycloalkyl allein oder als Teil einer anderen Gruppe, Cycloalkenyl oder Bicycloalkyl substituiert sein können mit einem C₆-C₁₀-carbocyclischem Aryl-C₁-C₆-Alkyl, (C₁-C₆-Alkoxy)carbonyl, C₁-C₆-Alkyl, C₁-C₆-Haloalkyl, aminosubstituiertem C₁-C₆-Alkyl, worin die Aminogruppen einen oder zwei C₁-C₆-Alkylsubstituenten oder einen Alphaaminosäure- oder Polypeptidrestsubstituenten enthalten können, hydroxysubstituiertem C₁-C₆-Alkyl, Hydroxy, C₁-C₆-Alkoxy oder Halo, oder einem Amino, mono- oder di-(C₁-C₆-Alkyl)amino, oder C₁-C₈-Alkanoylamino, worin die Aminogruppe mit einem C₁-C₈-Alkanoyl, einer Alphaminosäure oder einem Polypeptid mit zwei bis fünf Aminosäuren substituiert sein kann, für die Herstellung eines Medikaments zur Behandlung oder Prävention einer bakteriellen Infektion bei einem Menschen oder einem Wirtstier.

8. Die Verwendung einer Verbindung, die aus der Gruppe gewählt ist, die sich wie folgt zusammensetzt:
3-Fluor-9-(4-fluorphenyl)-2-(4-methylpiperazin-1-yl)-6(H)-6-oxo-pyrido-[1,2-a]pyrimidin-7-carbonsäure;
9-(2,4-Difluorphenyl)-3-fluor-2-(4-methylpiperazin-1-yl)-6(H)-6-oxo-pyrido[1,2-a]pyrimidin-7-carbonsäure;
3-Fluor-9-cyclopropyl-2-(4-methylpiperazin-1-yl)-6(H)-6-oxo-pyrido[1,2-a]pyrimidin-7-carbonsäure;
2-(3-Aminopyrrolidin-1-yl)-9-cyclopropyl-3-fluor-6H-6-oxo-pyrido[1,2-a]pyrimidin-7-carbonsäurehydrochloridsalz;
2-(3-Aminopyrrolidin-1-yl)-9-cyclopropyl-3-fluor-6H-6-oxo-pyrido[1,2-a]pyrimidin-7-carbonsäure;
9-(2,4-Difluorphenyl)-3-fluor-2-(4-methylpiperazin-1-yl)-6H-6-oxo-pyrido[1,2-a]pyrimidin-7-carbonsäure;
2-(3-Aminopyrrolidin-1-yl)-9-(2,4-difluorphenyl)-3-fluor-6H-6-oxo-pyrido[1,2-a]pyrimidin-7-carbonsäure;
2-(3-(N-*t*-Butoxycarbonyl)aminopyrrolidin-1-yl)-9-(2,4-difluorphenyl)-3-fluor-6H-6-oxopyrido[1,2-a]pyrimidin-7-carbonsäure;
2-(3-Aminopyrrolidin-1-yl)-9-(2,4-difluorphenyl)-3-fluor-6H-6-oxo-pyrido[1,2-a]pyrimidin-7-carbonsäure;
9-Cyclopropyl-3-fluor-2-(4-methylpiperazin-1-yl)-6H-6-oxo-pyrido-[1,2-a]pyrimidin-7-carbonsäure;
9-Cyclopropyl-3-fluor-2-(piperazin-1-yl)-6H-6-oxo-pyrido-[1,2-a]pyrimidin-7-carbonsäure;
9-Cyclopropyl-3-fluor-2-(morpholin-1-yl)-6H-6-oxo-pyrido-[1,2-a]pyrimidin-7-carbonsäure;
9-(2,4-Difluorphenyl)-3-fluor-2-(3-(N-(S)-norvalyl)aminopyrrolidin-1-yl)-6H-6-oxopyrido[1,2-a]pyrimidin-7-carbonsäurehydrochloridsalz;
2-(3-(N-(S)-Alanyl)aminopyrrolidin-1-yl)-9-(2,4-difluorphenyl)-3-fluor-6H-6-oxopyrido[1,2-a]pyrimidin-7-carbonsäurehydrochlorid;
2-(3-(N-(S)-Alanyl-(S)-alanyl)aminopyrrolidin-1-yl)-9-(2,4-difluorphenyl)-3-fluor-6H-6-oxopyrido[1,2-a]pyrimidin-7-carbonsäurehydrochlorid;
2-((2S,4S)-4-Acetamido-2-methylpyrrolidin-1-yl)-9-(2,4-difluorphenyl)-3-fluor-6H-6-oxopyrido[1,2-a]pyrimidin-7-carbonsäure;
9-(2,4-Difluorphenyl)-3-fluor-2-(3-hydroxypyrrolin-1-yl)-6H-6-oxopyrido[1,2-a]pyrimidin-7-carbonsäure; und
2-((2S,4S)-4-Amino-2-methylpyrrolidin-1-yl)-9-(2,4-difluorphenyl)-3-fluor-6H-6-oxopyrido[1,2-a]pyrimidin-7-carbonsäurehydrochlorid, für die Herstellung eines Medikaments zur Behandlung oder Prävention einer bakteriellen Infektion bei einem Menschen oder einem Wirtstier.

## Revendications (Revendications pour l'(les) Etat(s) contractant(s) suivant(s): AT, BE, CH, LI, DE, DK, FR, GB, IT, LU, NL, SE)

1. Composé de formule 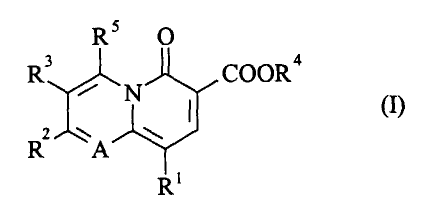 ou un de ses sels, esters ou amides pharmaceutiquement acceptables, dans laquelle
R¹ est choisi dans le groupe constitué par (a) alkyle en C₁-C₆, (b) alcényle en C₂-C₆, (c) halogénoalkyle en C₁-C₆, (d) alcoxy en C₁-C₆, (e) cycloalkyle en C₃-C₈, (f) phényle, (g) halogène, (h) cyano, (i) nitro, (j) bicycloalkyle ayant de 4 à 9 atomes de carbone dans le système cyclique dans lequel des atomes non adjacents sont pontés par 1 à 3 atomes de carbone supplémentaires, (k) alcynyle en C₂-C₆, (l) (alcoxy en C₁-C₆)carbonyle, (m) un hétérocycle aromatique contenant de l'azote ayant de 5 à 7 atomes cycliques et (n) un groupe de formule -NR⁷R⁸ dans laquelle R⁷ et R⁸ sont choisis indépendamment dans le groupe constitué par hydrogène, alkyle en C₁-C₆ et alcanoyle en C₁-C₈ ou, pris avec l'atome d'azote auquel ils sont liés, R⁷ et R⁸ forment un hétérocycle saturé à 5, 6 ou 7 chaînons éventuellement substitué par un groupe (aryl carbocyclique en C₆-C₁₀)alkyle en C₁-C₆, (alcoxy on C₁-C₆)carbonyle, alkyle en C₁-C₆, halogénoalkyle en C₁-C₆, alkyle en C₁-C₆ substitué par amino, dans lequel les groupes amino peuvent comporter un ou deux substituants alkyle en C₁-C₆ ou un substituant constitué par un acide alpha-aminé ou un résidu de polypeptide, alkyle en C₁-C₆ substitué par hydroxy, hydroxy, alcoxy en C₁-C₆ ou halogène ou un groupe amino, mono- ou di-(alkyl en C₁-C₆)amino ou (alcanoyl en C₁-C₈)amino, dans lequel le groupe amino peut être substitué par un groupe alcanoyle en C₁-C₈, un acide alpha-aminé ou un polypeptide comportant de deux à cinq acides aminés;
R² est choisi dans le groupe constitué par halogène, alcoxy en C₁-C₆, aryloxy carbocyclique on C₆-C₁₀, (aryl carbocyclique en C₆-C₁₀)alcoxy en C₁-C₆, alkyle en C₁-C₆, alcényle en C₂-C₆, cycloalkyle en C₃-C₈, cycloalcényle en C₄-C₈, (aryl carbocyclique en C₆-C₁₀)alkyle on C₁-C₆, (cycloalkyle en C₃-C₈)alkyle en C₁-C₆, phényle, amino, mono- ou di-(alkyl en C₁-C₆)amino, (aryl carbocyclique en C₆-C₁₀)(alkyl en C₁-C₆)amino, (alkyl en C₁-C₆ substitué par hydroxy)amino, un groupe hétérocyclique bicyclique contenant de l'azote choisi parmi l'une des formules 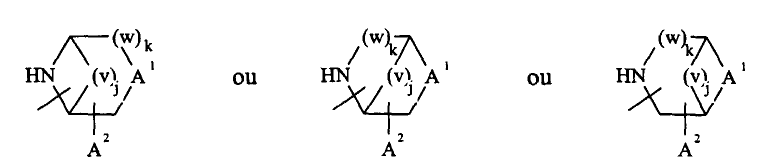 dans lesquelles v et w sont CH₂; j et k sont indépendamment 1, 2 ou 3; A¹ est un atome de carbone ou un hétéroatome choisi parmi S, O et N; et A² est un ou plusieurs substituants différents de l'hydrogène choisis indépendamment dans le groupe constitué par alkyle en C₁-C₆, halogénoalkyle en C₁-C₆, alkyle en C₁-C₆ substitué par hydroxy, hydroxy, halogène, alkyle en C₁-C₆ substitué par amino dans lequel les groupes amino peuvent comporter un ou deux substituants alkyle en C₁-C₆ ou un substituant constitué par un acide alpha-aminé ou un résidu de polypeptide, (alcanoyl en C₁-C₈)amino, phényle et un groupe de formule -NR⁷R⁸ dans laquelle R⁷ et R⁸ sont choisis indépendamment dans le groupe constitué par hydrogène et alkyle en C₁-C₆ ou, lorsque l'un est hydrogène, l'autre est un acide alpha-aminé ou un résidu de polypeptide comportant de 2 à 5 acides aminés ou R² peut être un hétérocycle contenant de l'azote ayant la formule 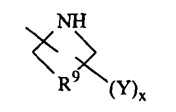 dans laquelle x est 0 à 3; R⁹ est choisi dans le groupe constitué par (a) -(CH₂)ₘ-, où m est 1, 2 ou 3; et (b) -(CH₂)ₙR¹⁰(CH₂)ₚ- où R¹⁰ est S, O ou N, n est 1 ou 2 et p est 1 ou 2; et Y est un substituant différent de l'hydrogène choisi indépendamment dans le groupe constitué par alkyle en C₁-C₆, halogénoalkyle en C₁-C₆, alcoxy en C₁-C₆, alkyle en C₁-C₆ substitué par hydroxy, hydroxy, alkyle en C₁-C₆ substitué par amino, dans lequel les groupes amino peuvent comporter un ou deux substituants alkyle en C₁-C₆ ou un substituant constitué par un acide alpha-aminé ou un résidu de polypeptide, halogène et un groupe de formule -NR¹¹R¹² dans laquelle R¹¹ et R¹² sont choisis indépendamment dans le groupe constitué par hydrogène et alkyle en C₁-C₆ ou, lorsque l'un est hydrogène, l'autre est choisi dans le groupe constitué par alcanoyle en C₁-C₈, un acide alpha-aminé et un résidu de polypeptide comportant de 2 à 5 acides aminés;
R³ est choisi dans le groupe constitué par hydrogène, halogène et alcoxy en C₁-C₆;
R⁴ est choisi dans le groupe constitué par hydrogène, alkyle en C₁-C₆, un cation pharmaceutiquement acceptable et un groupe ester de promédicament, ledit groupe ester de promédicament étant un groupe ester qui est hydrolysable dans des conditions physiologiques;
R⁵ est choisi dans le groupe constitué par hydrogène, halogène, hydroxy, alkyle en C₁-C₆, halogénoalkyle en C₁-C₆, alcoxy en C₁-C₆ et un groupe ayant la formule -NR¹³R¹⁴ dans laquelle R¹³ et R¹⁴ sont choisis indépendamment dans le groupe constitué par hydrogène, alkyle en C₁-C₆, alkyle en C₁-C₆ substitué par hydroxy, alkyle en C₁-C₆ substitué par alcoxy en C₁-C₆ et alcanoyle en C₁-C₈; et
A est N ou CR⁶, où R⁶ est choisi dans le groupe constitué par halogène, alkyle en C₁-C₆, halogénoalkyle en C₁-C₆, alkyle en C₁-C₆ substitué par hydroxy, (alcoxy en C₁-C₆)alkyle en C₁-C₆, alcoxy en C₁-C₆ et alkyle en C₁-C₆ substitué par amino dans lequel les groupes amino peuvent comporter un ou deux substituants alkyle en C₁-C₆ ou un substituant constitué par un acide alpha-aminé ou un résidu de polypeptide ou dans laquelle R¹ et R⁶ pris avec les atomes auxquels ils sont liés, forment un cycle saturé à 6 chaînons qui peut contenir un atome d'oxygène ou un atome de soufre et qui peut être substitué par un alkyle en C₁-C₆;
dans laquelle les groupes cycloalkyle en eux-mêmes ou en tant que partie d'un autre groupe, cycloalcényle ou bicycloalkyle peuvent être substitués par un groupe (aryl carbocyclique en C₆-C₁₀)alkyle en C₁-C₆, (alcoxy en C₁-C₆)carbonyle, alkyle en C₁-C₆, halogénoalkyle en C₁-C₆, alkyle en C₁-C₆ substitué par amino, dans lequel les groupes amino peuvent comporter un ou deux substituants alkyle en C₁-C₆ ou un substituant constitué par un acide alpha-aminé ou un résidu de polypeptide, alkyle en C₁-C₆ substitué par hydroxy, hydroxy, alcoxy en C₁-C₆ ou halogène ou un groupe amino, mono- ou di-(alkyl en C₁-C₆)amino ou (alcanoyl en C₁-C₈)amino, dans lequel le groupe amino peut être substitué par un groupe alcanoyle en C₁-C₈, un acide alpha-aminé ou un polypeptide ayant de deux à cinq acides aminés.

2. Composé selon la revendication 1, dans lequel A est N ou CR⁶ et R⁶ est choisi dans le groupe constitué par halogène, alkyle en C₁-C₆, halogénoalkyle en C₁-C₆, alkyle en C₁-C₆ substitué par hydroxy, (alcoxy en C₁-C₆)alkyle en C₁-C₆, alcoxy en C₁-C₆ et alkyle en C₁-C₆ substitué par amino, dans lequel les groupes amino peuvent comporter un ou deux substituants alkyle en C₁-C₆ ou un substituant constitué par un acide alpha-aminé ou un résidu de polypeptide.

3. Composé selon la revendication 2, dans lequel R² est un hétérocycle contenant de l'azote répondant à la formule 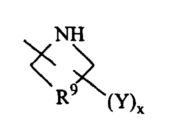 dans laquelle R⁹, Y et x sont tels que définis dans la revendication 1.

4. Composé selon la revendication 1 répondant à la formule 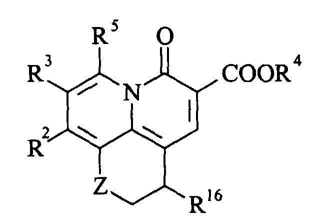 dans laquelle Z est choisi dans le groupe constitué par CH₂, O et S et R¹⁶ est alkyle en C₁-C₆.

5. Composé selon la revendication 4, dans lequel Z est O et R² est un hétérocycle contenant de l'azote répondant à la formule 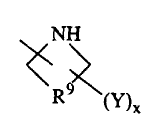 dans laquelle R⁹, Y et x sont tels que définis dans la revendication 1.

6. Composé choisi dans le groupe constitué par:
l'acide 3-fluoro-9-(4-fluorophényl)-2-(4-méthylpipérazin-1-yl)-6(H)-6-oxo-pyrido[1,2-a]pyrimidine-7-carboxylique;
l'acide 9-(2,4-difluorophényl)-3-fluoro-2-(4-méthylpipérazin-1-yl)-6(H)-6-oxo-pyrido[1,2-a]pyrimidine-7-carboxylique;
l'acide 3-fluoro-9-cyclopropyl-2-(4-méthylpipérazin-1-yl)-6(H)-6-oxo-pyrido[1,2-a]pyrimidine-7-carboxylique;
le chlorhydrate de l'acide 2-(3-aminopyrrolidin-1-yl)-9-cyclopropyl-3-fluoro-6H-6-oxo-pyrido[1,2-a]pyrimidine-7-carboxylique;
l'acide 2-(3-aminopyrrolidin-1-yl)-9-cyclopropyl-3-fluoro-6H-6-oxo-pyrido[1,2-a]pyrimidine-7-carboxylique;
l'acide 9-(2,4-difluorophényl)-3-fluoro-2-(4-méthylpipérazin-1-yl)-6H-6-oxo-pyrido[1,2-a]pyrimidine-7-carboxylique;
l'acide 2-(3-aminopyrrolidin-1-yl)-9-(2,4-difluorophényl)-3-fluoro-6H-6-oxo-pyrido[1,2-a]pyrimidine-7-carboxylique;
l'acide 2-(3-(N-tert-butoxycarbonyl)aminopyrrolidin-1-yl)-9-(2,4-difluorophényl)-3-fluoro-6H-6-oxo-pyrido[1,2-a]pyrimidine-7-carboxylique;
l'acide 2-(3-aminopyrrolidin-1-yl)-9-(2,4-difluorophényl)-3-fluoro-6H-6-oxo-pyrido[1,2-a]pyrimidine-7-carboxylique;
l'acide 9-cyclopropyl-3-fluoro-2-(4-méthylpipérazin-1-yl)-6H-6-oxo-pyrido[1,2-a]pyrimidine-7-carboxylique;
l'acide 9-cyclopropyl-3-fluoro-2-(pipérazin-1-yl)-6H-6-oxo-pyrido[1,2-a]pyrimidine-7-carboxylique;
l'acide 9-cyclopropyl-3-fluoro-2-(morpholin-1-yl)-6H-6-oxo-pyrido[1,2-a]pyrimidine-7-carboxylique;
le chlorhydrate de l'acide 9-(2,4-difluorophényl)-3-fluoro-2-(3-(N-(S)-norvalyl)aminopyrrolidin-1-yl)-6H-6-oxo-pyrido[1,2-a]pyrimidine-7-carboxylique;
le chlorhydrate de l'acide 2-(3-(N-(S)-alanyl)aminopyrrolidin-1-yl)-9-(2,4-difluorophényl)-3-fluoro-6H-6-oxo-pyrido[1,2-a]pyrimidine-7-carboxylique;
le chlorhydrate de l'acide 2-(3-(N-(S)-alanyl-(S)-alanyl)aminopyrrolidin-1-yl)-9-(2,4-difluorophényl)-3-fluoro-6H-6-oxo-pyrido[1,2-a]pyrimidine-7-carboxylique;
l'acide 2-((2S,4S)-4-acétamido-2-méthylpyrrolidin-1-yl)-9-(2,4-difluorophényl)-3-fluoro-6H-6-oxo-pyrido[1,2-a]pyrimidine-7-carboxylique;
l'acide 9-(2,4-difluorophényl)-3-fluoro-2-(3-hydroxypyrrolin-1-yl)-6H-6-oxo-pyrido[1,2-a]pyrimidine-7-carboxylique; et
le chlorhydrate de l'acide 2-((2S,4S)-4-amino-2-méthylpyrrolidin-1-yl)-9-(2,4-difluorophényl)-3-fluoro-6H-6-oxo-pyrido[1,2-a]pyrimidine-7-carboxylique.

7. Composition pharmaceutique pour le traitement ou la prévention des infections bactériennes comprenant une quantité thérapeutiquement efficace d'un composé selon la revendication 1 et un excipient pharmaceutiquement acceptable.

8. Composition pharmaceutique pour le traitement ou la prévention des infections bactériennes comprenant une quantité thérapeutiquement efficace d'un composé selon la revendication 6 et un excipient pharmaceutiquement acceptable.

9. Composé selon la revendication 1 ou 6 pour une utilisation en tant qu'agent thérapeutique.

10. Utilisation d'un composé selon la revendication 1 pour la fabrication d'un médicament pour le traitement ou la prévention des infections bactériennes chez un être humain ou un autre animal hôte.

11. Utilisation d'un composé selon la revendication 6 pour la fabrication d'un médicament pour le traitement ou la prévention des infections bactériennes chez un être humain ou un autre animal hôte.

## Revendications (Revendications pour l'(les) Etat(s) contractant(s) suivant(s): ES)

1. Procédé pour la préparation d'un composé de formule 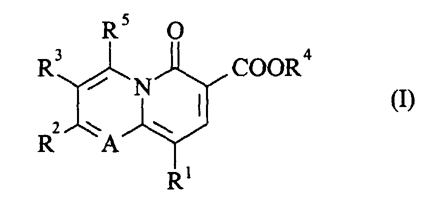 ou d'un de ses sels, esters ou amides pharmaceutiquement acceptables, dans laquelle
R¹ est choisi dans le groupe constitué par (a) alkyle en C₁-C₆, (b) alcényle en C₂-C₆, (c) halogénoalkyle en C₁-C₆, (d) alcoxy en C₁-C₆, (e) cycloalkyle en C₃-C₈, (f) phényle, (g) halogène, (h) cyano, (i) nitro, (j) bicycloalkyle ayant de 4 à 9 atomes de carbone dans le système cyclique dans lequel des atomes non adjacents sont pontés par 1 à 3 atomes de carbone supplémentaires, (k) alcynyle en C₂-C₆, (l) (alcoxy en C₁-C₆)carbonyle, (m) un hétérocycle aromatique contenant de l'azote ayant de 5 à 7 atomes cycliques et (n) un groupe de formule -NR⁷R⁸ dans laquelle R⁷ et R⁸ sont choisis indépendamment dans le groupe constitué par hydrogène, alkyle en C₁-C₆ et alcanoyle en C₁-C₈ ou, pris avec l'atome d'azote auquel ils sont liés, R⁷ et R⁸ forment un hétérocycle saturé à 5, 6 ou 7 chaînons éventuellement substitué par un groupe (aryl carbocyclique en C₆-C₁₀)alkyle en C₁-C₆, (alcoxy en C₁-C₆)carbonyle, alkyle en C₁-C₆, halogénoalkyle en C₁-C₆, alkyle en C₁-C₆ substitué par amino, dans lequel les groupes amino peuvent comporter un ou deux substituants alkyle en C₁-C₆ ou un substituant constitué par un acide alpha-aminé ou un résidu de polypeptide, alkyle en C₁-C₆ substitué par hydroxy, hydroxy, alcoxy en C₁-C₆ ou halogène ou un groupe amino, mono- ou di-(alkyl en C₁-C₆)amino ou (alcanoyl en C₁-C₈)amino, dans lequel le groupe amino peut être substitué par un groupe alcanoyle en C₁-C₈, un acide alpha-aminé ou un polypeptide comportant de deux à cinq acides aminés;
R² est choisi dans le groupe constitué par halogène, alcoxy en C₁-C₆, aryloxy carbocyclique en C₆-C₁₀, (aryl carbocyclique en C₆-C₁₀)alcoxy en C₁-C₆, alkyle en C₁-C₆, alcényle en C₂-C₆, cycloalkyle en C₃-C₈, cycloalcényle en C₄-C₈, (aryl carbocyclique en C₆-C₁₀)alkyle en C₁-C₆, (cycloalkyle en C₃-C₈)alkyle en C₁-C₆, phényle, amino, mono- ou di-(alkyl en C₁-C₆)amino, (aryl carbocyclique en C₆-C₁₀)(alkyl en C₁-C₆)amino, (alkyl en C₁-C₆ substitué par hydroxy)amino, un groupe hétérocyclique bicyclique contenant de l'azote choisi parmi l'une des formules 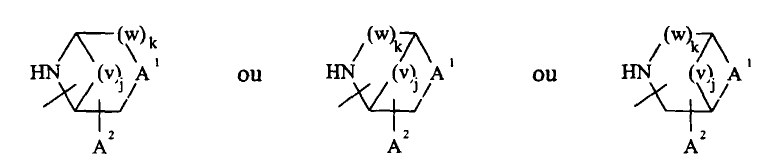 dans lesquelles v et w sont CH₂; j et k sont indépendamment 1, 2 ou 3; A¹ est un atome de carbone ou un hétéroatome choisi parmi S, O et N; et A² est un ou plusieurs substituants différents de l'hydrogène choisis indépendamment dans le groupe constitué par alkyle en C₁-C₆, halogénoalkyle en C₁-C₆, alkyle en C₁-C₆ substitué par hydroxy, hydroxy, halogène, alkyle en C₁-C₆ substitué par amino dans lequel les groupes amino peuvent comporter un ou deux substituants alkyle en C₁-C₆ ou un substituant constitué par un acide alpha-aminé ou un résidu de polypeptide, (alcanoyl en C₁-C₈)amino, phényle et un poupe de formule -NR⁷R⁸ dans laquelle R⁷ et R⁸ sont choisis indépendamment dans le groupe constitué par hydrogène et alkyle en C₁-C₆ ou, lorsque l'un est hydrogène, l'autre est un acide alpha-aminé ou un résidu de polypeptide comportant de 2 à 5 acides aminés ou R² peut être un hétérocycle contenant de l'azote ayant la formule 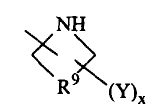 dans laquelle x est 0 à 3; R⁹ est choisi dans le poupe constitué par (a) -(CH₂)ₘ-, où m est 1, 2 ou 3; et (b) -(CH₂)ₙR¹⁰(CH₂)ₚ- où R¹⁰ est S, O ou N, n est 1 ou 2 et p est 1 ou 2; et Y est un substituant différent de l'hydrogène choisi indépendamment dans le groupe constitué par alkyle en C₁-C₆, halogénoalkyle en C₁-C₆, alcoxy en C₁-C₆, alkyle en C₁-C₆ substitué par hydroxy, hydroxy, alkyle en C₁-C₆ substitué par amino, dans lequel les poupes amino peuvent comporter un ou deux substituants alkyle en C₁-C₆ ou un substituant constitué par un acide alpha-aminé ou un résidu de polypeptide, halogène et un poupe de formule -NR¹¹R¹² dans laquelle R¹¹ et R¹² sont choisis indépendamment dans le groupe constitué par hydrogène et alkyle en C₁-C₆ ou, lorsque l'un est hydrogène, l'autre est choisi dans le poupe constitué par alcanoyle en C₁-C₈, un acide alpha-aminé et un résidu de polypeptide comportant de 2 à 5 acides aminés;
R³ est choisi dans le poupe constitué par hydrogène, halogène et alcoxy en C₁-C₆;
R⁴ est choisi dans le poupe constitué par hydrogène, alkyle en C₁-C₆, un cation pharmaceutiquement acceptable et un groupe ester de promédicament, ledit groupe ester de promédicament étant un groupe ester qui est hydrolysable dans des conditions physiologiques;
R⁵ est choisi dans le groupe constitué par hydrogène, halogène, hydroxy, alkyle en C₁-C₆, halogénoalkyle en C₁-C₆, alcoxy en C₁-C₆ et un groupe ayant la formule -NR¹³R¹⁴ dans laquelle R¹³ et R¹⁴ sont choisis indépendamment dans le groupe constitué par hydrogène, alkyle en C₁-C₆, alkyle en C₁-C₆ substitué par hydroxy, alkyle en C₁-C₆ substitué par alcoxy en C₁-C₆ et alcanoyle en C₁-C₈; et
A est N ou CR⁶, où R⁶ est choisi dans le groupe constitué par halogène, alkyle en C₁-C₆, halogénoalkyle en C₁-C₆, alkyle en C₁-C₆ substitué par hydroxy, (alcoxy en C₁-C₆)alkyle en C₁-C₆, alcoxy en C₁-C₆ et alkyle en C₁-C₆ substitué par amino dans lequel les groupes amino peuvent comporter un ou deux substituants alkyle en C₁-C₆ ou un substituant constitué par un acide alpha-aminé ou un résidu de polypeptide ou dans laquelle R¹ et R⁶, pris avec les atomes auxquels ils sont liés, forment un cycle saturé à 6 chaînons qui peut contenir un atome d'oxygène ou un atome de soufre et qui peut être substitué par un alkyle en C₁-C₆;
dans laquelle les groupes cycloalkyle en eux-mêmes ou en tant que partie d'un autre groupe, cycloalcényle ou bicycloalkyle peuvent être substitués par un groupe (aryl carbocyclique en C₆-C₁₀)alkyle en C₁-C₆, (alcoxy en C₁-C₆)carbonyle, alkyle en C₁-C₆, halogénoalkyle en C₁-C₆, alkyle en C₁-C₆ substitué par amino, dans lequel les groupes amino peuvent comporter un ou deux substituants alkyle en C₁-C₆ ou un substituant constitué par un acide alpha-aminé ou un résidu de polypeptide, alkyle en C₁-C₆ substitué par hydroxy, hydroxy, alcoxy en C₁-C₆ ou halogène ou un groupe amino, mono- ou di-(alkyl en C₁-C₆)amino ou (alcanoyl en C₁-C₈)amino, dans lequel le groupe amino peut être substitué par un groupe alcanoyle en C₁-C₈, un acide alpha-aminé ou un polypeptide ayant de deux à cinq acides aminés, ledit procédé comprenant l'étape d'hydrolyse d'un ester de l'acide carboxylique de formule I.

2. Procédé selon la revendication 1, dans lequel A est N ou CR⁶ et R⁶ est choisi dans le groupe constitué par halogène, alkyle en C₁-C₆, halogénoalkyle en C₁-C₆, alkyle en C₁-C₆ substitué par hydroxy, (alcoxy en C₁-C₆)alkyle en C₁-C₆, alcoxy en C₁-C₆ et alkyle en C₁-C₆ substitué par amino, dans lequel les groupes amino peuvent comporter un ou deux substituants alkyle en C₁-C₆ ou un substituant constitué par un acide alpha-aminé ou un résidu de polypeptide.

3. Procédé selon la revendication 2, dans lequel R² est un hétérocycle contenant de l'azote répondant à la formule 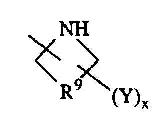 dans laquelle R⁹, Y et x sont tels que définis dans la revendication 1.

4. Procédé selon la revendication 1 pour la préparation d'un composé répondant à la formule 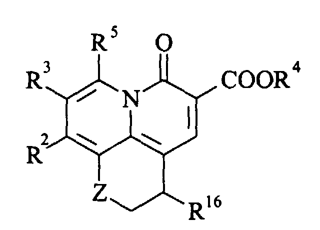 dans laquelle Z est choisi dans le groupe constitué par CH₂, O et S et R¹⁶ est alkyle en C₁-C₆.

5. Procédé selon la revendication 4, dans lequel Z est O et R² est un hétérocycle contenant de l'azote répondant à la formule 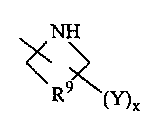 dans laquelle R⁹, Y et x sont tels que définis dans la revendication 1.

6. Procédé selon la revendication 1 pour la préparation d'un composé choisi dans le groupe constitué par:
l'acide 3-fluoro-9-(4-fluorophényl)-2-(4-méthylpipérazin-1-yl)-6(H)-6-oxo-pyrido[1,2-a]pyrimidine-7-carboxylique;
l'acide 9-(2,4-difluorophényl)-3-fluoro-2-(4-méthylpipérazin-1-yl)-6(H)-6-oxo-pyrido[1,2-a]pyrimidine-7-carboxylique;
l'acide 3-fluoro-9-cyclopropyl-2-(4-méthylpipérazin-1-yl)-6(H)-6-oxo-pyrido[1,2-a]pyrimidine-7-carboxylique;
le chlorhydrate de l'acide 2-(3-aminopyrrolidin-1-yl)-9-cyclopropyl-3-fluoro-6H-6-oxo-pyrido[1,2-a]pyrimidine-7-carboxylique;
l'acide 2-(3-aminopyrrolidin-1-yl)-9-cyclopropyl-3-fluoro-6H-6-oxo-pyrido[1,2-a]pyrimidine-7-carboxylique;
l'acide 9-(2,4-difluorophényl)-3-fluoro-2-(4-méthylpipérazin-1-yl)-6H-6-oxo-pyrido[1,2-a]pyrimidine-7-carboxylique;
l'acide 2-(3-aminopyrrolidin-1-yl)-9-(2,4-difluorophényl)-3-fluoro-6H-6-oxo-pyrido[1,2-a]pyrimidine-7-carboxylique;
l'acide 2-(3-(N-tert-butoxycarbonyl)aminopyrrolidin-1-yl)-9-(2,4-difluorophényl)-3-fluoro-6H-6-oxo-pyrido[1,2-a]pyrimidine-7-carboxylique;
l'acide 2-(3-aminopyrrolidin-1-yl)-9-(2,4-difluorophényl)-3-fluoro-6H-6-oxo-pyrido[1,2-a]pyrimidine-7-carboxylique;
l'acide 9-cyclopropyl-3-fluoro-2-(4-méthylpipérazin-1-yl)-6H-6-oxo-pyrido[1,2-a]pyrimidine-7-carboxylique;
l'acide 9-cyclopropyl-3-fluoro-2-(pipérazin-1-yl)-6H-6-oxo-pyrido[1,2-a]pyrimidine-7-carboxylique;
l'acide 9-cyclopropyl-3-fluoro-2-(morpholin-1-yl)-6H-6-oxo-pyrido[1,2-a]pyrimidine-7-carboxylique;
le chlorhydrate de l'acide 9-(2,4-difluorophényl)-3-fluoro-2-(3-(N-(S)-norvalyl)aminopyrrolidin-1-yl)-6H-6-oxo-pyrido[1,2-a]pyrimidine-7-carboxylique;
le chlorhydrate de l'acide 2-(3-(N-(S)-alanyl)aminopyrrolidin-1-yl)-9-(2,4-difluorophényl)-3-fluoro-6H-6-oxo-pyrido[1,2-a]pyrimidine-7-carboxylique;
le chlorhydrate de l'acide 2-(3-(N-(S)-alanyl-(S)-alanyl)aminopyrrolidin-1-yl)-9-(2,4-difluorophényl)-3-fluoro-6H-6-oxo-pyrido[1,2-a]pyrimidine-7-carboxylique;
l'acide 2-((2S,4S)-4-acétamido-2-méthylpyrrolidin-1-yl)-9-(2,4-difluorophényl)-3-fluoro-6H-6-oxo-pyrido[1,2-a]pyrimidine-7-carboxylique;
l'acide 9-(2,4-difluorophényl)-3-fluoro-2-(3-hydroxypyrrolin-1-yl)-6H-6-oxo-pyrido[1,2-a]pyrimidine-7-carboxylique; et
le chlorhydrate de l'acide 2-((2S,4S)-4-amino-2-méthylpyrrolidin-1-yl)-9-(2,4-difluorophényl)-3-fluoro-6H-6-oxo-pyrido[1,2-a]pyrimidine-7-carboxylique.

## Revendications (Revendications pour l'(les) Etat(s) contractant(s) suivant(s): GR)

1. Procédé pour la préparation d'un composé de formule 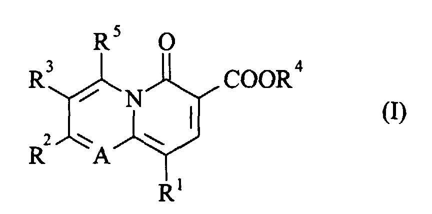 ou d'un de ses sels, esters ou amides pharmaceutiquement acceptables, dans laquelle
R¹ est choisi dans le groupe constitué par (a) alkyle en C₁-C₆, (b) alcényle en C₂-C₆, (c) halogénoalkyle en C₁-C₆, (d) alcoxy en C₁-C₆, (e) cycloalkyle en C₃-C₈, (f) phényle, (g) halogène, (h) cyano, (i) nitro, (j) bicycloalkyle ayant de 4 à 9 atomes de carbone dans le système cyclique dans lequel des atomes non adjacents sont pontés par 1 à 3 atomes de carbone supplémentaires, (k) alcynyle en C₂-C₆, (l) (alcoxy en C₁-C₆)carbonyle, (m) un hétérocycle aromatique contenant de l'azote ayant de 5 à 7 atomes cycliques et (n) un groupe de formule -NR⁷R⁸ dans laquelle R⁷ et R⁸ sont choisis indépendamment dans le groupe constitué par hydrogène, alkyle en C₁-C₆ et alcanoyle en C₁-C₈ ou, pris avec l'atome d'azote auquel ils sont liés, R⁷ et R⁸ forment un hétérocycle saturé à 5, 6 ou 7 chaînons éventuellement substitué par un groupe (aryl carbocyclique en C₆-C₁₀)alkyle en C₁-C₆, (alcoxy en C₁-C₆)carbonyle, alkyle en C₁-C₆, halogénoalkyle en C₁-C₆, alkyle en C₁-C₆ substitué par amino, dans lequel les groupes amino peuvent comporter un ou deux substituants alkyle en C₁-C₆ ou un substituant constitué par un acide alpha-aminé ou un résidu de polypeptide, alkyle en C₁-C₆ substitué par hydroxy, hydroxy, alcoxy en C₁-C₆ ou halogène ou un groupe amino, mono- ou di-(alkyl en C₁-C₆)amino ou (alcanoyl en C₁-C₈)amino, dans lequel le groupe amino peut être substitué par un groupe alcanoyle en C₁-C₈, un acide alpha-aminé ou un polypeptide comportant de deux à cinq acides aminés;
R² est choisi dans le groupe constitué par halogène, alcoxy en C₁-C₆, aryloxy carbocyclique en C₆-C₁₀, (aryl carbocyclique en C₆-C₁₀)alcoxy en C₁-C₆, alkyle en C₁-C₆, alcényle en C₂-C₆, cycloalkyle en C₃-C₈, cycloalcényle en C₄-C₈, (aryl carbocyclique en C₆-C₁₀)alkyle en C₁-C₆, (cycloalkyle en C₃-C₈)alkyle en C₁-C₆, phényle, amino, mono- ou di-(alkyl en C₁-C₆)amino, (aryl carbocyclique en C₆-C₁₀)(alkyl en C₁-C₆)amino, (alkyl en C₁-C₆ substitué par hydroxy)amino, un groupe hétérocyclique bicyclique contenant de l'azote choisi parmi l'une des formules 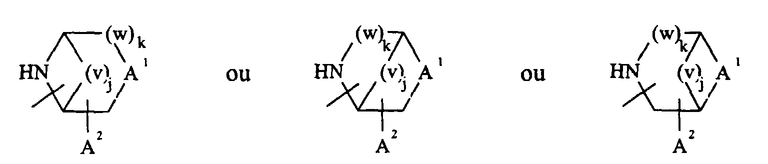 dans lesquelles v et w sont CH₂; j et k sont indépendamment 1, 2 ou 3; A¹ est un atome de carbone ou un hétéroatome choisi parmi S, O et N; et A² est un ou plusieurs substituants différents de l'hydrogène choisis indépendamment dans le groupe constitué par alkyle en C₁-C₆, halogénoalkyle en C₁-C₆, alkyle en C₁-C₆ substitué par hydroxy, hydroxy, halogène, alkyle en C₁-C₆ substitué par amino dans lequel les groupes amino peuvent comporter un ou deux substituants alkyle en C₁-C₆ ou un substituant constitué par un acide alpha-aminé ou un résidu de polypeptide, (alcanoyl en C₁-C₈)amino, phényle et un groupe de formule -NR⁷R⁸ dans laquelle R⁷ et R⁸ sont choisis indépendamment dans le groupe constitué par hydrogène et alkyle en C₁-C₆ ou, lorsque l'un est hydrogène, l'autre est un acide alpha-aminé ou un résidu de polypeptide comportant de 2 à 5 acides aminés ou R² peut être un hétérocycle contenant de l'azote ayant la formule 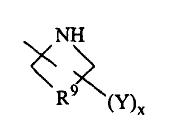 dans laquelle x est 0 à 3; R⁹ est choisi dans le groupe constitué par (a) -(CH₂)ₘ-, où m est 1, 2 ou 3; et (b) -(CH₂)ₙR¹⁰(CH₂)ₚ- où R¹⁰ est S, O ou N, n est 1 ou 2 et p est 1 ou 2; et Y est un substituant différent de l'hydrogène choisi indépendamment dans le groupe constitué par alkyle en C₁-C₆, halogénoalkyle en C₁-C₆, alcoxy en C₁-C₆, alkyle en C₁-C₆ substitué par hydroxy, hydroxy, alkyle en C₁-C₆ substitué par amino, dans lequel les groupes amino peuvent comporter un ou deux substituants alkyle en C₁-C₆ ou un substituant constitué par un acide alpha-aminé ou un résidu de polypeptide, halogène et un groupe de formule -NR¹¹R¹² dans laquelle R¹¹ et R¹² sont choisis indépendamment dans le groupe constitué par hydrogène et alkyle en C₁-C₆ ou, lorsque l'un est hydrogène, l'autre est choisi dans le groupe constitué par alcanoyle en C₁-C₈, un acide alpha-aminé et un résidu de polypeptide comportant de 2 à 5 acides aminés;
R³ est choisi dans le groupe constitué par hydrogène, halogène et alcoxy en
R⁴ est choisi dans le groupe constitué par hydrogène, alkyle en C₁-C₆, un cation pharmaceutiquement acceptable et un groupe ester de promédicament, ledit groupe ester de promédicament étant un groupe ester qui est hydrolysable dans des conditions physiologiques;
R⁵ est choisi dans le groupe constitué par hydrogène, halogène, hydroxy, alkyle en C₁-C₆, halogénoalkyle en C₁-C₆, alcoxy en C₁-C₆ et un groupe ayant la formule -NR¹³R¹⁴ dans laquelle R¹³ et R¹⁴ sont choisis indépendamment dans le groupe constitué par hydrogène, alkyle en C₁-C₆, alkyle en C₁-C₆ substitué par hydroxy, alkyle en C₁-C₆ substitué par alcoxy en C₁-C₆ et alcanoyle en C₁-C₈; et
A est N ou CR⁶, où R⁶ est choisi dans le groupe constitué par halogène, alkyle en C₁-C₆, halogénoalkyle en C₁-C₆, alkyle en C₁-C₆ substitué par hydroxy, (alcoxy en C₁-C₆)alkyle en C₁-C₆, alcoxy en C₁-C₆ et alkyle en C₁-C₆ substitué par amino dans lequel les groupes amino peuvent comporter un ou deux substituants alkyle en C₁-C₆ ou un substituant constitué par un acide alpha-aminé ou un résidu de polypeptide ou dans laquelle R¹ et R⁶, pris avec les atomes auxquels ils sont liés, forment un cycle saturé à 6 chaînons qui peut contenir un atome d'oxygène ou un atome de soufre et qui peut être substitué par un alkyle en C₁-C₆;
dans laquelle les groupes cycloalkyle en eux-mêmes ou en tant que partie d'un autre groupe, cycloalcényle ou bicycloalkyle peuvent être substitués par un groupe (aryl carbocyclique en C₆-C₁₀)alkyle en C₁-C₆, (alcoxy en C₁-C₆)carbonyle, alkyle en C₁-C₆, halogénoalkyle en C₁-C₆, alkyle en C₁-C₆ substitué par amino, dans lequel les groupes amino peuvent comporter un ou deux substituants alkyle en C₁-C₆ ou un substituant constitué par un acide alpha-aminé ou un résidu de polypeptide, alkyle en C₁-C₆ substitué par hydroxy, hydroxy, alcoxy en C₁-C₆ ou halogène ou un groupe amino, mono- ou di-(alkyl en C₁-C₆)amino ou (alcanoyl en C₁-C₈)amino, dans lequel le groupe amino peut être substitué par un groupe alcanoyle en C₁-C₈, un acide alpha-aminé ou un polypeptide ayant de deux à cinq acides aminés, ledit procédé comprenant l'étape d'hydrolyse d'un ester de l'acide carboxylique de formule I.

2. Procédé selon la revendication 1, dans lequel A est N ou CR⁶ et R⁶ est choisi dans le groupe constitué par halogène, alkyle en C₁-C₆, halogénoalkyle en C₁-C₆, alkyle en C₁-C₆ substitué par hydroxy, (alcoxy en C₁-C₆)alkyle en C₁-C₆, alcoxy en C₁-C₆ et alkyle en C₁-C₆ substitué par amino, dans lequel les groupes amino peuvent comporter un ou deux substituants alkyle en C₁-C₆ ou un substituant constitué par un acide alpha-aminé ou un résidu de polypeptide.

3. Procédé selon la revendication 2, dans lequel R² est un hétérocycle contenant de l'azote répondant à la formule 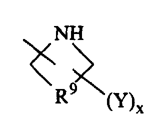 dans laquelle R⁹, Y et x sont tels que définis dans la revendication 1.

4. Procédé selon la revendication 1 pour la préparation d'un composé répondant à la formule 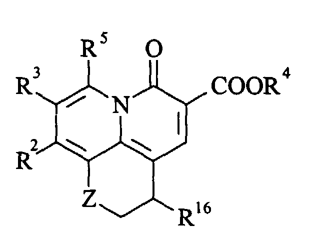 dans laquelle Z est choisi dans le groupe constitué par CH₂, O et S et R¹⁶ est alkyle en C₁-C₆.

5. Procédé selon la revendication 4, dans lequel Z est O et R² est un hétérocycle contenant de l'azote répondant à la formule 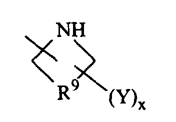 dans laquelle R⁹ Y et x sont tels que définis dans la revendication 1.

6. Procédé selon la revendication 1 pour la préparation d'un composé choisi dans le groupe constitué par:
l'acide 3-fluoro-9-(4-fluorophényl)-2-(4-méthylpipérazin-1-yl)-6(H)-6-oxo-pyrido[1,2-a]pyrimidine-7-carboxylique;
l'acide 9-(2,4-difluorophényl)-3-fluoro-2-(4-méthylpipérazin-1-yl)-6(H)-6-oxo-pyrido[1,2-a]pyrimidine-7-carboxylique;
l'acide 3-fluoro-9-cyclopropyl-2-(4-méthylpipérazin-1-yl)-6(H)-6-oxo-pyrido[1,2-a]pyrimidine-7-carboxylique;
le chlorhydrate de l'acide 2-(3-aminopyrrolidin-1-yl)-9-cyclopropyl-3-fluoro-6H-6-oxo-pyrido[1,2-a]pyrimidine-7-carboxylique;
l'acide 2-(3-aminopyrrolidin-1-yl)-9-cyclopropyl-3-fluoro-6H-6-oxo-pyrido[1,2-a]pyrimidine-7-carboxylique;
l'acide 9-(2,4-difluorophényl)-3-fluoro-2-(4-méthylpipérazin-1-yl)-6H-6-oxo-pyrido[1,2-a]pyrimidine-7-carboxylique;
l'acide 2-(3-aminopyrrolidin-1-yl)-9-(2,4-difluorophényl)-3-fluoro-6H-6-oxo-pyrido[1,2-a]pyrimidine-7-carboxylique;
l'acide 2-(3-(N-tert-butoxycarbonyl)aminopyrrolidin-1-yl)-9-(2,4-difluorophényl)-3-fluoro-6H-6-oxo-pyrido[1,2-a]pyrimidine-7-carboxylique;
l'acide 2-(3-aminopyrrolidin-1-yl)-9-(2,4-difluorophényl)-3-fluoro-6H-6-oxo-pyrido[1,2-a]pyrimidine-7-carboxylique;
l'acide 9-cyclopropyl-3-fluoro-2-(4-méthylpipérazin-1-yl)-6H-6-oxo-pyrido[1,2-a]pyrimidine-7-carboxylique;
l'acide 9-cyclopropyl-3-fluoro-2-(pipérazin-1-yl)-6H-6-oxo-pyrido[1,2-a]pyrimidine-7-carboxylique;
l'acide 9-cyclopropyl-3-fluoro-2-(morpholin-1-yl)-6H-6-oxo-pyrido[1,2-a]pyrimidine-7-carboxylique;
le chlorhydrate de l'acide 9-(2,4-difluorophényl)-3-fluoro-2-(3-(N-(S)-norvalyl)aminopyrrolidin-1-yl)-6H-6-oxo-pyrido[1,2-a]pyrimidine-7-carboxylique;
le chlorhydrate de l'acide 2-(3-(N-(S)-alanyl)aminopyrrolidin-1-yl)-9-(2,4-difluorophényl)-3-fluoro-6H-6-oxo-pyrido[1,2-a]pyrimidine-7-carboxylique;
le chlorhydrate de l'acide 2-(3-(N-(S)-alanyl-(S)-alanyl)aminopyrrolidin-1-yl)-9-(2,4-difluorophényl)-3-fluoro-6H-6-oxo-pyrido[1,2-a]pyrimidine-7-carboxylique;
l'acide 2-((2S,4S)-4-acétamido-2-méthylpyrrolidin-1-yl)-9-(2,4-difluorophényl)-3-fluoro-6H-6-oxo-pyrido[1,2-a]pyrimidine-7-carboxylique;
l'acide 9-(2,4-difluorophényl)-3-fluoro-2-(3-hydroxypyrrolin-1-yl)-6H-6-oxo-pyrido[1,2-a]pyrimidine-7-carboxylique; et
le chlorhydrate de l'acide 2-((2S,4S)-4-amino-2-méthylpyrrolidin-1-yl)-9-(2,4-difluorophényl)-3-fluoro-6H-6-oxo-pyrido[1,2-a]pyrimidine-7-carboxylique.

7. Utilisation d'un composé de formule 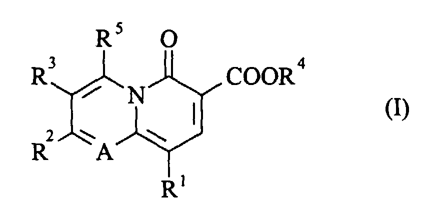 ou d'un de ses sels, esters ou amides pharmaceutiquement acceptables, dans laquelle
R¹ est choisi dans le groupe constitué par (a) alkyle en C₁-C₆, (b) alcényle en C₂-C₆, (c) halogénoalkyle en C₁-C₆, (d) alcoxy en C₁-C₆, (e) cycloalkyle en C₃-C₈, (f) phényle, (g) halogène, (h) cyano, (i) nitro, (j) bicycloalkyle ayant de 4 à 9 atomes de carbone dans le système cyclique dans lequel des atomes non adjacents sont pontés par 1 à 3 atomes de carbone supplémentaires, (k) alcynyle en C₂-C₆, (l) (alcoxy en C₁-C₆)carbonyle, (m) un hétérocycle aromatique contenant de l'azote ayant de 5 à 7 atomes cycliques et (n) un groupe de formule -NR⁷R⁸ dans laquelle R⁷ et R⁸ sont choisis indépendamment dans le groupe constitué par hydrogène, alkyle en C₁-C₆ et alcanoyle en C₁-C₈ ou, pris avec l'atome d'azote auquel ils sont liés, R⁷ et R⁸ forment un hétérocycle saturé à 5, 6 ou 7 chaînons éventuellement substitué par un groupe (aryl carbocyclique en C₆-C₁₀)alkyle en C₁-C₆, (alcoxy en C₁-C₆)carbonyle, alkyle en C₁-C₆, halogénoalkyle en C₁-C₆, alkyle en C₁-C₆ substitué par amino, dans lequel les groupes amino peuvent comporter un ou deux substituants alkyle en C₁-C₆ ou un substituant constitué par un acide alpha-aminé ou un résidu de polypeptide, alkyle en C₁-C₆ substitué par hydroxy, hydroxy, alcoxy en C₁-C₆ ou halogène ou un groupe amino, mono- ou di-(alkyl en C₁-C₆)amino ou (alcanoyl en C₁-C₆)amino, dans lequel le groupe amino peut être substitué par un groupe alcanoyle en C₁-C₈, un acide alpha-aminé ou un polypeptide comportant de deux à cinq acides aminés;
R² est choisi dans le groupe constitué par halogène, alcoxy en C₁-C₆, aryloxy carbocyclique en C₆-C₁₀, (aryl carbocyclique en C₆-C₁₀)alcoxy en C₁-C₆, alkyle en C₁-C₆, alcényle en C₂-C₆, cycloalkyle en C₃-C₈, cycloalcényle en C₄-C₈, (aryl carbocyclique en C₆-C₁₀)alkyle en C₁-C₆, (cycloalkyle en C₃-C₈)alkyle en C₁-C₆, phényle, amino, mono- ou di-(alkyl en C₁-C₆)amino, (aryl carbocyclique en C₆-C₁₀)(alkyl en C₁-C₆)amino, (alkyl en C₁-C₆ substitué par hydroxy)amino, un groupe hétérocyclique bicyclique contenant de l'azote choisi parmi l'une des formules 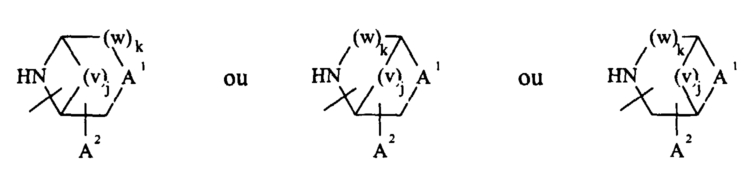 dans lesquelles v et w sont CH₂; j et k sont indépendamment 1, 2 ou 3; A¹ est un atome de carbone ou un hétéroatome choisi parmi S, O et N; et A² est un ou plusieurs substituants différents de l'hydrogène choisis indépendamment dans le groupe constitué par alkyle en C₁-C₆, halogénoalkyle en C₁-C₆, alkyle en C₁-C₆ substitué par hydroxy, hydroxy, halogène, alkyle en C₁-C₆ substitué par amino dans lequel les groupes amino peuvent comporter un ou deux substituants alkyle en C₁-C₆ ou un substituant constitué par un acide alpha-aminé ou un résidu de polypeptide, (alcanoyl en C₁-C₈)amino, phényle et un groupe de formule -NR⁷R⁸ dans laquelle R⁷ et R⁸ sont choisis indépendamment dans le groupe constitué par hydrogène et alkyle en C₁-C₆ ou, lorsque l'un est hydrogène, l'autre est un acide alpha-aminé ou un résidu de polypeptide comportant de 2 à 5 acides aminés ou R² peut être un hétérocycle contenant de l'azote ayant la formule 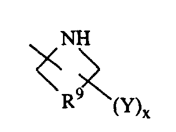 dans laquelle x est 0 à 3; R⁹ est choisi dans le groupe constitué par (a) -(CH₂)ₘ-, où m est 1, 2 ou 3; et (b) -(CH₂)ₙR¹⁰(CH₂)ₚ- où R¹⁰ est S, O ou N, n est 1 ou 2 et p est 1 ou 2; et Y est un substituant différent de l'hydrogène choisi indépendamment dans le groupe constitué par alkyle en C₁-C₆, halogénoalkyle en C₁-C₆, alcoxy en C₁-C₆, alkyle en C₁-C₆ substitué par hydroxy, hydroxy, alkyle en C₁-C₆ substitué par amino, dans lequel les groupes amino peuvent comporter un ou deux substituants alkyle en C₁-C₆ ou un substituant constitué par un acide alpha-aminé ou un résidu de polypeptide, halogène et un groupe de formule -NR¹¹R¹² dans laquelle R¹¹ et R¹² sont choisis indépendamment dans le groupe constitué par hydrogène et alkyle en C₁-C₆ ou, lorsque l'un est hydrogène, l'autre est choisi dans le groupe constitué par alcanoyle en C₁-C₈, un acide alpha-aminé et un résidu de polypeptide comportant de 2 à 5 acides aminés;
R³ est choisi dans le groupe constitué par hydrogène, halogène et alcoxy en
R⁴ est choisi dans le groupe constitué par hydrogène, alkyle en C₁-C₆, un cation pharmaceutiquement acceptable et un groupe ester de promédicament, ledit groupe ester de promédicament étant un groupe ester qui est hydrolysable dans des conditions physiologiques;
R⁵ est choisi dans le groupe constitué par hydrogène, halogène, hydroxy, alkyle en C₁-C₆, halogénoalkyle en C₁-C₆, alcoxy en C₁-C₆ et un groupe ayant la formule -NR¹³R¹⁴ dans laquelle R¹³ et R¹⁴ sont choisis indépendamment dans le groupe constitué par hydrogène, alkyle en C₁-C₆, alkyle en C₁-C₆ substitué par hydroxy, alkyle en C₁-C₆ substitué par alcoxy en C₁-C₆ et alcanoyle en C₁-C₈; et
A est N ou CR⁶, où R⁶ est choisi dans le groupe constitué par halogène, alkyle en C₁-C₆, halogénoalkyle en C₁-C₆, alkyle en C₁-C₆ substitué par hydroxy, (alcoxy en C₁-C₆)alkyle en C₁-C₆, alcoxy en C₁-C₆ et alkyle en C₁-C₆ substitué par amino dans lequel les groupes amino peuvent comporter un ou deux substituants alkyle en C₁-C₆ ou un substituant constitué par un acide alpha-aminé ou un résidu de polypeptide ou dans laquelle R¹ et R⁶ pris avec les atomes auxquels ils sont liés, forment un cycle saturé à 6 chaînons qui peut contenir un atome d'oxygène ou un atome de soufre et qui peut être substitué par un alkyle en C₁-C₆;
dans laquelle les groupes cycloalkyle en eux-mêmes ou en tant que partie d'un autre groupe, cycloalcényle ou bicycloalkyle peuvent être substitués par un groupe (aryl carbocyclique en C₆-C₁₀)alkyle en C₁-C₆, (alcoxy en C₁-C₆)carbonyle, alkyle en C₁-C₆, halogénoalkyle en C₁-C₆, alkyle en C₁-C₆ substitué par amino, dans lequel les groupes amino peuvent comporter un ou deux substituants alkyle en C₁-C₆ ou un substituant constitué par un acide alpha-aminé ou un résidu de polypeptide, alkyle en C₁-C₆ substitué par hydroxy, hydroxy, alcoxy en C₁-C₆ ou halogène ou un groupe amino, mono- ou di-(alkyl en C₁-C₆)amino ou (alcanoyl en C₁-C₈)amino, dans lequel le groupe amino peut être substitué par un groupe alcanoyle en C₁-C₈, un acide alpha-aminé ou un polypeptide ayant de deux à cinq acides aminés, pour la fabrication d'un médicament pour le traitement ou la prévention des infections bactériennes chez un être humain ou un autre animal hôte.

8. Utilisation d'un composé choisi dans le groupe constitué par:
l'acide 3-fluoro-9-(4-fluorophényl)-2-(4-méthylpipérazin-1-yl)-6(H)-6-oxo-pyrido[1,2-a]pyrimidine-7-carboxylique;
l'acide 9-(2,4-difluorophényl)-3-fluoro-2-(4-méthylpipérazin-1-yl)-6(H)-6-oxo-pyrido[1,2-a]pyrimidine-7-carboxylique;
l'acide 3-fluoro-9-cyclopropyl-2-(4-méthylpipérazin-1-yl)-6(H)-6-oxo-pyrido[1,2-a]pyrimidine-7-carboxylique; le chlorhydrate de l'acide 2-(3-aminopyrrolidin-1-yl)-9-cyclopropyl-3-fluoro-6H-6-oxo-pyrido[1,2-a]pyrimidine-7-carboxylique;
l'acide 2-(3-aminopyrrolidin-1-yl)-9-cyclopropyl-3-fluoro-6H-6-oxo-pyrido[1,2-a]pyrimidine-7-carboxylique;
l'acide 9-(2,4-difluorophényl)-3-fluoro-2-(4-méthylpipérazin-1-yl)-6H-6-oxo-pyrido[1,2-a]pyrimidine-7-carboxylique;
l'acide 2-(3-aminopyrrolidin-1-yl)-9-(2,4-difluorophényl)-3-fluoro-6H-6-oxo-pyrido[1,2-a]pyrimidine-7-carboxylique;
l'acide 2-(3-(N-tert-butoxycarbonyl)aminopyrrolidin-1-yl)-9-(2,4-difluorophényl)-3-fluoro-6H-6-oxo-pyrido[1,2-a]pyrimidine-7-carboxylique;
l'acide 2-(3-aminopyrrolidin-1-yl)-9-(2,4-difluorophényl)-3-fluoro-6H-6-oxo-pyrido[1,2-a]pyrimidine-7-carboxylique;
l'acide 9-cyclopropyl-3-fluoro-2-(4-méthylpipérazin-1-yl)-6H-6-oxo-pyrido[1,2-a]pyrimidine-7-carboxylique;
l'acide 9-cyclopropyl-3-fluoro-2-(pipérazin-1-yl)-6H-6-oxo-pyrido[1,2-a]pyrimidine-7-carboxylique;
l'acide 9-cyclopropyl-3-fluoro-2-(morpholin-1-yl)-6H-6-oxo-pyrido[1,2-a]pyrimidine-7-carboxylique;
le chlorhydrate de l'acide 9-(2,4-difluorophényl)-3-fluoro-2-(3-(N-(S)-norvalyl)aminopyrrolidin-1-yl)-6H-6-oxo-pyrido[1,2-a]pyrimidine-7-carboxylique;
le chlorhydrate de l'acide 2-(3-(N-(S)-alanyl)aminopyrrolidin-1-yl)-9-(2,4-difluorophényl)-3-fluoro-6H-6-oxo-pyrido[1,2-a]pyrimidine-7-carboxylique;
le chlorhydrate de l'acide 2-(3-(N-(S)-alanyl-(S)-alanyl)aminopyrrolidin-1-yl)-9-(2,4-difluorophényl)-3-fluoro-6H-6-oxo-pyrido[1,2-a]pyrimidine-7-carboxylique;
l'acide 2-((2S,4S)-4-acétamido-2-méthylpyrrolidin-1-yl)-9-(2,4-difluorophényl)-3-fluoro-6H-6-oxo-pyrido[1,2-a]pyrimidine-7-carboxylique;
l'acide 9-(2,4-difluorophényl)-3-fluoro-2-(3-hydroxypyrrolin-1-yl)-6H-6-oxo-pyrido[1,2-a]pyrimidine-7-carboxylique; et
le chlorhydrate de l'acide 2-((2S,4S)-4-amino-2-méthylpyrrolidin-1-yl)-9-(2,4-difluorophényl)-3-fluoro-6H-6-oxo-pyrido[1,2-a]pyrimidine-7-carboxylique, pour la fabrication d'un médicament pour le traitement ou la prévention des infections bactériennes chez un être humain ou un autre animal hôte.
